# EUROPEAN PATENT SPECIFICATION

(11) **EP 2 694 500 B1**
(45) Date of publication and mention of the grant of the patent: **27.05.2015**
(21) Application number: 12710745.6
(22) Date of filing: 28.03.2012
(51) Int. Cl.: C07D 471/04, A61K 31/437, A61P 35/00

(54) **SUBSTITUTED IMIDAZOPYRIDINES AND INTERMEDIATES THEREOF**
SUBSTITUIERTE IMIDAZOPYRIDINE UND DEREN ZWISCHENPRODUKTE
IMIDAZONAPHTHYRIDINES SUBSTITUEES ET DE PRODUITS INTERMÉDIAIRES DE & xA; CELLE-CI

(30) Priority: 06.04.2011 EP 11161332; 17.06.2011 EP 11170305; 26.08.2011 EP 11179044; 14.11.2011 EP 11188997
(43) Date of publication of application: 12.02.2014
(73) Proprietor: Bayer Pharma Aktiengesellschaft, 13353 Berlin (DE)
(72) Inventor: KOPPITZ, Marcus, 10115 Berlin (DE); KLAR, Ulrich, 13503 Berlin (DE); WENGNER, Antje, 10437 Berlin (DE); NEUHAUS, Roland, 12157 Berlin (DE); SIEMEISTER, Gerhard, 13503 Berlin (DE)
(74) Representative: BIP Patents
(86) International application number: PCT/EP2012/055471
(87) International publication number: WO 2012/136531

(56) References cited:
- WO-A2-2004/026867
- WO-A2-2007/032936

## Description

The present invention relates to substituted imidazopyridine compounds of general formula I as described and defined herein, to methods of preparing said compounds, to pharmaceutical compositions and combinations comprising said compounds, to the use of said compounds for manufacturing a pharmaceutical composition for the treatment or prophylaxis of a disease, as well as to intermediate compounds useful in the preparation of said compounds.

### BACKGROUND OF THE INVENTION

The present invention relates to chemical compounds that inhibit Mps-1 (Monopolar Spindle 1) kinase (also known as Tyrosine Threonine Kinase, TTK). Mps-1 is a dual specificity Ser/Thr kinase which plays a key role in the activation of the mitotic checkpoint (also known as spindle checkpoint, spindle assembly checkpoint) thereby ensuring proper chromosome segregation during mitosis [Abrieu A et al., Cell, 2001, 106, 83-93]. Every dividing cell has to ensure equal separation of the replicated chromosomes into the two daughter cells. Upon entry into mitosis, chromosomes are attached at their kinetochores to the microtubules of the spindle apparatus. The mitotic checkpoint is a surveillance mechanism that is active as long as unattached kinetochores are present and prevents mitotic cells from entering anaphase and thereby completing cell division with unattached chromosomes [Suijkerbuijk SJ and Kops GJ, Biochemica et Biophysica Acta, 2008, 1786, 24-31; Musacchio A and Salmon ED, Nat Rev Mol Cell Biol., 2007, 8, 379-93]. Once all kinetochores are attached in a correct amphitelic, *i.e.* bipolar, fashion with the mitotic spindle, the checkpoint is satisfied and the cell enters anaphase and proceeds through mitosis. The mitotic checkpoint consists of complex network of a number of essential proteins, including members of the MAD (mitotic arrest deficient, MAD 1-3) and Bub (Budding uninhibited by benzimidazole, Bub 1-3) families, the motor protein CENP-E, Mps-1 kinase as well as other components, many of these being over-expressed in proliferating cells (e.g. cancer cells) and tissues [Yuan B et al., Clinical Cancer Research, 2006, 12, 405-10]. The essential role of Mps-1 kinase activity in mitotic checkpoint signalling has been shown by shRNA-silencing, chemical genetics as well as chemical inhibitors of Mps-1 kinase [Jelluma N et al., PLos ONE, 2008, 3, e2415; Jones MH et al., Current Biology, 2005, 15, 160-65; Dorer RK et al., Current Biology, 2005, 15, 1070-76; Schmidt M et al., EMBO Reports, 2005, 6, 866-72].
There is ample evidence linking reduced but incomplete mitotic checkpoint function with aneuploidy and tumourigenesis [Weaver BA and Cleveland DW, Cancer Research, 2007, 67, 10103-5; King RW, Biochimica et Biophysica Acta, 2008, 1786, 4-14]. In contrast, complete inhibition of the mitotic checkpoint has been recognised to result in severe chromosome missegregation and induction of apoptosis in tumour cells [Kops GJ et al., Nature Reviews Cancer, 2005, 5, 773-85; Schmidt M and Medema RH, Cell Cycle, 2006, 5, 159-63; Schmidt M and Bastians H, Drug Resistance Updates, 2007, 10, 162-81]. Therefore, mitotic checkpoint abrogation through pharmacological inhibition of Mps-1 kinase or other components of the mitotic checkpoint represents a new approach for the treatment of proliferative disorders including solid tumours such as carcinomas and sarcomas and leukaemias and lymphoid malignancies or other disorders associated with uncontrolled cellular proliferation.

Established anti-mitotic drugs such as vinca alkaloids, taxanes or epothilones activate the SAC inducing a mitotic arrest either by stabilising or destabilising microtubule dynamics. This arrest prevents separation of sister chromatids to form the two daughter cells. Prolonged arrest in mitosis forces a cell either into mitotic exit without cytokinesis or into mitotic catastrophe leading to cell death.
In contrast, inhibitors of Mps-1 induce a SAC inactivation that accelerates progression of cells through mitosis resulting in severe chromosomal missegregation and finally in cell death.

These findings suggest that Mps-1 inhibitors should be of therapeutic value for the treatment of proliferative disorders associated with enhanced uncontrolled proliferative cellular processes such as, for example, cancer, inflammation, arthritis, viral diseases, neurodegenerative diseases such as Alzheimer's disease, cardiovascular diseases, or fungal diseases in a warm-blooded animal such as man.

Therefore, inhibitors of Mps-1 represent valuable compounds that should complement therapeutic options either as single agents or in combination with other drugs.

Different compounds have been disclosed in prior art which show an inhibitory effect on Mps-1 kinase. WO2010/124826A1 discloses substituted imidazoquinoxaline compounds as inhibitors of Mps-1 kinase or TTK. WO2011/026579A1 discloses substituted aminoquinoxalines as Mps-1 inhibitors. WO2011/013729A1 discloses fused imidazole derivatives as Mps-1 inhibitors. WO2011/063908A1, WO2011/064328A1 as well as WO2011063907 A1 disclose triazolopyridine derivates as inhibitors of Mps-1 kinase.

However, imidazopyridine derivatives have not been disclosed in the context of Mps-1 kinase inhibitors. Imidazopyridine derivates have been disclosed for the treatment or prophylaxis of different diseases:
WO2004/026867A2 and WO2007/032936A2 disclose substituted imidoazopyridines as cyclin dependent kinase (CDK) inhibitors for the treatment of diseases and disorders associated with CDKs.
WO2008/029152A2 discloses bicyclic heterorayl compounds for the treatment of duchenne muscular dystrophy. Among other compounds also substituted imidoazopyridines are mentioned.
WO2008/082490A2 discloses substituted imidazopyridines for the treatment of C-Jun-N-terminal kinase (JNK1) mediated diseases.
WO2008/134553A1 discloses substituted imidazopyridines and indolizines for the treatment of sodium channel-mediated diseases.

Thus, the state of the art described above does not describe the specifically substituted imidazopyridine compounds of general formula I of the present invention, or a stereoisomer, a tautomer, an N-oxide, a hydrate, a solvate, or a salt thereof, or a mixture of same, as described and defined herein, and as hereinafter referred to as "compounds of the present invention", or their pharmacological activity.
Furthermore, the state of the art described above does not describe the use of a compound of the present invention as Mps-1 inhibitor.

It has now been found, and this constitutes the basis of the present invention, that said compounds of the present invention have surprising and advantageous properties.

In particular, said compounds of the present invention have surprisingly been found to effectively inhibit Mps-1 kinase and may therefore be used for the treatment or prophylaxis of diseases of uncontrolled cell growth, proliferation and/or survival, inappropriate cellular immune responses, or inappropriate cellular inflammatory responses or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by Mps-1 kinase, such as, for example, haemotological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

### SUMMARY of the INVENTION

The present invention covers compounds of general formula I : 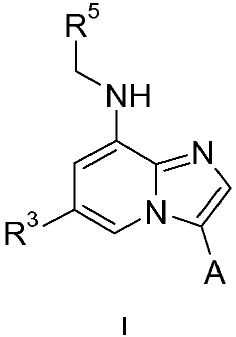 in which :
- A: represents a 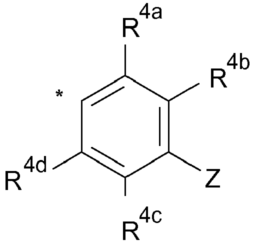 group ;
wherein * indicates the point of attachment of said groups with the rest of the molecule ;
- Z: represents a -C(=O)N(H)R¹ or a -C(=S)N(H)R¹ group ;
- R¹: represents a C₁-C₃-alkyl- or a cyclopropyl- group ;
wherein said cyclopropyl- group is optionally substituted, identically or differently, with 1, 2 or 3 groups selected from: halogen, -OH, -CN, C₁-C₃-alkyl- ;
wherein said C₁-C₃-alkyl- group is optionally substituted, identically or differently, with 1, 2 or 3 groups selected from: halogen, -OH, -CN, C₁-C₃-alkoxy-;
- R³: represents a hydrogen atom or a -CN, C₁-C₆-alkyl-, -(CH₂)ₘ-C₂-C₆-alkenyl, -(CH₂)ₘ-C₂-C₆-alkynyl, aryl-, heteroaryl-, aryl-X- group ;
wherein said C₁-C₆-alkyl-, -(CH₂)ₘ-C₂-C₆-alkenyl, -(CH₂)ₘ-C₂-C₆-alkynyl, aryl-, aryl-X- or heteroaryl- group is optionally substituted, identically or differently, with 1, 2 or 3 R⁷ groups;
- R^{4a}, R^{4b}, R^{4d}: represent a hydrogen atom;
- R^{4c}: represents a halogen atom, or a -CN, -OH, C₁-C₃-alkyl-, C₁-C₃-alkoxy-, halo-C₁-C₃-alkyl-, halo-C₁-C₃-alkoxy-, R^{6a}(R^{6b})N-C₁-C₃-alkyl-, HO-C₁-C₃-alkyl-, NC-C₁-C₃-alkyl-, C₁-C₃-alkoxy-C₁-C₃-alkyl-, halo-C₁-C₃-alkoxy-C₁-C₃-alkyl-group ;
- R⁵: represents a hydrogen atom, or a C₁-C₆-alkyl-, -(CH₂)ₙ-C₂-C₆-alkenyl, -(CH₂)ₙ-C₂-C₆-alkynyl, -(CH₂)ₘ-C₃-C₆-cycloalkyl, -(CH₂)ₘ-(3- to 7-membered heterocycloalkyl), aryl-C₁-C₆-alkyl-, heteroaryl-C₁-C₆-alkyl-, halo-C₁-C₆-alkyl-, R^{6a}(R^{6b})N-C₁-C₆-alkyl-, HO-C₁-C₆-alkyl-, -C₁-C₆-alkyl-CN, C₁-C₆-alkoxy-C₁-C₆-alkyl-, halo-C₁-C₆-alkoxy-C₁-C₆-alkyl-, C₃-C₆-cycloalkyl-, 3- to 7-membered heterocycloalkyl-, C₄-C₈-cycloalkenyl-, aryl- or heteroaryl-group ;
wherein said C₁-C₆-alkyl-, -(CH₂)ₙ-C₂-C₆-alkenyl, -(CH₂)ₙ-C₂-C₆-alkynyl, -(CH₂)ₘ-C₃-C₆-cycloalkyl, -(CH₂)ₘ-(3- to 7-membered heterocycloalkyl), aryl-C₁-C₆-alkyl-, heteroaryl-C₁-C₆-alkyl-, halo-C₁-C₆-alkyl-, R^{6a}(R^{6b})N-C₁-C₆-alkyl-, HO-C₁-C₆-alkyl-, -C₁-C₆-alkyl-CN, C₁-C₆-alkoxy-C₁-C₆-alkyl-, halo-C₁-C₆-alkoxy-C₁-C₆-alkyl-, C₃-C₆-cycloalkyl-, 3- to 7-membered heterocycloalkyl-, C₄-C₈-cycloalkenyl-, aryl- or heteroaryl-group is optionally substituted, identically or differently, with 1, 2, 3, 4 or 5 R⁸ groups ;
- R⁶, R^{6a}, R^{6b}, R^{6c}: represent, independently from each other, a hydrogen atom, or a C₁-C₆-alkyl-, C₃-C₆-cycloalkyl- or aryl-C₁-C₆-alkyl- group ;
wherein said C₃-C₆-cycloalkyl- group is optionally substituted, identically or differently with 1 or 2 groups selected from: halogen, -OH, -CN, C₁-C₆-alkyl-, HO-C₁-C₆-alkyl- ;
- R⁷: represents a halogen atom or a HO-, C₁-C₆-alkoxy-, C₁-C₆-alkyl-, halo-C₁-C₆-alkyl-, halo-C₁-C₆-alkoxy-, HO-C₁-C₆-alkyl-, -C(=O)N(H)R^{6a}, -N(R^{6a})R^{6b}, -O(C=O)OR⁶ or -OR⁶ group;
- R⁸: represents a hydrogen or halogen atom or a -CN, C₁-C₆-alkoxy-, C₁-C₆-alkyl-, halo-C₁-C₆-alkyl-, R^{6a}(R^{6b})N-C₁-C₆-alkyl-, HO-C₁-C₆-alkyl-, C₁-C₆-alkoxy-C₁-C₆-alkyl-, halo-C₁-C₆-alkoxy-C₁-C₆-alkyl-, C₂-C₆-alkenyl-, C₂-C₆-alkynyl-, 3- to 7-membered heterocycloalkyl-, aryl-, heteroaryl-, -C(=O)R⁶, -C(=O)N(H)R^{6a}, -C(=O)N(R^{6a})R^{6b}, -C(=O)O-R⁶, -N(R^{6a})R^{6b}, -NO₂, -N(H)C(=O)R⁶, -N(R^{6c})C(=O)R⁶, -N(H)C(=O)N(R^{6a})R^{6b}, -N(R^{6c})C(=O)N(R^{6a})R^{6b}, -N(H)C(=O)OR⁶, -N(R^{6c})C(=O)OR⁶, -N(H)S(=O)R⁶, -N(R^{6c})S(=O)R⁶, -N(H)S(=O)₂R⁶, -N(R^{6c})S(=O)₂R⁶, -N=S(=O)(R^{6a})R^{6b}, -OR⁶, -O(C=O)R⁶, -O(C=O)N(R^{6a})R^{6b}, -O(C=O)OR⁶, -SR⁶, -S(=O)R⁶, - S(=O)N(H)R⁶, -S(=O)N(R^{6a})R^{6b}, -S(=O₂)R⁶, -S(=O)₂N(H)R⁶, -S(=O)₂N(R^{6a})R^{6b}, -S(=O)(=NR^{6c})R⁶ or -S(=O)₂-(3- to 7-membered heterocycloalkyl) group ;
wherein said 3- to 7-membered heterocycloalkyl- or heteroaryl- group, is optionally substituted, identically or differently, with 1, 2, 3 or 4 C₁-C₆-alkyl-groups ;
- m: is an integer of 0, 1, 2, 3, 4, 5 or 6 ;
- n: is an integer of 0, 1, 2, 3, 4 or 5 ; and
- X: is S, S(=O), S(=O)₂, O, NR⁶, C(=O) or CR^{6a}R^{6b};
or a stereoisomer, a tautomer, an N-oxide, a hydrate, a solvate, or a salt thereof, or a mixture of same.

The present invention also relates to methods of preparing said compounds, to pharmaceutical compositions and combinations comprising said compounds, to the use of said compounds for manufacturing a pharmaceutical composition for the treatment or prophylaxis of a disease, as well as to intermediate compounds useful in the preparation of said compounds.

### DETAILED DESCRIPTION of the INVENTION

The terms as mentioned in the present text have preferably the following meanings :
The term "halogen atom" or "halo-" is to be understood as meaning a fluorine, chlorine, bromine, or iodine atom.

The term "C₁-C₆-alkyl" is to be understood as preferably meaning a linear or branched, saturated, monovalent hydrocarbon group having 1, 2, 3, 4, 5 or 6 carbon atoms, *e*.*g*. a methyl, ethyl, propyl, butyl, pentyl, hexyl, iso-propyl, iso-butyl, sec-butyl, tert-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl group, or an isomer thereof. Particularly, said group has 1, 2, 3 or 4 carbon atoms ("C₁-C₄-alkyl"), *e*.*g*. a methyl, ethyl, propyl, butyl, iso-propyl, iso-butyl, sec-butyl, tert-butyl group, more particularly 1, 2 or 3 carbon atoms ("C₁-C₃-alkyl"), *e*.*g*. a methyl, ethyl, n-propyl- or iso-propyl group.

The term "halo-C₁-C₆-alkyl" is to be understood as preferably meaning a linear or branched, saturated, monovalent C₁-C₆-alkyl group in which the term "C₁-C₆-alkyl" is defined *supra,* and in which one or more hydrogen atoms is replaced by a halogen atom, in identically or differently, *i.e.* one halogen atom being independent from another. Particularly, said halogen atom is F. Said halo-C₁-C₆-alkyl group is, for example, -CF₃, -CHF₂, -CH₂F, -CF₂CF₃, or -CH₂CF₃.

The term "C₁-C₆-alkoxy" is to be understood as preferably meaning a linear or branched, saturated, monovalent, group of formula -O-(C₁-C₆-alkyl), in which the term "alkyl" is defined *supra, e*.*g*. a methoxy, ethoxy, n-propoxy, iso-propoxy, n-butoxy, iso-butoxy, tert-butoxy, sec-butoxy, pentoxy, iso-pentoxy, or n-hexoxy group, or an isomer thereof.

The term "halo-C₁-C₆-alkoxy" is to be understood as preferably meaning a linear or branched, saturated, monovalent C₁-C₆-alkoxy group, as defined *supra,* in which one or more of the hydrogen atoms is replaced, in identically or differently, by a halogen atom. Particularly, said halogen atom is F. Said halo-C₁-C₆-alkoxy group is, for example, -OCF₃, -OCHF₂, -OCH₂F, -OCF₂CF₃, or -OCH₂CF₃.

The term "C₁-C₆-alkoxy-C₁-C₆-alkyl" is to be understood as preferably meaning a linear or branched, saturated, monovalent C₁-C₆-alkyl group, as defined *supra,* in which one or more of the hydrogen atoms is replaced, in identically or differently, by a C₁-C₆-alkoxy group, as defined *supra, e*.*g*. methoxyalkyl, ethoxyalkyl, propyloxyalkyl, iso-propoxyalkyl, butoxyalkyl, iso-butoxyalkyl, tert-butoxyalkyl, sec-butoxyalkyl, pentyloxyalkyl, iso-pentyloxyalkyl, hexyloxyalkyl group, or an isomer thereof.

The term "halo-C₁-C₆-alkoxy-C₁-C₆-alkyl" is to be understood as preferably meaning a linear or branched, saturated, monovalent C₁-C₆-alkoxy-C₁-C₆-alkyl group, as defined *supra,* in which one or more of the hydrogen atoms is replaced, in identically or differently, by a halogen atom. Particularly, said halogen atom is F. Said halo-C₁-C₆-alkoxy-C₁-C₆-alkyl group is, for example, -CH₂CH₂OCF₃, -CH₂CH₂OCHF₂, -CH₂CH₂OCH₂F, -CH₂CH₂OCF₂CF₃, or -CH₂CH₂OCH₂CF₃.

The term "C₂-C₆-alkenyl" is to be understood as preferably meaning a linear or branched, monovalent hydrocarbon group, which contains one or more double bonds, and which has 2, 3, 4, 5 or 6 carbon atoms, particularly 2 or 3 carbon atoms ("C₂-C₃-alkenyl"), it being understood that in the case in which said alkenyl group contains more than one double bond, then said double bonds may be isolated from, or conjugated with, each other. Said alkenyl group is, for example, a vinyl, allyl, (E)-2-methylvinyl, (Z)-2-methylvinyl, homoallyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (E)-3-methylpent-3-enyl, (Z)-3-methylpent-3-enyl, (E)-2-methylpent-3-enyl, (Z)-2-methylpent-3-enyl, (E)-1-methylpent-3-enyl, (Z)-1-methylpent-3-enyl, (E)-4-methylpent-2-enyl, (Z)-4-methylpent-2-enyl, (E)-3-methylpent-2-enyl, (Z)-3-methylpent-2-enyl, (E)-2-methylpent-2-enyl, (Z)-2-methylpent-2-enyl, (E)-1-methylpent-2-enyl, (Z)-1-methylpent-2-enyl, (E)-4-methylpent-1-enyl, (Z)-4-methylpent-1-enyl, (E)-3-methylpent-1-enyl, (Z)-3-methylpent-1-enyl, (E)-2-methylpent-1-enyl, (Z)-2-methylpent-1-enyl, (E)-1-methylpent-1-enyl, (Z)-1-methylpent-1-enyl, 3-ethylbut-3-enyl, 2-ethylbut-3-enyl, 1-ethylbut-3-enyl, (E)-3-ethylbut-2-enyl, (Z)-3-ethylbut-2-enyl, (E)-2-ethylbut-2-enyl, (Z)-2-ethylbut-2-enyl, (E)-1-ethylbut-2-enyl, (Z)-1-ethylbut-2-enyl, (E)-3-ethylbut-1-enyl, (Z)-3-ethylbut-1-enyl, 2-ethylbut-1-enyl, (E)-1-ethylbut-1-enyl, (Z)-1-ethylbut-1-enyl, 2-propylprop-2-enyl, 1-propylprop-2-enyl, 2-isopropylprop-2-enyl, 1-isopropylprop-2-enyl, (E)-2-propylprop-1-enyl, (Z)-2-propylprop-1-enyl, (E)-1-propylprop-1-enyl, (Z)-1-propylprop-1-enyl, (E)-2-isopropylprop-1-enyl, (Z)-2-isopropylprop-1-enyl, (E)-1-isopropylprop-1-enyl, (Z)-1-isopropylprop-1-enyl, (E)-3,3-dimethylprop-1-enyl, (Z)-3,3-dimethylprop-1-enyl, 1-(1,1-dimethylethyl)ethenyl, buta-1,3-dienyl, penta-1,4-dienyl, hexa-1,5-dienyl, or methylhexadienyl group. Particularly, said group is vinyl or allyl.

The term "C₂-C₆-alkynyl" is to be understood as preferably meaning a linear or branched, monovalent hydrocarbon group which contains one or more triple bonds, and which contains 2, 3, 4, 5 or 6 carbon atoms, particularly 2 or 3 carbon atoms ("C₂-C₃-alkynyl"). Said C₂-C₆-alkynyl group is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-inyl, hex-3-inyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-inyl, 1,1-dimethylbut-3-ynyl, 1,1-dimethylbut-2-ynyl, or 3,3-dimethylbut-1-ynyl group. Particularly, said alkynyl group is ethynyl, prop-1-ynyl, or prop-2-inyl.

The term "C₃-C₆-cycloalkyl" is to be understood as meaning a saturated, monovalent, monocyclic hydrocarbon ring which contains 3, 4, 5 or 6 carbon atoms ("C₃-C₆-cycloalkyl"). Said C₃-C₆-cycloalkyl group is for example a cyclopropyl, cyclobutyl, cyclopentyl, or cyclohexyl ring.

The term "C₄-C₈-cycloalkenyl" is to be understood as preferably meaning a monovalent, mono-, or bicyclic hydrocarbon ring which contains 4, 5, 6, 7 or 8 carbon atoms and one, two, three or four double bonds, in conjugation or not, as the size of said cycloalkenyl ring allows. Said C₄-C₈-cycloalkenyl group is for example, a monocyclic hydrocarbon ring, *e*.*g*. a cyclobutenyl, cyclopentenyl, or cyclohexenyl or a bicyclic hydrocarbon ring, *e*.*g*. a cylooctadienyl ring.

The term "3- to 7-membered heterocycloalkyl", is to be understood as meaning a saturated, monovalent, mono- or bicyclic hydrocarbon ring which contains 2, 3, 4, 5 or 6 carbon atoms, and one or more heteroatom-containing groups selected from C(=O), O, S, S(=O), S(=O)₂, NR^{a}, in which R^{a} represents a hydrogen atom, or a C₁-C₆-alkyl- or halo-C₁-C₆-alkyl- group ; it being possible for said heterocycloalkyl group to be attached to the rest of the molecule via any one of the carbon atoms or, if present, the nitrogen atom.

Particularly, said 3- to 7-membered heterocycloalkyl can contain 2, 3, 4, or 5 carbon atoms, and one or more of the above-mentioned heteroatom-containing groups (a "3- to 6-membered heterocycloalkyl"), more particularly said heterocycloalkyl can contain 4 or 5 carbon atoms, and one or more of the above-mentioned heteroatom-containing groups (a "5- to 6-membered heterocycloalkyl"), wherein two adjacent atoms of the 3- to 7-membered heterocycloalkyl-group are optionally substituted in such a way, that an aryl- or heteroaryl-group is formed.

Particularly, without being limited thereto, said heterocycloalkyl can be a 4-membered ring, such as an azetidinyl, oxetanyl, or a 5-membered ring, such as tetrahydrofuranyl, dioxolinyl, pyrrolidinyl, imidazolidinyl, pyrazolidinyl, pyrrolinyl, or a 6-membered ring, such as tetrahydropyranyl, piperidinyl, morpholinyl, dithianyl, thiomorpholinyl, piperazinyl, or trithianyl, or a 7-membered ring, such as a diazepanyl ring, for example. Optionally, said heterocycloalkyl can be benzo fused.

Said heterocyclyl can be bicyclic, such as, without being limited thereto, a 5,5-membered ring, e.g. a hexahydrocyclopenta[c]pyrrol-2(1H)-yl) ring, or a 5,6-membered bicyclic ring, e.g. a hexahydropyrrolo[1,2-a]pyrazin-2(1H)-yl ring.

As mentioned *supra,* said nitrogen atom-containing ring can be partially unsaturated, *i.e.* it can contain one or more double bonds, such as, without being limited thereto, a 2,5-dihydro-1H-pyrrolyl, 4H-[1,3,4]thiadiazinyl, 4,5-dihydrooxazolyl, or 4H-[1,4]thiazinyl ring, for example, or, it may be benzo-fused, such as, without being limited thereto, a dihydroisoquinolinyl ring, for example.

The term "4- to 8-membered heterocycloalkenyl", is to be understood as meaning an unsaturated, monovalent, mono- or bicyclic hydrocarbon ring which contains 4, 5, 6, 7 or 8 carbon atoms, and one or more heteroatom-containing groups selected from C(=O), O, S, S(=O), S(=O)₂, NR^{a}, in which R^{a} represents a hydrogen atom, or a C₁-C₆-alkyl- or halo-C₁-C₆-alkyl- group ; it being possible for said heterocycloalkenyl group to be attached to the rest of the molecule via any one of the carbon atoms or, if present, the nitrogen atom. Examples of said heterocycloalkenyl may contain one or more double bonds, *e*.*g*. 4H-pyranyl, 2H-pyranyl, 3H-diazirinyl, 2,5-dihydro-1H-pyrrolyl, [1,3]dioxolyl, 4H-[1,3,4]thiadiazinyl, 2,5-dihydrofuranyl, 2,3-dihydrofuranyl, 2,5-dihydrothiophenyl, 2,3-dihydrothiophenyl, 4,5-dihydrooxazolyl, or 4H-[1,4]thiazinyl group, or, it may be benzo fused.

The term "aryl" is to be understood as preferably meaning a monovalent, aromatic or partially aromatic, mono-, or bi- or tricyclic hydrocarbon ring having 6, 7, 8, 9, 10, 11, 12, 13 or 14 carbon atoms (a "C₆-C₁₄-aryl" group), particularly a ring having 6 carbon atoms (a "C₆-aryl" group), *e*.*g*. a phenyl group; or a biphenyl group, or a ring having 9 carbon atoms (a "C₉-aryl" group), *e*.*g*. an indanyl or indenyl group, or a ring having 10 carbon atoms (a "C₁₀-aryl" group), *e*.*g*. a tetralinyl, dihydronaphthyl, or naphthyl group, or a ring having 13 carbon atoms, (a "C₁₃-aryl" group), *e*.*g*. a fluorenyl group, or a ring having 14 carbon atoms, (a "C₁₄-aryl" group), *e*.*g*. an anthranyl group, wherein two adjacent atoms of the aryl-group are optionally substituted in such a way, that a 3- to 7-membered heterocycloalkyl-group is formed.

The term "heteroaryl" is understood as preferably meaning a monovalent, monocyclic- , bicyclic- or tricyclic aromatic ring system having 5, 6, 7, 8, 9, 10, 11, 12, 13 or 14 ring atoms (a "5- to 14-membered heteroaryl" group), particularly 5 or 6 or 9 or 10 atoms, and which contains at least one heteroatom which may be identical or different, said heteroatom being such as oxygen, nitrogen or sulfur, and in addition in each case can be benzocondensed, wherein two adjacent atoms of the heteroaryl-group are optionally substituted in such a way, that a 3- to 7-membered heterocycloalkyl-group is formed. Particularly, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl *etc.,* and benzo derivatives thereof, such as, for example, benzofuranyl, benzothienyl, benzoxazolyl, benzisoxazolyl, benzimidazolyl, benzotriazolyl, indazolyl, indolyl, isoindolyl, *etc*.; or pyridyl, pyridazinyl, pyrimidinyl, pyrazinyl, triazinyl, *etc.,* and benzo derivatives thereof, such as, for example, quinolinyl, quinazolinyl, isoquinolinyl, *etc*.; or azocinyl, indolizinyl, purinyl, *etc.,* and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, *etc..*

In general, and unless otherwise mentioned, the heteroarylic or heteroarylenic radicals include all the possible isomeric forms thereof, e.g. the positional isomers thereof. Thus, for some illustrative non-restricting example, the term pyridinyl or pyridinylene includes pyridin-2-yl, pyridin-2-ylene, pyridin-3-yl, pyridin-3-ylene, pyridin-4-yl and pyridin-4-ylene; or the term thienyl or thienylene includes thien-2-yl, thien-2-ylene, thien-3-yl and thien-3-ylene.

The term "C₁-C₆", as used throughout this text, *e*.*g*. in the context of the definition of "C₁-C₆-alkyl", "C₁-C₆-haloalkyl", "C₁-C₆-alkoxy", or "C₁-C₆-haloalkoxy" is to be understood as meaning an alkyl group having a finite number of carbon atoms of 1 to 6, *i.e.* 1, 2, 3, 4, 5, or 6 carbon atoms. It is to be understood further that said term "C₁-C₆" is to be interpreted as any sub-range comprised therein, *e*.*g*. C₁-C₆, C₂-C₅ , C₃-C₄, C₁-C₂, C₁-C₃, C₁-C₄, C₁-C₅, C₁-C₆; particularly C₁-C₂, C₁-C₃, C₁-C₄, C₁-C₅, C₁-C₆; more particularly C₁-C₄; in the case of "C₁-C₆-haloalkyl" or "C₁-C₆-haloalkoxy" even more particularly C₁-C₂.

Similarly, as used herein, the term "C₂-C₆", as used throughout this text, *e*.*g*. in the context of the definitions of "C₂-C₆-alkenyl" and "C₂-C₆-alkynyl", is to be understood as meaning an alkenyl group or an alkynyl group having a finite number of carbon atoms of 2 to 6, *i.e.* 2, 3, 4, 5, or 6 carbon atoms. It is to be understood further that said term "C₂-C₆" is to be interpreted as any sub-range comprised therein, *e.g*. C₂-C₆, C₃-C₅, C₃-C₄, C₂-C₃, C₂-C₄, C₂-C₅; particularly C₂-C₃.

Further, as used herein, the term "C₃-C₆", as used throughout this text, *e*.*g*. in the context of the definition of "C₃-C₆-cycloalkyl", is to be understood as meaning a cycloalkyl group having a finite number of carbon atoms of 3 to 6, *i.e.* 3, 4, 5 or 6 carbon atoms. It is to be understood further that said term "C₃-C₆" is to be interpreted as any sub-range comprised therein, *e*.*g*. C₃-C₆, C₄-C₅, C₃-C₅, C₃-C₄, C₄-C₆, C₅-C₆; particularly C₃-C₆.

As used herein, the term "leaving group" refers to an atom or a group of atoms that is displaced in a chemical reaction as stable species taking with it the bonding electrons. Preferably, a leaving group is selected from the group comprising: halo, in particular chloro, bromo or iodo, methanesulfonyloxy, p-toluenesulfonyloxy, trifluoromethanesulfonyloxy, nonafluorobutanesulfonyloxy, (4-bromo-benzene)sulfonyloxy, (4-nitro-benzene)sulfonyloxy, (2-nitro-benzene)-sulfonyloxy, (4-isopropyl-benzene)sulfonyloxy, (2,4,6-tri-isopropyl-benzene)-sulfonyloxy, (2,4,6-trimethyl-benzene)sulfonyloxy, (4-tertbutyl-benzene)sulfonyloxy, benzenesulfonyloxy, and (4-methoxy-benzene)sulfonyloxy.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

The term "optionally substituted" means optional substitution with the specified groups, radicals or moieties.

Ring system substituent means a substituent attached to an aromatic or nonaromatic ring system which, for example, replaces an available hydrogen on the ring system.

As used herein, the term "one or more times", *e*.*g*. in the definition of the substituents of the compounds of the general formulae of the present invention, is understood as meaning "one, two, three, four or five times, particularly one, two, three or four times, more particularly one, two or three times, even more particularly one or two times".

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulphur, fluorine, chlorine, bromine and iodine, such as ²H (deuterium), ³H (tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I and ¹³¹I, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

Where the plural form of the word compounds, salts, polymorphs, hydrates, solvates and the like, is used herein, this is taken to mean also a single compound, salt, polymorph, isomer, hydrate, solvate or the like.

By "stable compound' or "stable structure" is meant a compound that is sufficiently robust to survive isolation to a useful degree of purity from a reaction mixture, and formulation into an efficacious therapeutic agent.

In accordance with a first aspect, the present invention is directed to compounds of general formula I : 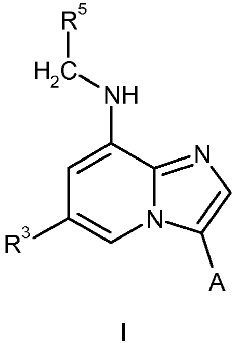 in which :
- A: represents a 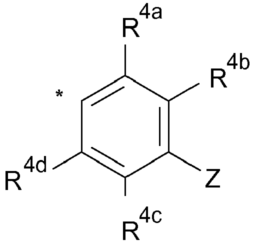 group ;
wherein * indicates the point of attachment of said groups with the rest of the molecule ;
- Z: represents a -C(=O)N(H)R¹ or a -C(=S)N(H)R¹ group ;
- R¹: represents a C₁-C₃-alkyl- or a cyclopropyl- group ;
wherein said cyclopropyl- group is optionally substituted, identically or differently, with 1, 2 or 3 groups selected from: halogen, -OH, -CN, C₁-C₃-alkyl-;
wherein said C₁-C₃-alkyl- group is optionally substituted, identically or differently, with 1, 2 or 3 groups selected from: halogen, -OH, -CN, C₁-C₃-alkoxy-;
- R³: represents a hydrogen atom or a -CN, C₁-C₆-alkyl-, -(CH₂)ₘ-C₂-C₆-alkenyl, -(CH₂)ₘ-C₂-C₆-alkynyl, aryl-, heteroaryl-, aryl-X- group ;
wherein said C₁-C₆-alkyl-, -(CH₂)ₘ-C₂-C₆-alkenyl, -(CH₂)ₘ-C₂-C₆-alkynyl, aryl-, aryl-X- or heteroaryl- group is optionally substituted, identically or differently, with 1, 2 or 3 R⁷ groups;
- R^{4a}, R^{4b}, R^{4d}: represent a hydrogen atom ;
- R^{4c}: represents a halogen atom, or a -CN, -OH, C₁-C₃-alkyl-, C₁-C₃-alkoxy-, halo-C₁-C₃-alkyl-, halo-C₁-C₃-alkoxy-, R^{6a}(R^{6b})N-C₁-C₃-alkyl-, HO-C₁-C₃-alkyl-, NC-C₁-C₃-alkyl-, C₁-C₃-alkoxy-C₁-C₃-alkyl-, halo-C₁-C₃-alkoxy-C₁-C₃-alkyl-group ;
- R⁵: represents a hydrogen atom, or a C₁-C₆-alkyl-, -(CH₂)ₙ-C₂-C₆-alkenyl, -(CH₂)ₙ-C₂-C₆-alkynyl, -(CH₂)ₘ-C₃-C₆-cycloalkyl, -(CH₂)ₘ-(3- to 7-membered heterocycloalkyl), aryl-C₁-C₆-alkyl-, heteroaryl-C₁-C₆-alkyl-, halo-C₁-C₆-alkyl-, R^{6a}(R^{6b})N-C₁-C₆-alkyl-, HO-C₁-C₆-alkyl-, -C₁-C₆-alkyl-CN, C₁-C₆-alkoxy-C₁-C₆-alkyl-, halo-C₁-C₆-alkoxy-C₁-C₆-alkyl-, C₃-C₆-cycloalkyl-, 3- to 7-membered heterocycloalkyl-, C₄-C₈-cycloalkenyl-, aryl- or heteroaryl-group ;
wherein said C₁-C₆-alkyl-, -(CH₂)ₙ-C₂-C₆-alkenyl, -(CH₂)ₙ-C₂-C₆-alkynyl, -(CH₂)ₘ-C₃-C₆-cycloalkyl, -(CH₂)ₘ-(3- to 7-membered heterocycloalkyl), aryl-C₁-C₆-alkyl-, heteroaryl-C₁-C₆-alkyl-, halo-C₁-C₆-alkyl-, R^{6a}(R^{6b})N-C₁-C₆-alkyl-, HO-C₁-C₆-alkyl-, -C₁-C₆-alkyl-CN, C₁-C₆-alkoxy-C₁-C₆-alkyl-, halo-C₁-C₆-alkoxy-C₁-C₆-alkyl-, C₃-C₆-cycloalkyl-, 3- to 7-membered heterocycloalkyl-, C₄-C₈-cycloalkenyl-, aryl- or heteroaryl-group is optionally substituted, identically or differently, with 1, 2, 3, 4 or 5 R⁸ groups ;
- R⁶, R^{6a}, R^{6b}, R^{6c}: represent, independently from each other, a hydrogen atom, or a C₁-C₆-alkyl-, C₃-C₆-cycloalkyl- or aryl-C₁-C₆-alkyl- group ;
wherein said C₃-C₆-cycloalkyl- group is optionally substituted, identically or differently with 1 or 2 groups selected from: halogen, -OH, -CN, C₁-C₆-alkyl-, HO-C₁-C₆-alkyl-;
- R⁷: represents a halogen atom or a HO-, C₁-C₆-alkoxy-, C₁-C₆-alkyl-, halo-C₁-C₆-alkyl-, halo-C₁-C₆-alkoxy-, HO-C₁-C₆-alkyl-, -C(=O)N(H)R^{6a}, -N(R^{6a})R^{6b}, -O(C=O)OR⁶ or -OR⁶ group ;
- R⁸: represents a hydrogen or halogen atom or a -CN, C₁-C₆-alkoxy-, C₁-C₆-alkyl-, halo-C₁-C₆-alkyl-, R^{6a}(R^{6b})N-C₁-C₆-alkyl-, HO-C₁-C₆-alkyl-, C₁-C₆-alkoxy-C₁-C₆-alkyl-, halo-C₁-C₆-alkoxy-C₁-C₆-alkyl-, C₂-C₆-alkenyl-, C₂-C₆-alkynyl-, 3- to 7-membered heterocycloalkyl-, aryl-, heteroaryl-, -C(=O)R⁶, -C(=O)N(H)R^{6a}, -C(=O)N(R^{6a})R^{6b}, -C(=O)O-R⁶, -N(R^{6a})R^{6b}, -NO₂, -N(H)C(=O)R⁶, -N(R^{6c})C(=O)R⁶, -N(H)C(=O)N(R^{6a})R^{6b}, -N(R^{6c})C(=O)N(R^{6a})R^{6b}, -N(H)C(=O)OR⁶, -N(R^{6c})C(=O)OR⁶, -N(H)S(=O)R⁶, -N(R^{6c})S(=O)R⁶, -N(H)S(=O)₂R⁶, -N(R^{6c})S(=O)₂R⁶, -N=S(=O)(R^{6a})R^{6b}, -OR⁶, -O(C=O)R⁶, -O(C=O)N(R^{6a})R^{6b}, -O(C=O)OR⁶, -SR⁶, -S(=O)R⁶, - S(=O)N(H)R⁶, -S(=O)N(R^{6a})R^{6b}, -S(=O₂)R⁶, -S(=O)₂N(H)R⁶, -S(=O)₂N(R^{6a})R^{6b}, -S(=O)(=NR^{6c})R⁶ or -S(=O)₂-(3- to 7-membered heterocycloalkyl) group ; wherein said 3- to 7-membered heterocycloalkyl- or heteroaryl- group, is optionally substituted, identically or differently, with 1, 2, 3 or 4 C₁-C₆-alkyl-groups ;
- m: is an integer of 0, 1, 2, 3, 4, 5 or 6 ;
- n: is an integer of 0, 1, 2, 3, 4 or 5 ; and
- X: is S, S(=O), S(=O)₂, O, NR⁶, C(=O) or CR^{6a}R^{6b} ;

In a preferred embodiment, with respect to compounds of formula I, *supra,*
- A: represents 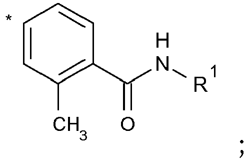 wherein * indicates the point of attachment of said group with the rest of the molecule.

In another preferred embodiment, with respect to compounds of formula I, *supra,*
- R¹: represents a methyl- or ethyl- group ;
wherein said methyl- or ethyl- group is optionally substituted, identically or differently, with 1, 2 or 3 groups selected from: halogen, -OH, -CN, C₁-C₃-alkoxy-.

In another preferred embodiment, with respect to compounds of formula I, *supra,*
- R¹: represents a methyl- or ethyl- group.

In another preferred embodiment, with respect to compounds of formula I, *supra,*
- R¹: is selected from:
methyl, ethyl, 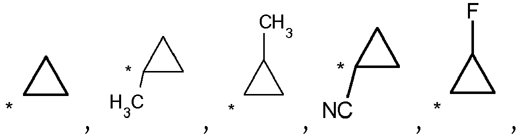 wherein * indicates the point of attachment of said group with the rest of the molecule.

In another preferred embodiment, with respect to compounds of formula I, *supra,*
- R¹: represents a methyl- group or a cyclopropyl- group ;
wherein said cyclopropyl- group is optionally substituted with one fluorine atom.

In another preferred embodiment, with respect to compounds of formula I, *supra,* R³ represents an aryl- group ; wherein said aryl- group is substituted, identically or differently, with 1 or 2 R⁷ groups. The aryl- group preferably is a substituted or unsubstituted phenyl- group.

In another preferred embodiment, with respect to compounds of formula I, *supra,* R³ represents an aryl-X- group; wherein said aryl-X- group is substituted, identically or differently, with 1 or 2 R⁷ groups. The aryl- group preferably is a substituted or unsubstituted phenyl- group.

In another preferred embodiment, with respect to compounds of formula I, *supra,* R³ represents an aryl-O- group; wherein said aryl-O- group is substituted, identically or differently, with 1 or 2 R⁷ groups. The aryl- group preferably is a substituted or unsubstituted phenyl- group.

In another preferred embodiment, with respect to compounds of formula I, *supra,* R³ represents an aryl-S(=O)ₚ- group; wherein said aryl-S(=O)ₚ- group is substituted, identically or differently, with 1 or 2 R⁷ groups. The integer p equals 0, 1 or 2. Preferably, p = 0 or p = 2. More preferably, p = 0. The aryl- group preferably is a substituted or unsubstituted phenyl- group.

In another preferred embodiment, with respect to compounds of formula I, *supra,* R³ represents a hydrogen atom or a -CN, C₁-C₆-alkyl-, -(CH₂)ₘ-C₂-C₆-alkenyl, -(CH₂)ₘ-C₂-C₆-alkynyl, aryl-, heteroaryl-, aryl-X- group ; wherein said C₁-C₆-alkyl-, -(CH₂)ₘ-C₂-C₆-alkenyl, -(CH₂)ₘ-C₂-C₆-alkynyl, aryl-, aryl-X-or heteroaryl- group is optionally substituted, identically or differently, with 1 or 2 R⁷ groups.

In another preferred embodiment, with respect to compounds of formula I, *supra,* R³ represents a heteroaryl- group which is optionally substituted, identically or differently, with 1 or 2 R⁷ groups. The heteroaryl- group preferably is a substituted or unsubstituted pyridyl- group.

In another preferred embodiment, with respect to compounds of formula I, *supra,* R³ is selected from:
H, H₃C-CH₂-, HO-CH₂-CH₂-, HO-CH₂-CH₂-CH₂-,
H₃C-CH(OH)-, H₂C=CH-, 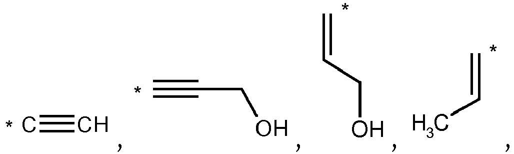 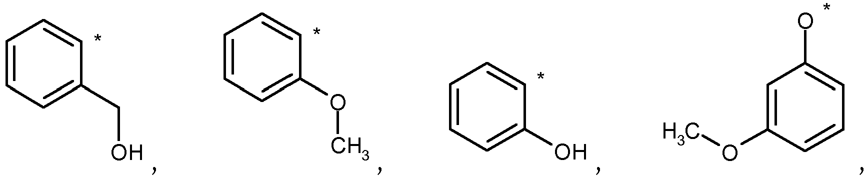 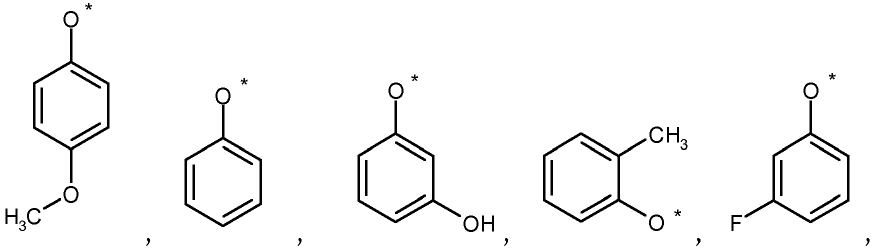 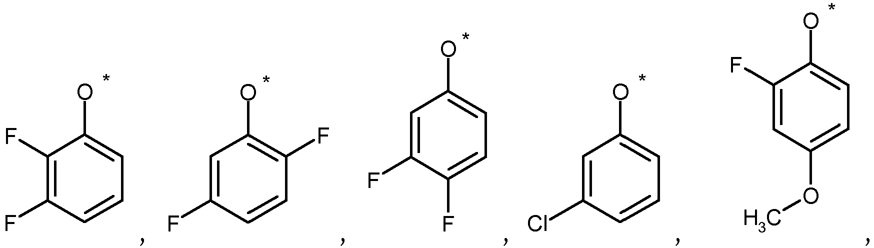 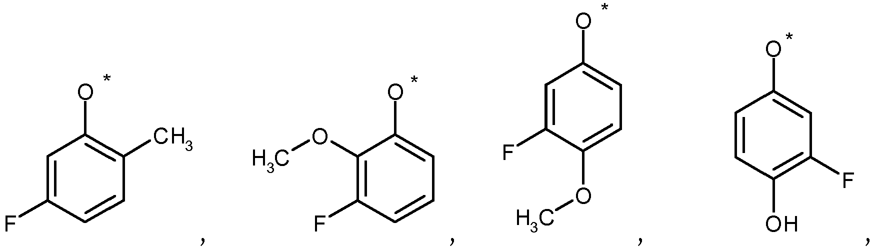 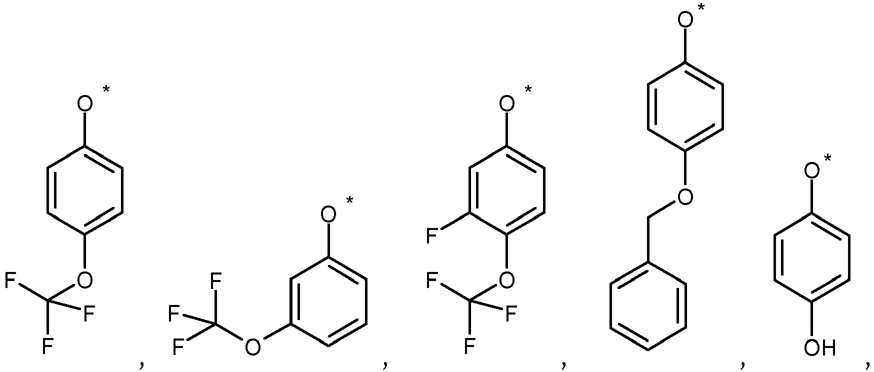 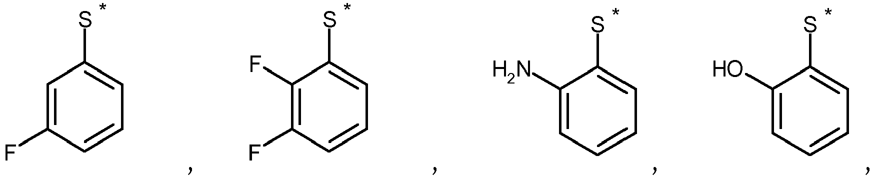 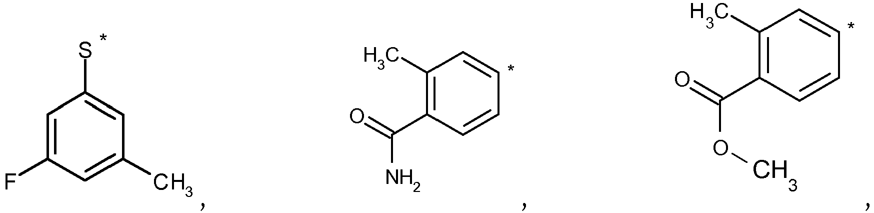 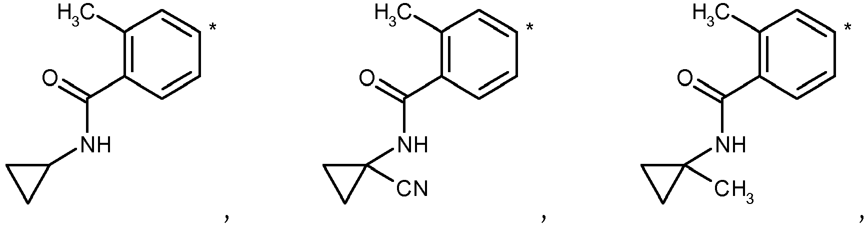 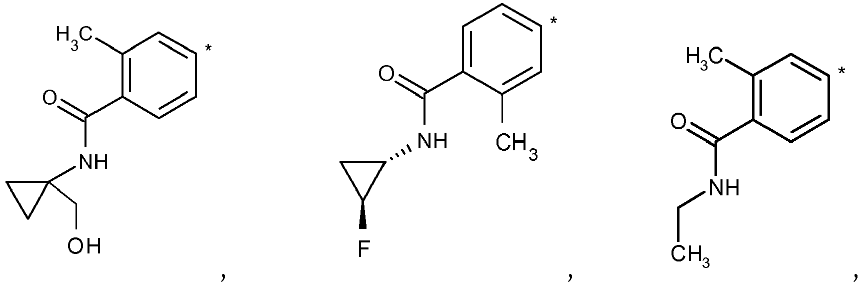 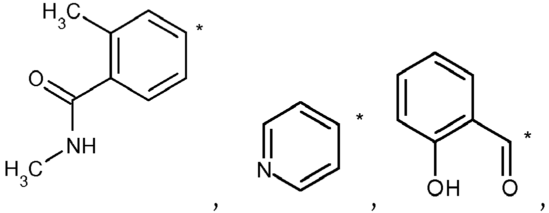 wherein * indicates the point of attachment of said groups with the rest of the molecule.

In another preferred embodiment, with respect to compounds of formula I, *supra,*
- R³: represents a pyridyl-, phenyl-, phenyl-O- or phenyl-S- group ;
wherein the phenyl- group is either substituted with a methyl- group and a -C(=O)N(H)R^{6a} group, or with a HO-CH₂- group;
wherein the phenyl-O- group is optionally substituted, identically or differently, with 1 or 2 groups selected from: -F, H₃C-O-, HO-, H₃C-; wherein the phenyl-S- group is optionally substituted, identically or differently, with 1 or 2 groups selected from: -F, H₃C-O-, HO-, H₃C-.

In another preferred embodiment of the present invention
R^{4a} = H, R^{4b} = H, R^{4c} = CH₃, and R^{4d} = H.

In another preferred embodiment, with respect to compounds of formula I, *supra,*
- R⁵: represents -(CH₂)ₙ-C₂-C₆-alkenyl, -(CH₂)ₙ-C₂-C₆-alkynyl, -(CH₂)ₘ-C₃-C₆-cycloalkyl, -(CH₂)ₘ-(3- to 7-membered heterocycloalkyl), aryl-C₁-C₆-alkyl-, heteroaryl-C₁-C₆-alkyl-, halo-C₁-C₆-alkyl-, R^{6a}(R^{6b})N-C₁-C₆-alkyl-, HO-C₁-C₆-alkyl-, -C₁-C₆-alkyl-CN, C₁-C₆-alkoxy-C₁-C₆-alkyl-, halo-C₁-C₆-alkoxy-C₁-C₆-alkyl-, C₃-C₆-cycloalkyl-, 3-to 7-membered heterocycloalkyl-, C₄-C₈-cycloalkenyl-, aryl- or heteroaryl-group ;
said (CH₂)ₙ-C₂-C₆-alkenyl, (CH₂)ₙ-C₂-C₆-alkynyl, -(CH₂)ₘ-C₃-C₆-cycloalkyl, -(CH₂)ₘ-(3- to 7-membered heterocycloalkyl), aryl-C₁-C₆-alkyl-, heteroaryl-C₁-C₆-alkyl-, halo-C₁-C₆-alkyl-, R^{6a}(R^{6b})N-C₁-C₆-alkyl-, HO-C₁-C₆-alkyl-, -C₁-C₆-alkyl-CN, C₁-C₆-alkoxy-C₁-C₆-alkyl-, halo-C₁-C₆-alkoxy-C₁-C₆-alkyl-, C₃-C₆-cycloalkyl-, 3- to 7-membered heterocycloalkyl-, C₄-C₈-cycloalkenyl-, aryl- or heteroaryl- group is optionally substituted, identically or differently, with 1, 2, 3, 4 or 5 R⁸ groups.

In another preferred embodiment, with respect to compounds of formula I, *supra,*
- R⁵: represents a C₁-C₆-alkyl-, -(CH₂)ₘ-(3- to 7-membered heterocycloalkyl), C₁-C₆-alkoxy-C₁-C₆-alkyl- or halo-C₁-C₆-alkyl- group ;
wherein said C₁-C₆-alkyl-, -(CH₂)ₘ-(3- to 7-membered heterocycloalkyl), C₁-C₆-alkoxy-C₁-C₆-alkyl- or halo-C₁-C₆-alkyl- group is optionally substituted, identically or differently, with 1, 2 or 3 R⁸ groups.

In another preferred embodiment, with respect to compounds of formula I, *supra,*
- R⁵: is selected from:
H, (CH₃)₂CH-, CHF₂-, CF₃-, CF₃-CH₂-, CF₃-CH₂-CH₂-, CF₃-CH(OH)-, HO-CH₂-, HO-C(CH₃)₂-, HO-C(CH₃)₂CH₂-, HO-CH₂-CH(OH)-, H₃C-O-CH₂-, H₂N-CH₂-CH₂-, H₂N-C(CH₃)₂-, (CH₃)₂N-CH₂-, (CH₃)₂N-CH₂-CH₂-, (CH₃)₂N-CH₂-CH₂-CH₂-, (CH₃)₂N-C(CH₃)₂-, H₃C-S(=O)₂-CH₂-, H₃C-S(=O)₂-CH₂-CH₂-, HO-S(=O)₂-CH₂-, HO-S(=O)₂-CH₂-CH₂-, NC-CH₂-, H₃C-C(=O)-N(H)-CH₂, H₃C-C(=O)-N(H)-CH₂-CH₂-, H₂N-C(=O)-CH₂-, (CH₃)₂N-C(=O)-CH₂-, H₃C-N(H)-C(=O)-N(CH₃)-CH₂-CH₂-, 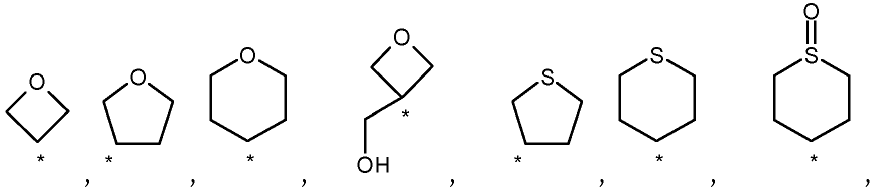 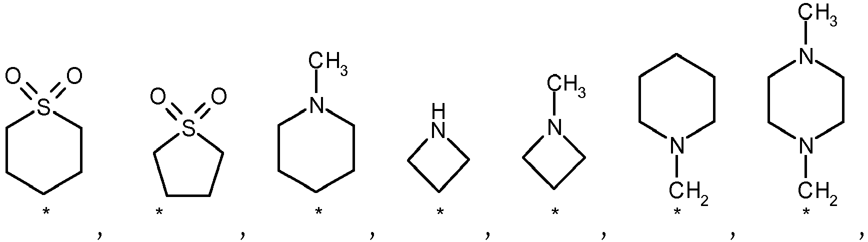 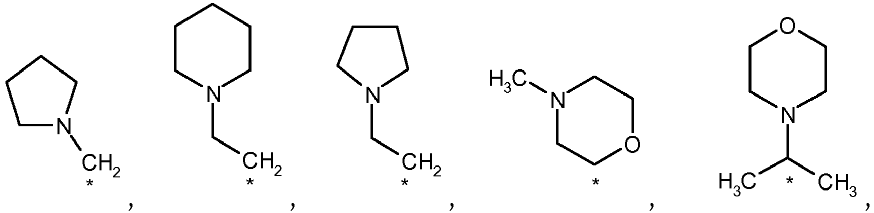 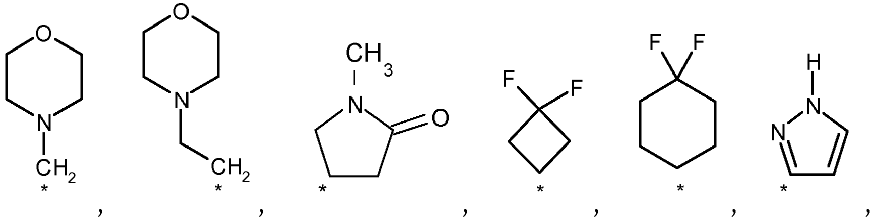 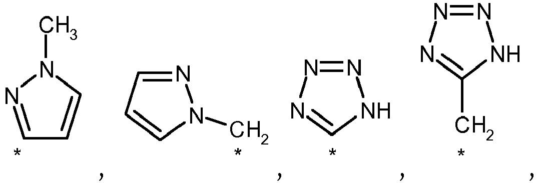 wherein * indicates the point of attachment of said groups with the rest of the molecule.

In another preferred embodiment, with respect to compounds of formula **I**, *supra,* R⁵ represents a group selected from: 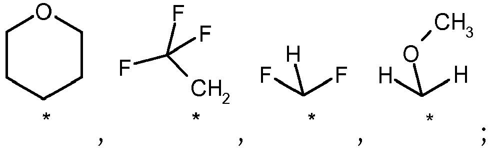 wherein * indicates the point of attachment of said groups with the rest of the molecule.

In another preferred embodiment, with respect to compounds of formula **I**, *supra,* R^{6a} represents a hydrogen atom or methyl- group or a C₃-C₆-cyclpropyl- group; wherein said C₃-C₆-cyclpropyl- group is optionally substituted with one -CN group.

In another preferred embodiment, with respect to compounds of formula I, *supra,* R⁸ represents a halogen atom, or a -CN, -OH, C₁-C₆-alkoxy-, C₁-C₆-alkyl-, HO-C₁-C₆-alkyl, -C(=O)N(R^{6a})R^{6b}, -N(R^{6a})R^{6b}, -N(H)C(=O)R⁶, -N(R^{6c})C(=O)N(R^{6a})R^{6b}, -S(=O)₂R⁶ or -S(=O)₂OH group.

In another preferred embodiment, the invention relates to compounds of formula I, wherein X is S.

In another preferred embodiment, with respect to compounds of formula I, *supra,* X is S(=O).

In another preferred embodiment, with respect to compounds of formula I, *supra,* X is S(=O)₂.

In another preferred embodiment, with respect to compounds of formula I, *supra,* X is O.

In another preferred embodiment, with respect to compounds of formula I, *supra,* X is NR⁶. Preferably, X is NH or N(CH₃). Most preferably, X is NH.

In another preferred embodiment, with respect to compounds of formula I, *supra,* X is CR^{6a}R^{6b}. Preferably, X is CH₂.

In another preferred embodiment, with respect to compounds of formula **I**, *supra,* X is C(=O).

In another preferred embodiment, with respect to compounds of formula **I**, *supra,* Z represents a -C(=O)N(H)R¹ group.

In another preferred embodiment, with respect to compounds of formula **I**, *supra,* n is 1.

In another preferred embodiment, with respect to compounds of formula **I**, *supra,* m is 0 or 1.

It is to be understood that the present invention relates also to any combination of the preferred embodiments described above.
One example of a combination is given hereinafter. However, the invention is not limited to this combination.

In a preferred embodiment the present invention is related to compounds of formula I : 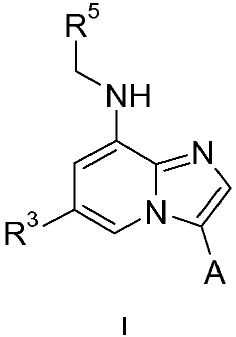 in which :
- A: represents 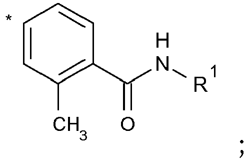 wherein * indicates the point of attachment of said group with the rest of the molecule ;
- R¹: represents a methyl- group or a cyclopropyl- group ;
wherein said cyclopropyl- group is optionally substituted with one fluorine atom;
- R³: represents a pyridyl-, phenyl-, phenyl-O- or phenyl-S- group ;
wherein the phenyl- group is either substituted with a methyl- group and a -C(=O)N(H)R^{6a} group, or with a HO-CH₂- group;
wherein the phenyl-O- group is optionally substituted, identically or differently, with 1 or 2 groups selected from: -F, H₃C-O-, HO-, H₃C-;
wherein the phenyl-S- group is optionally substituted, identically or differently, with 1 or 2 groups selected from: -F, H₃C-O-, HO-, H₃C-;
- R⁵: represents a group selected from: 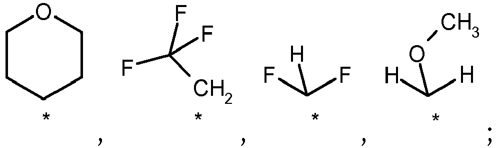 wherein * indicates the point of attachment of said groups with the rest of the molecule
- R^{6a}: represents a hydrogen atom or methyl- group or a C₃-C₆-cyclpropyl- group;
wherein said C₃-C₆-cyclpropyl- group is optionally substituted with one -CN group ;
or a stereoisomer, a tautomer, an N-oxide, a hydrate, a solvate, or a salt thereof, or a mixture of same.

It is to be understood that the present invention relates to any sub-combination within any embodiment of the present invention of compounds of general formula *I, supra.*

More particularly still, the present invention covers compounds of general formula I which are disclosed in the Experimental Section of this text, *infra.*

The compounds of this invention may contain one or more asymmetric centre, depending upon the location and nature of the various substituents desired. Asymmetric carbon atoms may be present in the (R) or (S) configuration, resulting in racemic mixtures in the case of a single asymmetric centre, and diastereomeric mixtures in the case of multiple asymmetric centres. In certain instances, asymmetry may also be present due to restricted rotation about a given bond, for example, the central bond adjoining two substituted aromatic rings of the specified compounds.

Substituents on a ring may also be present in either cis or trans form. It is intended that all such configurations (including enantiomers and diastereomers), are included within the scope of the present invention.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g.*, chiral HPLC columns), with or without conventional derivatisation, optimally chosen to maximise the separation of the enantiomers. Suitable chiral HPLC columns are manufactured by Diacel, *e.g.*, Chiracel OD and Chiracel OJ among many others, all routinely selectable. Enzymatic separations, with or without derivatisation, are also useful. The optically active compounds of this invention can likewise be obtained by chiral syntheses utilizing optically active starting materials.

In order to limit different types of isomers from each other reference is made to IUPAC Rules Section E (Pure Appl Chem 45, 11-30, 1976).

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulphur, fluorine, chlorine, bromine and iodine, such as ²H (deuterium), ³H (tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I ¹²⁴I, ¹²⁹I and ¹³¹I, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

The present invention includes all possible stereoisomers of the compounds of the present invention as single stereoisomers, or as any mixture of said stereoisomers, in any ratio. Isolation of a single stereoisomer, *e.g.* a single enantiomer or a single diastereomer, of a compound of the present invention may be achieved by any suitable state of the art method, such as chromatography, especially chiral chromatography, for example.

Further, the compounds of the present invention may exist as tautomers. For example, any compound of the present invention which contains a pyrazole moiety as a heteroaryl group for example can exist as a 1 H tautomer, or a 2H tautomer, or even a mixture in any amount of the two tautomers, or a triazole moiety for example can exist as a 1 H tautomer, a 2H tautomer, or a 4H tautomer, or even a mixture in any amount of said 1 H, 2H and 4H tautomers, namely :

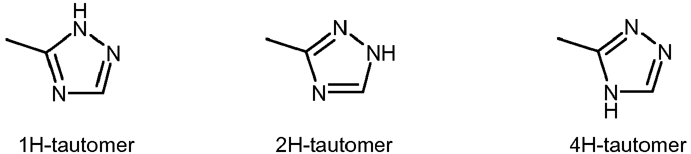

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

Furthermore, the present invention includes all possible crystalline forms, or polymorphs, of the compounds of the present invention, either as single polymorphs, or as a mixture of more than one polymorph, in any ratio.

The compounds of the present invention can exist as a hydrate, or as a solvate, wherein the compounds of the present invention contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

In an embodiment of the above-mentioned aspects, the invention relates to compounds of formula I, according to any of the above-mentioned embodiments, in the form of or a stereoisomer, a tautomer, an N-oxide, a hydrate, a solvate, or a salt thereof, or a mixture of same.

The compounds of the present invention have surprisingly been found to effectively inhibit Mps-1 kinase and may therefore be used for the treatment or prophylaxis of diseases of uncontrolled cell growth, proliferation and/or survival, inappropriate cellular immune responses, or inappropriate cellular inflammatory responses or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by Mps-1 kinase, such as, for example, haemotological tumours, solid tumours, and/or metastases thereof, *e.g*. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

Therefore, the compounds of formula I, *supra,* are expected to be valuable as therapeutic agents.

Accordingly, in another embodiment, the present invention is directed to a compound of general formula I, *supra,* or a stereoisomer, a tautomer, an N-oxide, a hydrate, a solvate, or a salt thereof, particularly a pharmaceutically acceptable salt thereof, or a mixture of same, for use in the treatment or prophylaxis of a disease.

In another embodiment, the present invention provides a method of treating disorders associated with enhanced uncontrolled proliferative cellular processes in a patient in need of such treatment, comprising administering to the patient an effective amount of a compound of formula I.

The term "treating" or "treatment" as stated throughout this document is used conventionally, e.g., the management or care of a subject for the purpose of combating, alleviating, reducing, relieving, improving the condition of a disease or disorder, such as a carcinoma.

The term "subject" or "patient" includes organisms which are capable of suffering from a cell proliferative disorder or who could otherwise benefit from the administration of a compound of the invention, such as human and non-human animals. Preferred humans include human patients suffering from or prone to suffering from a cell proliferative disorder or associated state, as described herein. The term "non-human animals" includes vertebrates, e.g., mammals, such as non-human primates, sheep, cow, dog, cat and rodents, e.g., mice, and non-mammals, such as chickens, amphibians, reptiles, etc.

The terms "cell proliferative disorder" or "disorder associated with enhanced uncontrolled proliferative cellular processes" include disorders involving the undesired or uncontrolled proliferation of a cell. The compounds of the present invention can be utilized to prevent, inhibit, block, reduce, decrease, control, etc., cell proliferation and/or cell division, and/or produce apoptosis. This method comprises administering to a subject in need thereof, including a mammal, including a human, an amount of a compound of this invention, or a pharmaceutically acceptable salt, isomer, polymorph, metabolite, hydrate or solvate thereof which is effective to treat or prevent the disorder.

In another embodiment, the present invention is directed to a compound of general formula I, or a stereoisomer, a tautomer, an N-oxide, a hydrate, a solvate, or a salt thereof, particularly a pharmaceutically acceptable salt thereof, or a mixture of same, for use in the treatment or prophylaxis of a disease, wherein said disease is a disease of uncontrolled cell growth, proliferation and/or survival, an inappropriate cellular immune response, or an inappropriate cellular inflammatory response, particularly in which the uncontrolled cell growth, proliferation and/or survival, inappropriate cellular immune response, or inappropriate cellular inflammatory response is mediated by the mitogen-activated protein kinase (MEK-ERK) pathway, more particularly in which the disease of uncontrolled cell growth, proliferation and/or survival, inappropriate cellular immune response, or inappropriate cellular inflammatory response is a haemotological tumour, a solid tumour and/or metastases thereof, *e.g.* leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

The present invention also relates to useful forms of the compounds as disclosed herein, such as metabolites, hydrates, solvates, prodrugs, salts, in particular pharmaceutically acceptable salts, *in vivo* hydrolysable esters, and co-precipitates.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, para-toluenesulfonic, methansulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric, or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides ; dialkyl sulfates like dimethyl, diethyl, and dibutyl sulfate ; and diamyl sulfates, long chain halides such as decyl, lauryl, myristyl and strearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention are prepared by reacting the compounds of the invention with the appropriate base via a variety of known methods.

The present invention includes all possible salts of the compounds of the present invention as single salts, or as any mixture of said salts, in any ratio.

As used herein, the term *"in vivo* hydrolysable ester" is understood as meaning an *in vivo* hydrolysable ester of a compound of the present invention containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, *e.g.* methoxymethyl, C₁-C₆ alkanoyloxymethyl esters, *e.g.* pivaloyloxymethyl, phthalidyl esters, C₃-C₈ cycloalkoxy-carbonyloxy-C₁-C₆ alkyl esters, *e.g.* 1-cyclohexylcarbonyloxyethyl ; 1,3-dioxolen-2-onylmethyl esters, *e.g.* 5-methyl-1,3-dioxolen-2-onylmethyl; and C₁-C₆-alkoxycarbonyloxyethyl esters, *e.g.* 1-methoxycarbonyloxyethyl, and may be formed at any carboxy group in the compounds of this invention.

An *in vivo* hydrolysable ester of a compound of the present invention containing a hydroxy group includes inorganic esters such as phosphate esters and [alpha]-acyloxyalkyl ethers and related compounds which as a result of the *in vivo* hydrolysis of the ester breakdown to give the parent hydroxy group. Examples of [alpha]-acyloxyalkyl ethers include acetoxymethoxy and 2,2-dimethylpropionyloxymethoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The present invention covers all such esters.

Compounds of formula I may be administered as the sole pharmaceutical agent or in combination with one or more additional therapeutic agents where the combination causes no unacceptable adverse effects. This combination therapy includes administration of a single pharmaceutical dosage formulation which contains a compound of formula I and one or more additional therapeutic agents, as well as administration of the compound of formula I and each additional therapeutic agent in its own separate pharmaceutical dosage formulation. For example, a compound of formula I and a therapeutic agent may be administered to the patient together in a single oral dosage composition such as a tablet or capsule, or each agent may be administered in separate dosage formulations.

Where separate dosage formulations are used, the compound of formula I and one or more additional therapeutic agents may be administered at essentially the same time (e.g., concurrently) or at separately staggered times (e.g., sequentially).

In another aspect, the invention provides a pharmaceutical composition comprising a compound of general formula I, or a stereoisomer, a tautomer, an N-oxide, a hydrate, a solvate, or a salt thereof, particularly a pharmaceutically acceptable salt thereof, or a mixture of same, and a pharmaceutically acceptable diluent or carrier.

Preferably, the pharmaceutical combination comprises :
- one or more compounds of general formula I, or a stereoisomer, a tautomer, an N-oxide, a hydrate, a solvate, or a salt thereof, particularly a pharmaceutically acceptable salt thereof, or a mixture of same; and
- one or more agents selected from : a taxane, such as Docetaxel, Paclitaxel, or Taxol; an epothilone, such as Ixabepilone, Patupilone, or Sagopilone; Mitoxantrone; Predinisolone; Dexamethasone; Estramustin; Vinblastin; Vincristin; Doxorubicin; Adriamycin; Idarubicin; Daunorubicin; Bleomycin; Etoposide; Cyclophosphamide; Ifosfamide; Procarbazine; Melphalan; 5-Fluorouracil; Capecitabine; Fludarabine; Cytarabine; Ara-C; 2-Chloro-2'-deoxyadenosine; Thioguanine; an anti-androgen, such as Flutamide, Cyproterone acetate, or Bicalutamide; Bortezomib; a platinum derivative, such as Cisplatin, or Carboplatin; Chlorambucil; Methotrexate; and Rituximab.

In still another aspect, the invention provides a process for preparing a pharmaceutical composition. The process includes the step of combining at least one compound of formula I as defined above with at least one pharmaceutically acceptable carrier, and bringing the resulting combination into a suitable administration form.

In still another aspect, the invention provides use of a compound of formula I as defined above for manufacturing a pharmaceutical composition for the treatment or prevention of a cell proliferative disorder. In certain embodiments, the cell proliferative disorder is cancer.

The active component of formula I can act systemically and/or locally. For this purpose, it can be applied in a suitable manner, for example orally, parenterally, pulmonally, nasally, sublingually, lingually, buccally, rectally, transdermally, conjunctivally, otically, or as an implant or stent.

When the compounds of the present invention are administered as pharmaceuticals, to humans and animals, they can be given per se or as a pharmaceutical composition containing, for example, 0.1 to 99.5% (more preferably, 0.5 to 90%) of active ingredient in combination with a pharmaceutically acceptable carrier.
Regardless of the route of administration selected, the compounds of the invention, which may be used in a suitable hydrated form, and/or the pharmaceutical compositions of the present invention, are formulated into pharmaceutically acceptable dosage forms by conventional methods known to those of skill in the art.

Actual dosage levels and time course of administration of the active ingredients in the pharmaceutical compositions of the invention may be varied so as to obtain an amount of the active ingredient which is effective to achieve the desired therapeutic response for a particular patient, composition, and mode of administration, without being toxic to the patient.

In accordance with another aspect, the present invention covers methods of preparing compounds of the present invention, said methods comprising the steps as described in the Experimental Section herein.

In accordance with another aspect, the present invention also relates to methods of preparing a compound of general formula I, *supra.*

In accordance with a first embodiment, the present invention relates to a method of preparing a compound of general formula I, the method comprises the step of allowing an intermediate compound of general formula IV : 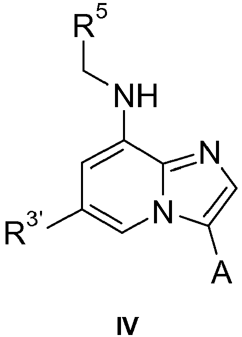 in which R⁵ and A are as defined for general formula I, *supra,* and R^{3'} is a halogen atom,
to react with a compound of general formula **IVa :**

R³-Y **IVa**

in which R³ is as defined for general formula I, *supra,* and Y is a substituent which is displaced in a coupling reaction, such as a hydrogen atom, or a boronic acid group, or an ester of a boronic acid group, for example,
thereby giving, upon optional deprotection, a compound of general formula I : 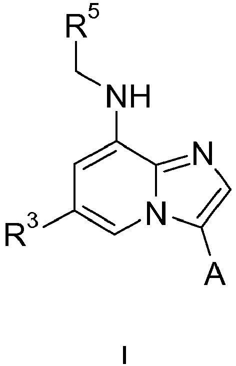 in which R³, R⁵ and A are as defined for general formula I, *supra.*

In accordance with a second embodiment, the present invention also relates to a method of preparing a compound of general formula I, *supra,* said method comprising the step of allowing an intermediate compound of general formula **II :** 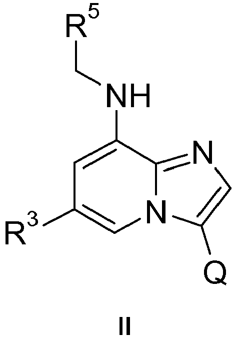 in which R³ and R⁵ are as defined for general formula I, *supra,* and Q is a halogen atom
to react with a compound of general formula **IIa :**

A-Y **IIa**

in which A is as defined for general formula I, *supra,* and Y is a substituent which is displaced in a coupling reaction, such as a boronic acid group, or an ester of a boronic acid group, for example,
thereby giving, upon optional deprotection, a compound of general formula I : 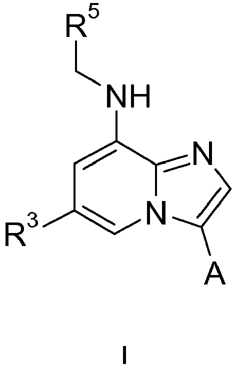 in which R³, R⁵ and A are as defined for general formula I, *supra.*

In accordance with a third embodiment, the present invention also relates to a method of preparing a compound of general formula I, *supra,* said method comprising the step of allowing an intermediate compound of general formula **VII :** 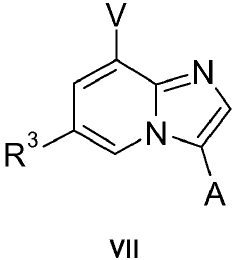 in which R³ and A are as defined for general formula I, *supra,* and V is a leaving group, for example a halogen atom,
to react with a compound of general formula **VIIa :**

R⁵-CH₂-NH₂ **VIIa**

in which R⁵ is as defined for general formula I, *supra,*
thereby giving, upon optional deprotection, a compound of general formula I : 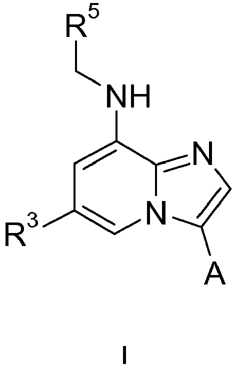 in which R³, R⁵ and A are as defined for general formula I, *supra.*

In accordance with a forth embodiment, the present invention also relates to a method of preparing a compound of general formula I, *supra,* said method comprising the step of allowing an intermediate compound of general formula **VII :** 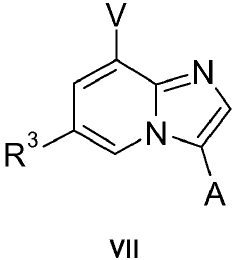 in which R³ and A are as defined for general formula I, *supra,* and V is a NH₂-group
to react with a compound of general formula **VIIb :**

O=CHR⁵ **VIIb**

in which R⁵ is as defined for general formula I, *supra,*
thereby giving, upon optional deprotection, a compound of general formula I : 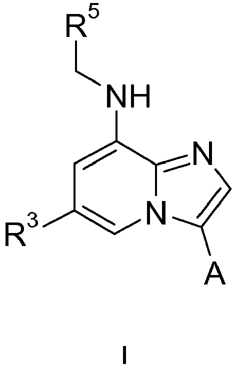 in which R³, R⁵ and A are as defined for general formula I, *supra.*

In accordance with a further aspect, the present invention covers intermediate compounds which are useful in the preparation of compounds of the present invention of general formula I, particularly in the methods described herein.

In particular, the present invention covers compounds of general formula IV : 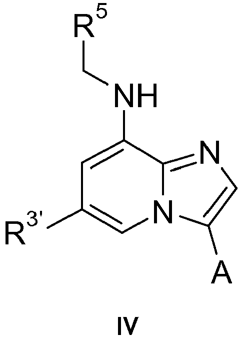 in which R⁵ and A are as defined for general formula I, *supra,* and R^{3'} is a halogen atom.

The present invention also covers compounds of general formula **VII :** 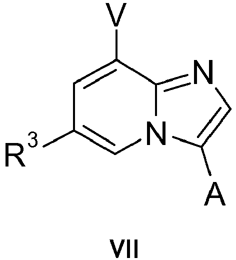 in which R³ and R⁵ are as defined for general formula I, *supra,* and V is a NH₂-group or a halogen atom.

### EXPERIMENTAL SECTION

As mentioned *supra,* another aspect of the present invention is a method which may be used for preparing the compounds according to the present invention.

The following Table lists the abbreviations used in this paragraph, and in the Examples section. NMR peak forms are stated as they appear in the spectra, possible higher order effects have not been considered.
Names of compounds were generated using the Autonom 2000 add-in of ISIS/Draw [MDL Information Systems Inc. (Elsevier MDL)] or the ICS naming tool 12.01 of ACD labs. In some cases generally accepted names of commercially available reagents were used.

| **Abbreviation** | **Meaning** |
|---|---|
| Ac | Acetyl |
| br | Broad |
| c- | cyclo- |
| d | Doublet |
| dd | doublet of doublets |
| DCE | Dichloroethane |
| DCM | Dichloromethane |
| DIPEA | N,N-diisopropylethylamine |
| DMAP | 4-Dimethylaminopyridine |
| DMF | N,N-dimethylformamide |
| DMSO | dimethyl sulfoxide |
| DIPEA | *N,N*-Diisopropylethylamine |
| dppf | 1,1'-bis(di-phenylphosphino)ferrocene |
| EDC | N-[3-(dimethylamino)propyl]-N'-ethylcarbodiimide |
| eq | Equivalent |
| ESI | electrospray ionisation |
| EtOAc | ethyl acetate |
| m | multiplet |
| MeOH | methanol |
| MS | mass spectrometry |
| MW | molecular weight |
| NIS | N-Iodosuccinimide |
| NMP | N-methylpyrrolidi none |
| NMR | nuclear magnetic resonance spectroscopy : chemical shifts (δ) are given in ppm. |
| HATU | 2-(1H-7-Azabenzotriazol-1-yl)--1,1,3,3-tetramethyl uronium hexafluorophosphate Methanaminium |
| HCl | hydrochloric acid |
| MPLC | middle performance liquid chromatography |
| MS | mass spectrometry |
| NMR | nuclear magnetic resonance spectroscopy : chemical shifts (δ) are given in ppm. The chemical shifts were corrected by setting the DMSO signal to 2.50 ppm using unless otherwise stated. |
| Pd₂dba₃ | Tris(dibenzylideneacetone)dipalladium(0) |
| Pd(dppf)Cl₂ | 1,1'-bis(diphenylphosphino)ferrocenedichloropalladium(II) |
| P(oTol)₃ | tri-o-tolylphosphine |
| q | quartet |
| rac-BINAP | racemic-2,2'-bis(diphenylphosphino)-1,1'-binaphthyl |
| rt | room temperature |
| RT | retention time in minutes |
| s | singlet |
| sept | septet |
| t | triplet |
| TEA | triethylamine |
| TFA | trifluoroacetic acid |
| THF | tetrahydrofuran |
| UPLC | ultra performance liquid chromatography |

Other abbreviations have their meanings customary per se to the skilled person. The various aspects of the invention described in this application are illustrated by the following examples which are not meant to limit the invention in any way.

The schemes and procedures described below illustrate general synthetic routes to the compounds of general formula **I** of the invention and are not intended to be limiting. It is clear to the person skilled in the art that the order of transformations as exemplified in the Schemes can be modified in various ways. The order of transformations exemplified in the Schemes is therefore not intended to be limiting. In addition, interconversion of any of the substituents, A, R³ or NH-CH₂-R⁵ can be achieved before and/or after the exemplified transformations. These modifications can be such as the introduction of protecting groups, cleavage of protecting groups, reduction or oxidation of functional groups, halogenation, metallation, substitution, cyclization, condensation or other reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wuts in Protective Groups in Organic Synthesis, 3^{rd} edition, Wiley 1999). Specific examples are described in the subsequent paragraphs. Further, it is possible that two or more successive steps may be performed without work-up being performed between said steps, *e.g.* a "one-pot" reaction, as is well-known to the person skilled in the art.

### Synthesis of compounds of general formula I of the present invention

Compounds of general formula **I** can be synthesized as depicted in the Scheme, with R³, R⁵, and A having the meaning as given for general formula I, *supra,* and R^{3'}, Q representing leaving groups and V represents an optionally protected NH₂-group or a leaving group. Examples for typical leaving groups include but are not limited to halogen atoms like a chlorine, bromine or iodine atom or S(O)ₚR⁶-groups like a methylsulfonyl-, triflate- or nonaflate-group, p being 0, 1 or 2.

The Scheme exemplifies routes that allow variations for R³, R^{3'}, R⁵, Q, V and A during the synthesis. Functional moieties in R³, R^{3'}, R⁵, Q, V and A can be converted at every suitable stage of the synthesis.

However, also other routes were used for synthesis of the target compounds.

Compounds of formula **XI** may be commercially available or can be synthesized according to procedures known to persons skilled in the art (see for example Tschitschibabin; Jegorow, Zhurnal Russkago Fiziko-Khimicheskago Obshchestva, 1928 , 60, 689; Fray, M. Jonathan et al., Journal of Medicinal Chemistry, 1995, 38, 3524 - 3535 or Cai, Sui Xiong et al., Journal of Medicinal Chemistry, 1997, 40, 3679 - 3686).
Compounds of formula X may be commercially available or can be synthesized according to procedures known to persons skilled in the art (see for example Loiseau, Philippe R. et al. European Journal of Medicinal Chemistry, 1987, 22, 457-462 or WO 200426867 A2, 2004, 32-33).
Compounds of formula **IX** may be commercially available or can be synthesized according to procedures known to persons skilled in the art (see for example Gudmundsson, Kristjan S.; Johns, Brian A., Organic Letters, 2003, 5, 1369 - 1372 or WO 201070008 A1, 2010, 68).

A leaving group Q can be introduced in compounds of general formula **X**, **VI** or **III** by procedures known to persons skilled in the art to give compounds of general formula **IX, V** or **II.** As an example, halogens can be introduced using halogenation reagents like N-iodosuccinimide, N-bromosuccinimide or N-chlorosuccinimide, in an inert solvent like N,N-dimethylformamide or 1-methylpyrrolidin-2-one, for example, at temperatures ranging from room temperature to the boiling point of the solvent, for example.

Compounds of general formula **I**, **IV** or **VIII** can be obtained from compounds of general formula **II, V** or **IX** via a coupling reaction between a reagent of formula Y-A, in which A is defined *supra* and Y represents a suitable functional group by which the group A can be transferred to the Q-group bearing carbon atom of compounds of formula **II, V** or **IX.** Examples of suitable functional groups for Y in A-Y include boronic acids A-B(OH)₂, or esters of boronic acids A-B(OC₁-C₆-alkyl)₂. Said coupling reactions are performed in the presence of suitable catalysts, such as, for example, palladium based catalysts like, for example, Palladium (II) acetate, tetrakis(triphenylphosphine)palladium (0), bis(triphenylphosphine)-palladium (II) chloride or (1,1,-bis(diphenylphosphino) ferrocene)-dichloropalladium (II) and optionally suitable additives such as, for example, phosphines like, for example, P(oTol)₃ or triphenylphosphine and optionally with a suitable base, such as, for example, potassium carbonate, sodium 2-methylpropan-2-olate, tetrabutylammonium fluoride or tribasic potassium phosphate in a suitable solvent, such as, for example, tetrahydrofuran.
Examples of such coupling reactions may be found in the textbook entitled "Metal-Catalyzed Cross-Coupling Reactions", Armin de Meijere (Editor), Francois Diederich (Editor) September 2004, Wiley Interscience ISBN: 978-3-527-30518-6.

Compounds of general formula I, **II, III** or **VII** can be obtained from compounds of general formula **IV, V**, **VI** or **VIII** via a coupling reaction using a reagent of formula Y-R³ in which R³ is defined *supra* and Y represents a suitable functional group by which the group R³ can be transferred to the R^{3'} bearing carbon atom of compounds of formula **IV, V**, **VI** or **VIII.** Examples of suitable functional groups Y for the use in coupling reactions are given *supra* for the preparation of compounds of general formula I, **IV** or **VIII** from compounds of general formula **II, V** or **IX**.
The coupling reactions include metal catalyzed coupling reactions like Sonogashira coupling reactions with alkynes for alkyne introduction, Heck coupling reactions with alkenes for alkene introduction, Hartwig Buchwald coupling reactions with amines for amine introduction.

Y in Y-R³ may also represent an acidic hydrogen that can be removed by suitable bases, for example sodium hydride, in a suitable solvent, such as DMSO or tetrahydrofuran at temperatures ranging from rt to the boiling point. The resulting nucleophiles like, for example, primary or secondary amines, alkoxides, thiolates or carbon anion bearing groups can be used to replace R^{3'} in compounds of general formula **IV, V**, **VI** or **VIII** to add secondary or tertiary amines, ethers, thioethers or carbon-atom attached groups to give compounds of general formula **I**, **II, III** or **VII.** Compounds of general formula **I**, **II, III** or **VII** containing primary or secondary amines, ethers or thioether can also be build by Ullmann-type coupling reactions in the presence of suitable catalysts, such as, for example, copper based catalysts like copper(II)diacetate in presence of a suitable base, like for example, caesium carbonate staring from compounds of general formula **IV, V**, **VI** or **VIII** in which R^{3'} represents a leaving group such as, for example, an iodine, bromine or chlorine atom. Optionally, suitable ligands like N,N-dimethylglycine or phenyl hydrogen pyrrolidin-2-ylphosphonate can be added.

In the case V represents a leaving group, the introduction of a R⁵-CH₂-group can be achieved by nucleophilic substitution of V in compounds of formula **VII, VIII, IX** or X i. e. by a reaction with suitable amines R⁵-CH₂-NH₂ in the presence of a suitable base, such as, for example DIPEA in a suitable solvent such as N,N-dimethylformamide or 1-methylpyrrolidin-2-one, at temperatures ranging from room temperature to the boiling point of the solvent to give amines of general formula **I, IV, V** or **VI**.
In the case V represents a leaving group, the introduction of a R⁵-CH₂-group can also be achieved in a coupling reaction in which V in compounds of formula **VII, VIII, IX** or **X** is reacted with suitable amines R⁵-CH₂-NH₂ optionally in the presence of a suitable catalyst, such as Pd₂dba₃ and BINAP for example, and optionally with a suitable base, such as, for example, sodium tert-butylate in a suitable solvent, such as, for example, N,N-dimethylformamide or 1-methylpyrrolidin-2-one to give amines of general formula **I, IV, V** or **VI.**

In the case V represents an optionally protected NH₂-group the introduction of a R⁵-CH₂-group, after deprotection to a NH₂-group, can be achieved by a reductive amination reaction using an aldehyde of formula O=CHR⁵, a suitable reducing agent, for example sodium tris(acetato-kappaO)(hydrido)borate or sodium cyanoborohydride in a suitable solvent like, for example, acetic acid at reaction temperatures ranging from room temperature to the boiling point of the solvent.

Residues in compounds of formula **I**, **II, III, IV, V**, **VI, VII, VIII, IX, X** or **XI** can be optionally modified. For example, thioethers can be oxidized using oxidation reagents like 3-chlorobenzenecarboperoxoic acid, oxone or dimethyldioxirane in inert solvents like dichloromethane or acetone, respectively. Depending on the stoichiometric ratio of oxidation reagent to the afore mentioned compounds sulfoxides or sulfones or mixtures thereof will be obtained.

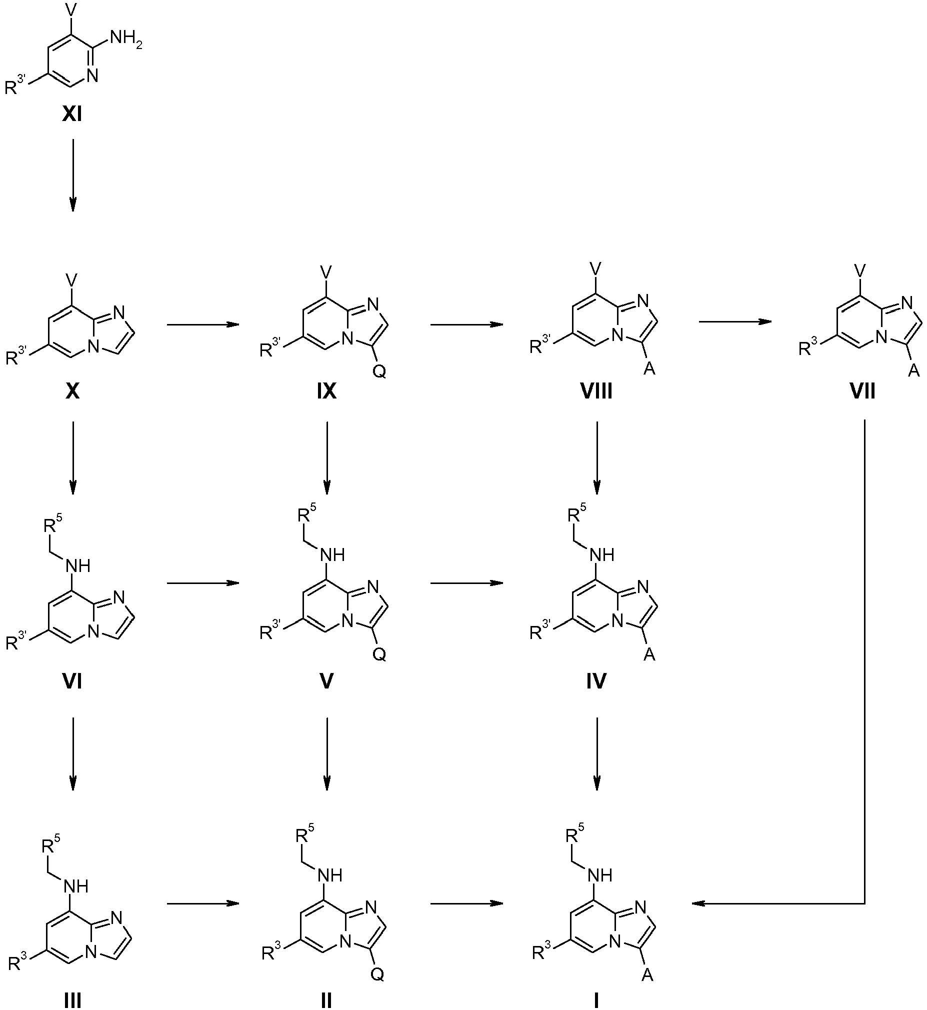

Further, the compounds of formula **I** of the present invention can be converted to any salt as described herein, by any method which is known to the person skilled in the art. Similarly, any salt of a compound of formula **I** of the present invention can be converted into the free compound, by any method which is known to the person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallisation. In some cases, impurities may be removed by stirring using a suitable solvent. In some cases, the compounds may be purified by chromatography, particularly flash chromatography, using for example pre-packed silica gel cartridges, *e.g.* from Separtis such as Isolute^{®} Flash silica gel or Isolute^{®} Flash NH2 silica gel in combination with a suitable chromatographic system such as a Flashmaster II (Separtis) or an Isolera system (Biotage) and eluents such as, for example, gradients of hexane/EtOAc or DCM/methanol. In some cases, the compounds may be purified by preparative HPLC using, for example, a Waters autopurifier equipped with a diode array detector and/or on-line electrospray ionisation mass spectrometer in combination with a suitable pre-packed reverse phase column and eluants such as, for example, gradients of water and acetonitrile which may contain additives such as trifluoroacetic acid, formic acid or aqueous ammonia.

### Analytical UPLC-MS was performed as follows:

Method A: System: UPLC Acquity (Waters) with PDA Detector und Waters ZQ mass spectrometer; Column: Acquity BEH C18 1.7µm 2.1x50mm; Temperature: 60°C; Solvent A: Water + 0.1% formic acid; Solvent B: acetonitrile; Gradient: 99 % A → 1 % A (1.6 min) → 1 % A (0.4 min) ; Flow: 0.8 mL/min; Injection Volume: 1.0 µl (0.1 mg-1mg/mL sample concentration); Detection: PDA scan range 210-400 nm - Fixed and ESI (+),scan range 170-800 m/z

### Intermediate Example 1-1: Preparation of 6,8-dibromo-imidazo[1,2-a]pyrazine

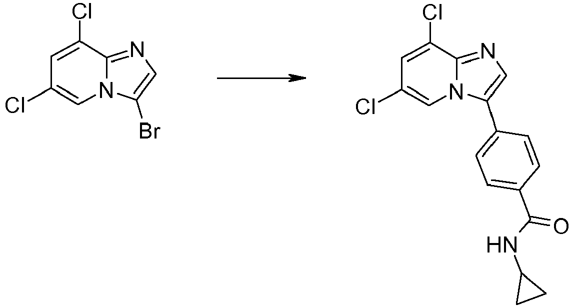

To a stirred solution of commercially available (Apollo) 3-bromo-6,8-dichloroimidazo[1,2-a]pyridine (1.00 g, 3.76 mmol) in NMP (40 mL) was subsequently added 2.31 g [4-[(cyclopropylamino)carbonyl]phenyl]-boronic acid (11.28 mmol, 3 eq), 614 mg Pd(dppf)Cl₂ (0.75mmol, 0.2 eq) and aqueous potassium carbonate solution (1M, 3 mL) in one portion at rt. After heating for 40 min at 130° C in a microwave oven, water was added and the precipitate was filtered off, washed and dried. Purification by preparative HPLC yielded 481 mg (36.95 %) N-cyclopropyl-4-(6,8-dichloroimidazo[1,2-a]pyridin-3-yl)benzamide: ¹H-NMR (300 MHz, CDCl₃): δ = 8.51 (1H), 8.00 - 7.91 (3H), 7.81 - 7.68 (4H), 2.85 (1H), 0.68 (1H), 0.57 (1H) ppm.

### Intermediate Example 2-1:

### Preparation of N-cyclopropyl-4-(6,8-dichloroimidazo[1,2-a]pyridin-3-yl)benzamide

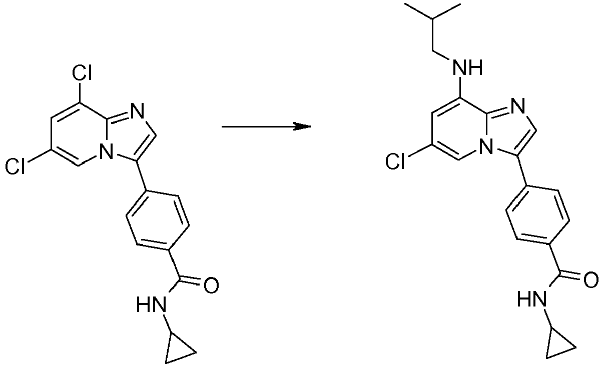

To a stirred solution of N-cyclopropyl-4-(6,8-dichloroimidazo[1,2-a]pyridin-3-yl)benzamide (320 mg, 0.92 mmol) in NMP (30 mL) was subsequently added 67.6 mg 2-methylpropan-1-amine (0.92 mmol, 1 eq), 169 mg (1E,4E)-1,5-diphenylpenta-1,4-dien-3-one - palladium (3:2) (0.185 mmol, 0.2 eq), 345.3 mg 1,1'-binaphthalene-2,2'-diylbis(diphenylphosphane) (0.55 mmol, 0.6 eq) and 222.1 mg sodium tert-butylate (2.31 mmol, 2.5 eq) in one portion at rt. After heating for 40 min at 150° C in a microwave oven, the solution was filtered, evaporated and the residue triturated with water. Purification of the residue by preparative HPLC yielded 70 mg (19.7 %) 4-{6-chloro-8-[(2-methylpropyl)amino]imidazo[1,2-a]pyridin-3-yl}-N-cyclopropylben zamide: ¹H-NMR (300 MHz, CDCl₃): δ 7.88 (2H), 7.68 (1H), 7.60 (2H), 7.55 (1H), 6.28 (1H), 6.10 (1H), 5.41 (1H), 3.09 (2H), 2.95 (1H), 1.05 (6H), 0.91 (2H), 0.66 (2H)

### Intermediate Example 3-1:

### Preparation of 6-bromoimidazo[1,2-a]pyridin-8-amine

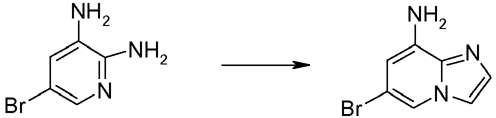

To a stirred solution of 5-bromopyridine-2,3-diamine (278 g, 1478 mmol) in isopropanol (2.2 L) at rt was added chloroacetaldehyde (255 g, 1626 mmol) in one portion. After stirring in an nitrogen atmosphere under reflux overnight, the mixture was stirred for an additional 60 min at rt. The suspension was filtered, and the remaining solid was washed with isopropanol and dried in vaccuo at 50°C. Redissolution in methanol and evaporation yielded 6-bromoimidazo[1,2-a]pyridin-8-amine as a brown solid (124 g, 40 %): ¹H-NMR (300 MHz, d₆-DMSO): δ =8.39 (2H), 8.12 (2H), 6.92 (1H) ppm.

### Intermediate Example 4-1:

### Preparation of 6-bromo-3-iodoimidazo[1,2-a]pyridin-8-amine

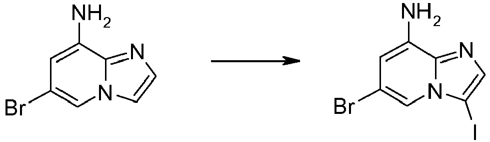

To a stirred suspension of 6-bromoimidazo[1,2-a]pyridin-8-amine (20.6 g, 97.15 mmol) in THF 100 mL) at 0°C was dropwise added a solution of NIS (25.4 g, 112.87 mmol, 1.16 eq) in 215 mL THF. After stirring for 2h, isolute sorbent (Biotage) was added (20 g) and the mixture was evaporated in vaccuo. The residue was loaded on a flash column and the crude product purified by flash chromatography (ethyl acetate / hexane) to yield 25.6 g 6-bromo-3-iodoimidazo[1,2-a]pyridin-8-amine (78.2 %). ¹H-NMR (300 MHz, d₆-DMSO): δ = 7.60 (1H), 7.54 (1 H, d), 6.38 (1H), 6.12 (2H) ppm. UPLC-MS: RT = 0.98 min; m/z (ES+) 339.0 [MH⁺]; required MW = 338.0.

### Intermediate Example 5-1:

### Preparation of 4-(8-amino-6-bromoimidazo[1,2-a]pyridin-3-yl)-N-cyclopropyl-2-methylbenzamide

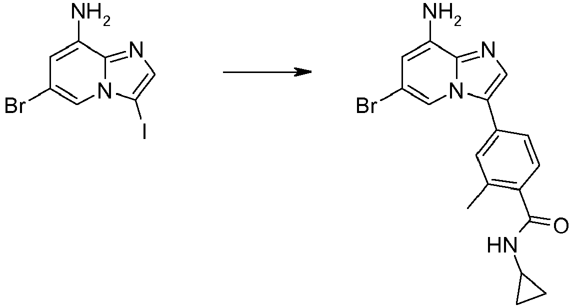

To a stirred solution of 6-bromo-3-iodoimidazo[1,2-a]pyridin-8-amine (8.52 g, 22.69 mmol) in THF (450 mL) was subsequently added 7.176 g N-cyclopropyl-2-methyl-4-(4,4,5,5-tetramethyl-1,3,2-dioxaborolan-2-yl)benzamide (23.82 mmol, 1.05 eq), 3.706 g Pd(dppf)Cl₂ (4.54 mmol, 0.2 eq) and aqueous potassium carbonate solution (1M, 68 mL) in one portion at rt. After stirring overnight at rt, the mixture was heated at 60°C for 24 h, and, after addition of 1.434 g N-cyclopropyl-2-methyl-4-(4,4,5,5-tetramethyl-1,3,2-dioxaborolan-2-yl)benzamide heated at 80° C for 48 h. After filtration through ALLOX and evaporation, the crude material was purified by flash chromatography to yield 4.93 g (56 %) 4-(8-amino-6-bromoimidazo[1,2-a]pyridin-3-yl)-N-cyclopropyl-2-methylbenzamide: ¹H-NMR (300 MHz, d₆-DMSO): δ = 8.31 (1H), 7.78 + 7.74 (1H), 7.59 (1 H), 7.45 + 7.44 (2H), 7.40 + 7.38 (1H), 6.41 + 6.37 (1H), 6.15 + 6.10 (2H), 2.81 (1H), 2.36 (3H), 0.66 (1H), 0.50 (1H) ppm. UPLC-MS: RT = 0.82 min; m/z (ES+) 386.3 [MH⁺]; required MW = 385.3.

### Intermediate Example 6-1:

### Preparation of 4-{6-bromo-8-[(3,3,3-trifluoropropyl)amino]imidazo[1,2-a]pyridin-3-yl}-N-cyclopropyl-2-methylbenzamide

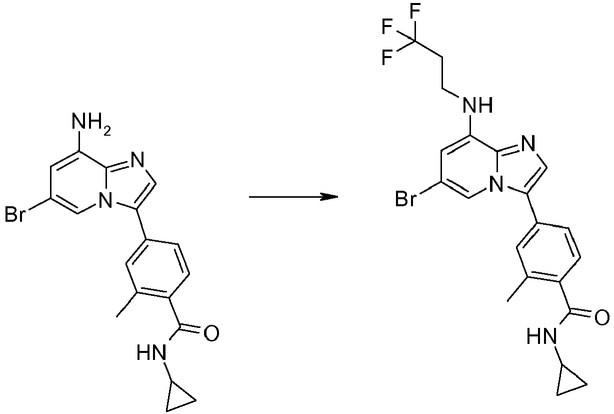

To a stirred solution of 4-(8-amino-6-bromoimidazo[1,2-a]pyridin-3-yl)-N-cyclopropyl-2-methylbenzamide (3 g, 5.87 mmol) in dichloroethane (60 mL) at rt was added 3,3,3-trifluoropropanal (4.58 g, 40.88 mmol, 7 eq). After stirring for 2 h, sodium triacetoxy borohydride (9.12 g, 40.88 mmol, 7 eq) and trifluoroacetic acid (3.33 g, 29.2 mmol, 5 eq) were added and the mixture was stirred for 1 h. After addition of DCM (100 mL), the organic phase was washed with water and evaporated. Purification of the residue by flash chromatography yielded 2.51 g 4-{6-bromo-8-[(3,3,3-trifluoropropyl)amino]imidazo[1,2-a]pyridin-3-yl}-N-cycloprop yl-2-methylbenzamide (66.9 %). ¹H-NMR (300 MHz, d₆-DMSO): δ = 8.32 (1H), 7.81 (1 H), 7.60 (1 H), 7.46 + 7.44 (2H), 7.41 + 7.39 (1 H), 6.63 (1 H), 6.32 (1 H), 3.49 (2H), 2.81 (1H), 2.64 (2H), 2.36 + 2.35 (3H), 0.66 (1H), 0.50 (1H) ppm. UPLC-MS: RT = 1.22 min; m/z (ES+) 482.3 [MH⁺]; required MW = 481.3.

### Intermediate Example 12:

### Preparation of [4-(cyclopropylcarbamoyl)-3-methylphenyl]boronic acid

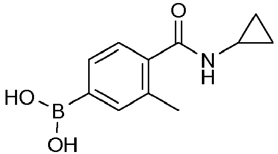

### Step A: Preparation of 4-bromo-N-cyclopropyl-2-methylbenzamide

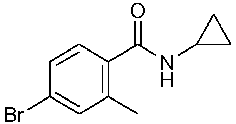

To a stirred solution of 4-bromo-2-methylbenzoic acid (300 g, 1.4 mol) in DCM (8.4 L) at rt was added cyclopropanamine (79.64 g, 1.4 mol) and EDC (320.9 g, 1.67 mol) in one portion. After stirring overnight, the solution was washed with water and the aqueous phase was reextracted with DCM. The combined organic phases were dried over sodium sulfate, filtered and evaporated. The remaining solid was triturated with diisopropyl ether, filtered, washed and dried in vaccuo to yield 260 g (73.4 %) 4-bromo-N-cyclopropyl-2-methylbenzamide: ¹H-NMR (300 MHz, CDCl₃): δ =7.34 (s, 1H), 7.27 (d, 1H), 7.14 (d, 1H), 5.96 (bs, 1H), 2.85 (m, 1H), 2.38 (s, 3H), 0.85 (m, 2H), 0.59 (m, 2H) ppm.

### Step B:

### Preparation of N-cyclopropyl-2-methyl-4-(4,4,5,5-tetramethyl-1,3,2-dioxaborolan-2-yl)benzamide

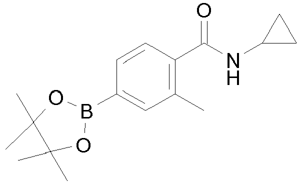

To a solution of 4-bromo-N-cyclopropyl-2-methylbenzamide (260 g, 1.02 mol) in dioxane (2 L) at rt was added bis-(pinacolato)-diboron (390 g, 1.53 mol), 2-dicyclohexylphosphino-2',4',6'-triisopropylbiphenyl (19.5 g, 40.9 mmol), potassium acetate (150.6 g, 1.53 mol) and tris-(dibenzylidenaceton)-dipalladium(0) (9.37 g, 10.2 mmol) and the mixture was refluxed for 6 h, After cooling to rt, water (3 L) and ethyl acetate (5 L) was added and the mixture stirred for 15 min. The organic phase was washed with water, dried over Na₂(SO₄), filtered and evaporated. Flash chromatography (ethyl acetate/hexane) yielded 308 g (56.3 %) N-cyclopropyl-2-methyl-4-(4,4,5,5-tetramethyl-1,3,2-dioxaborolan-2-yl)benzamide: ¹H-NMR (300 MHz, CDCl₃): δ =7.63 (s, 1 H), 7.60 (d, 1 H), 7.28 (d, 1 H), 5.94 (bs, 1 H), 2.87 (m, 1H), 2.41 (s, 3H), 1.33 (s, 6H), 0.85 (m, 2H), 0.59 (m, 2H) ppm.

### Step C: Preparation of [4-(cyclopropylcarbamoyl)-3-methylphenyl]boronic acid

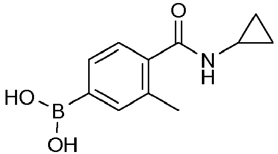

To a solution of N-cyclopropyl-2-methyl-4-(4,4,5,5-tetramethyl-1,3,2-dioxaborolan-2-yl)benzamide (20.2 g, 67.13 mol) in acetone (300 mL) at rt was added sodium periodate (43.1 g, 201.40 mol) and ammonium acetate (134.26 mol, 134 mL 1M aqueous solution) and the mixture was stirred for 3h. More water was added (120 mL), and the mixture was stirred at 40°C for 2 h more. After addition of 4 N HCl (32 mL), the organic phase was removed in vaccuo and the reminder was extracted with ethyl actate. The organic phase was washed with sat. sodium chloride solution, filtered through a Whatman filter and evaporated. The residue was redissolved in toluene and evaporated (two times) to yield 14.59 g (94.3 %) [4-(cyclopropylcarbamoyl)-3-methylphenyl]boronic acid: ¹H-NMR (300 MHz, d₆-DMSO): δ =8.21 (1H), 8.04 (2H), 7.56 (2H), 7.17 (1H), 2.77 (1H), 2.25 (3H), 0.62 (2H), 0.47 (2H) ppm.

### Example 1-1:

### Preparation of N-cyclopropyl-4-{8-[(2-methylpropyl)amino] imidazo[1,2-a]pyridin-3-yl}benzamide

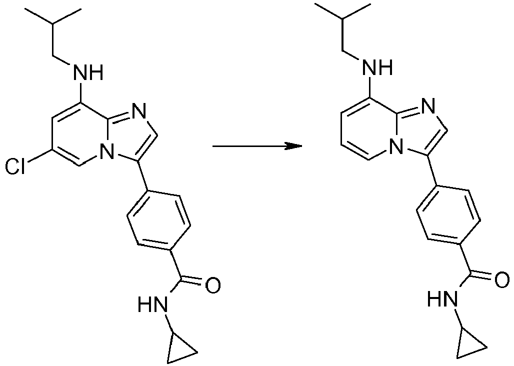

To a solution of 4-{6-chloro-8-[(2-methylpropyl)amino]imidazo[1,2-a]pyridin-3-yl}-N-cyclopropylben zamide (50 mg, 0.13 mmol) in ethanole (15 mL) was subsequently added 145 mg TEA (1.43 mmol) and 5 mg Pd/C (10%) at rt and the mixture subsequently stirred at rt in a hydrogen atmosphere at normal pressure for 2.5 h. After filtration, the solution was evaporated and the residue purified by preparative HPLC to yield 4.7 mg (10.3 %) N-cyclopropyl-4-{8-[(2-methylpropyl)amino]imidazo[1,2-a]pyridin-3-yl}benzamide: ¹H-NMR (300 MHz, d₆-DMSO): δ = 8.46 (1H), 7.93 (2H), 7.80 (1H), 7.71 - 7.65 (3H), 6.76 (1H), 6.15 (1H), 5.99 (1H), 3.03 (2H), 2.84 (1H), 0.92 (6H), 0.67 (2H), 0.56 (2H).

### Example 2-1:

### Preparation of N-cyclopropyl-4-{6-[2-(hydroxymethyl)phenyl]-8-[(3,3,3-trifluoropropyl)amino]imidazo[1,2-a]pyridin-3-yl}-2-methylbenzamide

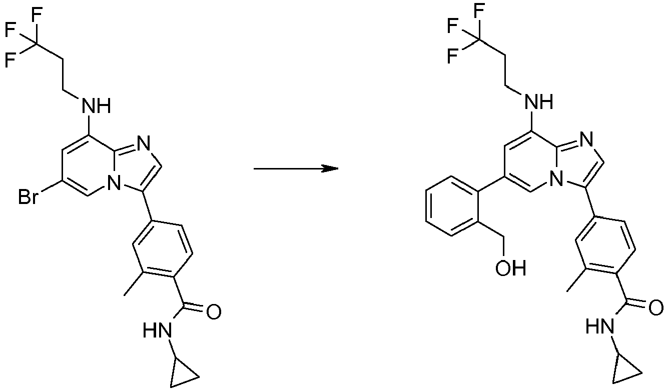

0.06 mmol 4-{6-bromo-8-[(3,3,3-trifluoropropyl)amino]imidazo[1,2-a]pyridin-3-yl}-N-cyclopropyl-2-methylbenzamide, 0.13 mmol [2-(hydroxymethyl)phenyl]boronic acid (18.9 mg, 2 eq), 0.012 mmol Pd(dppf)Cl₂ (10.18 mg, 0.2 eq), 2 mL NMP and 0.187 mmol potassium carbonate (0.19 mL, 1M in water, 3 eq) were combined in a sealed vial and heated at 130 °C under microwave irradiation for 40 min. After additional heating at 120°C overnight in a heating block and cooling to rt, the solution was filtered and subjected to preparative HPLC to give 17.3 mg (55 %) N-cyclopropyl-4-{6-[2-(hydroxymethyl)phenyl]-8-[(3,3,3-trifluoropropyl)amino]imid azo[1,2-a]pyridin-3-yl}-2-methylbenzamide: ¹H-NMR (300 MHz, CDCl₃): δ = 7.82 (1 H), 7.60 - 7.50 (3H), 7.48 - 7.30 (6H), 6.27 (1 H), 6.02 (1 H), 5.84 (1 H), 4.64 (2H), 3.58 (2H), 3.40 (2H, tr), 2.90 (1 H), 2.56 (2H), 2.45 (3H), 0.88 (2H), 0.61 (2H) ppm; UPLC-MS: RT = 1.02 min; m/z (ES+) 509.6 [MH⁺]; required MW = 508.6.

The following compound examples were prepared in analogy to the procedure described above using the appropriate intermediate example 6 and the appropriate boronic acid building block [LC-MS data such as retention time (RT in min) or observed mass peak were collected using LC-MS Method A unless explicitly stated]:

| Example | Structure | Name | Analytical Data |
|---|---|---|---|
| 2-2 | 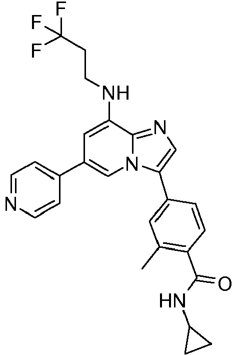 | N-cyclopropyl-2-methyl-4-{6-(pyridin-4-yl)-8-[(3,3,3-tr ifluoropropyl)amino]imidaz o[1,2-a]pyridin-3-yl}benza mide | RT = 0.85 MW_{found} = 480.5 MW_{calc} = 479.5 |
| | | | ¹H-NMR (300 MHz, d₆-DMSO): δ = 8.60 (2H), 8.34 (1H), 8.06 (1H), 7.71 (2H), 7.66 (1H), 7.59 - 7.53 (2H), 7.42 (1H), 6.54 (1H). 6.47 (1H) 3.60 (2H), 2.82 (1H), 2.71 (2H), 2.37 (3H), 0.66 (1H), 0.51 (1H) ppm |

### Example 3-1:

### Preparation of N-cyclopropyl-4-{6-[(3-fluoro-5-methylphenyl)sulfanyl]-8-[(3,3,3-trifluoropropyl)amino]imidazo[1,2-a]pyridin-3-yl}-2-methylbenzamide

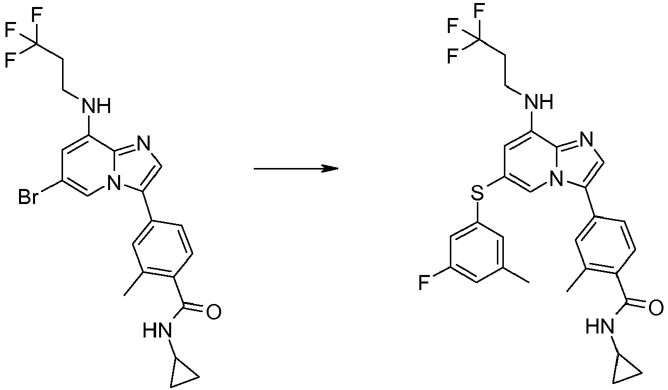

To a solution of 85.5 mg (0.6 mmol) 3-fluoro-5-methylbenzenethiol in 2.0 mL DMSO were added 24 mg (0.6 mmol) sodium hydride and the mixture was stirred for 1 h at rt.

48 mg (0.1 mmol) 4-{6-bromo-8-[(3,3,3-trifluoropropyl)amino]imidazo[1,2-a] pyridin-3-yl}-N-cyclopropyl-2-methylbenzamide were added and the mixture was heated at 160 °C for 1 h. The mixture was filtered and purified by HPLC to yield 20 mg (35 %) of the title compound. UPLC-MS: RT = 1.47 min; m/z (ES+) 543.6 [MH⁺]; required MW = 542.6. ¹H-NMR (300 MHz, d6-DMSO): δ = 8.31 (1H), 7.87 (1H), 7.66 (1H), 7.49 - 7.45 (2H), 7.39 (1H), 6.93 (1H), 6.85 (2H), 6.54 (1H), 6.13 (1H), 3.44 (2H), 2.81 (1H), 2.55 (2H), 2.35 (3H), 2.21 (3H), 0.66 (2H), 0.55 (2H) ppm.

The following compound examples were prepared analogously to the procedure described above using the appropriate thiol derivative and the appropriate Br-intermediate 6 [LC-MS data such as retention time (RT in min) or observed mass peak were collected using LC-MS Method A unless explicitly stated]:

| Example | Structure | Name | Analytical Data |
|---|---|---|---|
| 3-2 | 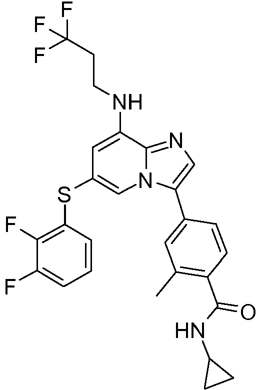 | N-cyclopropyl-4-{6-[(2,3-di fluorophenyl)sulfanyl]-8-[( 3,3,3-trifluoropropyl)amino]imidazo[1,2-a]pyridin-3-yl}-2-methylbenzamide | RT = 1.40 MW_{found} = 547.6 MW_{calc} = 546.6 |
| | | | ¹H-NMR (300 MHz, d₆-DMSO): δ = 8.29 (1 H), 7.90 (1H), 7.66 (1H), 7.48 - 7.42 (2H), 7.39 (1H), 7.30 (1H), 7.12 (1H), 7.01 (1H), 6.56 (1H)6.16 (1H), 3.43 (2H), 2.81 (1H), 2.54 (2H), 2.35 (3H), 0.66 (2H), 0.49 (2H) ppm |
| 3-3 | 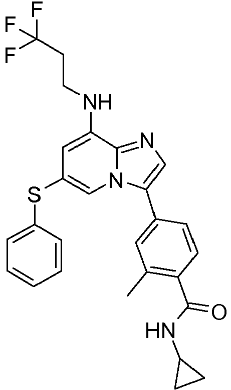 | N-cyclopropyl-2-methyl-4-{6-(phenylsulfanyl)-8-[(3,3,3-trifluoropropyl)amino]imidazo[1,2-a]pyridin-3-yl}benzamide | RT = 1.38 MW_{found} = 511.6 MW_{calc} = 510.6 |
| | | | ¹H-NMR (300 MHz, d₆-DMSO): δ = 8.32 (1 H), 7.75 (2H), 7.47 - 7.35 (3H), 7.31 (4H), 7.23 (1 H), 6.65 (1H), 6.23 (1H), 3.42 (2H), 2.80 (1 H), 2.55 (2H), 2.33 (3H), 0.65 (2H), 0.49 (2H) ppm |
| 3-4 | 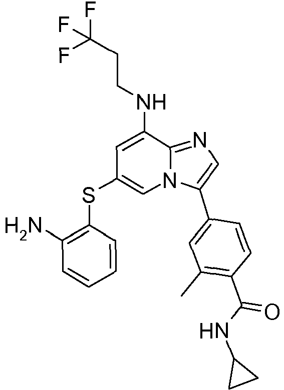 | 4-{6-[(2-aminophenyl)sulfanyl]-8-[(3,3,3-trifluoropropyl)amino]imidazo[1,2-a]pyridin-3-yl}-N-cyclopropyl-2-methylbenzamide | RT = 1.18 MW_{found} = 526.6 MW_{calc} = 525.6 |
| | | | ¹H-NMR (300 MHz, d₆-DMSO): δ = 8.29 (1H), 7.57 (1H), 7.42 (1H), 7.36 - 7.27 (3H), 7.12 (1H), 6.76 (1H), 6.53 (1H), 6.39 (1H), 6.06 (1H), 5.43 (2H), 5.40 (1H), 3.41 (2H), 2.81 (1H), 2.56 (2H), 2.30 (3H), 0.66 (2H), 0.50 (2H) ppm |
| 3-5 | 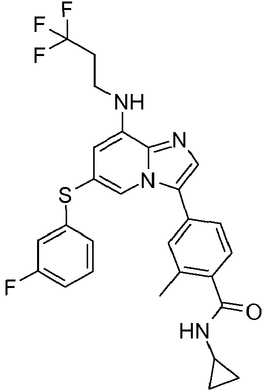 | N-cyclopropyl-4-{6-[(3-fluo rophenyl)sulfanyl]-8-[(3,3, 3-trifluoropropyl)amino]imidazo[1,2-a]pyridin-3-yl}-2-methylbenzamide | RT = 1.40 MW_{found} = 529.6 MW_{calc} = 528.6 |
| | | | ¹H-NMR (300 MHz, d₆-DMSO): δ = 8.30 (1H), 7.89 (1H), 7.70 (1H), 7.50 - 7.44 (2H), 7.39 (1H), 7.32 (1H), 7.08 (2H), 7.01 (1H), 6.58 (1H), 6.19 (1H), 3.42 (2H), 2.80 (1H), 2.55 (2H), 2.34 (3H), 0.65 (2H), 0.49 (2H) ppm |
| 3-6 | 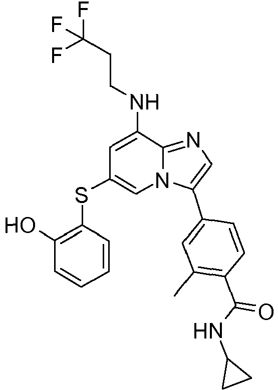 | N-cyclopropyl-4-{6-[(2-hydroxyphenyl)sulfanyl]-8-[(3, 3,3-trifluoropropyl)amino]imidazo[1,2-a]pyridin-3-yl}-2-methylbenzamide | RT = 1.14 MW_{found} = 527.6 MW_{calc} = 526.6 |
| | | | ¹H-NMR (300 MHz, d₆-DMSO): δ = 9.99 (1H), 7.67 (1H), 7.62 (1H), 7.41 - 7.35 (3H), 7.10 - 7.02 (2H), 6.85 (1H), 6.72 (1H), 6.43 (1H), 6.12 (1H), 3.43 (2H), 2.80 (1H), 2.57 (2H), 2.33 (3H), 0.65 (2H), 0.49 (2H) ppm |

### Example 4-1:

### Preparation of N-cyclopropyl-2-methyl-4-{6-(methylsulfanyl)-8-[(3,3,3-trifluoropropyl)amino]imidazo[1,2-a]pyridin-3-yl}benzamide

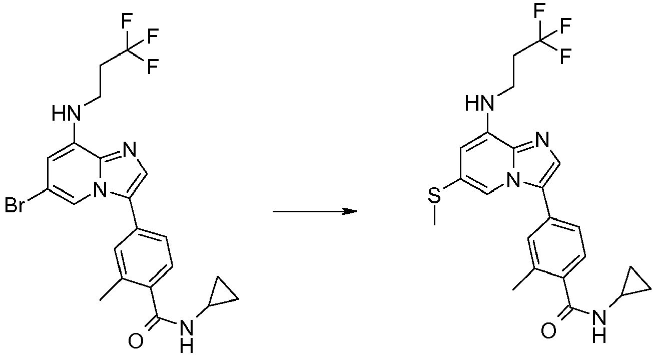

To a solution of 170.0 mg (0.35 mmol) 4-{6-bromo-8-[(3,3,3-trifluoropropyl)amino] imidazo[1,2-a]pyridin-3-yl}-N-cyclopropyl-2-methylbenzamide in 2.0 mL DMSO were added 124 mg (1.77 mmol) sodium thiomethylate and the mixture was stirred for 1 h at 70° C. After stirring at 100 °C for 3 h, water was added and the mixture was extracted with ethyl acetat. The organic phase was washed with water and sat sodium chloride solution, dried and evaporated to yield 158 mg of the title compound (100 %). UPLC-MS: RT = 1.08 min; m/z (ES+) 449.5 [MH⁺]; required MW = 448.5.

### Example 5-1:

### Preparation of N-cyclopropyl-4-{6-[(3-fluoro-5-methylphenyl)sulfanyl]-8-[(3,3,3-trifluoropropyl)amino]imidazo[1,2-a]pyridin-3-yl}-2-methylbenzamide

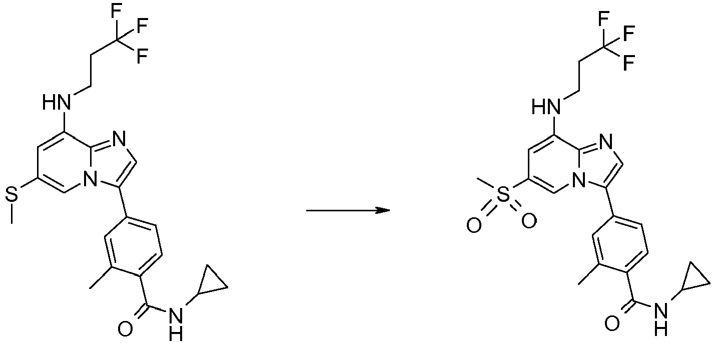

To a solution of 158 mg (0.35 mmol) N-cyclopropyl-2-methyl-4-{6-(methylsulfanyl)-8-[(3,3,3-trifluoropropyl)amino]imida zo[1,2-a]pyridin-3-yl}benzamide in 20 mL DMF were added 651 mg (1.06 mmol) potassium persulfate (oxone) and the mixture was stirred overnight at rt under nitrogen atmosphere. Water was added and the mixture was extracted with DCM. The organic phase was washed with water and sat sodium chloride solution, dried and evaporated to yield 150 mg of the title compound (88 %). UPLC-MS: RT = 1.08 min; m/z (ES+) 481.5 [MH⁺]; required MW = 480.5. ¹H-NMR (300 MHz, d6-DMSO): δ = 8.37 (1 H), 8.12 (1 H), 7.76 (1 H), 7.52 - 7.48 (2H), 7.45 (1 H), 6.82 (1 H), 6.50 (1 H), 3.56 (2H), 3.27 (3H), 2.81 (1H), 2.69 (2H), 2.37 (3H), 0.66 (2H), 0.51 (2H) ppm.

### Example 6-1:

### Preparation of N-cyclopropyl-4-{6-(3-fluorophenoxy)-8-[(3,3,3-trifluoropropyl)amino]imidazo[1,2-a]pyridin-3-yl}-2-methylbenzamide

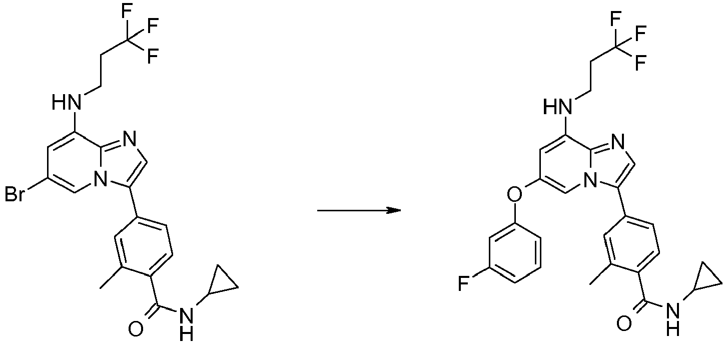

A mixture comprising 78 mg (162 µmol) 4-{6-bromo-8-[(3,3,3-trifluoropropyl)amino]imidazo[1,2-a]pyridin-3-yl}-N-cycloprop yl-2-methylbenzamide which was prepared according to intermediate example 6-1, 109 mg 3-fluorophenol 634 mg caesium carbonate, 3.3 mg N,N-dimethylglycine, 6.4 mg copper(I)chloride and 1.5 mL 1,4-dioxane was heated at 120°C using microwave irradiation for 4 hours. The mixture war poured into water and extracted with ethyl acetate. The organic layer was dried over sodium sulfate. After filtration and removal of the solvent the residue war purified by chromatography to give 1.2 mg (1%) of the title compound: m/z (ES+) 513 [MH⁺]; required MW = 512.2. ¹H-NMR (CDCl₃): δ= 0.62 (2H), 0.90 (2H), 2.49 (3H), 2.51 (2H), 2.92 (1H), 3.56 (2H), 5.50 (1 H), 5.88 (1 H), 5.99 (1 H), 6.75-6.83 (2H), 7.22-7.31 (2H), 7.35 (1 H), 7.37 (1 H), 7.43 (1 H), 7.55 (1 H), 7.60 (1 H) ppm.

### Example 6-2:

### Preparation of N-cyclopropyl-4-{6-(2-fluoro-4-methoxyphenoxy)-8-[(3,3,3-trifluoropropyl)amino]imidazo[1,2-a]pyridin-3-yl}-2-methylbenzamide

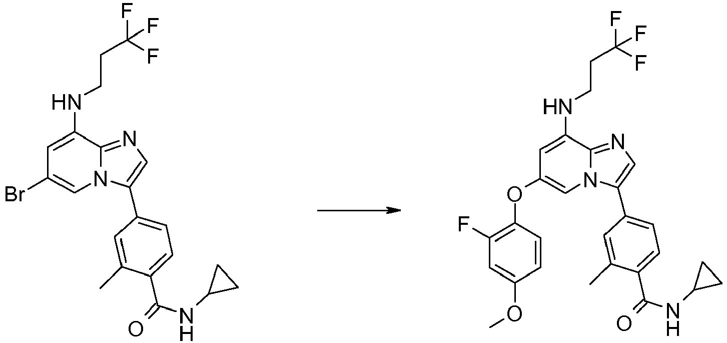

A mixture comprising 50.5 mg (105 µmol) 4-{6-bromo-8-[(3,3,3-trifluoropropyl)amino]imidazo[1,2-a]pyridin-3-yl}-N-cycloprop yl-2-methylbenzamide which was prepared according to intermediate example 6-1, 74.6 mg 2-fluoro-4-methoxyphenol 171 mg caesium carbonate, 4.77 mg (RS)-phenyl hydrogen pyrrolidin-2-ylphosphonate, 4.2 mg copper(I)chloride and 1 mL 1,4-dioxane was heated at 120°C using microwave irradiation for 2 hours. The mixture war poured into water and extracted with a mixture of ethyl acetate and methanol. The organic layer was dried over sodium sulfate. After filtration and removal of the solvent the residue war purified by chromatography to give 7.0 mg (14%) of the title compound: m/z (ES+) 543 [MH⁺]; required MW = 542.2.
¹H-NMR (DMSO-d6): δ= 0.49 (2H), 0.65 (2H), 2.29 (3H), 2.62 (2H), 2.80 (1H), 3.47 (2H), 3.72 (3H), 6.12 (1H), 6.54 (1H), 6.73 (1H), 7.00 (1H), 7.20 (1H), 7.29-7.36 (3H), 7.43 (1H), 7.59 (1H), 8.28 (1H) ppm.

### Example 7 (alternative route as described for Example 6-1)

### N-cyclopropyl-4-{6-(3-fluorophenoxy)-8-[(3,3,3-trifluoropropyl)amino]imidazo[1,2-a]pyridin-3-yl}-2-methylbenzamide

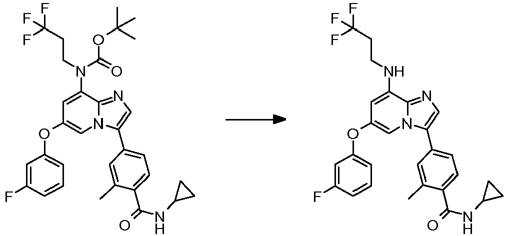

To a solution of 31 mg (51 µmol) tert-butyl {3-[4-(cyclopropylcarbamoyl)-3-methylphenyl]-6-(3-fluorophenoxy)imidazo[1,2-a]pyridin-8-yl}(3,3,3-trifluoropropyl)carbamate which was prepared according to intermediate example 7a in 0.5 mL dichloromethane was added 58.5 µL trifluoroacetic acid and the mixture was heated at 50°C under microwave irradiation for 1 hour. The solvents were removed and the residue purified by chromatography to give 19 mg (73%) of the title compound.
¹H-NMR (DMSO-d6): δ= 0.48 (2H), 0.64 (2H), 2.33 (3H), 2.61 (2H), 2.79 (1H), 3.45 (2H), 6.10 (1H), 6.58 (1H), 6.85-6.95 (3H), 7.29-7.48 (4H), 7.60-7.70 (2H), 8.28 (1H) ppm.

### Example 7a

### tert-Butyl {3-[4-(cyclopropylcarbamoyl)-3-methylphenyl]-6-(3-fluorophenoxy)imidazo[1,2-a]pyridin-8-yl}(3,3,3-trifluoropropyl)carbamate

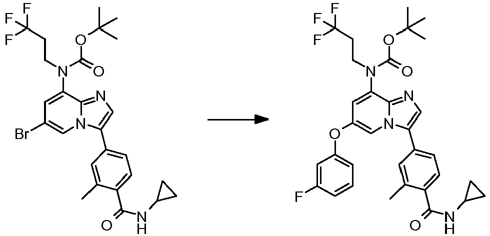

200 mg (344 µmol) tert-butyl {6-bromo-3-[4-(cyclopropylcarbamoyl)-3-methylphenyl]imidazo[1,2-a]pyridin-8-yl}(3,3,3-trifluoropropyl)carbamate which was prepared according to intermediate example 7b were transformed in analogy to example 6-1 to give after working up and purification 32 mg (15%) of the title compound.

### Example 7b

### tert-Butyl {6-bromo-3-[4-(cyclopropylcarbamoyl)-3-methylphenyl]imidazo[1,2-a]pyridin-8-yl}(3,3,3-trifluoropropyl)carbamate

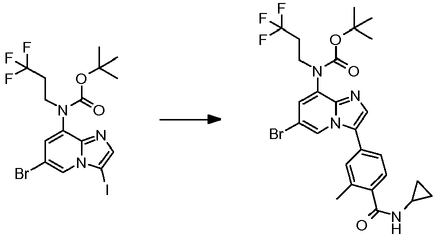

A mixture comprising 2.62 g (4.91 mmol) tert-butyl (6-bromo-3-iodoimidazo[1,2-a]pyridin-8-yl)(3,3,3-trifluoropropyl)carbamate which was prepared according to intermediate example 7c, 1.61 g [4-(cyclopropylcarbamoyl)-3-methylphenyl]boronic acid, which was prepared according to intermediate example 12, 100 mg (1,1,-bis(diphenylphosphino)ferrocene)-dichloropalladium (II), 6.1 mL aqueous 2M cesium carbonate solution and 30 mL tetrahydrofuran was stirred at 55°C for 2 hours. 50 mg (1,1,-bis(diphenylphosphino)ferrocene)-dichloropalladium (II) were added and stirring continued for additional 2 hours. Water was added and the mixture was extracted with ethyl acetate. The organic layer was washed with brine and dried over sodium sulfate. After filtration and removal of the solvent the residue was purified by chromatography to give 2.15 g (75%) of the title compound.

### Example 7c

### tert-Butyl (6-bromo-3-iodoimidazo[1,2-a]pyridin-8-yl)(3,3,3-trifluoropropyl)carbamate

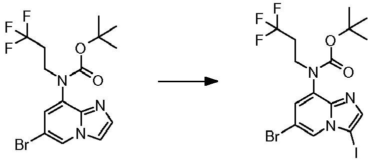

To a solution of 5.00 g (12.25 mmol) tert-butyl (6-bromoimidazo[1,2-a]pyridin-8-yl)(3,3,3-trifluoropropyl)carbamate which was prepared according to intermediate example 7d in 75 mL *N,N*-dimethylformamide were added 71.25 g N-iodosuccinimide and the mixture was stirred at 23°C for 1.5 hours. Ethyl acetate was added and the mixture was washed with saturated sodium thiosulfate solution, water and dried over sodium sulfate. After filtration and removal of solvent the residue was purified by chromatography to give 5.92 g (90%) of the title compound.

### Example 7d

### tert-Butyl (6-bromoimidazo[1,2-a]pyridin-8-yl)(3,3,3-trifluoropropyl)carbamate

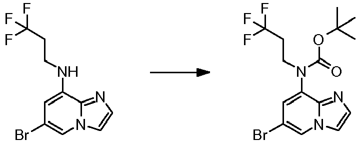

To a solution of 16.24 g (52,71 mmol) 6-bromo-N-(3,3,3-trifluoropropyl)imidazo[1,2-a]pyridin-8-amine which was prepared according to intermediate example 7e in 62 mL tetrahydrofuran were added 25.31 g di-tert-butyl dicarbonate, 644 mg N,N-dimethylpyridin-4-amine and the mixture was stirred at 55°C for 4 hours. Ethyl acetate was added and the mixture was washed with saturated sodium hydrogencarbonate solution and dried over sodium sulfate. After filtration and removal of solvent the residue was purified by chromatography to give 20.9 g (97%) of the title compound.

### Example 7e

### 6-Bromo-N-(3,3,3-trifluoropropyl)imidazo[1,2-a]pyridin-8-amine

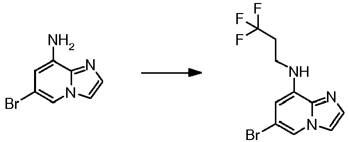

To a mixture comprising 21.7 g (102.3 mmol) 6-bromoimidazo[1,2-a]pyridin-8-amine which was prepared according to intermediate example 3-1, 13.05 mL 3,3,3-trifluoropropanal, 18.16 mL acetic acid in 1.3 L dichloromethane were added a total of 65.07 g sodium tris(acetato-kappaO)(hydrido)borate(1-) in portions. After the mixture was stirred at 23°C for 3 hours it was cooled to 3°C and 300 mL 4M aqueous ammonia was carefully added. The mixture was extracted with dichloromethane, the organic layer washed with brine and dried over sodium sulfate. After filtration and removal of solvent the residue was purified by chromatography to give 16.26 g (52%) of the title compound.

### Example 8

### 4-{6-(3-Fluorophenoxy)-8-[(3,3,3-trifluoropropyl)amino]imidazo[1,2-a]pyridin-3-yl}-N,2-dimethylbenzamide

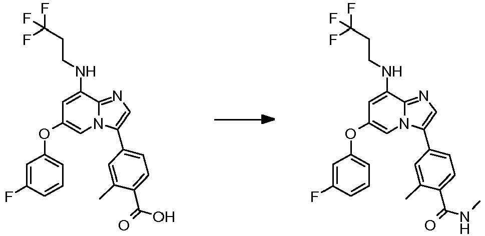

A mixture comprising 18 mg (38 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a, 28.5 µL methanamine solution in tetrahydrofuran (2M), 21.7 mg N-[(dimethylamino)(3H-[1,2,3]triazolo[4,5-b]pyridin-3-yloxy)methylene]-N-methylmethanaminium hexafluorophosphate, 7.0 mg *N,N-*dimethylpyridin-4-amine and 0.9 mL N,N-dimethylformamide was stirred at 23°C overnight. The solvent was removed and the residue purified by chromatography to give 16.1 mg (83%) of title compound.
¹H-NMR (CDCl₃): δ= 2.41-2.68 (2H), 2.49 (3H), 3.02 (3H), 3.55 (2H), 5.49 (1 H), 5.82 (1H), 5.99 (1H), 6.71 (1H), 6.75-6.83 (2H), 7.27 (1H), 7.35 (1H), 7.37 (1H), 7.46 (1 H), 7.56 (1 H), 7.61 (1 H) ppm.

### Example 8a

### 4-{6-(3-Fluorophenoxy)-8-[(3,3,3-trifluoropropyl)amino]imidazo[1,2-a]pyridin-3-yl}-2-methylbenzoic acid

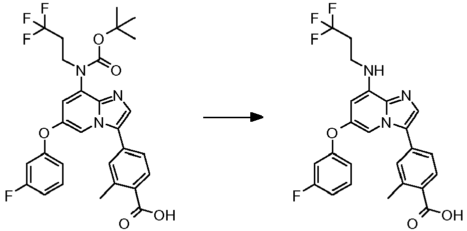

100 mg (205 µmol) 4-{8-[(tert-butoxycarbonyl)(3,3,3-trifluoropropyl)amino]-6-(3-fluorophenoxy)imidazo[1,2-a]pyridin-3-yl}-2-methylbenzoic acid which was prepared according to intermediate example 8b were transformed in analogy to example 7 to give after working up 97 mg (100%) of the title compound.

### Example 8b

### 4-{8-[(tert-Butoxycarbonyl)(3,3,3-trifluoropropyl)amino]-6-(3-fluorophenoxy)imidazo[1,2-a]pyridin-3-yl}-2-methylbenzoic acid

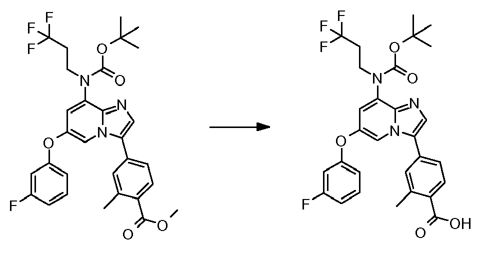

To a solution of 50 mg (85 µmol) methyl 4-{8-[(tert-butoxycarbonyl)(3,3,3-trifluoropropyl)amino]-6-(3-fluorophenoxy)imidazo[1,2-a]pyridin-3-yl}-2-methylbenzoate which was prepared according to intermediate example 8c in 1.8 mL tetrahydrofurane and 0.5 mL methanol were added 1.28 mL of a 1M aqueous lithium hydroxide solution and the mixture was stirred at 23°C for 1 hour. Water was added, the mixture was acidified by the addition of a 1M hydrochloric acid and extracted with dichloromethane and methanol. The organic layer was washed with brine and dried over sodium sulfate. After filtration and removal of solvent the residue was purified by chromatography to give 107 mg (100%) of the title compound.

### Example 8c

### Methyl 4-{8-[(tert-butoxycarbonyl)(3,3,3-trifluoropropyl)amino]-6-(3-fluorophenoxy)imidazo[1,2-a]pyridin-3-yl}-2-methylbenzoate

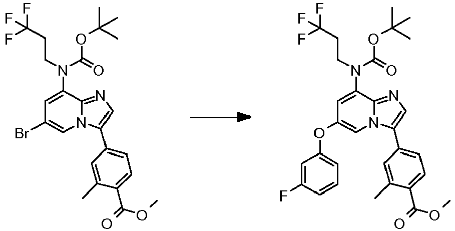

550 mg (988 µmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to intermediate example 7a to give after working up and purification 287 mg (54%) of the title compound.

### Methyl 4-{6-bromo-8-[(tert-butoxycarbonyl)(3,3,3-trifluoropropyl)amino]imidazo[1,2-a]pyridin-3-yl}-2-methylbenzoate

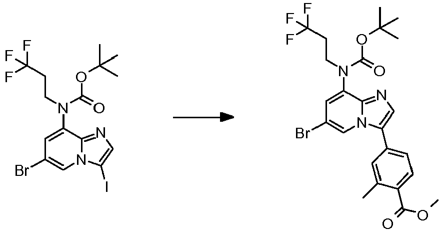

2.50 g (4.68 mmol) tert-butyl (6-bromo-3-iodoimidazo[1,2-a]pyridin-8-yl)(3,3,3-trifluoropropyl)carbamate which was prepared according to intermediate example 7c were transformed in analogy to intermediate example 7b using [4-(methoxycarbonyl)-3-methylphenyl]boronic acid to give after working up and purification 1.83 g (67%) of the title compound.

### Example 9

### 4,4'-{8-[(Tetrahydro-2H-pyran-4-ylmethyl)amino]imidazo[1,2-a]pyridine-3,6-diyl}bis(N-cyclopropyl-2-methylbenzamide)

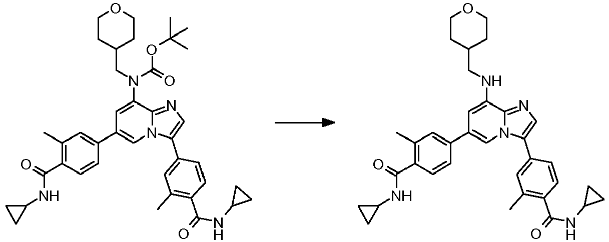

91 mg (134 µmol) tert-butyl {3,6-bis[4-(cyclopropylcarbamoyl)-3-methylphenyl]imidazo[1,2-a]pyridin-8-yl}(tetrahydro-2H-pyran-4-ylmethyl)carbamate which was prepared according to intermediate example 9a were transformed in analogy to example 7 to give after working up and purification 42.3 mg (52%) of the title compound.
¹H-NMR (DMSO-d6): δ= 0.45-0.54 (4H), 0.60-0.70 (4H), 1.17-1.32 (2H), 1.59-1.69 (2H), 1.95 (1H), 2.35 (3H), 2.36 (3H), 2.75-2.87 (2H), 3.18-3.27 (4H), 3.78-3.87 (2H), 6.22 (1 H), 6.39 (1 H), 7.31 (1 H), 7.39-7.55 (5H), 7.61 (1 H), 7.84 (1 H), 8.24 (1 H), 8.32 (1 H) ppm.

### Example 9a

### tert-Butyl {3,6-bis[4-(cyclopropylcarbamoyl)-3-methylphenyl]imidazo[1,2-a]pyridin-8-yl}(tetrahydro-2H-pyran-4-ylmethyl)carbamate

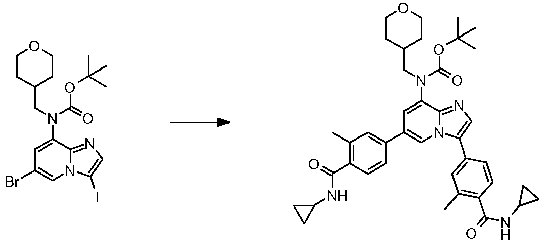

1.00 g (1.865 mmol) tert-butyl (6-bromo-3-iodoimidazo[1,2-a]pyridin-8-yl)(tetrahydro-2H-pyran-4-ylmethyl)carbamate which was prepared according to intermediate example 9b were transformed in analogy to intermediate example 7b at 120°C to give after working up and purification 670 mg (53%) of the title compound.

### Example 9b

### tert-Butyl (6-bromo-3-iodoimidazo[1,2-a]pyridin-8-yl)(tetrahydro-2H-pyran-4-ylmethyl)carbamate

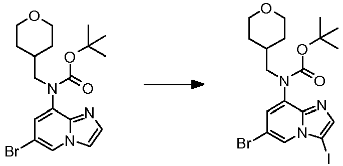

2.22 g (5.41 mmol) tert-butyl (6-bromoimidazo[1,2-a]pyridin-8-yl)(tetrahydro-2H-pyran-4-ylmethyl)carbamate which was prepared according to intermediate example 9c were transformed in analogy to intermediate example 7c to give after working up and purification 2.87 g (99%) of the title compound.

### Example 9c

### tert-Butyl (6-bromoimidazo[1,2-a]pyridin-8-yl)(tetrahydro-2H-pyran-4-ylmethyl)carbamate

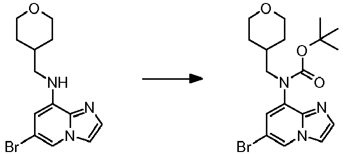

1.85 g (5.964 mmol) 6-bromo-N-(tetrahydro-2H-pyran-4-ylmethyl)imidazo[1,2-a]pyridin-8-amine which was prepared according to intermediate example 9d were transformed in analogy to intermediate example 7d to give after working up and purification 1.41 g (58%) of the title compound.

### Example 9d

### 6-Bromo-N-(tetrahydro-2H-pyran-4-ylmethyl)imidazo[1,2-a]pyridin-8-amine

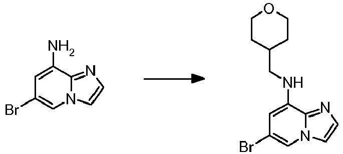

3.00 g (14.15 mmol) 6-bromoimidazo[1,2-a]pyridin-8-amine which was prepared according to intermediate example 3-1 were transformed in analogy to intermediate example 7e using tetrahydro-2H-pyran-4-carbaldehyde to give after working up and purification 2.57 g (53%) of the title compound.

### Example 10

### N-ethyl-4-{6-(3-fluorophenoxy)-8-[(3,3,3-trifluoropropyl)amino]imidazo[1,2-a]pyridin-3-yl}-2-methylbenzamide

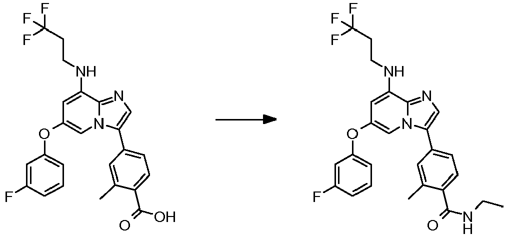

18 mg (38 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using ethanamine to give after working up and purification 15.3 mg (76%) of the title compound.
¹H-NMR (CDCl₃): δ= 1.26 (3H), 2.46-2.58 (2H), 2.50 (3H), 3.50 (2H), 3.56 (2H), 5.48 (1H), 5.75 (1H), 5.99 (1H), 6.72 (1H), 6.76-6.82 (2H), 7.27 (1H), 7.36 (1H), 7.38 (1 H), 7.46 (1 H), 7.56 (1 H), 7.61 (1 H) ppm.

### Example 11

### 4-{6-(3-Fluorophenoxy)-8-[(3,3,3-trifluoropropyl)amino]imidazo[1,2-a]pyridin-3-yl}-2-methyl-N-(1-methylcyclopropyl)benzamide

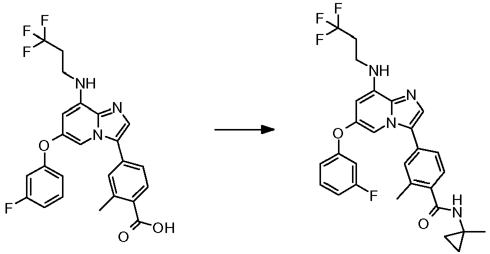

18 mg (38 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using 1-methylcyclopropanaminium chloride to give after working up and purification 13.7 mg (65%) of the title compound.
¹H-NMR (CDCl₃): δ= 0.76 (2H), 0.87 (2H), 1.52 (3H), 2.48 (3H), 2.51 (2H), 3.56 (2H), 5.47 (1H), 5.99 (1H), 6.08 (1H), 6.69-6.82 (3H), 7.27 (1H), 7.34 (1H), 7.36 (1H), 7.40 (1 H), 7.55 (1 H), 7.59 (1 H) ppm.

### Example 12

### N-[rel-(1S,2S)-2-fluorocyclopropyl]-4-{6-(3-fluorophenoxy)-8-[(3,3,3-trifluoropropyl)amino]imidazo[1,2-a]pyridin-3-yl}-2-methylbenzamide (rac-A), N-{[(1R,2R) or (1S,2S)]-2-fluorocyclopropyl}-4-{6-(3-fluorophenoxy)-8-[(3,3,3-trifluoropropyl)amino]imidazo[1,2-a]pyridin-3-yl}-2-methylbenzamide (ent-A or A) and N-{[(1S,2S) or (1R,2R)]-2-fluorocyclopropyl}-4-{6-(3-fluorophenoxy)-8-[(3,3,3-trifluoropropyl)amino]imidazo[1,2-a]pyridin-3-yl}-2-methylbenzamide (A or ent-A)

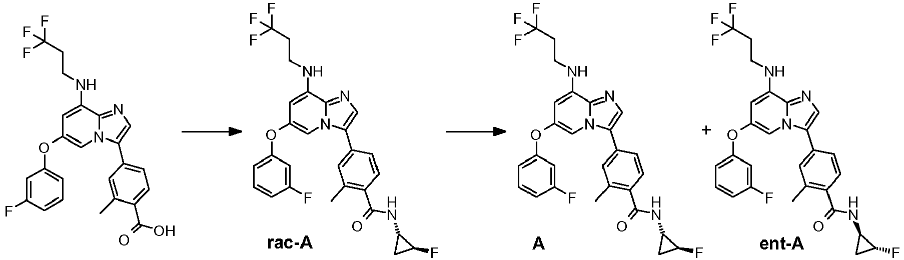

18 mg (38 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using rel-(1S,2S)-2-fluorocyclopropanaminium chloride to give after working up and purification 14.1 mg (66%) of the racemic title compound A.
¹H-NMR (CDCl₃): δ= 1.02 (1 H), 1.27 (1 H), 2.46-2.58 (2H), 2.51 (3H), 3.06 (1 H), 3.56 (2H), 4.76 (1H), 5.48 (1H), 5.99 (1H), 6.03 (1H), 6.72 (1H), 6.75-6.83 (2H), 7.27 (1 H), 7.37 (1 H), 7.39 (1 H), 7.49 (1 H), 7.56 (1 H), 7.61 (1 H) ppm. 8.7 mg of rac-A (16 µmol) were separated by HPLC using a chiral column to give 2.2 mg (25%) ent-A or A and 2.1 mg (24%) A or ent-A.

### Example 13

### N-(1-cyanocyclopropyl)-4-{6-(3-fluorophenoxy)-8-[(3,3,3-trifluoropropyl)amino]imidazo[1,2-a]pyridin-3-yl}-2-methylbenzamide

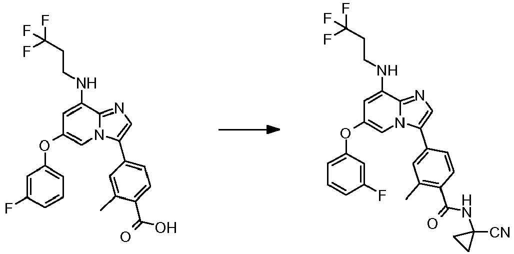

18 mg (38 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using 1-cyanocyclopropanaminium chloride to give after working up and purification 7.5 mg (35%) of the title compound.
¹H-NMR (CDCl₃): δ= 1.38 (2H), 1.66 (2H), 2.43-2.63 (2H), 2.51 (3H), 3.55 (2H), 5.49 (1H), 6.00 (1H), 6.44 (1H), 6.71 (1H), 6.75-6.84 (2H), 7.27 (1H), 7.33-7.47 (3H), 7.55 (1 H), 7.60 (1 H).

### Example 14

### 4-{6-ethynyl-8-[(3,3,3-trifluoropropyl)amino]imidazo[1,2-a]pyridin-3-yl}-N,2-dimethylbenzamide

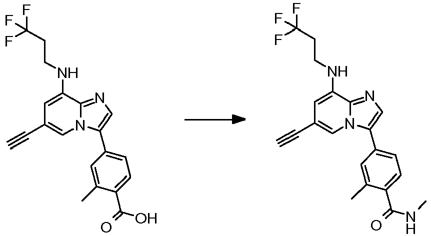

14 mg (36 µmol) 4-{6-ethynyl-8-[(3,3,3-trifluoropropyl)amino]imidazo[1,2-a]pyridin-3-yl}-2-methylbenzoic acid which was prepared according to intermediate example 14a were transformed in analogy to example 8 to give after working up and purification 12.4 mg (86%) of the title compound.
¹H-NMR (CDCl₃): δ= 2.45-2.61 (2H), 2.54 (3H), 3.04 (4H), 3.61 (2H), 5.36 (1 H), 5.82 (1 H), 6.17 (1 H), 7.37 (1 H), 7.39 (1 H), 7.50 (1 H), 7.55 (1 H), 7.91 (1 H) ppm.

### Example 14a

### 4-{6-Ethynyl-8-[(3,3,3-trifluoropropyl)amino]imidazo[1,2-a]pyridin-3-yl}-2-methylbenzoic acid

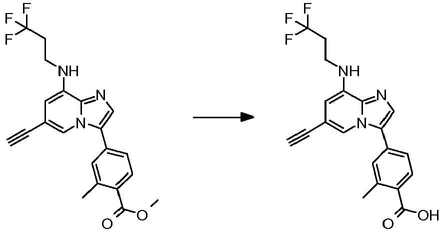

93 mg (232 µmol) methyl 4-{6-ethynyl-8-[(3,3,3-trifluoropropyl)amino]imidazo[1,2-a]pyridin-3-yl}-2-methylbenzoate which was prepared according to intermediate example 14b were transformed in analogy to intermediate example 8b to give after working up and purification 70.8 mg (79%) of the title compound.

### Example 14b

### Methyl 4-{6-ethynyl-8-[(3,3,3-trifluoropropyl)amino]imidazo[1,2-a]pyridin-3-yl}-2-methylbenzoate

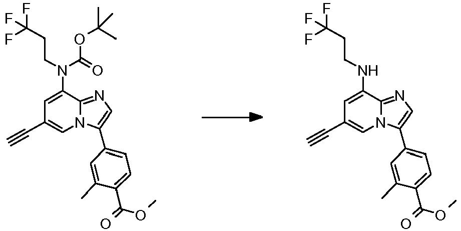

122.9 mg (245 µmol) methyl 4-{8-[(tert-butoxycarbonyl)(3,3,3-trifluoropropyl)amino]-6-ethynylimidazo[1,2-a]pyridin-3-yl}-2-methylbenzoate which was prepared according to intermediate example 14c were transformed in analogy to example 7 to give after working up and purification 93 mg (85%) of the title compound.

### Example 14c

### Methyl 4-{8-[(tert-butoxycarbonyl)(3,3,3-trifluoropropyl)amino]-6-ethynylimidazo[1,2-a]pyridin-3-yl}-2-methylbenzoate

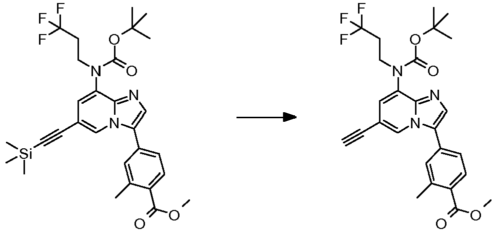

A mixture comprising 169 mg (295 µmol) methyl 4-{8-[(tert-butoxycarbonyl)(3,3,3-trifluoropropyl)amino]-6-[(trimethylsilyl)ethynyl]imidazo[1,2-a]pyridin-3-yl}-2-methylbenzoate which was prepared according to intermediate example 14d, 295 µL tetra-n-butylammonium fluoride in tetrahydrofuran (1M) and 1.4 mL tetrahydrofuran was stirred at 23°C for 20 minutes. Saturated ammoniumchloride solution was added the mixture extracted with ethyl acetate. The organic layer was dried over sodium sulfate. After filtration and removal of solvent the residue was purified by chromatography to give 122.9 mg (75%) of the title compound.

### Example 14d

### Methyl 4-{8-[(tert-butoxycarbonyl)(3,3,3-trifluoropropyl)amino]-6-[(trimethylsilyl)ethynyl]imidazo[1,2-a]pyridin-3-yl}-2-methylbenzoate

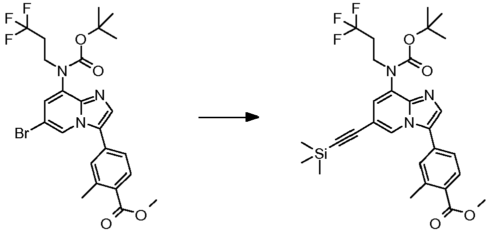

A mixture comprising 50 mg (90 µmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d, 35.3 mg ethynyl(trimethyl)silane, 6.3 mg bis(triphenylphosphine)palladium(II) chloride, 1.7 mg copper(I) iodide, 0.82 mL N-isopropylpropan-2-amine and 0.82 mL dioxane was heated at 80°C for 10 minutes under microwave irradiation. The solvents were removed and the residue purified by chromatography to give 59.9 mg (99%) of the title compound.

### Example 15

### N-ethyl-4-{6-ethynyl-8-[(3,3,3-trifluoropropyl)amino]imidazo[1,2-a]pyridin-3-yl}1-2-methylbenzamide

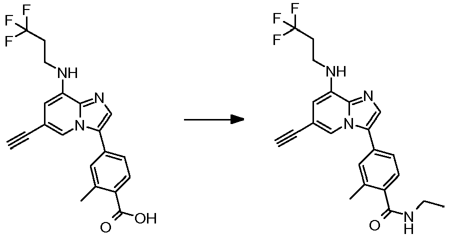

14 mg (36 µmol) 4-{6-ethynyl-8-[(3,3,3-trifluoropropyl)amino]imidazo[1,2-a]pyridin-3-yl}-2-methylbenzoic acid which was prepared according to intermediate example 14a were transformed in analogy to example 8 using ethanamine to give after working up and purification 12.1 mg (81%) of the title compound.
¹H-NMR (CDCl₃): δ= 1.28 (3H), 2.48-2.61 (2H), 2.54 (3H), 3.04 (1 H), 3.52 (2H), 3.61 (2H), 5.36 (1H), 5.78 (1H), 6.17 (1H), 7.38 (1H), 7.39 (1H), 7.50 (1H), 7.54 (1H), 7.91 (1 H) ppm.

### Example 16

### 4-{6-ethynyl-8-[(3,3,3-trifluoropropyl)amino]imidazo[1,2-a]pyridin-3-yl}-N-[rel-(1R,2R)-2-fluorocyclopropyl]-2-methylbenzamide

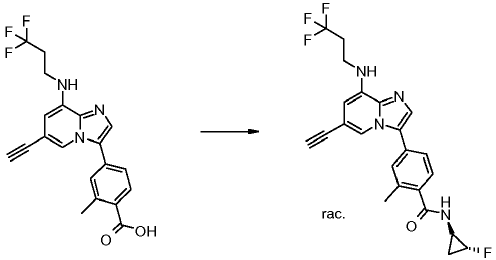

14 mg (36 µmol) 4-{6-ethynyl-8-[(3,3,3-trifluoropropyl)amino]imidazo[1,2-a]pyridin-3-yl}-2-methylbenzoic acid which was prepared according to intermediate example 14a were transformed in analogy to example 8 using rel-(1S,2S)-2-fluorocyclopropanaminium chloride to give after working up and purification 12.3 mg (77%) of the title compound.
¹H-NMR (CDCl₃): δ= 1.04 (1 H), 1.29 (1 H), 2.49-2.60 (2H), 2.55 (3H), 3.05 (1 H), 3.08 (1H), 3.61 (2H), 4.78 (1H), 5.37 (1H), 6.04 (1H), 6.17 (1H), 7.38 (1H), 7.40 (1H), 7.51-7.58 (2H), 7.91 (1H) ppm.

### Example 17

### 4-{6-ethynyl-8-[(3,3,3-trifluoropropyl)amino]imidazo[1,2-a]pyridin-3-yl}-2-methyl-N-(1-methylcyclopropyl)benzamide

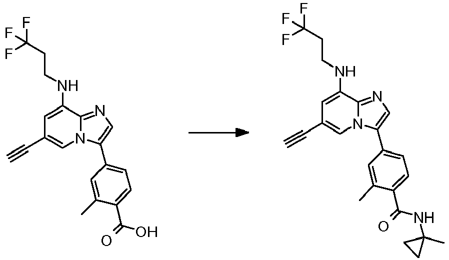

14 mg (36 µmol) 4-{6-ethynyl-8-[(3,3,3-trifluoropropyl)amino]imidazo[1,2-a]pyridin-3-yl}-2-methylbenzoic acid which was prepared according to intermediate example 14a were transformed in analogy to example 8 using 1-methylcyclopropanaminium chloride to give after working up and purification 10.1 mg (63%) of the title compound.
¹H-NMR (CDCl₃): δ= 0.77 (2H), 0.89 (2H), 1.54 (3H), 2.45-2.60 (2H), 2.51 (3H), 3.04 (1H), 3.61 (2H), 5.36 (1 H), 6.10 (1H), 6.16 (1 H), 7.35 (1 H), 7.37 (1 H), 7.44 (1 H), 7.53 (1 H), 7.89 (1 H) ppm.

### Example 18

### N-(1-cyanocyclopropyl)-4-{6-ethynyl-8-[(3,3,3-trifluoropropyl)amino]imidazo[1,2-a]pyridin-3-yl}-2-methylbenzamide

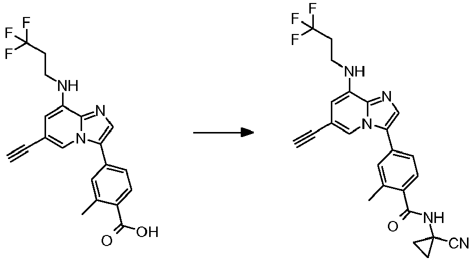

14 mg (36 µmol) 4-{6-ethynyl-8-[(3,3,3-trifluoropropyl)amino]imidazo[1,2-a]pyridin-3-yl}-2-methylbenzoic acid which was prepared according to intermediate example 14a were transformed in analogy to example 8 using 1-cyanocyclopropanaminium chloride to give after working up and purification 11.8 mg (72%) of the title compound.
¹H-NMR (CDCl₃): δ= 1.40 (2H), 1.68 (2H), 2.48-2.61 (2H), 2.55 (3H), 3.05 (1 H), 3.61 (2H), 5.37 (1 H), 6.17 (1 H), 6.46 (1 H), 7.36-7.50 (3H), 7.54 (1 H), 7.89 (1 H) ppm.

### Example 19

### N-cyclopropyl-4-{6-(5-fluoro-2-methylphenoxy)-8-[(3,3,3-trifluoropropyl)amino]imidazo[1,2-a]pyridin-3-yl}-2-methylbenzamide

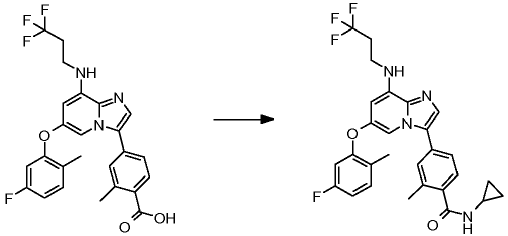

20 mg (41 µmol) 4-{6-(5-fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using cyclopropanamine to give after working up and purification 20.1 mg (93%) of the title compound.
¹H-NMR (CDCl₃): δ= 0.62 (2H), 0.89 (2H), 2.27 (3H), 2.42-2.60 (2H), 2.48 (3H), 2.92 (1H), 3.57 (2H), 5.50 (1H), 5.92 (1H), 6.00 (1H), 6.57 (1H), 6.73 (1H), 7.17 (1H), 7.30-7.37 (2H), 7.41 (1H), 7.45 (1H), 7.53 (1H) ppm.

### Example 19a

### 4-{6-(5-Fluoro-2-methylphenoxy)-8-[(3,3,3-trifluoropropyl)amino]imidazo[1,2-a]pyridin-3-yl}-2-methylbenzoic acid

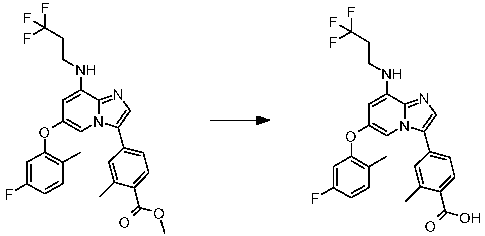

805 mg (1.61 mmol) methyl 4-{6-(5-fluoro-2-methylphenoxy)-8-[(3,3,3-trifluoropropyl)amino]imidazo[1,2-a]pyridin-3-yl}-2-methylbenzoate which was prepared according to intermediate example 19b were transformed in analogy to intermediate example 8b to give after working up and purification 736 mg (89%) of the title compound.

### Example 19b

### Methyl 4-{6-(5-fluoro-2-methylphenoxy)-8-[(3,3,3-trifluoropropyl)amino]imidazo[1,2-a]pyridin-3-yl}-2-methylbenzoate

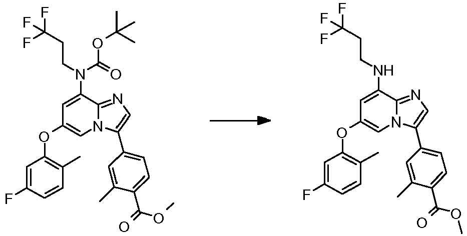

1.06 g (1.77 mmol) methyl 4-{8-[(tert-butoxycarbonyl)(3,3,3-trifluoropropyl)amino]-6-(5-fluoro-2-methylphenoxy)imidazo[1,2-a]pyridin-3-yl}-2-methylbenzoate which was prepared according to intermediate example 19c were transformed in analogy to example 7 to give after working up and purification 861 mg (97%) of the title compound.

### Example 19c

### Methyl 4-{8-[(tert-butoxycarbonyl)(3,3,3-trifluoropropyl)amino]-6-(5-fluoro-2-methylphenoxy)imidazo[1,2-a]pyridin-3-yl}-2-methylbenzoate

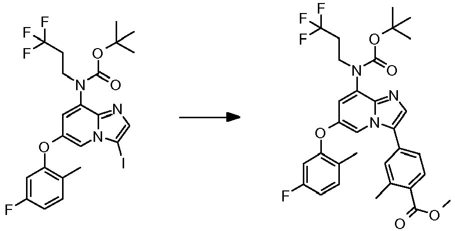

1.36 g (2.34 mmol) tert-butyl [6-(5-fluoro-2-methylphenoxy)-3-iodoimidazo[1,2-a]pyridin-8-yl](3,3,3-trifluoropropyl)carbamate which was prepared according to intermediate example 19d were transformed in analogy to intermediate example 7b using [4-(methoxycarbonyl)-3-methylphenyl]boronic acid to give after working up and purification 1.12 g (79%) of the title compound.

### Example 19d

### tert-Butyl [6-(5-fluoro-2-methylphenoxy)-3-iodoimidazo[1,2-a]pyridin-8-yl](3,3,3-trifluoropropyl)carbamate

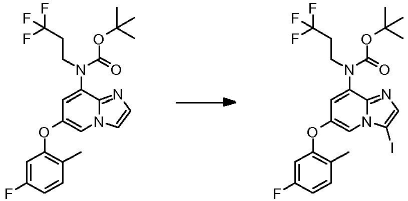

1.10 g (2.43 mmol) tert-butyl [6-(5-fluoro-2-methylphenoxy)imidazo[1,2-a]pyridin-8-yl](3,3,3-trifluoropropyl)carbamate which was prepared according to intermediate example 19e were transformed in analogy to intermediate example 7c to give after working up and purification 1.37 g (97%) of the title compound.

### Example 19e

### tert-Butyl [6-(5-fluoro-2-methylphenoxy)-3-iodoimidazo[1,2-a]pyridin-8-yl](3,3,3-trifluoropropyl)carbamate

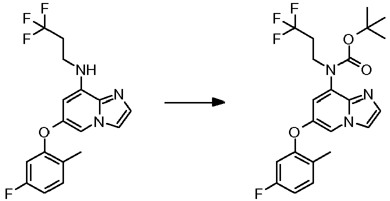

1.06 g (3.00 mmol) 6-(5-fluoro-2-methylphenoxy)-N-(3,3,3-trifluoropropyl)imidazo[1,2-a]pyridin-8-amine which was prepared according to intermediate example 19f were transformed in analogy to intermediate example 7d to give after working up and purification 1.17 g (82%) of the title compound.

### Example 19f

### 6-(5-Fluoro-2-methylphenoxy)-N-(3,3,3-trifluoropropyl)imidazo[1,2-a]pyridin-8-amine

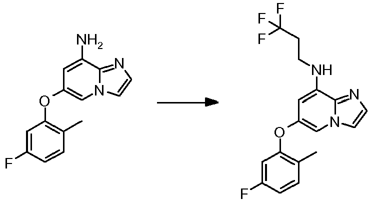

1.06 g (4.11 mmol) 6-(5-fluoro-2-methylphenoxy)imidazo[1,2-a]pyridin-8-amine which was prepared according to intermediate example 19g were transformed in analogy to intermediate example 7e to give after working up and purification 1.06 g (73%) of the title compound.

### Example 19g

### 6-(5-Fluoro-2-methylphenoxy)imidazo[1,2-a]pyridin-8-amine

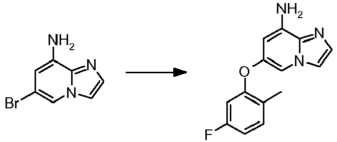

3.00 g (14.15 mmol) 6-bromoimidazo[1,2-a]pyridin-8-amine which was prepared according to intermediate example 3-1 were transformed in analogy to example 6-1 using 5-fluoro-2-methylphenol to give after working up and purification 1.10 g (30%) of the title compound.

### Example 20

### 4-{6-(3-hydroxyprop-1-yn-1-yl)-8-[(3,3,3-trifluoropropyl)amino]imidazo[1,2-a]pyridin-3-yl}-N,2-dimethylbenzamide

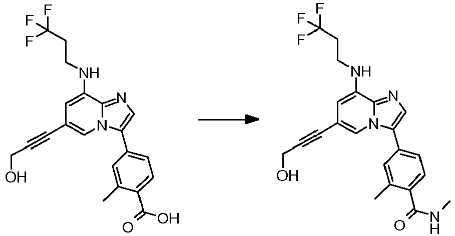

14 mg (34 µmol) 4-{6-(3-hydroxyprop-1-yn-1-yl)-8-[(3,3,3-trifluoropropyl)amino]imidazo[1,2-a]pyridin-3-yl}-2-methylbenzoic acid which was prepared according to intermediate example 20a were transformed in analogy to example 8 using to give after working up and purification 7.3 mg (51%) of the title compound.
¹H-NMR (CD₃OD): δ= 2.46 (3H), 2.59 (2H), 2.92 (3H), 3.58 (2H), 4.36 (2H), 6.26 (1H), 7.41-7.47 (2H), 7.49 (1H), 7.56 (1H), 7.89 (1H) ppm.

### Example 20a

### 4-{6-(3-Hydroxyprop-1-yn-1-yl)-8-[(3,3,3-trifluoropropyl)amino]imidazo[1,2-a]pyridin-3-yl}-2-methylbenzoic acid

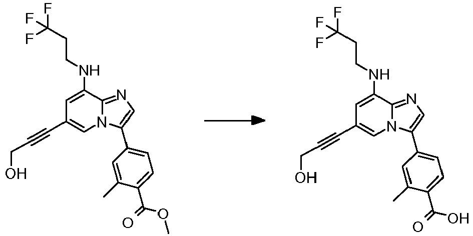

83 mg (192 µmol) methyl 4-{6-(3-hydroxyprop-1-yn-1-yl)-8-[(3,3,3-trifluoropropyl)amino]imidazo[1,2-a]pyridin-3-yl}-2-methylbenzoate which was prepared according to intermediate example 20b were transformed in analogy to intermediate example 8b to give after working up and purification 58.1 mg (72%) of the title compound.

### Example 20b

### Methyl 4-{6-(3-hydroxyprop-1-yn-1-yl)-8-[(3,3,3-trifluoropropyl)amino]imidazo[1,2-a]pyridin-3-yl}-2-methylbenzoate

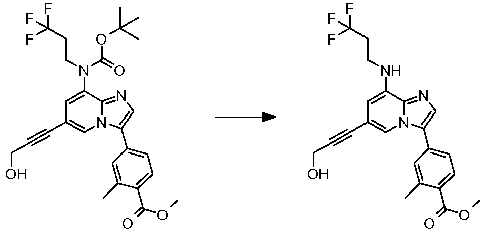

146 mg (275 µmol) methyl 4-{8-[(tert-butoxycarbonyl)(3,3,3-trifluoropropyl)amino]-6-(3-hydroxyprop-1-yn-1-yl)imidazo[1,2-a]pyridin-3-yl}-2-methylbenzoate which was prepared according to intermediate example 20c were transformed in analogy to example 7 to give after working up and purification 82.9 mg (63%) of the title compound.

### Example 20c

### Methyl 4-{8-[(tert-butoxycarbonyl)(3,3,3-trifluoropropyl)amino]-6-(3-hydroxyprop-1-yn-1-yl)imidazo[1,2-a]pyridin-3-yl}-2-methylbenzoate

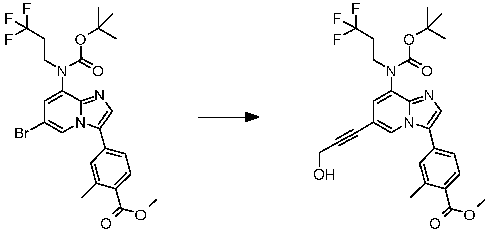

200 mg (359 µmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to intermediate example 14d using prop-2-yn-1-ol to give after working up and purification 146.1 mg (76%) of the title compound.

### Example 21

### N-ethyl-4-{6-(3-hydroxyprop-1-yn-1-yl)-8-[(3,3,3-trifluoropropyl)amino]imidazo[1,2-a]pyridin-3-yl}-2-methylbenzamide

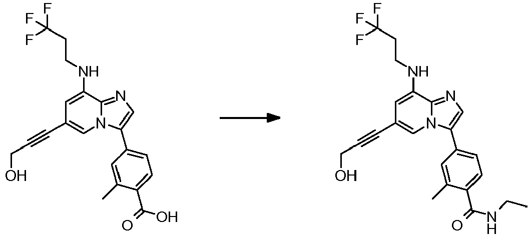

14 mg (34 µmol) 4-{6-(3-hydroxyprop-1-yn-1-yl)-8-[(3,3,3-trifluoropropyl)amino]imidazo[1,2-a]pyridin-3-yl}-2-methylbenzoic acid which was prepared according to intermediate example 20a were transformed in analogy to example 8 using ethanamine to give after working up and purification 11.2 mg (75%) of the title compound.
¹H-NMR (CDCl₃): δ= 1.28 (3H), 2.36-2.60 (2H), 2.52 (3H), 3.52 (2H), 3.59 (2H), 4.49 (2H), 5.39 (1 H), 5.86 (1 H), 6.12 (1 H), 7.34 (1 H), 7.36 (1 H), 7.47 (1 H), 7.53 (1 H), 7.83 (1 H) ppm.

### Example 22

### 4-{6-(3-hydroxyprop-1-yn-1-yl)-8-[(3,3,3-trifluoropropyl)amino]imidazo[1,2-a]pyridin-3-yl}-2-methyl-N-(1-methylcyclopropyl)benzamide

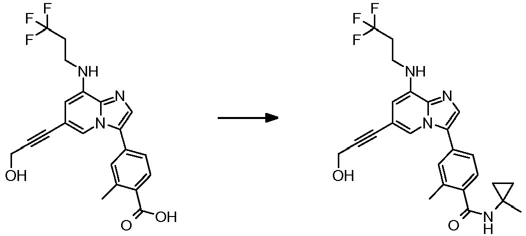

14 mg (34 µmol) 4-{6-(3-hydroxyprop-1-yn-1-yl)-8-[(3,3,3-trifluoropropyl)amino]imidazo[1,2-a]pyridin-3-yl}-2-methylbenzoic acid which was prepared according to intermediate example 20a were transformed in analogy to example 8 using 1-methylcyclopropanaminium chloride to give after working up and purification 11.6 mg (74%) of the title compound.
¹H-NMR (CDCl₃): δ= 0.77 (2H), 0.89 (2H), 1.53 (3H), 2.34-2.60 (2H), 2.50 (3H), 3.59 (2H), 4.49 (2H), 5.30 (1H), 5.39 (1H), 6.12 (1H), 6.16 (1H), 7.31 (1H), 7.34 (1H), 7.41 (1 H), 7.52 (1 H), 7.81 (1 H) ppm.

### Example 23

### N-(1-cyanocyclopropyl)-4-{6-(3-hydroxyprop-1-yn-1-yl)-8-[(3,3,3-trifluoropropyl)amino]imidazo[1,2-a]pyridin-3-yl}-2-methylbenzamide

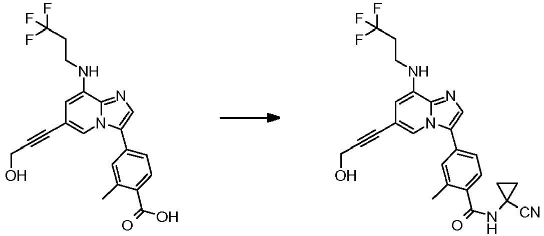

14 mg (34 µmol) 4-{6-(3-hydroxyprop-1-yn-1-yl)-8-[(3,3,3-trifluoropropyl)amino]imidazo[1,2-a]pyridin-3-yl}-2-methylbenzoic acid which was prepared according to intermediate example 20a were transformed in analogy to example 8 using 1-cyanocyclopropanaminium chloride to give after working up and purification 4.0 mg (25%) of the title compound.
¹H-NMR (CDCl₃): δ= 1.41 (2H), 1.68 (2H), 2.46-2.61 (2H), 2.49 (3H), 3.58 (2H), 4.48 (2H), 5.38 (1H), 6.10 (1H), 6.79 (1H), 7.25-7.45 (4H), 7.51 (1H), 7.76 (1H) ppm.

### Example 24

### N-cyclobutyl-4-{6-(3-fluorophenoxy)-8-[(3,3,3-trifluoropropyl)amino]imidazo[1,2-a]pyridin-3-yl}-2-methylbenzamide

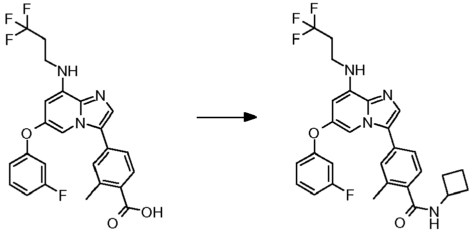

17 mg (36 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using cyclobutanamine hydrochloride to give after working up and purification 16.5 mg (83%) of the title compound.
¹H-NMR (CDCl₃): δ= 1.78 (2H), 1.94 (2H), 2.38-2.59 (4H), 2.49 (3H), 3.52 (2H), 4.59 (1H), 5.50 (1H), 5.92 (1H), 5.99 (1H), 6.71 (1H), 6.76-6.84 (2H), 7.27 (1H), 7.35 (1 H), 7.37 (1 H), 7.46 (1 H), 7.56 (1 H), 7.60 (1 H) ppm.

### Example 25

### 4-{6-(5-Fluoro-2-methylphenoxy)-8-[(3,3,3-trifluoropropyl)amino]imidazo[1,2-a]pyridin-3-yl}-N,2-dimethylbenzamide

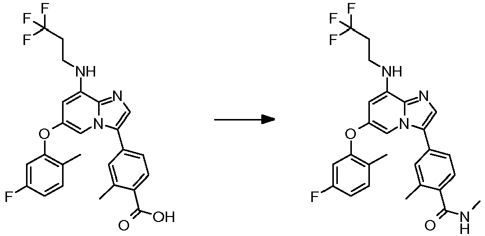

20 mg (41 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 to give after working up and purification 16.0 mg (78%) of the title compound.
¹H-NMR (CDCl₃): δ= 2.28 (3H), 2.40-2.61 (2H), 2.49 (3H), 3.02 (3H), 3.57 (2H), 5.50 (1H), 5.80 (1H), 5.99 (1H), 6.58 (1H), 6.73 (1H), 7.17 (1H), 7.33 (1H), 7.35 (1H), 7.45 (1 H), 7.47 (1 H), 7.54 (1 H) ppm.

### Example 26

### N-ethyl-4-{6-(5-fluoro-2-methylphenoxy)-8-[(3,3,3-trifluoropropyl)amino]imidazo[1,2-a]pyridin-3-yl}-2-methylbenzamide

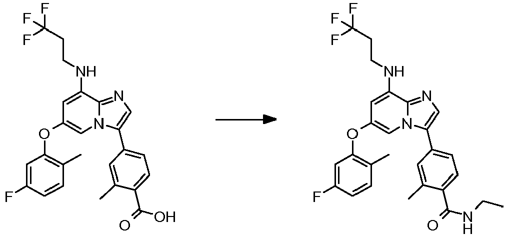

20 mg (41 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using ethanamine to give after working up and purification 18.8 mg (85%) of the title compound.
¹H-NMR (CDCl₃): δ= 1.26 (3H), 2.28 (3H), 2.49 (3H), 2.50-2.59 (2H), 3.50 (2H), 3.57 (2H), 5.49 (1 H), 5.74 (1 H), 5.99 (1 H), 6.58 (1 H), 6.73 (1 H), 7.17 (1 H), 7.34 (1 H), 7.35 (1 H), 7.45 (1 H), 7.47 (1 H), 7.55 (1 H) ppm.

### Example 27

### 4-{6-(5-Fluoro-2-methylphenoxy)-8-[(3,3,3-trifluoropropyl)amino]imidazo[1,2-a]pyridin-3-yl}-2-methyl-N-(1-methylcyclopropyl)benzamide

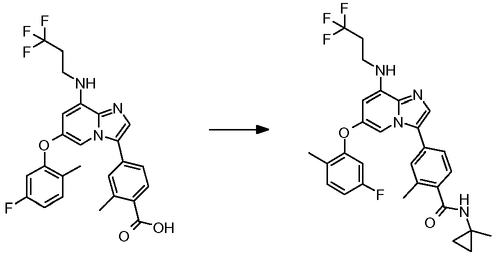

20 mg (41 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using 1-methylcyclopropanaminium chloride to give after working up and purification 18.6 mg (80%) of the title compound.
¹H-NMR (CDCl₃): δ= 0.76 (2H), 0.87 (2H), 1.52 (3H), 2.28 (3H), 2.43-2.62 (2H), 2.46 (3H), 3.57 (2H), 5.48 (1H), 5.99 (1H), 6.06 (1H), 6.58 (1H), 6.74 (1H), 7.18 (1H), 7.32 (1 H), 7.33 (1 H), 7.39 (1 H), 7.45 (1 H), 7.54 (1 H) ppm.

### Example 28

### N-(1-cyanocyclopropyl)-4-{6-(5-fluoro-2-methylphenoxy)-8-[(3,3,3-trifluoropropyl)amino]imidazo[1,2-a]pyridin-3-yl}-2-methylbenzamide

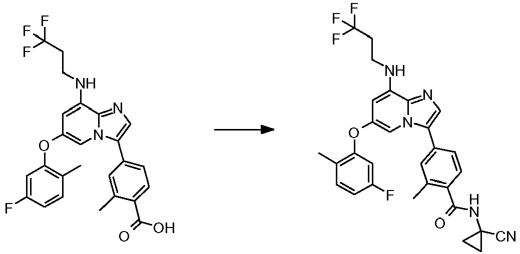

20 mg (41 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using 1-cyanocyclopropanaminium chloride to give after working up and purification 13.3 mg (59%) of the title compound.
¹H-NMR (CD₃OD): δ= 1.33 (2H), 1.57 (2H), 2.23 (3H), 2.42 (3H), 2.58 (2H), 3.57 (2H), 6.16 (1H), 6.69 (1H), 6.79 (1H), 7.24 (1H), 7.38-7.45 (4H), 7.56 (1H) ppm.

### Example 29

### N-[rel-(1S,2S)-2-fluorocyclopropyl]-4-{6-(5-fluoro-2-methylphenoxy)-8-[(3,3,3-trifluoropropyl)amino]imidazo[1,2-a]pyridin-3-yl}-2-methylbenzamide

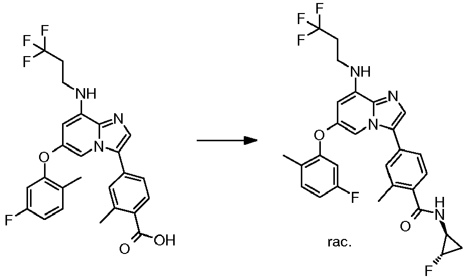

20 mg (41 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using rel-(1S,2S)-2-fluorocyclopropanaminium chloride to give after working up and purification 19.0 mg (85%) of the title compound.
¹H-NMR (CDCl₃): δ= 1.02 (1 H), 1.27 (1 H), 2.28 (3H), 2.42-2.61 (2H), 2.50 (3H), 3.05 (1H), 3.57 (2H), 4.73 (1H), 5.52 (1H), 5.96-6.08 (2H), 6.57 (1H), 6.73 (1H), 7.17 (1 H), 7.35 (1 H), 7.36 (1 H), 7.47 (1 H), 7.48 (1 H), 7.54 (1 H) ppm.

### Example 30

### N-cyclopropyl-4-{6-(2,3-difluorophenoxy)-8-[(3,3,3-trifluoropropyl)amino]imidazo[1,2-a]pyridin-3-yl}-2-methylbenzamide

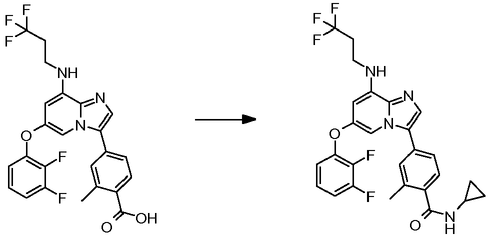

56 mg (114 µmol) 4-{6-(2,3-difluorophenoxy)-8-[(3,3,3-trifluoropropyl)amino]imidazo[1,2-a]pyridin-3-yl}-2-methylbenzoic acid which was prepared according to intermediate example 30a were transformed in analogy to example 8 using cyclopropanamine to give after working up and purification 44.2 mg (69%) of the title compound.
¹H-NMR (CDCl₃): δ= 0.63 (2H), 0.90 (2H), 2.43-2.59 (2H), 2.48 (3H), 2.92 (1H), 3.57 (2H), 5.49 (1H), 5.89 (1H), 6.03 (1H), 6.77 (1H), 6.90-7.03 (2H), 7.33 (1H), 7.35 (1 H), 7.42 (1 H), 7.54 (2H) ppm.

### Example 30a

### 4-{6-(2,3-Difluorophenoxy)-8-[(3,3,3-trifluoropropyl)amino]imidazo[1,2-a]pyridin-3-yl}-2-methylbenzoic acid

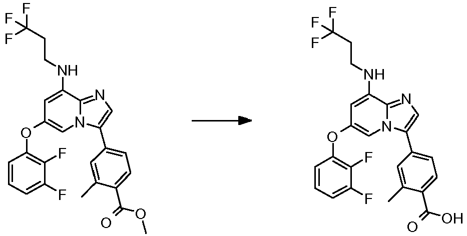

153 mg (303 µmol) methyl 4-{6-(2,3-difluorophenoxy)-8-[(3,3,3-trifluoropropyl)amino]imidazo[1,2-a]pyridin-3-yl}-2-methylbenzoate which was prepared according to intermediate example 30b were transformed in analogy to intermediate example 8b to give after working up and purification 112.7 mg (76%) of the title compound.

### Example 30b

### Methyl 4-{6-(2,3-difluorophenoxy)-8-[(3,3,3-trifluoropropyl)amino]imidazo[1,2-a]pyridin-3-yl}-2-methylbenzoate

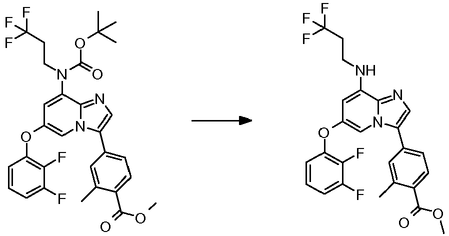

191 mg (316 µmol) methyl 4-{8-[(tert-butoxycarbonyl)(3,3,3-trifluoropropyl)amino]-6-(2,3-difluorophenoxy)imidazo[1,2-a]pyridin-3-yl}-2-methylbenzoate which was prepared according to intermediate example 30c were transformed in analogy to example 7 to give after working up and purification 170 mg (96%) of the title compound.

### Example 30c

### Methyl 4-{8-[(tert-butoxycarbonyl)(3,3,3-trifluoropropyl)amino]-6-(2,3-difluorophenoxy)imidazo[1,2-a]pyridin-3-yl}-2-methylbenzoate

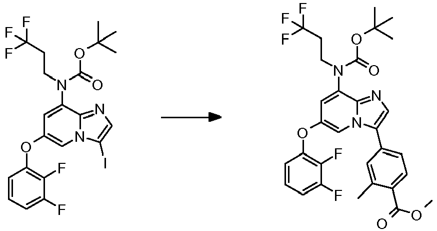

305 mg (523 µmol) tert-butyl [6-(2,3-difluorophenoxy)-3-iodoimidazo[1,2-a]pyridin-8-yl](3,3,3-trifluoropropyl)carbamate which was prepared according to intermediate example 30d were transformed in analogy to intermediate example 7b using [4-(methoxycarbonyl)-3-methylphenyl]boronic acid to give after working up and purification 202 mg (61%) of the title compound.

### Example 30d

### tert-Butyl [6-(2,3-difluorophenoxy)-3-iodoimidazo[1,2-a]pyridin-8-yl](3,3,3-trifluoropropyl)carbamate

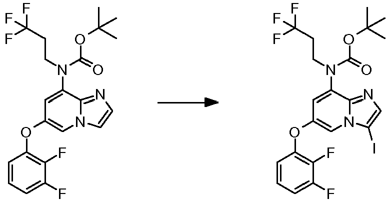

288 mg (630 µmol) tert-butyl [6-(2,3-difluorophenoxy)imidazo[1,2-a]pyridin-8-yl](3,3,3-trifluoropropyl)carbamate which was prepared according to intermediate example 30e were transformed in analogy to intermediate example 7c to give after working up and purification 308 mg (84%) of the title compound.

### Example 30e

### tert-Butyl [6-(2,3-difluorophenoxy)imidazo[1,2-a]pyridin-8-yl](3,3,3-trifluoropropyl)carbamate

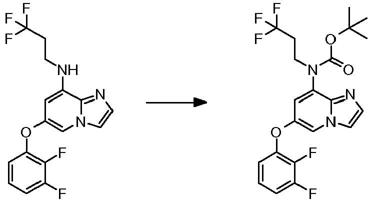

290 mg (812 µmol) 6-(2,3-difluorophenoxy)-N-(3,3,3-trifluoropropyl)imidazo[1,2-a]pyridin-8-amine which was prepared according to intermediate example 30f were transformed in analogy to intermediate example 7d to give after working up and purification 291 mg (74%) of the title compound.

### Example 30f

### 6-(2,3-Difluorophenoxy)-N-(3,3,3-trifluoropropyl)imidazo[1,2-a]pyridin-8-amine

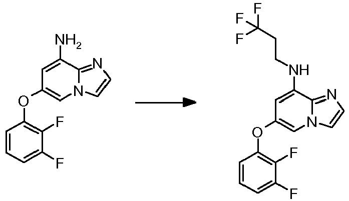

373 mg (1.43 mmol) 6-(2,3-difluorophenoxy)imidazo[1,2-a]pyridin-8-amine which was prepared according to intermediate example 30g were transformed in analogy to intermediate example 7e to give after working up 500 mg of a crude material that contained some incomplete reduction product. The compound mixture was solved in 20 mL ethanol, 76 mg palladium on charcoal (10%) was added and the mixture was stirred under an atmosphere of hydrogen overnight. After filtration and removal of the solvent the residue was purified by chromatography to give 294.8 mg (52%) of the title compound.

### Example 30g

### 6-(2,3-Difluorophenoxy)imidazo[1,2-a]pyridin-8-amine

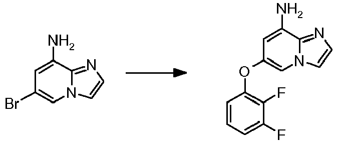

5.00 g (23.6 mmol) 6-bromoimidazo[1,2-a]pyridin-8-amine which was prepared according to intermediate example 3-1 were transformed in analogy to example 6-1 using 2,3-difluorophenol to give after working up and purification 373 mg (6%) of the title compound.

### Example 31

### 4-{6-(3-chlorophenoxy)-8-[(3,3,3-trifluoropropyl)amino]imidazo[1,2-a]pyridin-3-yl}-N-cyclopropyl-2-methylbenzamide

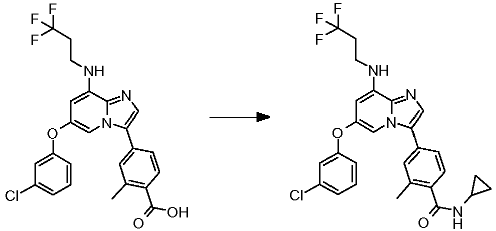

20 mg (41 µmol) 4-{6-(3-chlorophenoxy)-8-[(3,3,3-trifluoropropyl)amino]imidazo[1,2-a]pyridin-3-yl}-2-methylbenzoic acid which was prepared according to intermediate example 31 a were transformed in analogy to example 8 using cyclopropanamine to give after working up and purification 12.7 mg (56%) of the title compound.
¹H-NMR (CDCl₃): δ= 0.62 (2H), 0.99 (2H), 2.42-2.60 (2H), 2.49 (3H), 2.92 (1H), 3.56 (2H), 5.49 (1H), 5.90 (1H), 5.98 (1H), 6.90 (1H), 6.99 (1H), 7.06 (1H), 7.24 (1H), 7.34 (1H), 7.37 (1H), 7.43 (1H), 7.55 (1H), 7.58 (1H) ppm.

### Example 31 a

### 4-{6-(3-Chlorophenoxy)-8-[(3,3,3-trifluoropropyl)amino]imidazo[1,2-a]pyridin-3-yl}-2-methylbenzoic acid

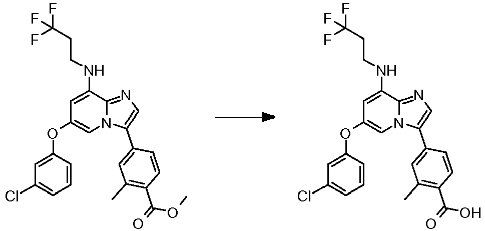

272 mg (540 µmol) methyl 4-{6-(3-chlorophenoxy)-8-[(3,3,3-trifluoropropyl)amino]imidazo[1,2-a]pyridin-3-yl}-2-methylbenzoate which was prepared according to intermediate example 31 b were transformed in analogy to intermediate example 8b to give after working up and purification 260 mg (98%) of the title compound.

### Example 31 b

### Methyl 4-{6-(3-chlorophenoxy)-8-[(3,3,3-trifluoropropyl)amino]imidazo[1,2-a]pyridin-3-yl}-2-methylbenzoate

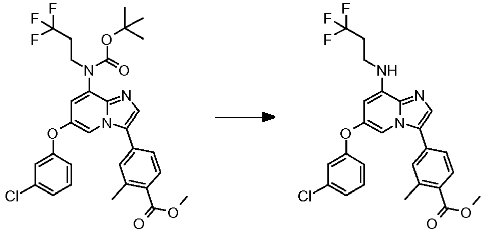

330 mg (546 µmol) methyl 4-{8-[(tert-butoxycarbonyl)(3,3,3-trifluoropropyl)amino]-6-(3-chlorophenoxy)imidazo[1,2-a]pyridin-3-yl}-2-methylbenzoate which was prepared according to intermediate example 31 c were transformed in analogy to example 7 to give after working up and purification 277 mg (100%) of the title compound.

### Example 31 c

### Methyl 4-{8-[(tert-butoxycarbonyl)(3,3,3-trifluoropropyl)amino]-6-(3-chlorophenoxy)imidazo[1,2-a]pyridin-3-yl}-2-methylbenzoate

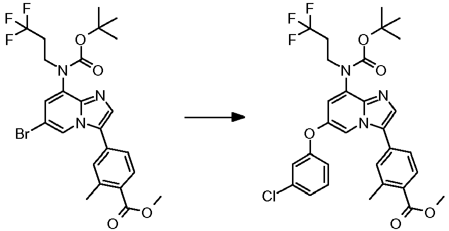

1.00 g (1.80 mmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to example 6-1 using 3-chlorophenol to give after working up and purification 344 mg (30%) of the title compound.

### Example 32

### 4,4'-{8-[(3,3,3-trifluoropropyl)amino]imidazo[1,2-a]pyridine-3,6-diyl}bis(N-cyclopropyl-2-methylbenzamide)

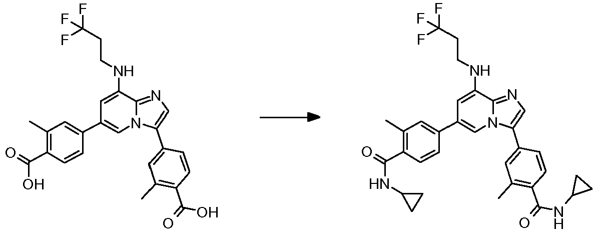

20 mg (40 µmol) 4,4'-{8-[(3,3,3-trifluoropropyl)amino]imidazo[1,2-a]pyridine-3,6-diyl}bis(2-methylbenzoic acid) which was prepared according to intermediate example 32a were transformed in analogy to example 8 using cyclopropanamine to give after working up and purification 6.0 mg (25%) of the title compound. ¹H-NMR (DMSO-d6): δ= 0.50 (4H), 0.66 (4H), 2.35 (3H), 2.37 (3H), 2.69 (2H), 2.81 (2H), 3.59 (2H), 6.36 (1H), 6.44 (1H), 7.32 (1H), 7.41 (1H), 7.46-7.55 (4H), 7.63 (1H), 7.90 (1H), 8.23 (1H), 8.31 (1H) ppm.

### Example 32a

### 4,4'-{8-[(3,3,3-Trifluoropropyl)amino]imidazo[1,2-a]pyridine-3,6-diyl}bis(2-methylbenzoic acid)

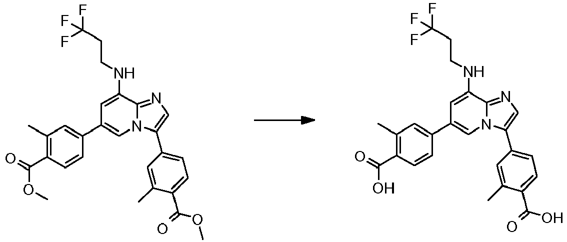

930 mg (1.77 mmol) dimethyl 4,4'-{8-[(3,3,3-trifluoropropyl)amino]imidazo[1,2-a]pyridine-3,6-diyl}bis(2-methylbenzoate) which was prepared according to intermediate example 32b were transformed in analogy to intermediate example 8b to give after working up and purification 743 mg (80%) of the title compound.

### Example 32b

### Dimethyl 4,4'-{8-[(3,3,3-trifluoropropyl)amino]imidazo[1,2-a]pyridine-3,6-diyl}bis(2-methylbenzoate)

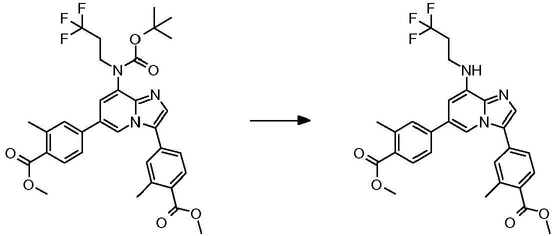

1.15 g (1.84 mmol) dimethyl 4,4'-{8-[(tert-butoxycarbonyl)(3,3,3-trifluoropropyl)amino]imidazo[1,2-a]pyridine-3,6-diyl}bis(2-methylbenzoate) which was prepared according to intermediate example 32c were transformed in analogy to example 7 to give after working up and purification 956 mg (94%) of the title compound.

### Example 32c

### Dimethyl 4,4'-{8-[(tert-butoxycarbonyl)(3,3,3-trifluoropropyl)amino]imidazo[1,2-a]pyridine-3,6-diyl}bis(2-methylbenzoate)

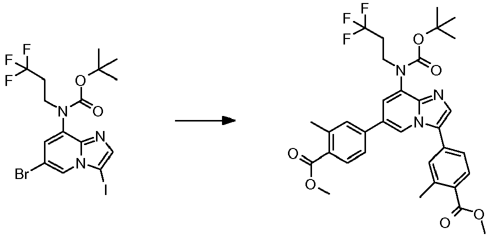

1.31 g (2.45 mmol) tert-butyl (6-bromo-3-iodoimidazo[1,2-a]pyridin-8-yl)(3,3,3-trifluoropropyl)carbamate which was prepared according to intermediate example 7c were transformed in analogy to intermediate example 7b at 120°C using [4-(methoxycarbonyl)-3-methylphenyl]boronic acid to give after working up and purification 1.16 g (72%) of the title compound.

### Example 33

### N-cyclopropyl-2-methyl-4-{6-phenoxy-8-[(3,3,3-trifluoropropyl)amino]imidazo[1,2-a]pyridin-3-yl}benzamide

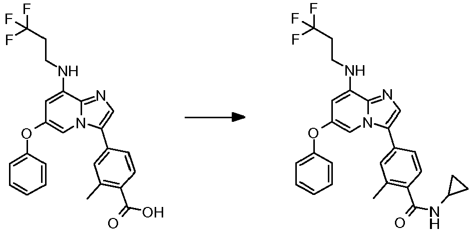

10.0 mg (22 µmol) 2-methyl-4-{6-phenoxy-8-[(3,3,3-trifluoropropyl)amino]imidazo[1,2-a]pyridin-3-yl}benzoic acid which was prepared according to intermediate example 33a were transformed in analogy to example 8 using cyclopropanamine to give after working up and purification 5.6 mg (49%) of the title compound.
¹H-NMR (CDCl₃): δ= 0.62 (2H), 0.89 (2H), 2.40-2.60 (2H), 2.47 (3H), 2.92 (1 H), 3.54 (2H), 5.45 (1H), 5.92 (1H), 6.02 (1H), 7.01 (2H), 7.09 (1H), 7.28-7.44 (5H), 7.55 (2H), ppm.

### Example 33a

### 2-Methyl-4-{6-phenoxy-8-[(3,3,3-trifluoropropyl)amino]imidazo[1,2-a]pyridin-3-yl}benzoic acid

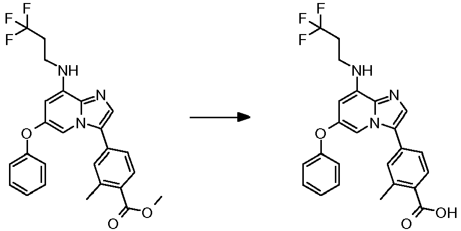

492 mg (1.05 mmol) methyl 2-methyl-4-{6-phenoxy-8-[(3,3,3-trifluoropropyl)amino]imidazo[1,2-a]pyridin-3-yl}benzoate which was prepared according to intermediate example 33b were transformed in analogy to intermediate example 8b to give after working up and purification 352 mg (70%) of the title compound.

### Example 33b

### Methyl 2-methyl-4-{6-phenoxy-8-[(3,3,3-trifluoropropyl)amino]imidazo[1,2-a]pyridin-3-yl}benzoate

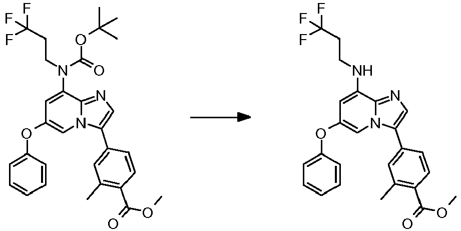

602 mg (1.06 mmol) methyl 4-{8-[(tert-butoxycarbonyl)(3,3,3-trifluoropropyl)amino]-6-phenoxyimidazo[1,2-a]pyridin-3-yl}-2-methylbenzoate which was prepared according to intermediate example 33c were transformed in analogy to example 7 to give after working up and purification 492 mg (99%) of the title compound.

### Example 33c

### Methyl 4-{8-[(tert-butoxycarbonyl)(3,3,3-trifluoropropyl)amino]-6-phenoxyimidazo[1,2-a]pyridin-3-yl}-2-methylbenzoate

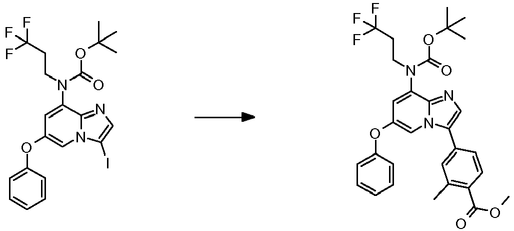

692 mg (1.26 mmol) tert-butyl (3-iodo-6-phenoxyimidazo[1,2-a]pyridin-8-yl)(3,3,3-trifluoropropyl)carbamate which was prepared according to intermediate example 33d were transformed in analogy to intermediate example 7b using [4-(methoxycarbonyl)-3-methylphenyl]boronic acid to give after working up and purification 606 mg (84%) of the title compound.

### Example 33d

### tert-Butyl (3-iodo-6-phenoxyimidazo[1,2-a]pyridin-8-yl)(3,3,3-trifluoropropyl)carbamate

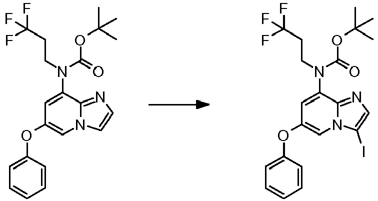

590 mg (1.40 mmol) tert-butyl (6-phenoxyimidazo[1,2-a]pyridin-8-yl)(3,3,3-trifluoropropyl)carbamate which was prepared according to intermediate example 33e were transformed in analogy to intermediate example 7c to give after working up and purification 696 mg (91%) of the title compound.

### Example 33e

### tert-butyl (6-phenoxyimidazo[1,2-a]pyridin-8-yl)(3,3,3-trifluoropropyl)carbamate

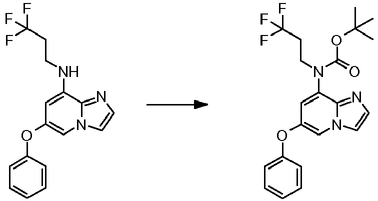

535 mg (1.67 mmol) 6-phenoxy-N-(3,3,3-trifluoropropyl)imidazo[1,2-a]pyridin-8-amine which was prepared according to intermediate example 33f were transformed in analogy to intermediate example 7d to give after working up and purification 590 mg (80%) of the title compound.

### Example 33f

### 6-Phenoxy-N-(3,3,3-trifluoropropyl)imidazo[1,2-a]pyridin-8-amine

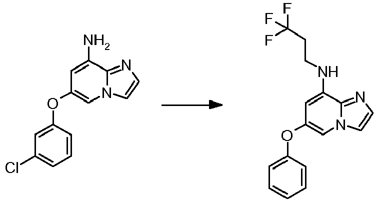

850 mg (3.27 mmol) 6-(3-chlorophenoxy)imidazo[1,2-a]pyridin-8-amine which was prepared according to intermediate example 33g were transformed in analogy to intermediate example 30f to give after working up and purification 638 mg (61 %) of the title compound.

### Example 33g

### 6-(3-Chlorophenoxy)imidazo[1,2-a]pyridin-8-amine

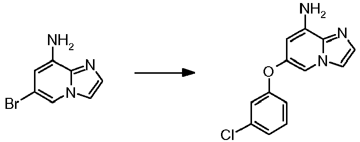

3.00 g (14.15 mmol) 6-bromoimidazo[1,2-a]pyridin-8-amine which was prepared according to intermediate example 3-1 were transformed in analogy to example 6-1 using 3-chlorophenol to give after working up and purification 854 mg (216%) of the title compound.

### Example 34

### 4-{6-(3-chlorophenoxy)-8-[(3,3,3-trifluoropropyl)amino]imidazo[1,2-a]pyridin-3-yl}-N,2-dimethylbenzamide

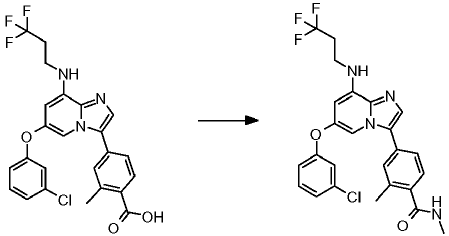

20 mg (41 µmol) 4-{6-(3-chlorophenoxy)-8-[(3,3,3-trifluoropropyl)amino]imidazo[1,2-a]pyridin-3-yl}-2-methylbenzoic acid which was prepared according to intermediate example 31 a were transformed in analogy to example 8 to give after working up and purification 14.3 mg (66%) of the title compound.
¹H-NMR (CDCl₃): δ= 2.42-2.59 (2H), 2.50 (3H), 3.02 (3H), 3.56 (2H), 5.50 (1 H), 5.82 (1H), 5.98 (1H), 6.90 (1H), 7.00 (1H), 7.06 (1H), 7.24 (1H), 7.36 (1H), 7.38 (1H), 7.46 (1H), 7.56 (1H), 7.59 (1H) ppm.

### Example 35

### 4-{6-(3-chlorophenoxy)-8-[(3,3,3-trifluoropropyl)amino]imidazo[1,2-a]pyridin-3-yl}-N-ethyl-2-methylbenzamide

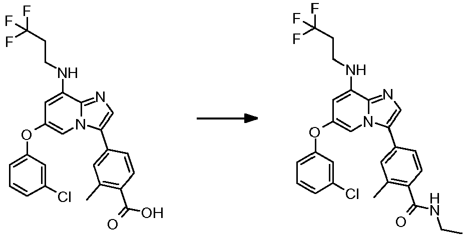

20 mg (41 µmol) 4-{6-(3-chlorophenoxy)-8-[(3,3,3-trifluoropropyl)amino]imidazo[1,2-a]pyridin-3-yl}-2-methylbenzoic acid which was prepared according to intermediate example 31 a were transformed in analogy to example 8 using ethanamine to give after working up and purification 13.4 mg (60%) of the title compound.
¹H-NMR (CDCl₃): δ= 1.26 (3H), 2.46-2.59 (2H), 2.50 (3H), 3.47-3.61 (4H), 5.51 (1H), 5.76 (1H), 5.98 (1H), 6.90 (1H), 7.00 (1H), 7.07 (1H), 7.23 (1H), 7.36 (1H), 7.38 (1 H), 7.46 (1 H), 7.56 (1 H), 7.59 (1 H) ppm.

### Example 36

### 4-{6-(3-chlorophenoxy)-8-[(3,3,3-trifluoropropyl)amino]imidazo[1,2-a]pyridin-3-yl}-2-methyl-N-(1-methylcyclopropyl)benzamide

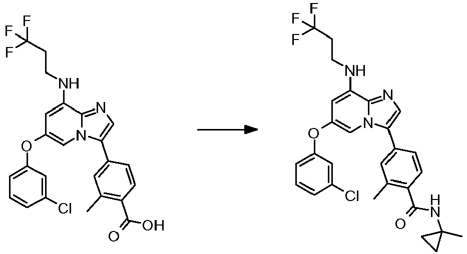

20 mg (41 µmol) 4-{6-(3-chlorophenoxy)-8-[(3,3,3-trifluoropropyl)amino]imidazo[1,2-a]pyridin-3-yl}-2-methylbenzoic acid which was prepared according to intermediate example 31 a were transformed in analogy to example 8 using 1-methylcyclopropanaminium chloride to give after working up and purification 13.3 mg (57%) of the title compound.
¹H-NMR (CDCl₃): δ= 0.76 (2H), 0.87 (2H), 1.52 (3H), 2.45-2.58 (2H), 2.48 (3H), 3.56 (2H), 5.48 (1H), 5.98 (1H), 6.07 (1H), 6.90 (1H), 6.99 (1H), 7.07 (1H), 7.23 (1H), 7.34 (1H), 7.36 (1H), 7.40 (1H), 7.55 (1H), 7.58 (1H) ppm.

### Example 37

### 4-{6-(3-chlorophenoxy)-8-[(3,3,3-trifluoropropyl)amino]imidazo[1,2-a]pyridin-3-yl}-N-(1-cyanocyclopropyl)-2-methylbenzamide

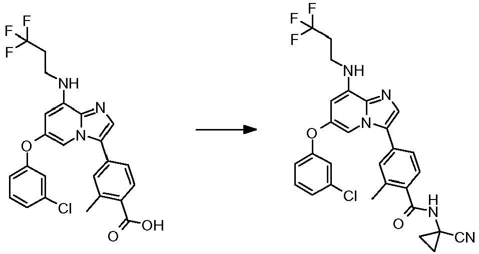

35 mg (71 µmol) 4-{6-(3-chlorophenoxy)-8-[(3,3,3-trifluoropropyl)amino]imidazo[1,2-a]pyridin-3-yl}-2-methylbenzoic acid which was prepared according to intermediate example 31 a were transformed in analogy to example 8 using 1-cyanocyclopropanaminium chloride to give after working up and purification 20.4 mg (57%) of the title compound.
¹H-NMR (CDCl₃): δ= 1.37 (2H), 1.66 (2H), 2.43-2.64 (2H), 2.51 (3H), 3.55 (2H), 5.49 (1H), 5.98 (1H), 6.48 (1H), 6.90 (1H), 6.99 (1H), 7.07 (1H), 7.25 (1H), 7.33-7.46 (3H), 7.55 (1H), 7.58 (1H) ppm.

### Example 38

### 4-{6-(3-fluorophenoxy)-8-[(3,3,3-trifluoropropyl)amino]imidazo[1,2-a]pyridin-3-yl}-2-methyl-N-(1-methylcyclobutyl)benzamide

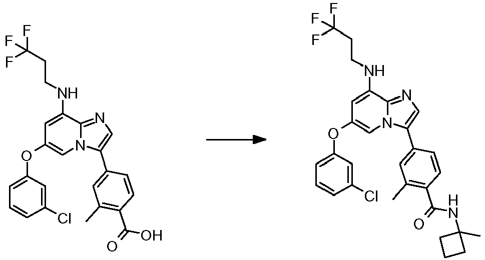

20 mg (41 µmol) 4-{6-(3-chlorophenoxy)-8-[(3,3,3-trifluoropropyl)amino]imidazo[1,2-a]pyridin-3-yl}-2-methylbenzoic acid which was prepared according to intermediate example 31 a were transformed in analogy to example 8 using 1-methylcyclobutylamin to give after working up and purification 12.5 mg (52%) of the title compound.
¹H-NMR (CDCl₃): δ= 1.60 (3H), 1.85-2.01 (2H), 2.09-2.18 (2H), 2.42 (2H), 2.47-2.58 (2H), 2.50 (3H), 3.56 (2H), 5.50 (1 H), 5.83 (1 H), 5.99 (1H), 6.72 (1H), 6.75-6.83 (2H), 7.27 (1 H), 7.35 (1 H), 7.36 (1 H), 7.46 (1 H), 7.56 (1 H), 7.61 (1 H) ppm.

### Example 39

### 4-{6-(3-chlorophenoxy)-8-[(3,3,3-trifluoropropyl)amino]imidazo[1,2-a]pyridin-3-yl}-N-[rel-(1R,2R)-2-fluorocyclopropyl]-2-methylbenzamide (rac-A), 4-{6-(3-chlorophenoxy)-8-[(3,3,3-trifluoropropyl)amino]imidazo[1,2-a]pyridin-3-yl}-N-{[(1S,2S) or (1R,2R)]-2-fluorocyclopropyl}-2-methylbenzamide (A or ent-A) and 4-{6-(3-chlorophenoxy)-8-[(3,3,3-trifluoropropyl)amino]imidazo[1,2-a]pyridin-3-yl}-N-{[(1R,2R) or (1S,2S)]-2-fluorocyclopropyl}-2-methylbenzamide (ent-A or A)

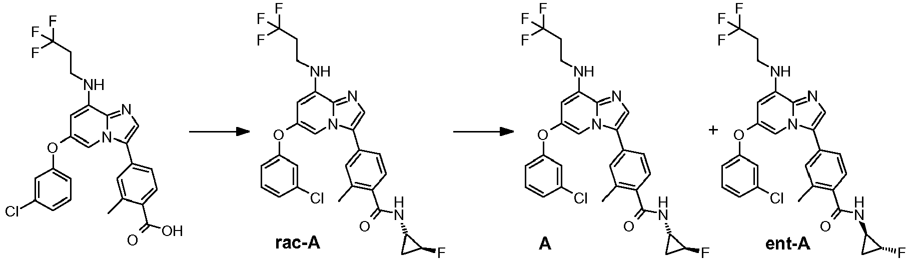

35 mg (71 µmol) 4-{6-(3-chlorophenoxy)-8-[(3,3,3-trifluoropropyl)amino]imidazo[1,2-a]pyridin-3-yl}-2-methylbenzoic acid which was prepared according to intermediate example 31 a were transformed in analogy to example 8 using rel-(1S,2S)-2-fluorocyclopropanaminium chloride to give after working up and purification 22.9 mg (56%) of the title compound.
¹H-NMR (CDCl₃): δ= 1.02 (1 H), 1.27 (1 H), 2.46-2.57 (2H), 2.52 (3H), 3.06 (1 H), 3.56 (2H), 4.77 (1H), 5.51 (1H), 5.97-6.04 (2H), 6.90 (1H), 7.00 (1H), 7.07 (1H), 7.23 (1 h), 7.37 (1 H), 7.39 (1 H), 7.49 (1 H), 7.57 (1 H), 7.60 (1 H) ppm. 16.9 mg of rac-A (31 µmol) were separated by HPLC using a chiral column to give 7.2 mg (43%) ent.-A or A and 7.7 mg (46%) A or ent.-A.

### Example 40

### N-(2,6-diethylphenyl)-4-{6-(3-fluorophenoxy)-8-[(3,3,3-trifluoropropyl)amino]imidazo[1,2-a]pyridin-3-yl}-2-methylbenzamide

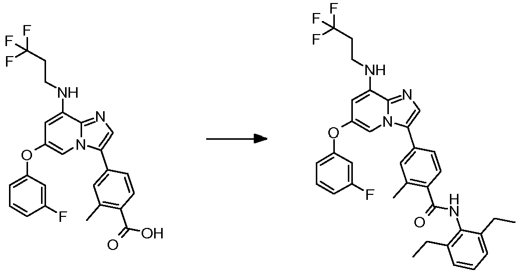

17 mg (36 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using 2,6-diethylaniline to give after working up and purification 15.0 mg (66%) of the title compound.
¹H-NMR (CDCl₃): δ= 1.27 (6H), 2.48 (2H), 2.62 (3H), 2.73 (4H), 3.53 (2H), 5.44 (1 H), 6.01 (1H), 6.71-6.84 (3H), 7.20 (2H), 7.24-7.32 (3H), 7.44 (1H), 7.46 (1H), 7.50 (1 H), 7.65 (1 H), 7.68 (1H) ppm.

### Example 41

### 4,4'-{8-[methyl(3,3,3-trifluoropropyl)amino]imidazo[1,2-a]pyridine-3,6-diyl}bis(N,2-dimethylbenzamide)

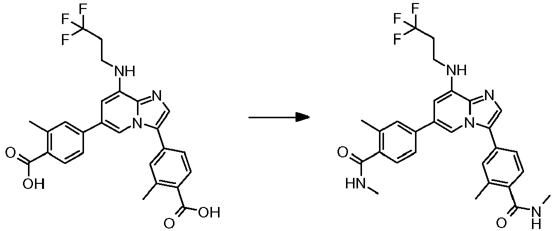

20 mg (40 µmol) 4,4'-{8-[(3,3,3-trifluoropropyl)amino]imidazo[1,2-a]pyridine-3,6-diyl}bis(2-methylbenzoic acid) which was prepared according to intermediate example 32a were transformed in analogy to example 8 to give after working up and purification 4.9 mg (22%) of the title compound.
¹H-NMR (DMSO-d6): δ= 2.37 (3H), 2.39 (3H), 2.70 (2H), 2.74 (6H), 3.59 (2H), 6.36 (1H), 6.46 (1H), 7.36 (1H), 7.45 (1H), 7.48-7.56 (4H), 7.64 (1H), 7.92 (1H), 8.12 (1 H), 8.20 (1 H) ppm.

### Example 42

### N-cyclopropyl-4-{6-(3-fluoro-4-methoxyphenoxy)-8-[(3,3,3-trifluoropropyl)amino]imidazo[1,2-a]pyridin-3-yl}-2-methylbenzamide

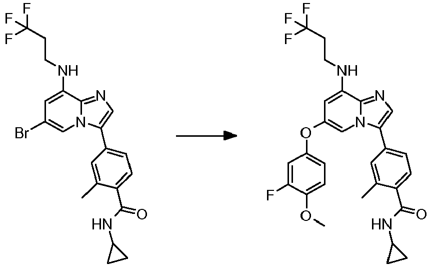

A mixture comprising 200 mg (416 µmol) 4-{6-bromo-8-[(3,3,3-trifluoropropyl)amino]imidazo[1,2-a]pyridin-3-yl}-N-cyclopropyl-2-methylbenzamide which was prepared according to intermediate example 42a, 472 mg 3-fluoro-4-methoxyphenol3-fluorophenol, 975 mg caesium carbonate, 18.9 mg (RS) phenyl hydrogen pyrrolidin-2-ylphosphonate, 16.4 mg copper(I)chloride and 4 mL 1,4-dioxane was heated at 130°C using microwave irradiation for 4 hours. The mixture was poured into water and extracted with ethyl acetate and methanol. The organic layer was dried over sodium sulfate. After filtration and removal of the solvent the residue war purified by chromatography to give 23.2 mg (10%) of the title compound.
¹H-NMR (CDCl₃): δ= 0.62 (2H), 0.90 (2H), 2.45-2.57 (2H), 2.48 (3H), 2.92 (1H), 3.56 (2H), 3.87 (3H), 5.44 (1H), 5.90 (1H), 5.98 (1H), 6.74 (1H), 6.83 (1H), 6.91 (1H), 7.33 (1 H), 7.35 (1 H), 7.42 (1 H), 7.49 (1 H), 7.53 (1 H) ppm.

### Example 42a

### 4-{6-bromo-8-[(3,3,3-trifluoropropyl)amino]imidazo[1,2-a]pyridin-3-yl}-N-cyclopropyl-2-methylbenzamide

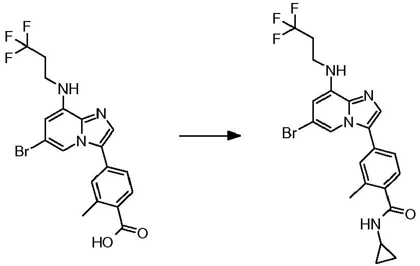

750 mg (1.70 mmol) 4-{6-bromo-8-[(3,3,3-trifluoropropyl)amino]imidazo[1,2-a]pyridin-3-yl}-2-methylbenzoic acid which was prepared according to intermediate example 42b were transformed in analogy to example 8 using cyclopropanamine to give after working up and purification 589 mg (69%) of the title compound.

### Example 42b

### 4-{6-bromo-8-[(3,3,3-trifluoropropyl)amino]imidazo[1,2-a]pyridin-3-yl}-2-methylbenzoic acid

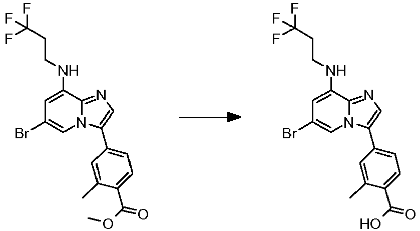

787 mg (1.73 mmol) methyl 4-{6-bromo-8-[(3,3,3-trifluoropropyl)amino]imidazo[1,2-a]pyridin-3-yl}-2-methylbenzoate which was prepared according to intermediate example 42c were transformed in analogy to intermediate example 8b to give after working up 769 mg (96%) of the title compound.

### Example 42c

### methyl 4-{6-bromo-8-[(3,3,3-trifluoropropyl)amino]imidazo[1,2-a]pyridin-3-yl}-2-methylbenzoate

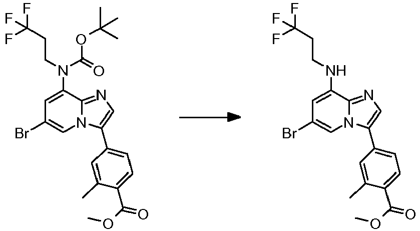

1000 mg (1.80 mmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to example 7 to give after working up and purification 801 mg (93%) of the title compound.

### Example 43

### 4,4'-{8-[methyl(3,3,3-trifluoropropyl)amino]imidazo[1,2-a]pyridine-3,6-diyl}bis[N-(1-cyanocyclopropyl)-2-methylbenzamide]

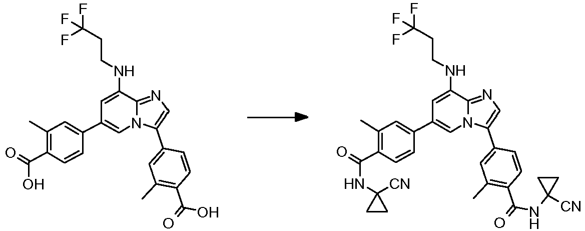

20 mg (40 µmol) 4,4'-{8-[(3,3,3-trifluoropropyl)amino]imidazo[1,2-a]pyridine-3,6-diyl}bis(2-methylbenzoic acid) which was prepared according to intermediate example 32a were transformed in analogy to example 8 using 1-cyanocyclopropanaminium chlorid to give after working up and purification 2.9 mg (4%) of the title compound.
¹H-NMR (DMSO-d6): δ= 1.25 (4H), 1.53 (4H), 2.38 (3H), 2.40 (3H), 2.62-2.79 (2H), 3.59 (2H), 6.38 (1 H), 6.46 (1 H), 7.39 (1 H), 7.49 (1 H), 7.54 (1 H), 7.56-7.61 (3H), 7.67 (1 H), 7.94 (1 H), 9.13 (1 H), 9.21 (1 H) ppm.

### Example 44

### 4,4'-{8-[methyl(3,3,3-trifluoropropyl)amino]imidazo[1, 2-a]pyridine-3,6-diyl}bis[2-methyl-N-(1-methylcyclopropyl)benzamide]

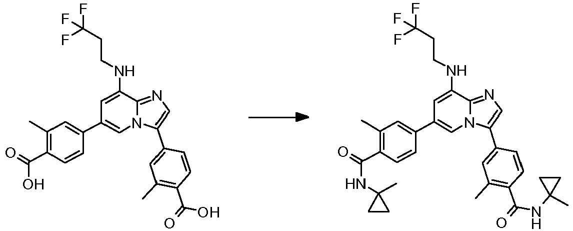

20 mg (40 µmol) 4,4'-{8-[(3,3,3-trifluoropropyl)amino]imidazo[1,2-a]pyridine-3,6-diyl}bis(2-methylbenzoic acid) which was prepared according to intermediate example 32a were transformed in analogy to example 8 using 1-methylcyclopropanaminium chloride to give after working up and purification 8.4 mg (33%) of the title compound.
¹H-NMR (DMSO-d6): δ= 0.56 (4H), 0.69 (4H), 1.36 (3H), 1.37 (3H), 2.33 (3H), 2.35 (3H), 2.49-2.77 (2H), 3.59 (2H), 6.36 (1H), 6.43 (1H), 7.27 (1H), 7.37 (1H), 7.43-7.54 (4H), 7.62 (1 H), 7.88 (1 H), 8.37 (1 H), 8.46 (1 H) ppm.

### Example 45

### 4,4'-{8-[methyl(3,3,3-trifluoropropyl)amino]imidazo[1,2-a]pyridine-3,6-diyl}bis(N-ethyl-2-methylbenzamide)

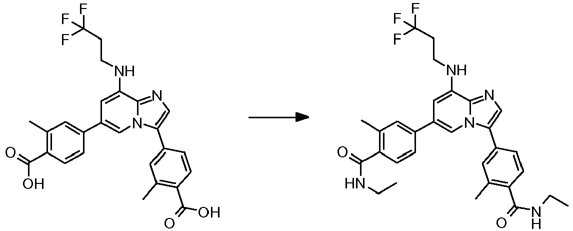

20 mg (40 µmol) 4,4'-{8-[(3,3,3-trifluoropropyl)amino]imidazo[1,2-a]pyridine-3,6-diyl}bis(2-methylbenzoic acid) which was prepared according to intermediate example 32a were transformed in analogy to example 8 using ethanamine to give after working up and purification 5.3 mg (23%) of the title compound.
¹H-NMR (DMSO-d6): δ= 1.09 (3H), 1.10 (3H), 2.37 (3H), 2.38 (3H), 2.70 (2H), 3.23 (4H), 3.59 (2H), 6.36 (1 H), 6.45 (1 H), 7.34 (1 H), 7.44 (1 H), 7.48-7.56 (4H), 7.64 (1H), 7.91 (1H), 8.18 (1H), 8.27 (1H) ppm.

### Example 46

### N-(2,6-diethylphenyl)-4-{6-(5-fluoro-2-methylphenoxy)-8-[(3,3,3-trifluoropropyl)amino]imidazo[1,2-a]pyridin-3-yl}-2-methylbenzamide

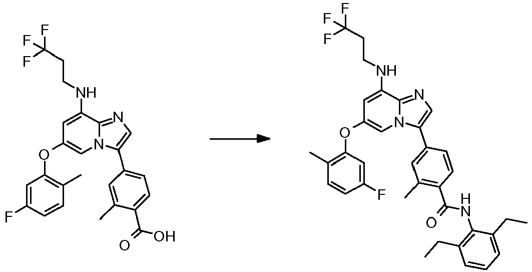

15 mg (31 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using 2,6-diethylaniline to give after working up and purification 6.7 mg (35%) of the title compound.
¹H-NMR (CDCl₃): δ= 1.27 (6H), 2.29 (3H), 2.45 (2H), 2.60 (3H), 2.74 (4H), 3.51 (2H), 5.35 (1 H), 6.00 (1 H), 6.58 (1 H), 6.74 (1 H), 7.14-7.24 (3H), 7.29 (1 H), 7.37-7.40 (4H), 7.50 (1 H), 7.66 (1 H) ppm.

### Example 47

### N-cyclobutyl-4-{6-(5-fluoro-2-methylphenoxy)-8-[(3,3,3-trifluoropropyl)amino]imidazo[1,2-a]pyridin-3-yl}-2-methylbenzamide

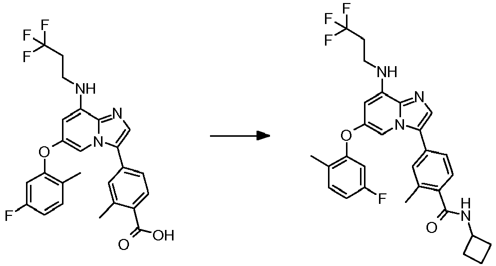

15 mg (31 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using cyclobutanamine to give after working up and purification 14.7 mg (88%) of the title compound.
¹H-NMR (CDCl₃): δ= 1.70-1.84 (2H), 1.85-2.03 (2H), 2.28 (3H), 2.42-2.60 (4H), 2.48 (3H), 3.57 (2H), 4.60 (1 H), 5.46 (1 H), 5.89 (1 H), 5.99 (1 H), 6.57 (1 H), 6.73 (1 H), 7.17 (1 H), 7.34 (1 H), 7.35 (1 H), 7.42-7.49 (2H), 7.55 (1 H) ppm.

### Example 48

### Rel-4-{6-(3-fluorophenoxy)-8-[(3,3,3-trifluoropropyl)amino]imidazo[1,2-a]pyridin-3-yl}-2-methyl-N-[(1R,2R)-2-methylcyclopropyl]benzamide

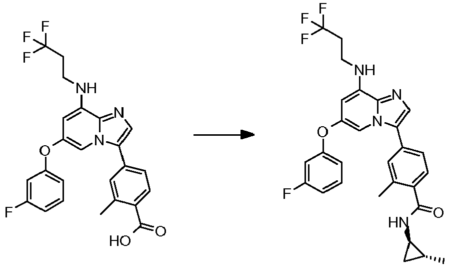

20 mg (42 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using rel-(1R,2R)-2-methylcyclopropanamine to give after working up and purification 15.5 mg (66%) of the title compound.
¹H-NMR (CDCl₃): δ= 0.66 (1H), 0.75 (1H), 0.98 (1H), 1.16 (3H), 2.44-2.58 (2H), 2.49 (3H), 2.60 (1H), 3.56 (2H), 5.50 (1H), 5.85 (1H), 5.99 (1H), 6.72 (1H), 6.79-6.82 (2H), 7.27 (1 H), 7.34 (1 H), 7.36 (1 H), 7.42 (1 H), 7.55 (1 H), 7.60 (1 H) ppm.

### Example 49

### N-[1,1'-bi(cyclopropyl)-1-yl]-4-{6-(3-fluorophenoxy)-8-[(3,3,3-trifluoropropyl)amino]imidazo[1,2-a]pyridin-3-yl}-2-methylbenzamide

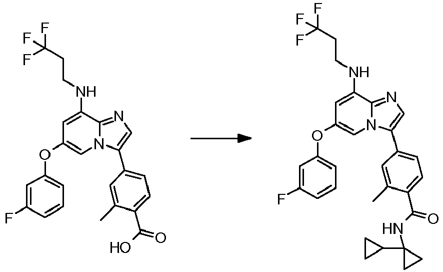

20 mg (42 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using 1,1'-bi(cyclopropyl)-1-aminium chloride to give after working up and purification 14.3 mg (58%) of the title compound.
¹H-NMR (CDCl₃): δ= 0.22 (2H), 0.49 (2H), 0.72 (2H), 0.82 (2H), 1.57 (1 H), 2.44-2.59 (2H), 2.49 (3H), 3.56 (2H), 5.48 (1H), 5.99 (1H), 6.10 (1H), 6.71 (1H), 6.75-6.83 (2H), 7.27 (1 H), 7.34 (1 H), 7.36 (1 H), 7.43 (1 H), 7.55 (1 H), 7.60 (1 H) ppm.

### Example 50

### 4-{6-(3-Fluorophenoxy)-8-[(3,3,3-trifluoropropyl)amino]imidazo[1,2-a]pyridin-3-yl}-N-[1-(hydroxymethyl)cyclopropyl]-2-methylbenzamide

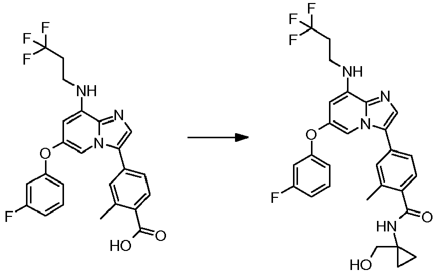

30 mg (63 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using 1-(hydroxymethyl)cyclopropanaminium chloride to give after working up and purification 23.3 mg (64%) of the title compound.
¹H-NMR (DMSO-d6): δ= 0.65 (2H), 0.72 (2H), 2.33 (3H), 2.54-2.67 (2H), 3.46 (2H), 3.52 (2H), 4.70 (1H), 6.10 (1H), 6.56 (1H), 6.86-6.94 (3H), 7.31-7.44 (4H), 7.63 (1 H), 7.64 (1 H), 8.43 (1 H) ppm.

### Example 51

### N-(1-cyanocyclobutyl)-4-{6-(3-fluorophenoxy)-8-[(3,3,3-trifluoropropyl)amino]imidazo[1,2-a]pyridin-3-yl}-2-methylbenzamide

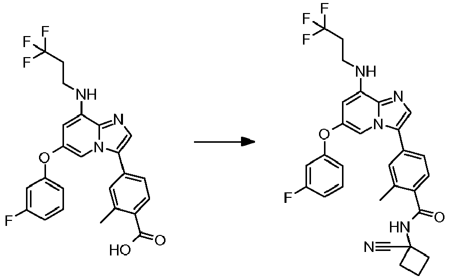

20 mg (42 µmol) 4-{6-(3-fluorophenoxy)-8-[(3,3,3-trifluoropropyl)amino]imidazo[1,2-a]pyridin-3-yl}-2-methylbenzoic acid which was prepared according to intermediate example 8a were transformed in analogy to example 8 using 1-aminocyclobutanecarbonitrile to give after working up and purification 13.7 mg (56%) of the title compound.
¹H-NMR (CDCl₃): δ= 2.11-2.36 (2H), 2.43-2.58 (4H), 2.53 (3H), 2.83-2.95 (2H), 3.56 (2H), 5.49 (1H), 6.00 (1H), 6.23 (1H), 6.72 (1H), 6.77-6.83 (2H), 7.28 (1H), 7.39 (1 H), 7.41 (1 H), 7.51 (1 H), 7.57 (1 H), 7.60 (1 H) ppm.

### Example 52

### N-cyclopropyl-4-{6-(3-methoxyphenoxy)-8-[(3,3,3-trifluoropropyl)amino]imidazo[1,2-a]pyridin-3-yl}-2-methylbenzamide

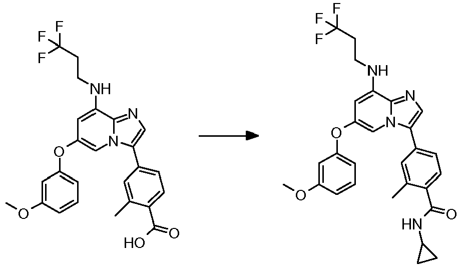

20 mg (41 µmol) 4-{6-(3-methoxyphenoxy)-8-[(3,3,3-trifluoropropyl)amino]imidazo[1,2-a]pyridin-3-yl}-2-methylbenzoic acid which was prepared according to intermediate example 52a were transformed in analogy to example 8 using cyclopropanamine to give after working up and purification 13.1 mg (58%) of the title compound.
¹H-NMR (CDCl₃): δ= 0.62 (2H), 0.89 (2H), 2.42-2.60 (2H), 2.48 (3H), 2.91 (1 H), 3.54 (2H), 3.78 (3H), 5.48 (1H), 5.92 (1H), 6.02 (1H), 6.54-6.68 (3H), 7.21 (1H), 7.33 (1 H), 7.36 (1 H), 7.41 (1 H), 7.54 (1 H), 7.58 (1 H) ppm.

### Example 52a

### 4-{6-(3-Methoxyphenoxy)-8-[(3,3,3-trifluoropropyl)amino]imidazo[1,2-a]pyridin-3-yl}-2-methylbenzoic acid

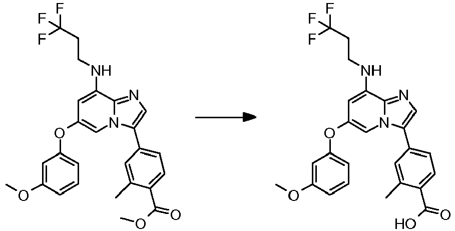

231 mg (95 µmol) methyl 4-{6-(3-methoxyphenoxy)-8-[(3,3,3-trifluoropropyl)amino]imidazo[1,2-a]pyridin-3-yl}-2-methylbenzoate which was prepared according to intermediate example 52b were transformed in analogy to intermediate example 8b to give after working up and purification 211 mg (99%) of the title compound.

### Example 52b

### Methyl 4-{6-(3-methoxyphenoxy)-8-[(3,3,3-trifluoropropyl)amino]imidazo[1,2-a]pyridin-3-yl}-2-methylbenzoate

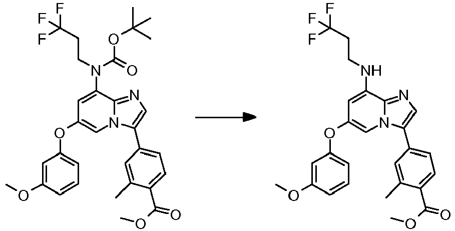

536 mg (894µmol) methyl 4-{8-[(tert-butoxycarbonyl)(3,3,3-trifluoropropyl)amino]-6-(3-methoxyphenoxy)imidazo[1,2-a]pyridin-3-yl}-2-methylbenzoate which was prepared according to intermediate example 52c were transformed in analogy to example 7 to give after working up and purification 236 mg (53%) of the title compound.

### Example 52c

### Methyl 4-{8-[(tert-butoxycarbonyl)(3,3,3-trifluoropropyl)amino]-6-(3-methoxyphenoxy)imidazo[1,2-a]pyridin-3-yl}-2-methylbenzoate

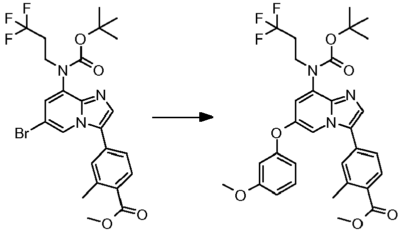

1.00 g (1,80 mmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to example 6-1 using 3-methoxyphenol to give after working up and purification 536 mg (50%) of the title compound.

### Example 53

### N-(1-cyanocyclopropyl)-4-{6-(3-methoxyphenoxy)-8-[(3,3,3-trifluoropropyl)amino]imidazo[1,2-a]pyridin-3-yl}-2-methylbenzamide

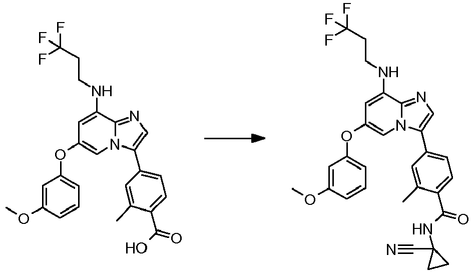

35 mg (72 µmol) 4-{6-(3-methoxyphenoxy)-8-[(3,3,3-trifluoropropyl)amino]imidazo[1,2-a]pyridin-3-yl}-2-methylbenzoic acid which was prepared according to intermediate example 52a were transformed in analogy to example 8 using 1-cyanocyclopropanaminium chloride to give after working up and purification 15.1 mg (36%) of the title compound.
¹H-NMR (CDCl₃): δ= 1.37 (2H), 1.66 (2H), 2.43-2.59 (2H), 2.50 (3H), 3.55 (2H), 3.78 (3H), 5.44 (1 H), 6.03 (1 H), 6.42 (1 H), 6.54-6.68 (3H), 7.22 (1 H), 7.35 (1 H), 7.39 (1 H), 7.42 (1 H), 7.54 (1 H), 7.57 (1 H) ppm.

### Example 54

### 4-{6-(3-Methoxyphenoxy)-8-[(3,3,3-trifluoropropyl)amino]imidazo[1,2-a]pyridin-3-yl}-2-methyl-N-(1-methylcyclopropyl)benzamide

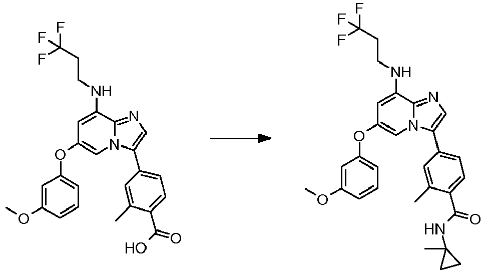

20 mg (41 µmol) 4-{6-(3-methoxyphenoxy)-8-[(3,3,3-trifluoropropyl)amino]imidazo[1,2-a]pyridin-3-yl}-2-methylbenzoic acid which was prepared according to intermediate example 52a were transformed in analogy to example 8 using 1-methylcyclopropanaminium chloride to give after working up and purification 13.0 mg (56%) of the title compound.
¹H-NMR (CDCl₃): δ= 0.76 (2H), 0.86 (2H), 1.52 (3H), 2.42-2.59 (2H), 2.47 (3H), 3.55 (2H), 3.78 (3H), 5.46 (1H), 6.02 (1H), 6.08 (1H), 6.55-6.68 (3H), 7.22 (1H), 7.33 (1 H), 7.35 (1 H), 7.39 (1 H), 7.53 (1 H), 7.57 (1 H) ppm.

### Example 55

### 4-{6-(3-Methoxyphenoxy)-8-[(3,3,3-trifluoropropyl)amino]imidazo[1,2-a]pyridin-3-yl}-N,2-dimethylbenzamide

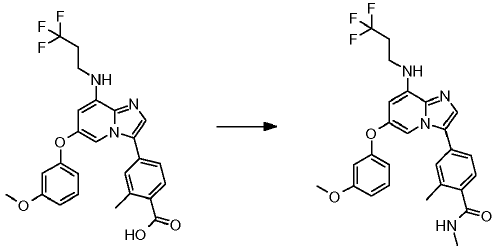

20 mg (41 µmol) 4-{6-(3-methoxyphenoxy)-8-[(3,3,3-trifluoropropyl)amino]imidazo[1,2-a]pyridin-3-yl}-2-methylbenzoic acid which was prepared according to intermediate example 52a were transformed in analogy to example 8 to give after working up and purification 12.5 mg (58%) of the title compound.
¹H-NMR (CDCl₃): δ= 2.42-2.59 (2H), 2.49 (3H), 3.02 (3H), 3.55 (2H), 3.78 (3H), 5.48 (1 H), 5.82 (1 H), 6.02 (1 H), 6.55-6.67 (3H), 7.22 (1 H), 7.35 (1 H), 7.37 (1 H), 7.45 (1 H), 7.55 (1 H), 7.59 (1 H) ppm.

### Example 56

### N-ethyl-4-{6-(3-methoxyphenoxy)-8-[(3,3,3-trifluoropropyl)amino]imidazo[1,2-a]pyridin-3-yl}-2-methylbenzamide

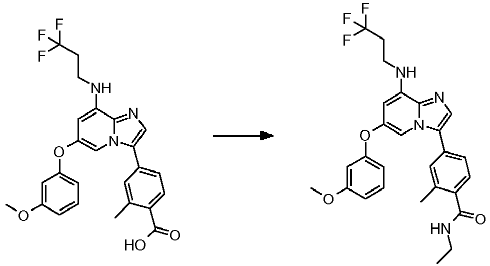

20 mg (41 µmol) 4-{6-(3-methoxyphenoxy)-8-[(3,3,3-trifluoropropyl)amino]imidazo[1,2-a]pyridin-3-yl}-2-methylbenzoic acid which was prepared according to intermediate example 52a were transformed in analogy to example 8 using ethanamine to give after working up and purification 12.6 mg (57%) of the title compound.
¹H-NMR (CDCl₃): δ= 1.26 (3H), 2.42-2.59 (2H), 2.49 (3H), 3.45-3.61 (4H), 3.78 (3H), 5.47 (1H), 5.77 (1 H), 6.02 (1H), 6.54-6.68 (3H), 7.22 (1H), 7.35 (1H), 7.37 (1H), 7.45 (1 H), 7.55 (1 H), 7.59 (1 H) ppm.

### Example 57

### N-(1-cyanocyclopropyl)-2-methyl-4-{6-phenoxy-8-[(3,3,3-trifluoropropyl)amino]imidazo[1,2-a]pyridin-3-yl}benzamide

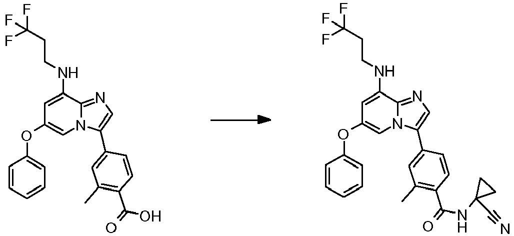

10 mg (22 µmol) 2-methyl-4-{6-phenoxy-8-[(3,3,3-trifluoropropyl)amino]imidazo[1,2-a]pyridin-3-yl}benzoic acid which was prepared according to intermediate example 33a were transformed in analogy to example 8 using 1-cyanocyclopropanaminium chloride to give after working up and purification 7.6 mg (63%) of the title compound.
¹H-NMR (CDCl₃): δ= 1.37 (2H), 1.65 (2H), 2.42-2.60 (2H), 2.49 (3H), 3.54 (2H), 5.45 (1 H), 6.03 (1 H), 6.51 (1 H), 7.01 (2H), 7.10 (1 H), 7.28-7.44 (5H), 7.54 (2H) ppm.

### Example 58

### 2-Methyl-N-(1-methylcyclopropyl)-4-{6-phenoxy-8-[(3,3,3-trifluoropropyl)amino]imidazo[1,2-a]pyridin-3-yl}benzamide

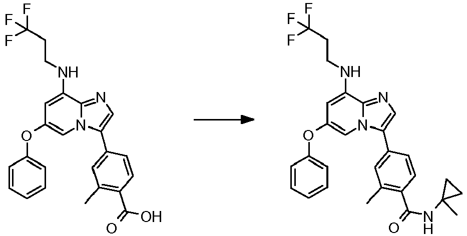

10 mg (22 µmol) 2-methyl-4-{6-phenoxy-8-[(3,3,3-trifluoropropyl)amino]imidazo[1,2-a]pyridin-3-yl}benzoic acid which was prepared according to intermediate example 33a were transformed in analogy to example 8 using 1-methylcyclopropanaminium chloride to give after working up and purification 8.3 mg (71%) of the title compound.
¹H-NMR (CDCl₃): δ= 0.75 (2H), 0.86 (2H), 1.51 (3H), 2.42-2.59 (2H), 2.46 (3H), 3.54 (2H), 5.46 (1 H), 6.02 (1 H), 6.08 (1 H), 7.01 (2H), 7.09 (1 H), 7.29-7.42 (5H), 7.53 (1 H), 7.55 (1 H) ppm.

### Example 59

### N,2-dimethyl-4-{6-phenoxy-8-[(3,3,3-trifluoropropyl)amino]imidazo[1,2-a]pyridin-3-yl}benzamide

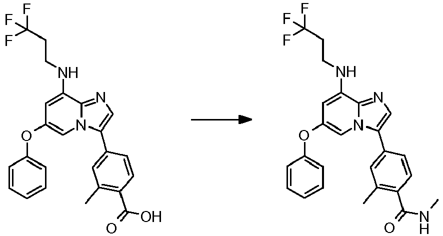

10 mg (22 µmol) 2-methyl-4-{6-phenoxy-8-[(3,3,3-trifluoropropyl)amino]imidazo[1,2-a]pyridin-3-yl}benzoic acid which was prepared according to intermediate example 33a were transformed in analogy to example 8 to give after working up and purification 6.7 mg (62%) of the title compound.
¹H-NMR (CDCl₃): δ= 2.42-2.59 (2H), 2.48 (3H), 3.01 (3H), 3.54 (2H), 5.46 (1 H), 5.83 (1 H), 6.02 (1 H), 7.01 (2H), 7.09 (1 H), 7.29-7.39 (4H), 7.44 (1 H), 7.54 (1 H), 7.57 (1 H) ppm.

### Example 60

### N-ethyl-2-methyl-4-{6-phenoxy-8-[(3,3,3-trifluoropropyl)amino]imidazo[1,2-a]pyridin-3-yl}benzamide

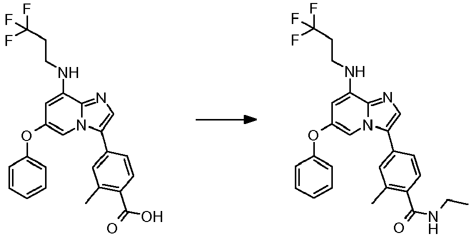

10 mg (22 µmol) 2-methyl-4-{6-phenoxy-8-[(3,3,3-trifluoropropyl)amino]imidazo[1,2-a]pyridin-3-yl}benzoic acid which was prepared according to intermediate example 33a were transformed in analogy to example 8 using ethanamine to give after working up and purification 6.4 mg (57%) of the title compound.
¹H-NMR (CDCl₃): δ= 1.26 (3H), 2.41-2.59 (2H), 2.48 (3H), 3.44-3.60 (4H), 5.46 (1H), 5.76 (1 H), 6.03 (1 H), 7.01 (2H), 7.09 (1 H), 7.29-7.39 (4H), 7.44 (1 H), 7.55 (1 H), 7.57 (1 H) ppm.

### Example 61

### Rel-4-{6-(5-fluoro-2-methylphenoxy)-8-[(3,3,3-trifluoropropyl)amino]imidazo[1,2-a]pyridin-3-yl}-2-methyl-N-[(1R,2R)-2-methylcyclopropyl]benzamide

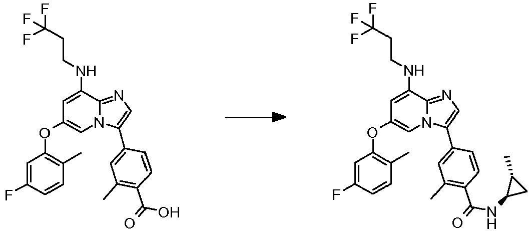

10 mg (21 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using rel-(1R,2R)-2-methylcyclopropanamine to give after working up and purification 9.6 mg (82%) of the title compound.
¹H-NMR (CDCl₃): δ= 0.66 (1H), 0.75 (1H), 0.98 (1H), 1.16 (3H), 2.28 (3H), 2.46-2.57 (2H), 2.48 (3H), 2.60 (1 H), 3.57 (2H), 5.47 (1 H), 5.86 (1 H), 5.99 (1 H), 6.57 (1 H), 6.73 (1 H), 7.17 (1 H), 7.32 (1 H), 7.34 (1 H), 7.40 (1 H), 7.46 (1 H), 7.54 (1 H) ppm.

### Example 62

### N-[1,1'-bi(cyclopropyl)-1-yl]-4-{6-(5-fluoro-2-methylphenoxy)-8-[(3,3,3-trifluoropropyl)amino]imidazo[1,2-a]pyridin-3-yl}-2-methylbenzamide

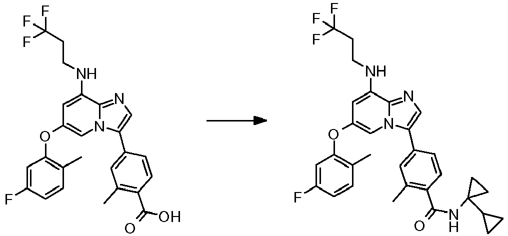

10 mg (21 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using 1,1'-bi(cyclopropyl)-1-aminium chloride to give after working up and purification 9.5 mg (78%) of the title compound.
¹H-NMR (CDCl₃): δ= 0.23 (2H), 0.49 (2H), 0.72 (2H), 0.82 (2H), 1.57 (1 H), 2.28 (3H), 2.46-2.59 (2H), 2.48 (3H), 3.57 (2H), 5.47 (1 H), 5.99 (1 H), 6.10 (1 H), 6.58 (1 H), 6.73 (1 H), 7.17 (1 H), 7.33 (1 H), 7.34 (1 H), 7.41 (1 H), 7.46 (1 H), 7.54 (1 H) ppm.

### Example 63

### N-(1-cyanocyclobutyl)-4-{6-(5-fluoro-2-methylphenoxy)-8-[(3,3,3-trifluoropropyl)amino]imidazo[1,2-a]pyridin-3-yl}-2-methylbenzamide

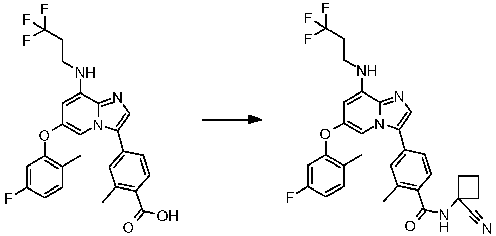

10 mg (21 µmol) 4-{6-(5-Fluoro-2-methylphenoxy)-8-[(3,3,3-trifluoropropyl)amino]imidazo[1,2-a]pyridin-3-yl}-2-methylbenzoic acid which was prepared according to intermediate example 19a were transformed in analogy to example 8 using 1-aminocyclobutanecarbonitrile to give after working up and purification 8.1 mg (66%) of the title compound.
¹H-NMR (CDCl₃): δ= 2.11-2.35 (2H), 2.28 (3H), 2.43-2.59 (4H), 2.52 (3H), 2.89 (2H), 3.57 (2H), 5.48 (1H), 6.00 (1H), 6.25 (1H), 6.58 (1H), 6.74 (1H), 7.18 (1H), 7.36 (1 H), 7.38 (1 H), 7.46 (1 H), 7.49 (1 H), 7.55 (1 H) ppm.

### Example 64

### N-cyclopropyl-2-methyl-4-{6-[4-(trifluoromethoxy)phenoxy]-8-[(3,3,3-trifluoropropyl)amino]imidazo[1,2-a]pyridin-3-yl}benzamide

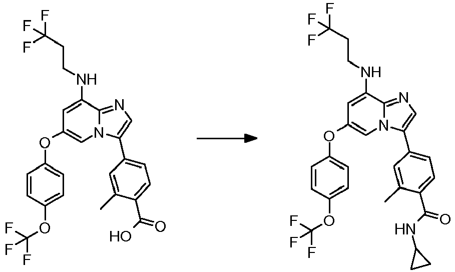

20 mg (37 µmol) 2-methyl-4-{6-[4-(trifluoromethoxy)phenoxy]-8-[(3,3,3-trifluoropropyl)amino]imidazo[1,2-a]pyridin-3-yl}benzoic acid which was prepared according to intermediate example 64a were transformed in analogy to example 8 using cyclopropanamine to give after working up and purification 13.3 mg (59%) of the title compound.
¹H-NMR (CDCl₃): δ= 0.62 (2H), 0.90 (2H), 2.43-2.59 (2H), 2.49 (3H), 2.92 (1H), 3.56 (2H), 5.49 (1 H), 5.89 (1 H), 5.98 (1 H), 7.00 (2H), 7.18 (2H), 7.33 (1 H), 7.35 (1 H), 7.42 (1 H), 7.55 (1 H), 7.56 (1 H) ppm.

### Example 64a

### 2-Methyl-4-{6-[4-(trifluoromethoxy)phenoxy]-8-[(3,3,3-trifluoropropyl)amino]imidazo[1,2-a]pyridin-3-yl}benzoic acid

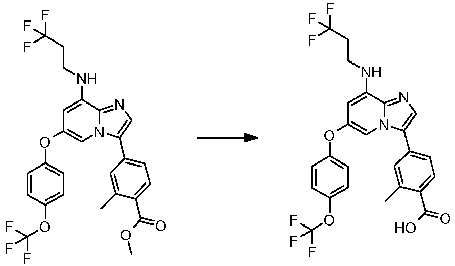

233 mg (421 µmol) methyl 2-methyl-4-{6-[4-(trifluoromethoxy)phenoxy]-8-[(3,3,3-trifluoropropyl)amino]imidazo[1,2-a]pyridin-3-yl}benzoate which was prepared according to intermediate example 64b were transformed in analogy to intermediate example 8b to give after working up 234 mg (max. 100%) of the title compound.

### Example 64b

### Methyl 2-methyl-4-{6-[4-(trifluoromethoxy)phenoxy]-8-[(3,3,3-trifluoropropyl)amino]imidazo[1,2-a]pyridin-3-yl}benzoate

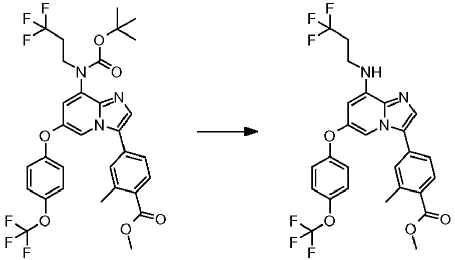

292 mg (447 µmol) methyl 4-{8-[(tert-butoxycarbonyl)(3,3,3-trifluoropropyl)amino]-6-[4-(trifluoromethoxy)phenoxy]imidazo[1,2-a]pyridin-3-yl}-2-methylbenzoate which was prepared according to intermediate example 64c were transformed in analogy to example 7 to give after working up and purification 239 mg (97%) of the title compound.

### Example 64c

### Methyl 4-{8-[(tert-butoxycarbonyl)(3,3,3-trifluoropropyl)amino]-6-[4-(trifluoromethoxy)phenoxy]imidazo[1,2-a]pyridin-3-yl}-2-methylbenzoate

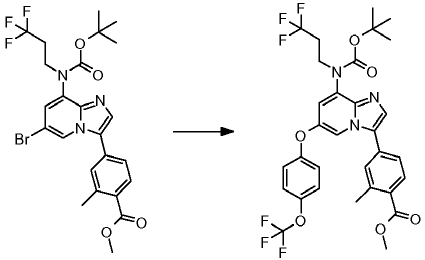

750 mg (1.35 mmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to example 6-1 using 4-(trifluoromethoxy)phenol to give after working up and purification 296 mg (34%) of the title compound.

### Example 65

### N-(1-cyanocyclopropyl)-2-methyl-4-{6-[4-(trifluoromethoxy)phenoxy]-8-[(3,3,3-trifluoropropyl)amino]imidazo[1,2-a]pyridin-3-yl}benzamide

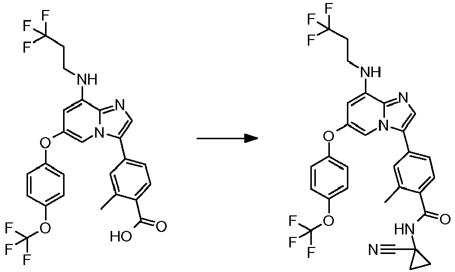

30 mg (56 µmol) 2-methyl-4-{6-[4-(trifluoromethoxy)phenoxy]-8-[(3,3,3-trifluoropropyl)amino]imidazo[1,2-a]pyridin-3-yl}benzoic acid which was prepared according to intermediate example 64a were transformed in analogy to example 8 using 1-cyanocyclopropanaminium chloride to give after working up and purification 19.1 mg (54%) of the title compound.
¹H-NMR (CDCl₃): δ= 1.37 (2H), 1.66 (2H), 2.42-2.63 (2H), 2.50 (3H), 3.55 (2H), 5.50 (1H), 5.99 (1H), 6.45 (1H), 7.00 (2H), 7.18 (2H), 7.32-7.47 (3H), 7.55 (1H), 7.57 (1 H) ppm.

### Example 66

### 2-Methyl-N-(1-methylcyclopropyl)-4-{6-[4-(trifluoromethoxy)phenoxy]-8-[(3,3,3-trifluoropropyl)amino]imidazo[1,2-a]pyridin-3-yl}benzamide

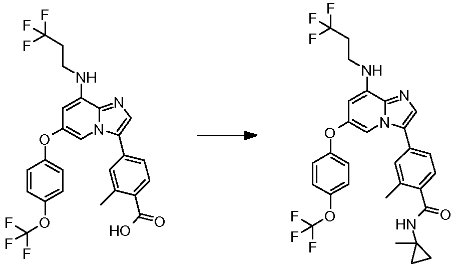

20 mg (37 µmol) 2-methyl-4-{6-[4-(trifluoromethoxy)phenoxy]-8-[(3,3,3-trifluoropropyl)amino]imidazo[1,2-a]pyridin-3-yl}benzoic acid which was prepared according to intermediate example 64a were transformed in analogy to example 8 using 1-methylcyclopropanaminium chloride to give after working up and purification 13.8 mg (60%) of the title compound.
¹H-NMR (CDCl₃): δ= 0.76 (2H), 0.86 (2H), 1.52 (3H), 2.43-2.59 (2H), 2.47 (3H), 3.56 (2H), 5.50 (1H), 5.98 (1H), 6.06 (1H), 7.01 (2H), 7.18 (2H), 7.32 (1H), 7.34 (1H), 7.39 (1 H), 7.55 (1 H), 7.56 (1 H) ppm.

### Example 67

### N,2-dimethyl-4-{6-[4-(trifluoromethoxy)phenoxy]-8-[(3,3,3-trifluoropropyl)amino]imidazo[1,2-a]pyridin-3-yl}benzamide

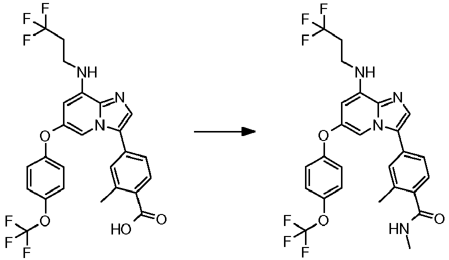

20 mg (37 µmol) 2-methyl-4-{6-[4-(trifluoromethoxy)phenoxy]-8-[(3,3,3-trifluoropropyl)amino]imidazo[1,2-a]pyridin-3-yl}benzoic acid which was prepared according to intermediate example 64a were transformed in analogy to example 8 to give after working up and purification 11.8 mg (55%) of the title compound. ¹H-NMR (CDCl₃): δ= 2.43-2.59 (2H), 2.49 (3H), 3.02 (3H), 3.56 (2H), 5.50 (1H), 5.78 (1 H), 5.99 (1 H), 7.01 (2H), 7.18 (2H), 7.35 (1 H), 7.36 (1 H), 7.46 (1 H), 7.56 (1 H), 7.58 (1 H) ppm.

### Example 68

### N-ethyl-2-methyl-4-{6-[4-(trifluoromethoxy)phenoxy]-8-[(3,3,3-trifluoropropyl)amino]imidazo[1,2-a]pyridin-3-yl}benzamide

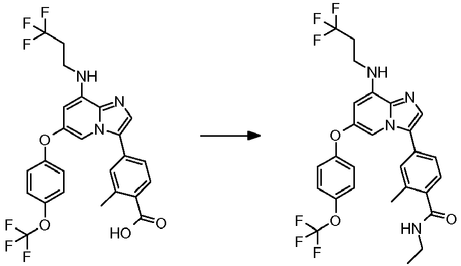

20 mg (37 µmol) 2-methyl-4-{6-[4-(trifluoromethoxy)phenoxy]-8-[(3,3,3-trifluoropropyl)amino]imidazo[1,2-a]pyridin-3-yl}benzoic acid which was prepared according to intermediate example 64a were transformed in analogy to example 8 using ethanamine to give after working up and purification 12.6 mg (57%) of the title compound.
¹H-NMR (CDCl₃): δ= 1.26 (3H), 2.42-2.60 (2H), 2.49 (3H), 3.45-3.61 (4H), 5.50 (1H), 5.75 (1 H), 5.99 (1 H), 7.01 (2H), 7.18 (2H), 7.35 (1 H), 7.36 (1 H), 7.46 (1 H), 7.56 (1 H), 7.58 (1 H) ppm.

### Example 69

### N-cyclopropyl-4-{6-(2-fluoro-4-methoxyphenoxy)-8-[(3,3,3-trifluoropropyl)amino]imidazo[1,2-a]pyridin-3-yl}-2-methylbenzamide

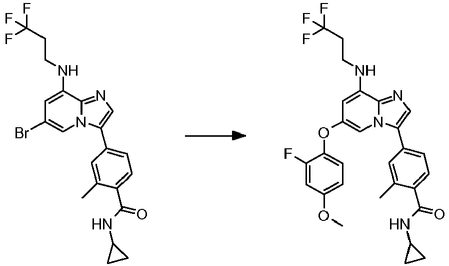

20 mg (37 µmol) 4-{6-bromo-8-[(3,3,3-trifluoropropyl)amino]imidazo[1,2-a]pyridin-3-yl}-N-cyclopropyl-2-methylbenzamide which was prepared according to intermediate example 42a were transformed in analogy to example 42 using 2-fluoro-4-methoxyphenol to give after working up and purification 17.8 mg (8%) of the title compound.
¹H-NMR (CDCl₃): δ= 0.62 (2H), 0.90 (2H), 2.41-2.60 (2H), 2.46 (3H), 2.92 (1 H), 3.57 (2H), 3.80 (3H), 5.43 (1 H), 5.91 (1 H), 6.06 (1 H), 6.64 (1 H), 6.75 (1 H), 7.02 (1 H), 7.29 (1H), 7.31 (1H), 7.36 (1H), 7.39 (1H), 7.50 (1H) ppm.

### Example 70

### N-(1-cyanocyclopropyl)-4-{6-(2,3-difluorophenoxy)-8-[(3,3,3-trifluoropropyl)amino]imidazo[1,2-a]pyridin-3-yl}-2-methylbenzamide

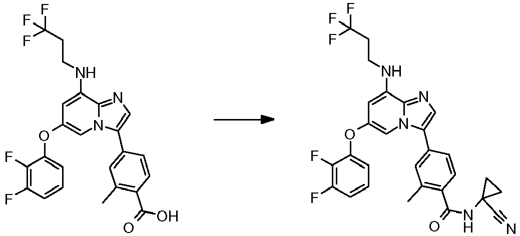

10 mg (20 µmol) 4-{6-(2,3-difluorophenoxy)-8-[(3,3,3-trifluoropropyl)amino]imidazo[1,2-a]pyridin-3-yl}-2-methylbenzoic acid which was prepared according to intermediate example 30a were transformed in analogy to example 8 using 1-cyanocyclopropanaminium chloride to give after working up and purification 8.4 mg (71%) of the title compound.
¹H-NMR (DMSO-d6): δ= 1.23 (2H), 1.52 (2H), 2.35 (3H), 2.55-2.67 (2H), 3.46 (2H), 6.18 (1H), 6.60 (1H), 6.94 (1H), 7.07-7.18 (2H), 7.42 (1H), 7.47 (1H), 7.48 (1H), 7.67 (1H), 7.70 (1H), 9.17 (1H) ppm.

### Example 71

### 4-{6-(2,3-Difluorophenoxy)-8-[(3,3,3-trifluoropropyl)amino]imidazo[1,2-a]pyridin-3-yl}-2-methyl-N-(1-methylcyclopropyl)benzamide

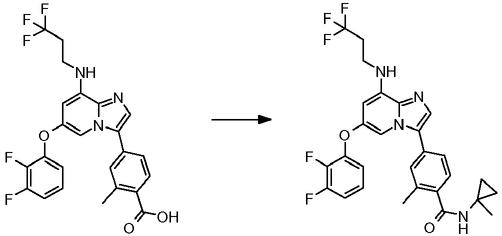

10 mg (20 µmol) 4-{6-(2,3-difluorophenoxy)-8-[(3,3,3-trifluoropropyl)amino]imidazo[1,2-a]pyridin-3-yl}-2-methylbenzoic acid which was prepared according to intermediate example 30a were transformed in analogy to example 8 using 1-methylcyclopropanaminium chloride to give after working up and purification 8.9 mg (76%) of the title compound.
¹H-NMR (CDCl₃): δ= 0.76 (2H), 0.87 (2H), 1.52 (3H), 2.45-2.59 (2H), 2.47 (3H), 3.57 (2H), 5.49 (1H), 6.03 (1H), 6.07 (1H), 6.77 (1H), 6.90-7.03 (2H), 7.32 (1H), 7.34 (1 H), 7.39 (1 H), 7.52-7.56 (2H) ppm.

### Example 72

### 4-{6-(2,3-Difluorophenoxy)-8-[(3,3,3-trifluoropropyl)amino]imidazo[1,2-a]pyridin-3-yl}-N,2-dimethylbenzamide

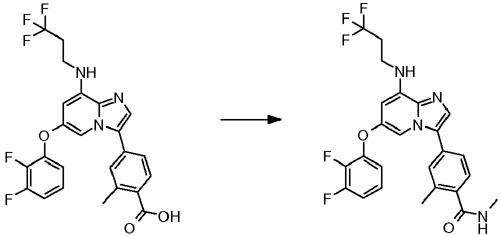

10 mg (20 µmol) 4-{6-(2,3-difluorophenoxy)-8-[(3,3,3-trifluoropropyl)amino]imidazo[1,2-a]pyridin-3-yl}-2-methylbenzoic acid which was prepared according to intermediate example 30a were transformed in analogy to example 8 to give after working up and purification 9.2 mg (85%) of the title compound.
¹H-NMR (CDCl₃): δ= 2.46-2.58 (2H), 2.49 (3H), 3.02 (3H), 3.57 (2H), 5.51 (1 H), 5.80 (1H), 6.03 (1H), 6.77 (1H), 6.90-7.03 (2H), 7.34 (1H), 7.36 (1H), 7.45 (1H), 7.55 (1 H), 7.56 (1 H) ppm.

### Example 73

### 4-{6-(2,3-Difluorophenoxy)-8-[(3,3,3-trifluoropropyl)amino]imidazo[1,2-a]pyridin-3-yl}-N-ethyl-2-methylbenzamide

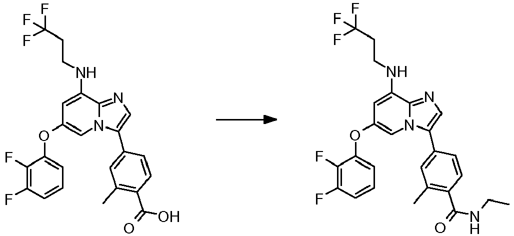

10 mg (20 µmol) 4-{6-(2,3-difluorophenoxy)-8-[(3,3,3-trifluoropropyl)amino]imidazo[1,2-a]pyridin-3-yl}-2-methylbenzoic acid which was prepared according to intermediate example 30a were transformed in analogy to example 8 using ethanamine to give after working up and purification 8.8 mg (79%) of the title compound.
¹H-NMR (CDCl₃): δ= 1.26 (3H), 2.46-2.58 (2H), 2.49 (3H), 3.51 (2H), 3.57 (2H), 5.49 (1H), 5.75 (1H), 6.03 (1H), 6.78 (1H), 6.90-7.03 (2H), 7.34 (1H), 7.36 (1H), 7.45 (1 H), 7.55 (1 H), 7.56 (1 H) ppm.

### Example 74

### N-cyclopropyl-2-methyl-4-{6-[3-(trifluoromethoxy)phenoxy]-8-[(3,3,3-trifluoropropyl)amino]imidazo[1,2-a]pyridin-3-yl}benzamide

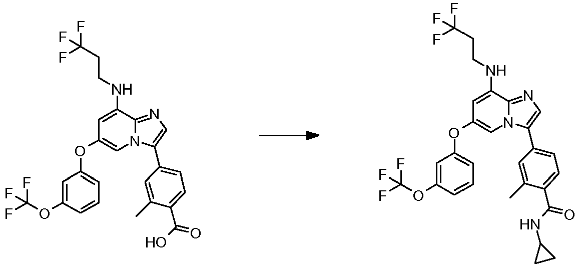

20 mg (37 µmol) 2-methyl-4-{6-[3-(trifluoromethoxy)phenoxy]-8-[(3,3,3-trifluoropropyl)amino]imidazo[1,2-a]pyridin-3-yl}benzoic acid which was prepared according to intermediate example 74a were transformed in analogy to example 8 using cyclopropanamine to give after working up and purification 12.2 mg (54%) of the title compound.
¹H-NMR (CDCl₃): δ= 0.62 (2H), 0.89 (2H), 2.43-2.59 (2H), 2.49 (3H), 2.92 (1H), 3.56 (2H), 5.52 (1H), 5.89 (1H), 5.98 (1H), 6.88 (1H, 6.90-6.98 (2H), 7.31 (1H), 7.34 (1 H), 7.36 (1 H), 7.43 (1 H), 7.56 (1 H), 7.59 (1 H) ppm.

### Example 74a

### 2-Methyl-4-{6-[3-(trifluoromethoxy)phenoxy]-8-[(3,3,3-trifluoropropyl)amino]imidazo[1,2-a]pyridin-3-yl}benzoic acid

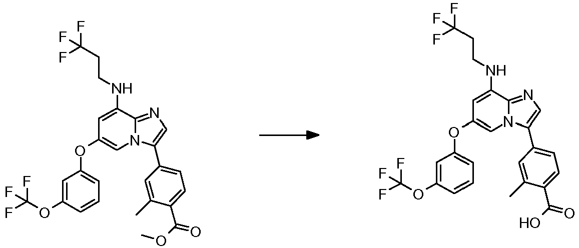

231 mg (417 µmol) methyl 2-methyl-4-{6-[3-(trifluoromethoxy)phenoxy]-8-[(3,3,3-trifluoropropyl)amino]imidazo[1,2-a]pyridin-3-yl}benzoate which was prepared according to intermediate example 74b were transformed in analogy to intermediate example 8b to give after working up 233 mg (max. 100%) of the title compound.

### Example 74b

### Methyl 2-methyl-4-{6-[3-(trifluoromethoxy)phenoxy]-8-[(3,3,3-trifluoropropyl)amino]imidazo[1,2-a]pyridin-3-yl}benzoate

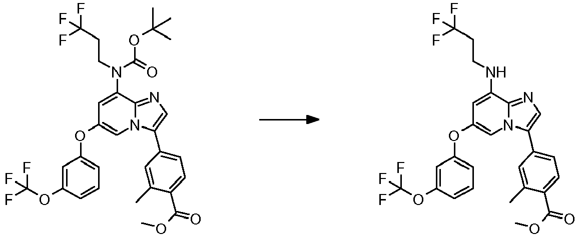

300 mg (459 µmol) methyl 4-{8-[(tert-butoxycarbonyl)(3,3,3-trifluoropropyl)amino]-6-[3-(trifluoromethoxy)phenoxy]imidazo[1,2-a]pyridin-3-yl}-2-methylbenzoate which was prepared according to intermediate example 74c were transformed in analogy to example 7 to give after working up and purification 237 mg (93%) of the title compound.

### Example 74c

### Methyl 4-{8-[(tert-butoxycarbonyl)(3,3,3-trifluoropropyl)amino]-6-[3-(trifluoromethoxy)phenoxy]imidazo[1,2-a]pyridin-3-yl}-2-methylbenzoate

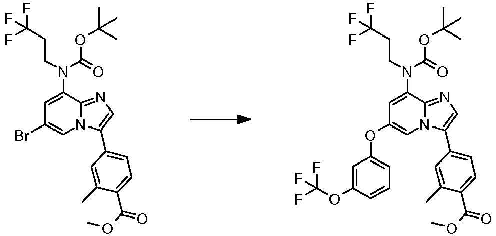

750 mg (1.35 mmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to example 6-1 using 3-(trifluoromethoxy)phenol to give after working up and purification 301 mg (32%) of the title compound.

### Example 75

### N-(1-cyanocyclopropyl)-2-methyl-4-{6-[3-(trifluoromethoxy)phenoxy]-8-[(3,3,3-trifluoropropyl)amino]imidazo[1,2-a]pyridin-3-yl}benzamide

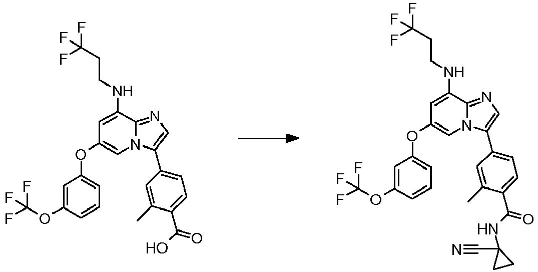

30 mg (56 µmol) 2-methyl-4-{6-[3-(trifluoromethoxy)phenoxy]-8-[(3,3,3-trifluoropropyl)amino]imidazo[1,2-a]pyridin-3-yl}benzoic acid which was prepared according to intermediate example 74a were transformed in analogy to example 8 using 1-cyanocyclopropanaminium chloride to give after working up and purification 18.5 mg (52%) of the title compound.
¹H-NMR (CDCl₃): δ= 1.37 (2H), 1.66 (2H), 2.43-2.58 (2H), 2.51 (3H), 3.55 (2H), 5.51 (1H), 5.99 (1H), 6.45 (1H), 6.85-7.01 (3H), 7.30-7.46 (4H), 7.56 (1H), 7.60 (1H) ppm.

### Example 76

### 2-Methyl-N-(1-methylcyclopropyl)-4-{6-[3-(trifluoromethoxy)phenoxy]-8-[(3,3,3-trifluoropropyl)amino]imidazo[1,2-a]pyridin-3-yl}benzamide

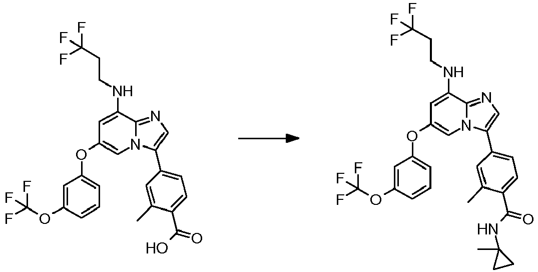

20 mg (37 µmol) 2-methyl-4-{6-[3-(trifluoromethoxy)phenoxy]-8-[(3,3,3-trifluoropropyl)amino]imidazo[1,2-a]pyridin-3-yl}benzoic acid which was prepared according to intermediate example 74a were transformed in analogy to example 8 using 1-methylcyclopropanaminium chloride to give after working up and purification 13.0 mg (56%) of the title compound.
¹H-NMR (CDCl₃): δ= 0.76 (2H), 0.87 (2H), 1.52 (3H), 2.43-2.59 (2H), 2.48 (3H), 3.56 (2H), 5.50 (1H), 5.98 (1H), 6.06 (1H), 6.86-6.98 (3H), 7.29-7.43 (4H), 7.55 (1H), 7.59 (1 H) ppm.

### Example 77

### N-ethyl-2-methyl-4-{6-[3-(trifluoromethoxy)phenoxy]-8-[(3,3,3-trifluoropropyl)amino]imidazo[1,2-a]pyridin-3-yl}benzamide

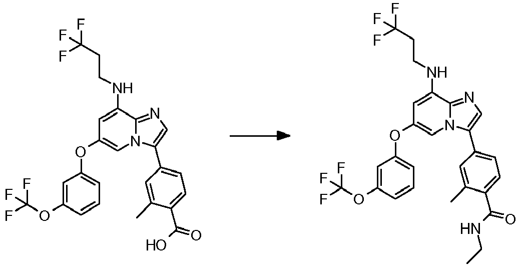

20 mg (37 µmol) 2-methyl-4-{6-[3-(trifluoromethoxy)phenoxy]-8-[(3,3,3-trifluoropropyl)amino]imidazo[1,2-a]pyridin-3-yl}benzoic acid which was prepared according to intermediate example 74a were transformed in analogy to example 8 using ethanamine to give after working up and purification 13.0 mg (59%) of the title compound.
¹H-NMR (CDCl₃): δ= 1.26 (3H), 2.42-2.59 (2H), 2.50 (3H), 3.45-3.62 (4H), 5.52 (1H), 5.76 (1H), 5.98 (1H), 6.86-7.00 (3H), 7.29-7.40 (3H), 7.46 (1H), 7.56 (1H), 7.61 (1H) ppm.

### Example 78

### N,2-dimethyl-4-{6-[3-(trifluoromethoxy)phenoxy]-8-[(3,3,3-trifluoropropyl)amino]imidazo[1,2-a]pyridin-3-yl}benzamide

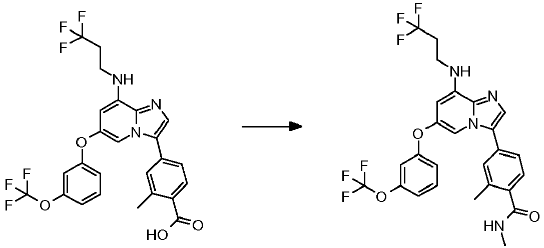

20 mg (37 µmol) 2-methyl-4-{6-[3-(trifluoromethoxy)phenoxy]-8-[(3,3,3-trifluoropropyl)amino]imidazo[1,2-a]pyridin-3-yl}benzoic acid which was prepared according to intermediate example 74a were transformed in analogy to example 8 to give after working up and purification 12.5 mg (58%) of the title compound.
¹H-NMR (CDCl₃): δ= 2.43-2.59 (2H), 2.50 (3H), 3.02 (3H), 3.56 (2H), 5.51 (1H), 5.79 (1H), 5.98 (1 H), 6.86-6.98 (3H), 7.29-7.40 (3H), 7.46 (1H), 7.56 (1H), 7.61 (1H) ppm.

### Example 79

### N-cyclopropyl-4-{6-(2-methoxyphenyl)-8-[(3,3,3-trifluoropropyl)amino]imidazo[1,2-a]pyridin-3-yl}-2-methylbenzamide

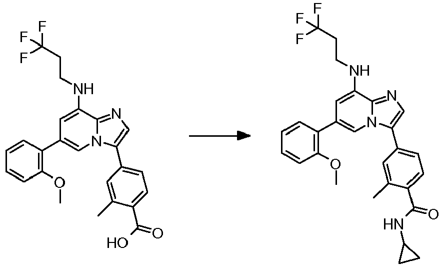

50 mg (107 µmol) 4-{6-(2-methoxyphenyl)-8-[(3,3,3-trifluoropropyl)amino]imidazo[1,2-a]pyridin-3-yl}-2-methylbenzoic acid which was prepared according to intermediate example 79a were transformed in analogy to example 8 using cyclopropanamine to give after working up and purification 41.1 mg (72%) of the title compound.
¹H-NMR (CDCl₃): δ= 0.62 (2H), 0.89 (2H), 2.49-2.62 (2H), 2.51 (3H), 2.92 (1 H), 3.64 (2H), 3.85 (3H), 5.31 (1 H), 5.97 (1 H), 6.38 (1 H), 7.01 (1 H), 7.04 (1 H), 7.30-7.45 (5H), 7.53 (1H), 7.88 (1H) ppm.

### Example 79a

### 4-{6-(2-Methoxyphenyl)-8-[(3,3,3-trifluoropropyl)amino]imidazo[1,2-a]pyridin-3-yl}-2-methylbenzoic acid

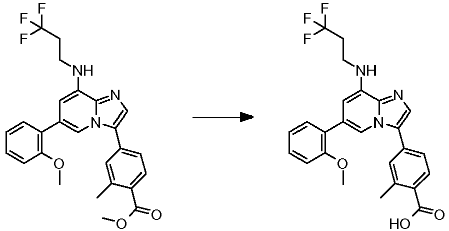

628 mg (max. 1.30 mmol) methyl 4-{6-(2-methoxyphenyl)-8-[(3,3,3-trifluoropropyl)amino]imidazo[1,2-a]pyridin-3-yl}-2-methylbenzoate which was prepared according to intermediate example 79b were transformed in analogy to intermediate example 8b to give after working up and purification 337 mg (55%) of the title compound.

### Example 79b

### Methyl 4-{6-(2-methoxyphenyl)-8-[(3,3,3-trifluoropropyl)amino]imidazo[1,2-a]pyridin-3-yl}-2-methylbenzoate

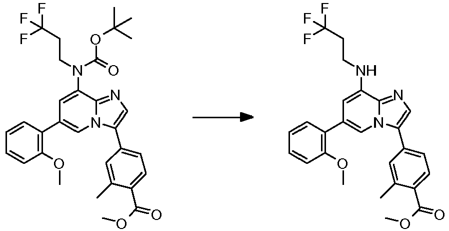

759 mg (1.30 mmol) methyl 4-{8-[(tert-butoxycarbonyl)(3,3,3-trifluoropropyl)amino]-6-(2-methoxyphenyl)imidazo[1,2-a]pyridin-3-yl}-2-methylbenzoate which was prepared according to intermediate example 79c were transformed in analogy to example 7 to give after working up 872 mg of crude title compound.

### Example 79c

### Methyl 4-{8-[(tert-butoxycarbonyl)(3,3,3-trifluoropropyl)amino]-6-(2-methoxyphenyl)imidazo[1,2-a]pyridin-3-yl}-2-methylbenzoate

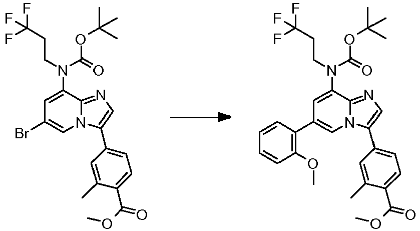

A mixture comprising 750 g (1.35 mmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d, 410 mg (2-methoxyphenyl)boronic acid, 18 mL n-propanol, 1.35 mL of an aqueous 2M potassium carbonate solution, 1.0 mL 1-methyl-2-pyrrolidon, 35.4 mg triphenylphosphine, and 94.9 mg bis(triphenylphosphine)palladium was stirred at 120°C for 2 hours under microwave irradiation. The solution was cooled, water added and extracted with ethylacetate and methanol. The organic phase was washed with brine and dried over sodium sulfate. After filtration and removal of solvent the residue (915 mg) was used without further purification.

### Example 80

### 4-{6-(2-Methoxyphenyl)-8-[(3,3,3-trifluoropropyl)amino]imidazo[1,2-a]pyridin-3-yl}-2-methyl-N-(1-methylcyclopropyl)benzamide

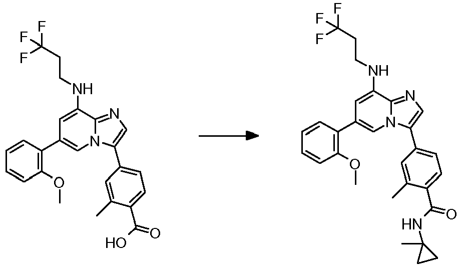

50 mg (107 µmol) 4-{6-(2-methoxyphenyl)-8-[(3,3,3-trifluoropropyl)amino]imidazo[1,2-a]pyridin-3-yl}-2-methylbenzoic acid which was prepared according to intermediate example 79a were transformed in analogy to example 8 using 1-methylcyclopropanaminium chloride to give after working up and purification 43.2 mg (74%) of the title compound.
¹H-NMR (CDCl₃): δ= 0.76 (2H), 0.87 (2H), 1.52 (3H), 2.48-2.63 (2H), 2.49 (3H), 3.64 (2H), 3.85 (3H), 5.30 (1 H), 6.12 (1 H), 6.38 (1 H), 7.01 (1 H), 7.04 (1 H), 7.30-7.44 (5H), 7.53 (1 H), 7.87 (1 H) ppm.

### Example 81

### 4-{6-(2-Methoxyphenyl)-8-[(3,3,3-trifluoropropyl)amino]imidazo[1,2-a]pyridin-3-yl}-N,2-dimethylbenzamide

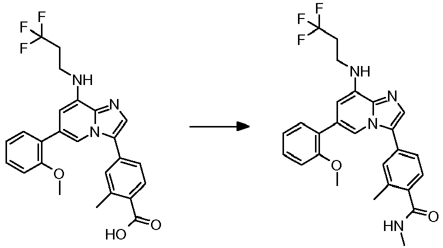

50 mg (107 µmol) 4-{6-(2-methoxyphenyl)-8-[(3,3,3-trifluoropropyl)amino]imidazo[1,2-a]pyridin-3-yl}-2-methylbenzoic acid which was prepared according to intermediate example 79a were transformed in analogy to example 8 to give after working up and purification 38.7 mg (72%) of the title compound.
¹H-NMR (CDCl₃): δ= 2.50-2.63 (2H), 2.51 (3H), 3.02 (3H), 3.64 (2H), 3.85 (3H), 5.30 (1 H), 5.86 (1 H), 6.38 (1 H), 7.01 (1 H), 7.05 (1 H), 7.31-7.48 (5H), 7.54 (1 H), 7.89 (1 H) ppm.

### Example 82

### N-ethyl-4-{6-(2-methoxyphenyl)-8-[(3,3,3-trifluoropropyl)amino]imidazo[1,2-a]pyridin-3-yl}-2-methylbenzamide

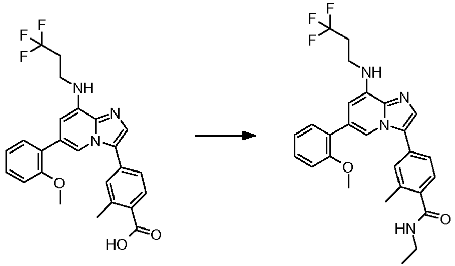

50 mg (107 µmol) 4-{6-(2-methoxyphenyl)-8-[(3,3,3-trifluoropropyl)amino]imidazo[1,2-a]pyridin-3-yl}-2-methylbenzoic acid which was prepared according to intermediate example 79a were transformed in analogy to example 8 using ethanamine to give after working up and purification 40.1 mg (72%) of the title compound.
¹H-NMR (CDCl₃): δ= 1.26 (3H), 2.50-2.62 (2H), 2.52 (3H), 3.51 (2H), 3.64 (2H), 3.85 (3H), 5.31 (1 H), 5.81 (1 H), 6.38 (1 H), 7.01 (1 H), 7.05 (1 H), 7.31-7.48 (5H), 7.55 (1 H), 7.89 (1 H) ppm.

### Example 83

### N-cyclopropyl-4-{6-[3-fluoro-4-(trifluoromethoxy)phenoxy]-8-[(3,3,3-trifluoropropyl)amino]imidazo[1,2-a]pyridin-3-yl}-2-methylbenzamide

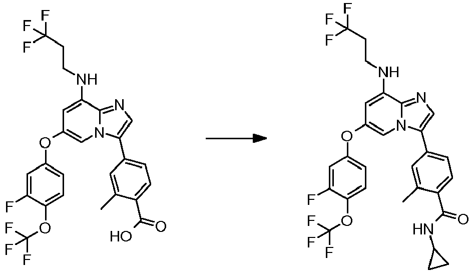

20 mg (36 µmol) 4-{6-[3-fluoro-4-(trifluoromethoxy)phenoxy]-8-[(3,3,3-trifluoropropyl)amino]imidazo[1,2-a]pyridin-3-yl}-2-methylbenzoic acid which was prepared according to intermediate example 83a were transformed in analogy to example 8 using cyclopropanamine to give after working up and purification 13.0 mg (58%) of the title compound.
¹H-NMR (CDCl₃): δ= 0.62 (2H), 0.90 (2H), 2.44-2.60 (2H), 2.50 (3H), 2.92 (1 H), 3.57 (2H), 5.53 (1H), 5.89 (1H), 5.96 (1H), 6.77 (1H)=, 6.83 (1H), 7.24 (1H), 7.34 (1H), 7.37 (1 H), 7.43 (1 H), 7.56 (1 H), 7.61 (1 H) ppm.

### Example 83a

### 4-{6-[3-fluoro-4-(trifluoromethoxy)phenoxy]-8-[(3,3,3-trifluoropropyl)amino]imidazo[1,2-a]pyridin-3-yl}-2-methylbenzoic acid

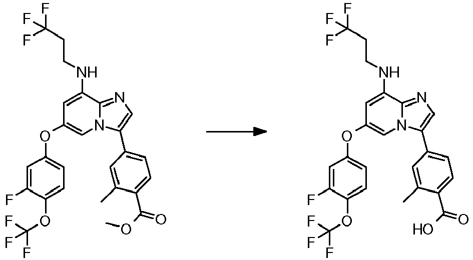

221 mg (387 µmol) methyl 4-{6-[3-fluoro-4-(trifluoromethoxy)phenoxy]-8-[(3,3,3-trifluoropropyl)amino]imidazo[1,2-a]pyridin-3-yl}-2-methylbenzoate which was prepared according to intermediate example 83b were transformed in analogy to intermediate example 8b to give after working up and purification 220 mg (max. 100%) of the title compound.

### Example 83b

### Methyl 4-{6-[3-fluoro-4-(trifluoromethoxy)phenoxy]-8-[(3,3,3-trifluoropropyl)amino]imidazo[1,2-a]pyridin-3-yl}-2-methylbenzoate

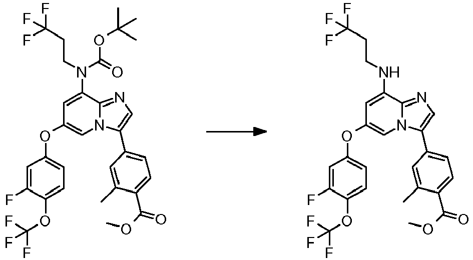

286 mg (426 µmol) methyl 4-{8-[(tert-butoxycarbonyl)(3,3,3-trifluoropropyl)amino]-6-[3-fluoro-4-(trifluoromethoxy)phenoxy]imidazo[1,2-a]pyridin-3-yl}-2-methylbenzoate which was prepared according to intermediate example 83c were transformed in analogy to example 7 to give after working up and purification 227 mg (93%) of the title compound.

### Example 83c

### Methyl 4-{8-[(tert-butoxycarbonyl)(3,3,3-trifluoropropyl)amino]-6-[3-fluoro-4-(trifluoromethoxy)phenoxy]imidazo[1,2-a]pyridin-3-yl}-2-methylbenzoate

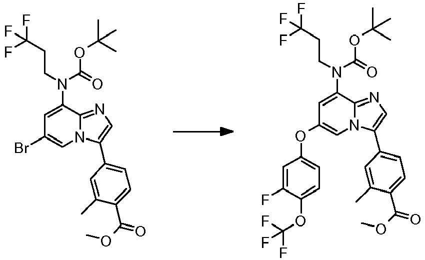

750 mg (1.35 mmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to example 6-1 using 3-fluoro-4-(trifluoromethoxy)phenol to give after working up and purification 293 mg (32%) of the title compound.

### Example 84

### N-(1-cyanocyclopropyl)-4-{6-[3-fluoro-4-(trifluoromethoxy)phenoxy]-8-[(3,3,3-trifluoropropyl)amino]imidazo[1,2-a]pyridin-3-yl}-2-methylbenzamide

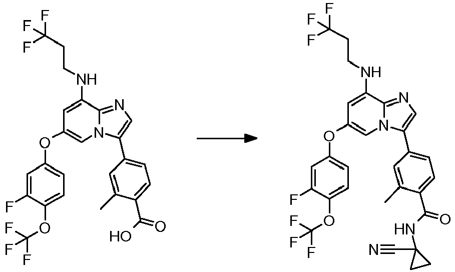

30 mg (54 µmol) 4-{6-[3-fluoro-4-(trifluoromethoxy)phenoxy]-8-[(3,3,3-trifluoropropyl)amino]imidazo[1,2-a]pyridin-3-yl}-2-methylbenzoic acid which was prepared according to intermediate example 83a were transformed in analogy to example 8 using 1-cyanocyclopropanaminium chloride to give after working up and purification 19.3 mg (55%) of the title compound.
¹H-NMR (CDCl₃): δ= 1.37 (2H), 1.67 (2H), 2.44-2.61 (2H), 2.52 (3H), 3.56 (2H), 5.53 (1H), 5.96 (1H), 6.43 (1H), 6.77 (1H), 6.83 (1H), 7.24 (1H), 7.36 (1H), 7.40 (1H), 7.44 (1 H), 7.57 (1 H), 7.61 (1 H) ppm.

### Example 85

### 4-{6-[3-Fluoro-4-(trifluoromethoxy)phenoxy]-8-[(3,3,3-trifluoropropyl)amino]imidazo[1,2-a]pyridin-3-yl}-2-methyl-N-(1-methylcyclopropyl)benzamide

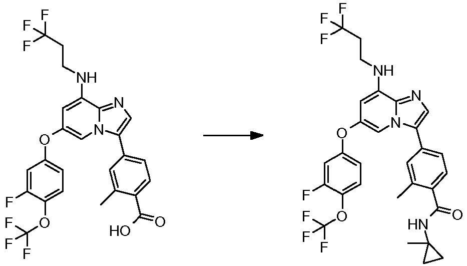

20 mg (36 µmol) 4-{6-[3-fluoro-4-(trifluoromethoxy)phenoxy]-8-[(3,3,3-trifluoropropyl)amino]imidazo[1,2-a]pyridin-3-yl}-2-methylbenzoic acid which was prepared according to intermediate example 83a were transformed in analogy to example 8 using 1-methylcyclopropanaminium chloride to give after working up and purification 13.2 mg (55%) of the title compound.
¹H-NMR (CDCl₃): δ= 0.76 (2H), 0.87 (2H), 1.52 (3H), 2.44-2.60 (2H), 2.48 (3H), 3.57 (2H), 5.53 (1 H), 5.96 (1 H), 6.06 (1H), 6.77 (1 H), 6.84 (1 H), 7.24 (1 H), 7.33 (1 H), 7.35 (1 H), 7.41 (1 H), 7.56 (1 H), 7.60 (1 H) ppm.

### Example 86

### 4-{6-[3-Fluoro-4-(trifluoromethoxy)phenoxy]-8-[(3,3,3-trifluoropropyl)amino]imidazo[1,2-a]pyridin-3-yl}-N,2-dimethylbenzamide

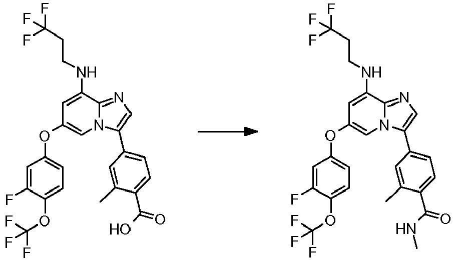

20 mg (36 µmol) 4-{6-[3-fluoro-4-(trifluoromethoxy)phenoxy]-8-[(3,3,3-trifluoropropyl)amino]imidazo[1,2-a]pyridin-3-yl}-2-methylbenzoic acid which was prepared according to intermediate example 83a were transformed in analogy to example 8 to give after working up and purification 12.3 mg (57%) of the title compound.
¹H-NMR (CDCl₃): δ= 2.45-2.60 (2H), 2.51 (3H), 3.02 (3H), 3.57 (2H), 5.53 (1H), 5.78 (1H), 5.96 (1H), 6.78 (1H), 6.84 (1H), 7.24 (1H), 7.36 (1H), 7.38 (1H), 7.47 (1H), 7.57 (1 H), 7.62 (1 H) ppm.

### Example 87

### N-ethyl-4-{6-[3-fluoro-4-(trifluoromethoxy)phenoxy]-8-[(3,3,3-trifluoropropyl)amino]imidazo[1,2-a]pyridin-3-yl}-2-methylbenzamide

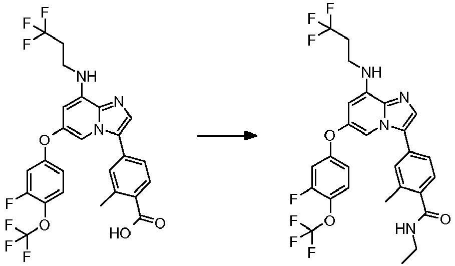

20 mg (36 µmol) 4-{6-[3-fluoro-4-(trifluoromethoxy)phenoxy]-8-[(3,3,3-trifluoropropyl)amino]imidazo[1,2-a]pyridin-3-yl}-2-methylbenzoic acid which was prepared according to intermediate example 83a were transformed in analogy to example 8 using ethanamine to give after working up and purification mg (%) of the title compound.
¹H-NMR (CDCl₃): δ= 1.26 (3H), 2.43-2.61 (2H), 2.50 (3H), 3.45-3.62 (4H), 5.53 (1H), 5.76 (1H), 5.96 (1H), 6.77 (1H), 6.84 (1H), 7.24 (1H), 7.36 (1H), 7.37 (1H), 7.47 (1 H), 7.57 (1 H), 7.62 (1 H) ppm.

### Example 88

### N,2-dimethyl-4-{6-(pyridin-4-yl)-8-[(3,3,3-trifluoropropyl)amino]imidazo[1,2-a]pyridin-3-yl}benzamide

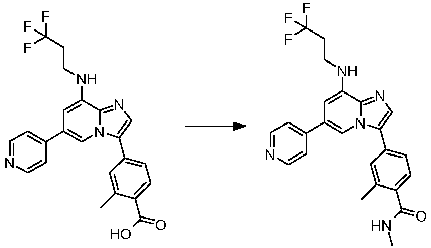

20 mg (45 µmol) 2-methyl-4-{6-(pyridin-4-yl)-8-[(3,3,3-trifluoropropyl)amino]imidazo[1,2-a]pyridin-3-yl}benzoic acid which was prepared according to intermediate example 88a were transformed in analogy to example 8 to give after working up and purification 10.5 mg (48%) of the title compound.
¹H-NMR (DMSO-d6): δ= 2.39 (3H), 2.62-2.79 (2H), 2.74 (3H), 3.60 (2H), 6.47 (1H), 6.54 (1H), 7.45 (1H), 7.52-7.61 (2H), 7.67 (1H), 7.72 (2H), 8.08 (1H), 8.22 (1H), 8.59 (2H) ppm.

### Example 88a

### 2-Methyl-4-{6-(pyridin-4-yl)-8-[(3,3,3-trifluoropropyl)amino]imidazo[1,2-a]pyridin-3-yl}benzoic acid

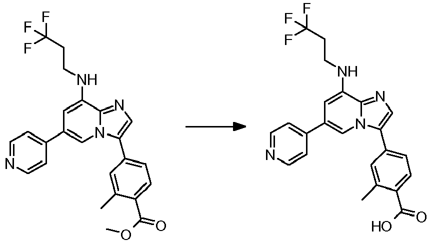

399 mg (878 µmol) methyl 2-methyl-4-{6-(pyridin-4-yl)-8-[(3,3,3-trifluoropropyl)amino]imidazo[1,2-a]pyridin-3-yl}benzoate which was prepared according to intermediate example 88b were transformed in analogy to intermediate example 8b to give after working up and purification 300 mg (78%) of the title compound.

### Example 88b

### Methyl 2-methyl-4-{6-(pyridin-4-yl)-8-[(3,3,3-trifluoropropyl)amino]imidazo[1,2-a]pyridin-3-yl}benzoate

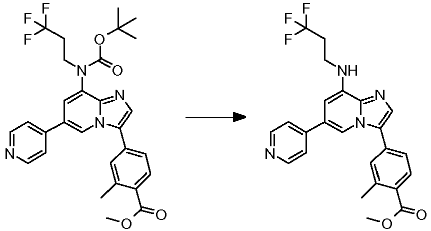

487 mg (878 µmol) methyl 4-{8-[(tert-butoxycarbonyl)(3,3,3-trifluoropropyl)amino]-6-(pyridin-4-yl)imidazo[1,2-a]pyridin-3-yl}-2-methylbenzoate which was prepared according to intermediate example 88c were transformed in analogy to example 7 to give after working up 532 mg (max. 100%) of the crude title compound.

### Example 88c

### Methyl 4-{8-[(tert-butoxycarbonyl)(3,3,3-trifluoropropyl)amino]-6-(pyridin-4-yl)imidazo[1,2-a]pyridin-3-yl}-2-methylbenzoate

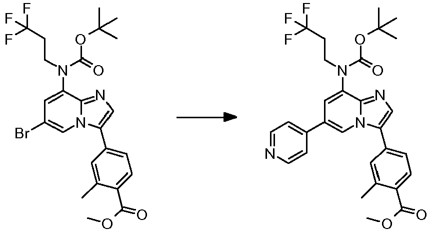

500 mg (899 µmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to intermediate example 79c using pyridin-4-ylboronic acid to give after working up 601 mg (max. 100%) of the crude title compound.

### Example 89

### N-ethyl-2-methyl-4-{6-(pyridin-4-yl)-8-[(3,3,3-trifluoropropyl)amino]imidazo[1,2-a]pyridin-3-yl}benzamide

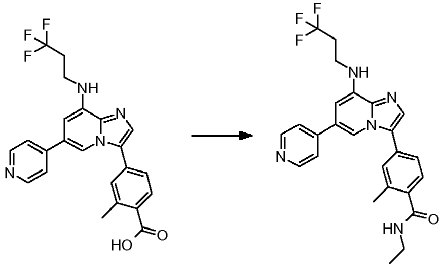

20 mg (45 µmol) 2-methyl-4-{6-(pyridin-4-yl)-8-[(3,3,3-trifluoropropyl)amino]imidazo[1,2-a]pyridin-3-yl}benzoic acid which was prepared according to intermediate example 88a were transformed in analogy to example 8 using ethanamine to give after working up and purification 9.7 mg (43%) of the title compound.
¹H-NMR (DMSO-d6): δ= 1.09 (3H), 2.39 (3H), 2.62-2.79 (2H), 3.24 (2H), 3.60 (2H), 6.47 (1H), 6.54 (1H), 7.44 (1H), 7.52-7.61 (2H), 7.66 (1H), 7.71 (2H), 8.08 (1H), 8.29 (1 H), 8.59 (2H) ppm.

### Example 90

### 2-Methyl-N-(1-methylcyclopropyl)-4-{6-(pyridin-4-yl)-8-[(3,3,3-trifluoropropyl)amino]imidazo[1,2-a]pyridin-3-yl}benzamide

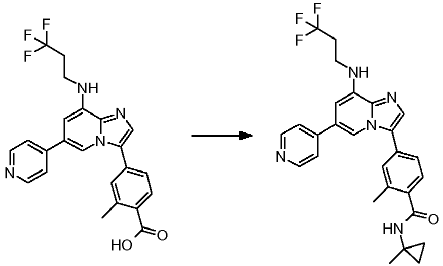

20 mg (45 µmol) 2-methyl-4-{6-(pyridin-4-yl)-8-[(3,3,3-trifluoropropyl)amino]imidazo[1,2-a]pyridin-3-yl}benzoic acid which was prepared according to intermediate example 88a were transformed in analogy to example 8 using 1-methylcyclopropanaminium chloride to give after working up and purification 7.1 mg (32%) of the title compound.
¹H-NMR (DMSO-d6): δ= 0.56 (2H), 0.70 (2H), 1.37 (3H), 2.36 (3H), 2.62-2.78 (2H), 3.60 (2H), 6.47 (1 H), 6.54 (1 H), 7.38 (1 H), 7.50-7.58 (2H), 7.65 (1 H), 7.71 (2H), 8.06 (1 H), 8.48 (1 H), 8.59 (2H) ppm.

### Example 91

### N-(1-cyanocyclopropyl)-2-methyl-4-{6-(pyridin-4-yl)-8-[(3,3,3-trifluoropropyl)amino]imidazo[1,2-a]pyridin-3-yl}benzamide

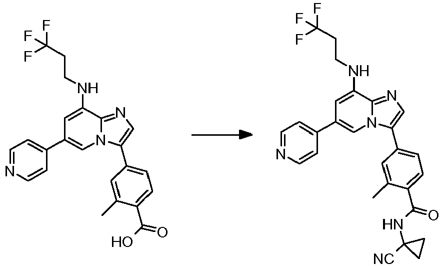

30 mg (68 µmol) 2-methyl-4-{6-(pyridin-4-yl)-8-[(3,3,3-trifluoropropyl)amino]imidazo[1,2-a]pyridin-3-yl}benzoic acid which was prepared according to intermediate example 88a were transformed in analogy to example 8 using 1-cyanocyclopropanaminium chloride to give after working up and purification 4.2 mg (11%) of the title compound.
¹H-NMR (DMSO-d6): δ= 1.25 (2H), 1.54 (2H), 2.40 (3H), 2.49-2.78 (2H), 3.60 (2H), 6.48 (1 H), 6.55 (1 H), 7.49 (1 H), 7.59 (1 H), 7.62 (1 H), 7.69 (1 H), 7.72 (2H), 8.08 (1 H), 8.59 (2H), 9.23 (1 H) ppm.

### Example 92

### N-cyclopropyl-2-methyl-4-{8-[(3,3,3-trifluoropropyl)amino]-6-vinylimidazo[1,2-a]pyridin-3-yl}benzamide

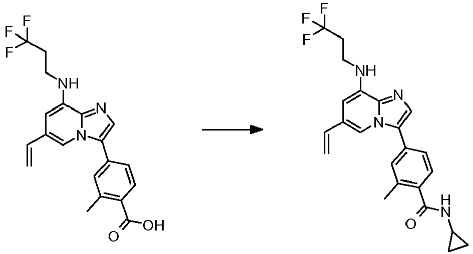

10 mg (26 µmol) 2-methyl-4-{8-[(3,3,3-trifluoropropyl)amino]-6-vinylimidazo[1,2-a]pyridin-3-yl}benzoic acid which was prepared according to intermediate example 92a were transformed in analogy to example 8 using cyclopropanamine to give after working up and purification 9.1 mg (83%) of the title compound.
¹H-NMR (CDCl₃): δ= 0.64 (2H), 0.91 (2H), 2.49-2.63 (2H), 2.52 (3H), 2.94 (1 H), 3.64 (2H), 5.28 (1H), 5.33 (1H), 5.68 (1H), 5.98 (1H), 6.32 (1H), 6.57 (1H), 7.36 (1H), 7.39 (1 H), 7.45 (1 H), 7.47 (1 H), 7.63 (1 H) ppm.

### Example 92a

### 2-Methyl-4-{8-[(3,3,3-trifluoropropyl)amino]-6-vinylimidazo[1,2-a]pyridin-3-yl}benzoic acid

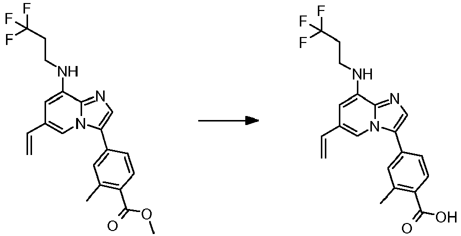

240 mg (595 µmol) methyl 2-methyl-4-{8-[(3,3,3-trifluoropropyl)amino]-6-vinylimidazo[1,2-a]pyridin-3-yl}benzoate which was prepared according to intermediate example 92b were transformed in analogy to intermediate example 8b to give after working up and purification 183.7 mg (75%) of the title compound.

### Example 92b

### Methyl 2-methyl-4-{8-[(3,3,3-trifluoropropyl)amino]-6-vinylimidazo[1,2-a]pyridin-3-yl}benzoate

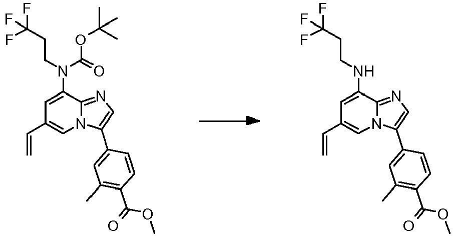

363 mg (721 µmol) methyl 4-{8-[(tert-butoxycarbonyl)(3,3,3-trifluoropropyl)amino]-6-vinylimidazo[1,2-a]pyridin-3-yl}-2-methylbenzoate which was prepared according to intermediate example 92c were transformed in analogy to example 7 to give after working up and purification 242.9 mg (79%) of the title compound.

### Example 92c

### Methyl 4-{8-[(tert-butoxycarbonyl)(3,3,3-trifluoropropyl)amino]-6-vinylimidazo[1,2-a]pyridin-3-yl}-2-methylbenzoate

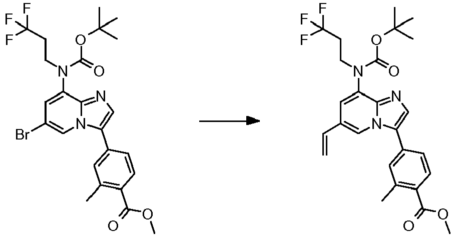

500 mg (899 µmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to intermediate example 79c using using 4,4,5,5-tetramethyl-2-vinyl-1,3,2-dioxaborolane to give after working up and purification 367 mg (81%) of the title compound.

### Example 93

### N,2-dimethyl-4-{8-[(3,3,3-trifluoropropyl)amino]-6-vinylimidazo[1,2-a]pyridin-3-yl}benzamide

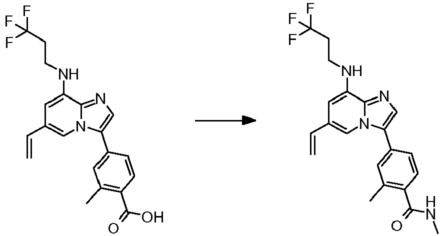

10 mg (26 µmol) 2-methyl-4-{8-[(3,3,3-trifluoropropyl)amino]-6-vinylimidazo[1,2-a]pyridin-3-yl}benzoic acid which was prepared according to intermediate example 92a were transformed in analogy to example 8 to give after working up and purification 8.1 mg (78%) of the title compound.
¹H-NMR (CDCl₃): δ= 2.49-2.63 (2H), 2.53 (3H), 3.04 (3H), 3.65 (2H), 5.29 (1 H), 5.34 (1 H), 5.69 (1 H), 5.88 (1 H), 6.33 (1 H), 6.58 (1 H), 7.38 (1 H), 7.40 (1 H), 7.47-7.53 (2H), 7.65 (1H) ppm.

### Example 94

### N-ethyl-2-methyl-4-{8-[(3,3,3-trifluoropropyl)amino]-6-vinylimidazo[1,2-a]pyridin-3-yl}benzamide

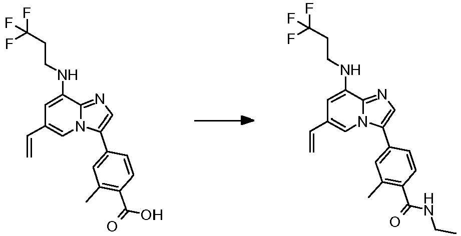

10 mg (26 µmol) 2-methyl-4-{8-[(3,3,3-trifluoropropyl)amino]-6-vinylimidazo[1,2-a]pyridin-3-yl}benzoic acid which was prepared according to intermediate example 92a were transformed in analogy to example 8 using ethanamine to give after working up and purification 9.4 mg (88%) of the title compound.
¹H-NMR (CDCl₃): δ= 1.28 (3H), 2.50-2.62 (2H), 2.53 (3H), 3.52 (2H), 3.65 (2H), 5.28 (1 H), 5.34 (1 H), 5.69 (1 H), 5.84 (1 H), 6.32 (1 H), 6.57 (1 H), 7.38 (1 H), 7.39 (1 H), 7.48 (1 H). 7.49 (1 H), 7.64 (1 H) ppm.

### Example 95

### 2-Methyl-N-(1-methylcyclopropyl)-4-{8-[(3,3,3-trifluoropropyl)amino]-6-vinylimidazo[1,2-a]pyridin-3-yl}benzamide

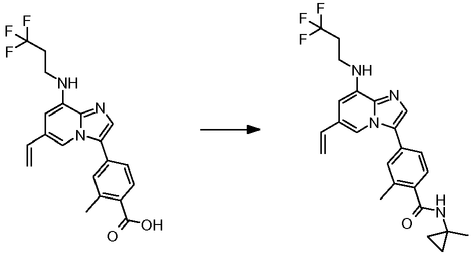

10 mg (26 µmol) 2-methyl-4-{8-[(3,3,3-trifluoropropyl)amino]-6-vinylimidazo[1,2-a]pyridin-3-yl}benzoic acid which was prepared according to intermediate example 92a were transformed in analogy to example 8 using 1-methylcyclopropanaminium chloride to give after working up and purification 6.8 mg (60%) of the title compound.
¹H-NMR (CDCl₃): δ= 0.77 (2H), 0.89 (2H), 1.53 (3H), 2.49-2.63 (2H), 2.51 (3H), 3.65 (2H), 5.29 (1 H), 3.32 (1 H), 5.68 (1 H), 6.13 (1 H), 6.32 (1 H), 6.57 (1 H), 7.36 (1 H), 7.38 (1 H), 7.44 (1 H), 7.48 (1 H), 7.63 (1 H) ppm.

### Example 96

### N-(1-cyanocyclopropyl)-2-methyl-4-{8-[(3,3,3-trifluoropropyl)amino]-6-vinylimidazo[1,2-a]pyridin-3-yl}benzamide

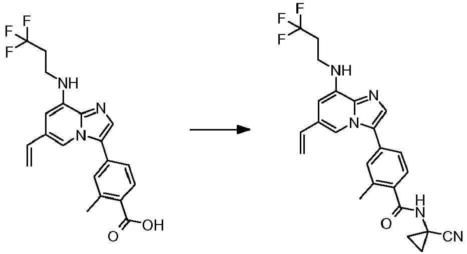

10 mg (26 µmol) 2-methyl-4-{8-[(3,3,3-trifluoropropyl)amino]-6-vinylimidazo[1,2-a]pyridin-3-yl}benzoic acid which was prepared according to intermediate example 92a were transformed in analogy to example 8 using 1-cyanocyclopropanaminium chloride to give after working up and purification 8.6 mg (74%) of the title compound.
¹H-NMR (CDCl₃): δ= 1.39 (2H), 1.67 (2H), 2.48-2.63 (2H), 2.52 (3H), 3.64 (2H), 5.28 (1 H), 5.31 (1 H), 5.69 (1 H), 6.33 (1 H), 6.56 (1 H), 6.66 (1 H), 7.36 (1 H), 7.40 (1 H), 7.42-7.48 (2H), 7.63 (1H) ppm.

### Example 97

### N-[rel-(1S,2S)-2-fluorocyclopropyl]-2-methyl-4-{8-[(3,3,3-trifluoropropyl)amino]-6-vinylimidazo[1,2-a]pyridin-3-yl}benzamide

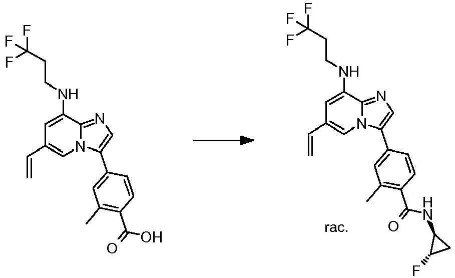

10 mg (26 µmol) 2-methyl-4-{8-[(3,3,3-trifluoropropyl)amino]-6-vinylimidazo[1,2-a]pyridin-3-yl}benzoic acid which was prepared according to intermediate example 92a were transformed in analogy to example 8 using rel-(1S,2S)-2-fluorocyclopropanaminium chloride to give after working up and purification 10.2 mg (89%) of the title compound.
¹H-NMR (CDCl₃): δ= 1.04 (1 H), 1.28 (1 H), 2.48-2.64 (2H), 2.54 (3H), 3.07 (1 H), 3.64 (2H), 4.78 (1H), 5.29 (1H), 5.33 (1 H), 5.69 (1 H), 6.10 (1 H), 6.32 (1 H), 6.57 (1 H), 7.38 (1H), 7.40 (1H), 7.48 (1H), 7.52 (1H), 7.64 (1H) ppm.

### Example 98

### 2-Methyl-N-[rel-(1S,2S)-2-methylcyclopropyl]-4-{8-[(3,3,3-trifluoropropyl)amino]-6-vinylimidazo[1,2-a]pyridin-3-yl}benzamide

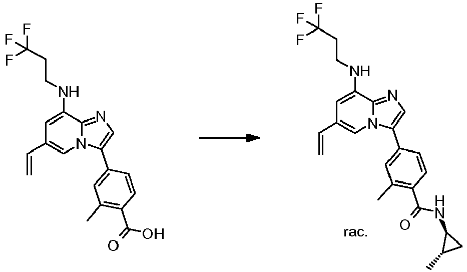

10 mg (26 µmol) 2-methyl-4-{8-[(3,3,3-trifluoropropyl)amino]-6-vinylimidazo[1,2-a]pyridin-3-yl}benzoic acid which was prepared according to intermediate example 92a were transformed in analogy to example 8 using rel-(1R,2R)-2-methylcyclopropanamine to give after working up and purification 10.0 mg (88%) of the title compound.
¹H-NMR (CDCl₃): δ= 0.67 (1H), 0.77 (1H), 0.99 (1H), 1.17 (3H), 2.45-2.66 (3H), 2.51 (3H), 3.64 (2H), 5.28 (1H), 5.34 (1H), 5.68 (1H), 5.96 (1H), 6.32 (1H), 6.57 (1H), 7.35 (1H), 7.37 (1H), 7.44 (1H), 7.47 (1H), 7.63 (1H) ppm.

### Example 99

### N-cyclopropyl-4-{6-ethyl-8-[(3,3,3-trifluoropropyl)amino]imidazo[1,2-a]pyridin-3-yl}-2-methylbenzamide

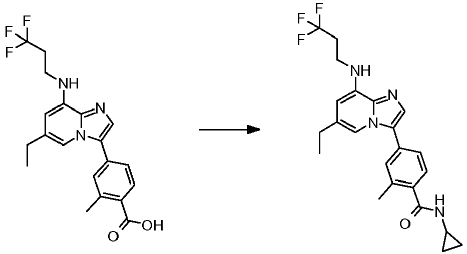

10 mg (26 µmol) 4-{6-ethyl-8-[(3,3,3-trifluoropropyl)amino]imidazo[1,2-a]pyridin-3-yl}-2-methylbenzoic acid which was prepared according to intermediate example 99a were transformed in analogy to example 8 using cyclopropanamine to give after working up and purification 6.8 mg (62%) of the title compound.
¹H-NMR (CDCl₃): δ= 0.64 (2H), 0.91 (2H), 1.24 (3H), 2.46-2.65 (4H), 2.52 (3H), 2.93 (1H), 3.60 (2H), 5.24 (1H), 5.97 (1H), 6.04 (1H), 7.37 (1H), 7.39 (1H), 7.45 (1H), 7.47 (1 H), 7.53 (1 H) ppm.

### Example 99a

### 4-{6-Ethyl-8-[(3,3,3-trifluoropropyl)amino]imidazo[1,2-a]pyridin-3-yl}-2-methylbenzoic acid

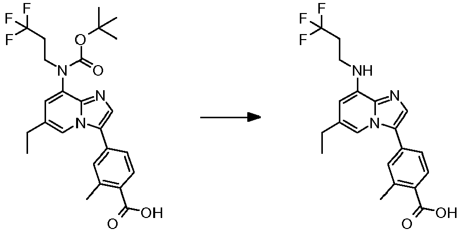

183 mg (372 µmol) 4-{8-[(tert-butoxycarbonyl)(3,3,3-trifluoropropyl)amino]-6-ethylimidazo[1,2-a]pyridin-3-yl}-2-methylbenzoic acid which was prepared according to intermediate example 99b were transformed in analogy to example 7 to give after working up and purification 150.8 mg (93%) of the title compound.

### Example 99b

### 4-{8-[(tert-Butoxycarbonyl)(3,3,3-trifluoropropyl)amino]-6-ethylimidazo[1,2-a]pyridin-3-yl}-2-methylbenzoic acid

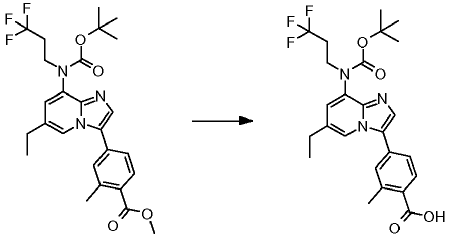

201 mg (398 µmol) methyl 4-{8-[(tert-butoxycarbonyl)(3,3,3-trifluoropropyl)amino]-6-ethylimidazo[1,2-a]pyridin-3-yl}-2-methylbenzoate which was prepared according to intermediate example 99c were transformed in analogy to intermediate example 8b to give after working up and purification 183 mg (93%) of the title compound.

### Example 99c

### Methyl 4-{8-[(tert-butoxycarbonyl)(3,3,3-trifluoropropyl)amino]-6-ethylimidazo[1,2-a]pyridin-3-yl}-2-methylbenzoate

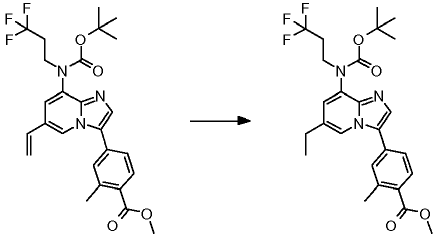

To a solution of 132 mg (262 µmol) methyl 4-{8-[(tert-butoxycarbonyl)(3,3,3-trifluoropropyl)amino]-6-vinylimidazo[1,2-a]pyridin-3-yl}-2-methylbenzoate which was prepared according to intermediate example 92c in 5 mL ethanol were added 13.9 mg palladium on charcoal (10%) and the mixture was vigorously stirred under an atmosphere of hydrogen for 1.5 hours at 23 °C. After filtration and removal of the solvent the residue was purified by chromatography to give 112.1 mg (80%) of the title compound.

### Example 100

### 4-{6-Ethyl-8-[(3,3,3-trifluoropropyl)amino]imidazo[1,2-a]pyridin-3-yl}-N,2-dimethylbenzamide

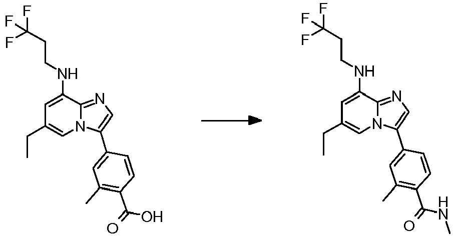

10 mg (26 µmol) 4-{6-ethyl-8-[(3,3,3-trifluoropropyl)amino]imidazo[1,2-a]pyridin-3-yl}-2-methylbenzoic acid which was prepared according to intermediate example 99a were transformed in analogy to example 8 to give after working up and purification 5.4 mg (52%) of the title compound.
¹H-NMR (CDCl₃): δ= 1.24 (3H), 2.48-2.65 (4H), 2.53 (3H), 3.04 (3H), 3.60 (2H), 5.25 (1 H), 5.85 (1 H), 6.05 (1 H), 7.39 (1 H), 7.40 (1 H), 7.49 (2H), 7.54 (1 H) ppm.

### Example 101

### N-ethyl-4-{6-ethyl-8-[(3,3,3-trifluoropropyl)amino]imidazo[1,2-a]pyridin-3-yl}-2-methylbenzamide

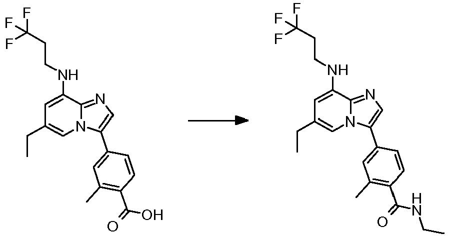

10 mg (26 µmol) 4-{6-ethyl-8-[(3,3,3-trifluoropropyl)amino]imidazo[1,2-a] pyridin-3-yl}-2-methylbenzoic acid which was prepared according to intermediate example 99a were transformed in analogy to example 8 using ethanamine to give after working up and purification 7.6 mg (71%) of the title compound.
¹H-NMR (CDCl₃): δ= 1.24 (3H), 1.27 (3H), 2.48-2.63 (4H), 2.53 (3H), 3.52 (2H), 3.60 (2H), 5.25 (1 H), 5.82 (1 H), 6.04 (1 H), 7.39 (1 H), 7.40 (1 H), 7.45-7.52 (2H), 7.54 (1 H) ppm.

### Example 102

### 4-{6-Ethyl-8-[(3,3,3-trifluoropropyl)amino]imidazo[1,2-a]pyridin-3-yl}-2-methyl-N-(1-methylcyclopropyl)benzamide

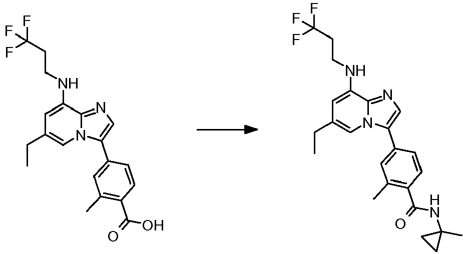

10 mg (26 µmol) 4-{6-ethyl-8-[(3,3,3-trifluoropropyl)amino]imidazo[1,2-a] pyridin-3-yl}-2-methylbenzoic acid which was prepared according to intermediate example 99a were transformed in analogy to example 8 using 1-methylcyclopropanaminium chloride to give after working up and purification 8.7 mg (77%) of the title compound.
¹H-NMR (CDCl₃): δ= 0.77 (2H), 0.88 (2H), 1.24 (3H), 1.53 (3H), 2.47-2.64 (4H), 2.50 (3H), 3.60 (2H), 5.26 (1 H), 6.04 (1 H), 6.15 (1 H), 7.36 (1 H), 7.38 (1 H), 7.43 (1 H), 7.47 (1 H), 7.52 (1 H) ppm.

### Example 103

### N-(1-cyanocyclopropyl)-4-{6-ethyl-8-[(3,3,3-trifluoropropyl)amino]imidazo[1,2-a]pyridin-3-yl}-2-methylbenzamide

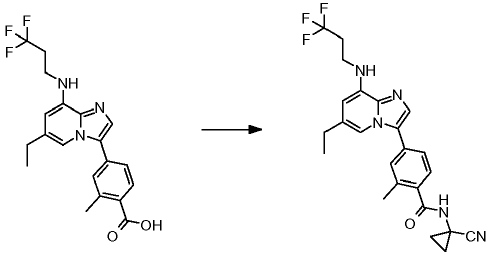

10 mg (26 µmol) 4-{6-ethyl-8-[(3,3,3-trifluoropropyl)amino]imidazo[1,2-a] pyridin-3-yl}-2-methylbenzoic acid which was prepared according to intermediate example 99a were transformed in analogy to example 8 using 1-cyanocyclopropanaminium chloride to give after working up and purification 9.5 mg (82%) of the title compound.
¹H-NMR (CDCl₃): δ= 1.24 (3H), 1.39 (2H), 1.67 (2H), 2.48-2.63 (4H), 2.53 (3H), 3.61 (2H), 5.28 (1 H), 6.05 (1 H), 6.64 (1 H), 7.36-7.48 (4H), 7.53 (1 H) ppm.

### Example 104

### 4-{6-Ethyl-8-[(3,3,3-trifluoropropyl)amino]imidazo[1,2-a]pyridin-3-yl}-N-[rel-(1S,2S)-2-fluorocyclopropyl]-2-methylbenzamide

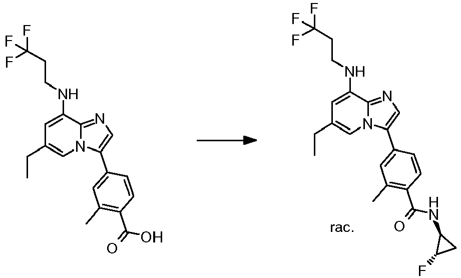

10 mg (26 µmol) 4-{6-ethyl-8-[(3,3,3-trifluoropropyl)amino]imidazo[1,2-a]pyridin-3-yl}-2-methylbenzoic acid which was prepared according to intermediate example 99a were transformed in analogy to example 8 using rel-(1S,2S)-2-fluorocyclopropanaminium chloride to give after working up and purification 9.3 mg (81%) of the title compound.
¹H-NMR (CDCl₃): δ= 1.04 (1 H), 1.19-1.34 (1 H), 1.25 (3H), 2.47-2.63 (4H), 2.54 (3H), 3.07 (1H), 3.61 (2H), 4.78 (1H), 5.28 (1H), 6.03-6.13 (2H), 7.38-7.56 (5H) ppm.

### Example 105

### 4-{6-Ethyl-8-[(3,3,3-trifluoropropyl)amino]imidazo[1,2-a]pyridin-3-yl}-2-methyl-N-[rel-(1S,2S)-2-methylcyclopropyl]benzamide

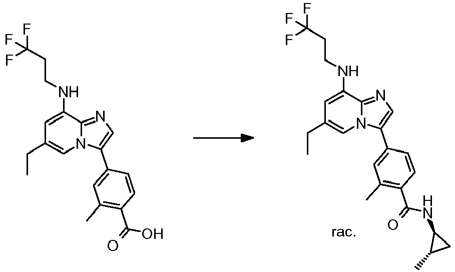

10 mg (26 µmol) 4-{6-ethyl-8-[(3,3,3-trifluoropropyl)amino]imidazo[1,2-a]pyridin-3-yl}-2-methylbenzoic acid which was prepared according to intermediate example 99a were transformed in analogy to example 8 using rel-(1R,2R)-2-methylcyclopropanamine to give after working up and purification 10.4 mg (92%) of the title compound.
¹H-NMR (CDCl₃): δ= 0.68 (1H), 0.77 (1H), 0.99 (1H), 1.17 (3H), 1.24 (3H), 2.46-2.65 (5H), 2.52 (3H), 3.60 (2H), 5.27 (1 H), 5.94 (1 H), 6.04 (1 H), 7.36 (1 H), 7.39 (1 H), 7.44 (1 H), 7.47 (1 H), 7.53 (1 H) ppm.

### Example 106

### 4-{6-(3-Fluoro-4-methoxyphenoxy)-8-[(3,3,3-trifluoropropyl)amino]imidazo[1,2-a]pyridin-3-yl}-N,2-dimethylbenzamide

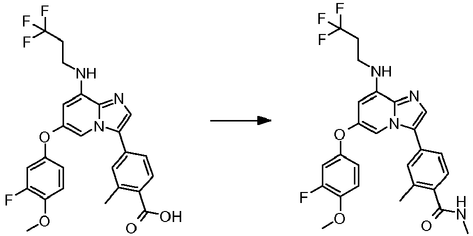

30 mg (60 µmol) 4-{6-(3-fluoro-4-methoxyphenoxy)-8-[(3,3,3-trifluoropropyl)amino]imidazo[1,2-a]pyridin-3-yl}-2-methylbenzoic acid which was prepared according to intermediate example 106a were transformed in analogy to example 8 to give after working up and purification 12.5 mg (39%) of the title compound.
¹H-NMR (CDCl₃): δ= 2.42-2.60 (2H), 2.49 (3H), 3.02 (3H), 3.55 (2H), 3.87 (3H), 5.47 (1H), 5.83 (1 H), 5.98 (1 H), 6.74 (1 H), 6.82 (1 H), 6.92 (1 H), 7.33 (1 H), 7.35 (1 H), 7.45 (1 H), 7.50 (1 H), 7.53 (1 H) ppm.

### Example 106a

### 4-{6-(3-Fluoro-4-methoxyphenoxy)-8-[(3,3,3-trifluoropropyl)amino]imidazo[1,2-a]pyridin-3-yl}-2-methylbenzoic acid

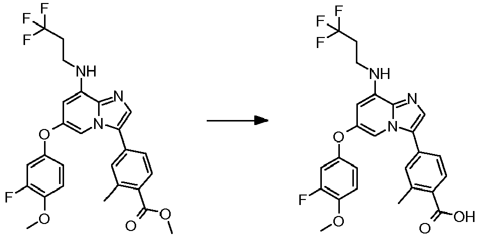

966 mg (1.87 mmol) methyl 4-{6-(3-fluoro-4-methoxyphenoxy)-8-[(3,3,3-trifluoropropyl)amino]imidazo[1,2-a]pyridin-3-yl}-2-methylbenzoate which was prepared according to intermediate example 106b were transformed in analogy to intermediate example 8b to give after working up and purification 201 mg (21 %) of the title compound.

### Example 106b

### Methyl 4-{6-(3-fluoro-4-methoxyphenoxy)-8-[(3,3,3-trifluoropropyl)amino]imidazo[1,2-a]pyridin-3-yl}-2-methylbenzoate

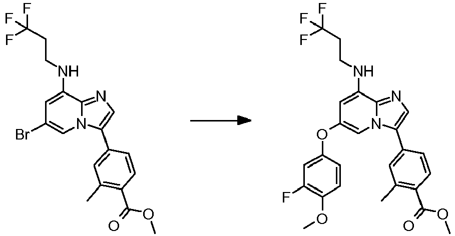

850 mg (1.86 mmol) methyl 4-{6-bromo-8-[(3,3,3-trifluoropropyl)amino]imidazo[1,2-a]pyridin-3-yl}-2-methylbenzoate which was prepared according to intermediate example 106c were transformed in analogy to intermediate example 6-1 using 3-fluoro-4-methoxyphenol to give after working up 2.3 g of a crude product that contained about 40% of the title compound and was used without further purification.

### Example 106c

### Methyl 4-{6-bromo-8-[(3,3,3-trifluoropropyl)amino]imidazo[1,2-a]pyridin-3-yl}-2-methylbenzoate

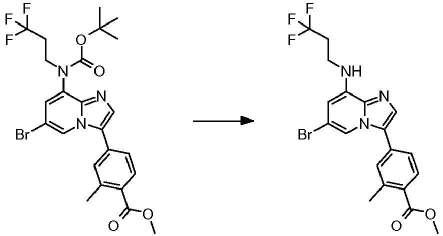

2.90 g (5.21 mmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to example 7 to give after working up and purification 2.33 mg (98%) of the title compound.

### Example 107

### N-ethyl-4-{6-(3-fluoro-4-methoxyphenoxy)-8-[(3,3,3-trifluoropropyl)amino]imidazo[1,2-a]pyridin-3-yl}-2-methylbenzamide

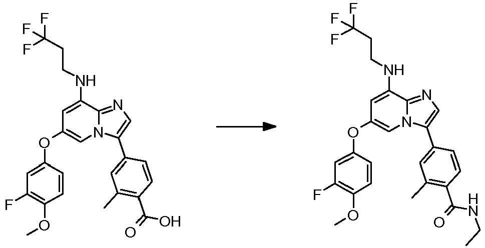

30 mg (60 µmol) 4-{6-(3-fluoro-4-methoxyphenoxy)-8-[(3,3,3-trifluoropropyl)amino]imidazo[1,2-a]pyridin-3-yl}-2-methylbenzoic acid which was prepared according to intermediate example 106a were transformed in analogy to example 8 using ethanamine to give after working up and purification 11.6 mg (35%) of the title compound.
¹H-NMR (CDCl₃): δ= 1.26 (3H), 2.42-2.59 (2H), 2.49 (3H), 3.45-3.61 (4H), 3.87 (3H), 5.48 (1 H), 5.78 (1 H), 5.99 (1 H), 6.74 (1 H), 6.83 (1 H), 6.91 (1 H), 7.34 (1 H), 7.35 (1 H), 7.45 (1 H), 7.50 (1 H), 7.54 (1 H) ppm.

mg (85%) of the title compound.
¹H-NMR (CDCl₃): δ= 0.62 (2H), 0.89 (2H), 2.44-2.58 (2H), 2.47 (3H), 2.92 (1H), 3.55 (2H), 5.04 (2H), 5.48 (1 H), 5.92 (1 H), 6.02 (1 H), 6.92-7.00 (4H), 7.29-7.48 (9H), 7.51 (1 H) ppm.

### Example 109a

### 4-{6-[4-(Benzyloxy)phenoxy]-8-[(3,3,3-trifluoropropyl)amino]imidazo[1,2-a] pyridin-3-yl}-2-methylbenzoic acid

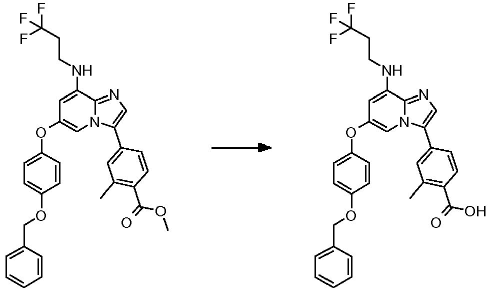

Methyl 4-{6-[4-(benzyloxy)phenoxy]-8-[(3,3,3-trifluoropropyl)amino]imidazo[1,2-a]pyridin-3-yl}-2-methylbenzoate which was prepared according to intermediate example 109b were transformed in analogy to intermediate example 8b to give after working up and purification 16.3 mg (3%) of the title compound.

### Example 109b

### Methyl 4-{6-[4-(benzyloxy)phenoxy]-8-[(3,3,3-trifluoropropyl)amino]imidazo[1,2-a]pyridin-3-yl}-2-methylbenzoate

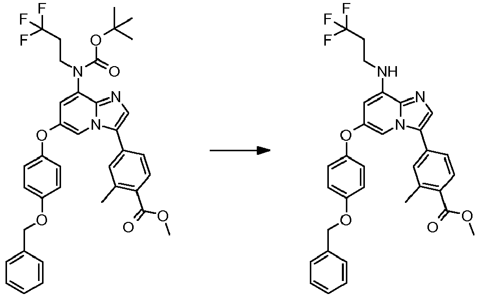

Methyl 4-{6-[4-(benzyloxy)phenoxy]-8-[(tert-butoxycarbonyl)(3,3,3-trifluoropropyl)amino]imidazo[1,2-a]pyridin-3-yl}-2-methylbenzoate which was prepared according to intermediate example 109c were transformed in analogy to example 7 to give after working up the title compound as crude product that was used without further purification.

### Example 109c

### Methyl 4-{6-[4-(benzyloxy)phenoxy]-8-[(tert-butoxycarbonyl)(3,3,3-trifluoropropyl)amino]imidazo[1,2-a]pyridin-3-yl}-2-methylbenzoate

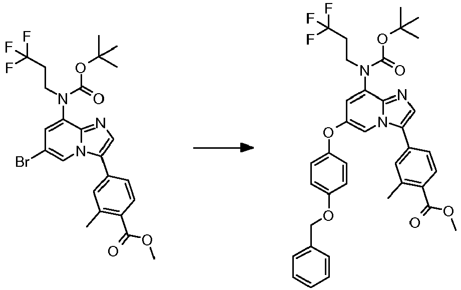

500 mg (899 µmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to example 6-1 using 4-(benzyloxy)phenol to give after working up the title compound that was used without further purification.

### Example 110

### N-cyclopropyl-4-{6-(4-hydroxyphenoxy)-8-[(3,3,3-trifluoropropyl)amino]imidazo[1,2-a]pyridin-3-yl}-2-methylbenzamide

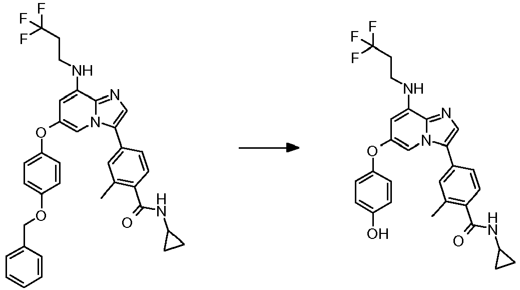

To a solution of 8.0 mg (13 µmol) 4-{6-[4-(benzyloxy)phenoxy]-8-[(3,3,3-trifluoropropyl)amino]imidazo[1,2-a]pyridin-3-yl}-N-cyclopropyl-2-methylbenzamide which was prepared according to example 109 in 254 µL ethanol were added 0.54 µL pyridine and 1 mg palladium on charcoal. The mixture was vigorously stirred under an atmosphere of hydrogen at 23°C for 16 hours. After filtration and removal of the solvents the residue was purified by chromatography to give 4.6 mg (68%) of the title compound.
¹H-NMR (CDCl₃): δ= 0.63 (2H), 0.89 (2H), 2.43-2.56 (2H), 2.46 (3H), 2.91 (1H), 3.54 (2H), 3.86 (1H), 5.46 (1H), 5.97 (1H), 6.04 (1H), 6.83 (2H), 6.93 (2H), 7.30 (1H), 7.32 (1 H), 7.39 (1 H), 7.41 (1 H), 7.52 (1 H) ppm.

### Example 111

### N-cyclopropyl-2-methyl-4-{6-[(1E)-prop-1-en-1-yl]-8-[(3,3,3-trifluoropropyl)amino]imidazo[1,2-a]pyridin-3-yl}benzamide

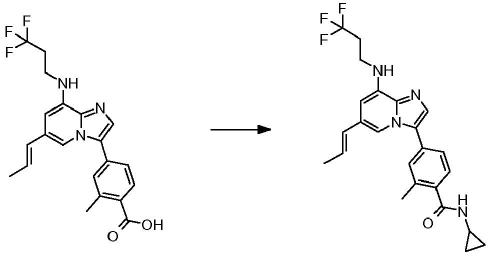

10 mg (25 µmol) 2-methyl-4-{6-[(1E)-prop-1-en-1-yl]-8-[(3,3,3-trifluoropropyl)amino]imidazo[1,2-a]pyridin-3-yl}benzoic acid which was prepared according to intermediate example 111 a were transformed in analogy to example 8 using cyclopropanamine to give after working up and purification 7.7 mg (70%) of the title compound.
¹H-NMR (CDCl₃): δ= 0.64 (2H), 0.91 (2H), 1.89 (3H), 2.48-2.62 (2H), 2.52 (3H), 2.94 (1 H), 3.63 (2H), 5.29 (1H), 5.97 (1 H), 6.11-6.33 (3H), 7.36 (1 H), 7.39 (1 H), 7.43-7.49 (2H), 7.57 (1 H) ppm.

### Example 111 a

### 2-Methyl-4-{6-[(1E)-prop-1-en-1-yl]-8-[(3,3,3-trifluoropropyl)amino]imidazo[1,2-a]pyridin-3-yl}benzoic acid

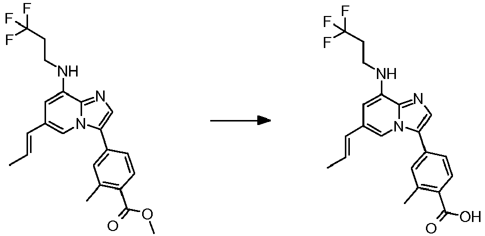

400 mg (958 µmol) methyl 2-methyl-4-{6-[(1E)-prop-1-en-1-yl]-8-[(3,3,3-trifluoropropyl)amino]imidazo[1,2-a]pyridin-3-yl}benzoate which was prepared according to intermediate example 111 b were transformed in analogy to intermediate example 8b to give after working up and purification 259.3 mg (64%) of the title compound.

### Example 111 b

### Methyl 2-methyl-4-{6-[(1E)-prop-1-en-1-yl]-8-[(3,3,3-trifluoropropyl)amino]imidazo[1,2-a]pyridin-3-yl}benzoate

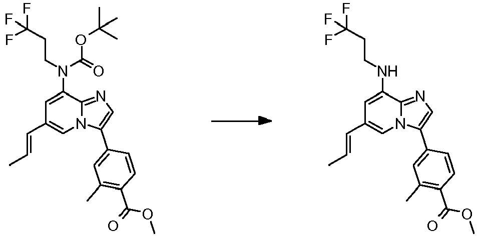

522 mg (1.01 mmol) methyl 4-{8-[(tert-butoxycarbonyl)(3,3,3-trifluoropropyl)amino]-6-[(1E)-prop-1-en-1-yl]imidazo[1,2-a]pyridin-3-yl}-2-methylbenzoate which was prepared according to intermediate example 111c were transformed in analogy to example 7 to give after working up and purification 403 mg (91%) of the title compound.

### Example 111 c

### Methyl 4-{8-[(tert-butoxycarbonyl)(3,3,3-trifluoropropyl)amino]-6-[(1E)-prop-1-en-1-yl]imidazo[1,2-a]pyridin-3-yl}-2-methylbenzoate

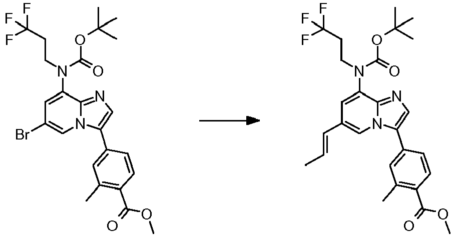

500 mg (899 µmol) methyl 4-{6-bromo-8-[(tert-butoxycarbonyl)(3,3,3-trifluoropropyl)amino]imidazo[1,2-a]pyridin-3-yl}-2-methylbenzoate which was prepared according to intermediate example 8d were transformed in analogy to intermediate example 79c using (1E)-prop-1-en-1-ylboronic acid to give after working up and purification 465 mg (100%) of the title compound.

### Example 112

### N,2-dimethyl-4-{6-[(1E)-prop-1-en-1-yl]-8-[(3,3,3-trifluoropropyl)amino]imidazo[1,2-a]pyridin-3-yl}benzamide

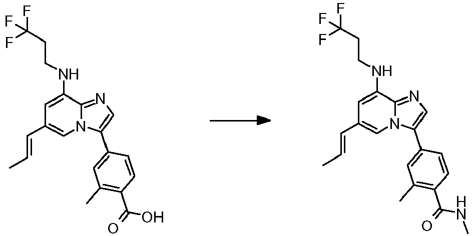

10 mg (25 µmol) 2-methyl-4-{6-[(1E)-prop-1-en-1-yl]-8-[(3,3,3-trifluoropropyl)amino]imidazo[1,2-a]pyridin-3-yl}benzoic acid which was prepared according to intermediate example 111 a were transformed in analogy to example 8 to give after working up and purification 8.7 mg (84%) of the title compound. ¹H-NMR (CDCl₃): δ= 1.89 (3H), 2.49-2.62 (2H), 2.53 (3H), 3.04 (3H), 3.63 (2H), 5.29 (1 H), 5.89 (1 H), 6.12-6.32 (3H), 7.37 (1 H), 7.39 (1 H), 7.47 (1 H), 7.49 (1 H), 7.58 (1 H) ppm.

### Example 113

### N-ethyl-2-methyl-4-{6-[(1E)-prop-1-en-1-yl]-8-[(3,3,3-trifluoropropyl)amino]imidazo[1,2-a]pyridin-3-yl}benzamide

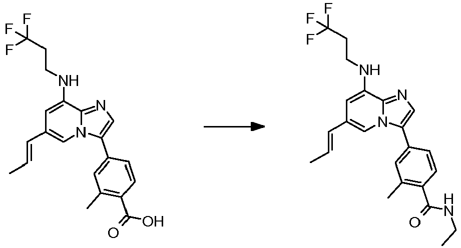

10 mg (25 µmol) 2-methyl-4-{6-[(1E)-prop-1-en-1-yl]-8-[(3,3,3-trifluoropropyl)amino]imidazo[1,2-a]pyridin-3-yl}benzoic acid which was prepared according to intermediate example 111 a were transformed in analogy to example 8 using ethanamine to give after working up and purification 9.4 mg (88%) of the title compound.
¹H-NMR (CDCl₃): δ= 1.28 (3H), 1.90 (3H), 2.48-2.63 (2H), 2.53 (3H), 3.52 (2H), 3.63 (2H), 5.29 (1 H), 5.82 (1 H), 6.12-6.32 (3H), 7.38 (1 H), 7.39 (1 H), 7.47 (1 H), 7.49 (1 H), 7.58 (1 H) ppm.

### Example 114

### 2-Methyl-N-(1-methylcyclopropyl)-4-{6-[(1E)-prop-1-en-1-yl]-8-[(3,3,3-trifluoropropyl)amino]imidazo[1,2-a]pyridin-3-yl}benzamide

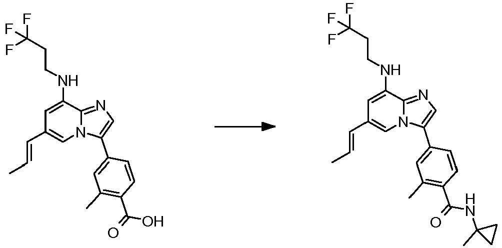

10 mg (25 µmol) 2-methyl-4-{6-[(1E)-prop-1-en-1-yl]-8-[(3,3,3-trifluoropropyl)amino]imidazo[1,2-a]pyridin-3-yl}benzoic acid which was prepared according to intermediate example 111 a were transformed in analogy to example 8 using 1-methylcyclopropanaminium chloride to give after working up and purification 10.5 mg (93%) of the title compound.
¹H-NMR (CDCl₃): δ= 0.77 (2H), 0.89 (2H), 1.54 (3H), 1.90 (3H), 2.46-2.63 (2H), 2.51 (3H), 3.63 (2H), 5.26 (1 H), 6.08-6.31 (4H), 7.36 (1 H), 7.38 (1 H), 7.43 (1 H), 7.47 (1 H), 7.56 (1 H) ppm.

### Example 115

### N-(1-cyanocyclopropyl)-2-methyl-4-{6-[(1E)-prop-1-en-1-yl]-8-[(3,3,3-trifluoropropyl)amino]imidazo[1,2-a]pyridin-3-yl}benzamide

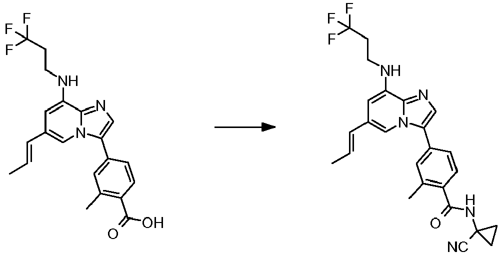

10 mg (25 µmol) 2-methyl-4-{6-[(1E)-prop-1-en-1-yl]-8-[(3,3,3-trifluoropropyl)amino]imidazo[1,2-a]pyridin-3-yl}benzoic acid which was prepared according to intermediate example 111 a were transformed in analogy to example 8 using 1-cyanocyclopropanaminium chloride to give after working up and purification 9.4 mg (81%) of the title compound.
¹H-NMR (CDCl₃): δ= 1.39 (2H), 1.68 (2H), 1.90 (3H), 2.48-2.63 (2H), 2.54 (3H), 3.63 (2H), 5.26 (1H), 6.11-6. 31 (3H), 6.48 (1H), 7.38 (1H), 7.42 (1H), 7.45 (1H), 7.46 (1 H), 7.57 (1 H) ppm.

### Example 116

### N-[rel-(1S,2S)-2-fluorocyclopropyl]-2-methyl-4-{6-[(1E)-prop-1-en-1-yl]-8-[(3,3,3-trifluoropropyl)amino]imidazo[1,2-a]pyridin-3-yl}benzamide

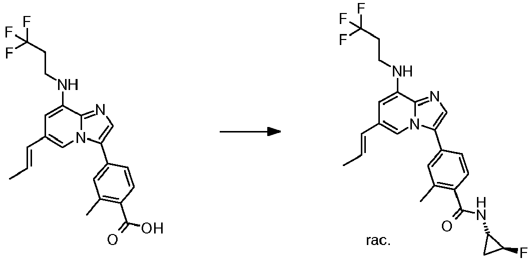

10 mg (25 µmol) 2-methyl-4-{6-[(1E)-prop-1-en-1-yl]-8-[(3,3,3-trifluoropropyl)amino]imidazo[1,2-a]pyridin-3-yl}benzoic acid which was prepared according to intermediate example 111a were transformed in analogy to example 8 using rel-(1S,2S)-2-fluorocyclopropanaminium chloride to give after working up and purification 7.5 mg (66%) of the title compound.
¹H-NMR (CDCl₃): δ= 1.04 (1H), 1.28 (1H), 1.89 (3H), 2.47-2.63 (2H), 2.54 (3H), 3.08 (1H), 3.63 (2H), 4.78 (1H), 5.27 (1H), 6.06 (1H), 6.10-6.32 (3H), 7.39 (1H), 7.40 (1 H), 7.47 (1 H), 7.52 (1 H), 7.58 (1 H) ppm.

### Example 117

### 2-Methyl-N-[rel-(1S,2S)-2-methylcyclopropyl]-4-{6-[(1E)-prop-1-en-1-yl]-8-[(3,3,3-trifluoropropyl)amino]imidazo[1,2-a]pyridin-3-yl}benzamide

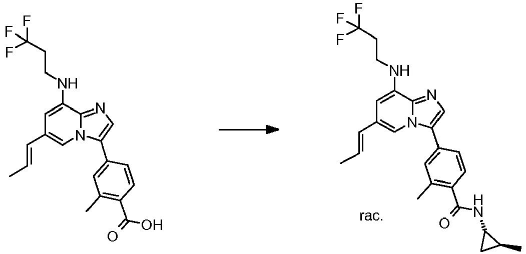

10 mg (25 µmol) 2-methyl-4-{6-[(1E)-prop-1-en-1-yl]-8-[(3,3,3-trifluoropropyl)amino]imidazo[1,2-a]pyridin-3-yl}benzoic acid which was prepared according to intermediate example 111 a were transformed in analogy to example 8 using rel-(1 R,2R)-2-methylcyclopropanamine to give after working up and purification 9.4 mg (83%) of the title compound.
¹H-NMR (CDCl₃): δ= 0.67 (1H), 0.77 (1H), 1.00 (1H), 1.17 (3H), 1.90 (3H), 2.46-2.65 (2H), 2.52 (3H), 3.63 (2H), 5.26 (1 H), 5.89 (1 H), 6.11-6.30 (3H), 7.36 (1 H), 7.38 (1 H), 7.45 (1 H), 7.47 (1 H), 7.57 (1 H) ppm.

### Example 118

### N-[rel-(1R,2R)-2-fluorocyclopropyl]-4-{6-[(1Z)-3-hydroxyprop-1-en-1-yl]-8-[(3,3,3-trifluoropropyl)amino]imidazo[1,2-a]pyridin-3-yl}-2-methylbenzamide

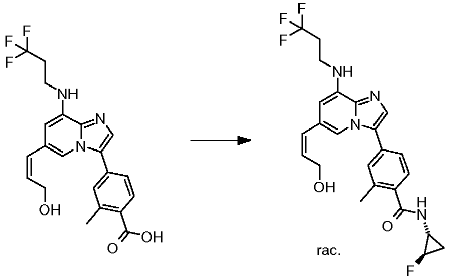

10 mg (24 µmol) 4-{6-[(1Z)-3-hydroxyprop-1-en-1-yl]-8-[(3,3,3-trifluoropropyl)amino]imidazo[1,2-a]pyridin-3-yl}-2-methylbenzoic acid which was prepared according to intermediate example 108a were transformed in analogy to example 8 using rel-(1S,2S)-2-fluorocyclopropanaminium chloride to give after working up and purification 8.1 mg (71%) of the title compound.
¹H-NMR (CDCl₃): δ= 1.05 (1 H), 1.28 (1 H), 2.48-2.60 (2H), 2.52 (3H), 3.06 (1 H), 3.60 (2H), 4.38 (2H), 4.77 (1H), 5.37 (1H), 5.94 (1H), 6.07 (1H), 6.15 (1H), 6.46 (1H), 7.36 (1 H), 7.40 (1 H), 7.49 (1 H), 7.51 (1 H), 7.66 (1 H), ppm.

### Example 119

### 4-{6-[(1Z)-3-hydroxyprop-1-en-1-yl]-8-[(3,3,3-trifluoropropyl)amino]imidazo[1,2-a]pyridin-3-yl}-N,2-dimethylbenzamide

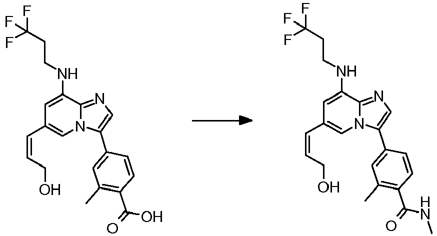

10 mg (24 µmol) 4-{6-[(1Z)-3-hydroxyprop-1-en-1-yl]-8-[(3,3,3-trifluoropropyl)amino]imidazo[1,2-a]pyridin-3-yl}-2-methylbenzoic acid which was prepared according to intermediate example 108a were transformed in analogy to example 8 to give after working up and purification 8.5 mg (82%) of the title compound.
¹H-NMR (CDCl₃): δ= 2.48-2.61 (2H), 2.50 (3H), 3.02 (3H), 3.59 (2H), 4.37 (2H), 5.41 (1H), 5.90-6.01 (2H), 6.07 (1H), 6.45 (2H), 7.34 (1H), 7.39 (1H), 7.45 (1H), 7.50 (1 H), 7.66 (1 H) ppm.

### Example 120

### N-ethyl-4-{6-[(1Z)-3-hydroxyprop-1-en-1-yl]-8-[(3,3,3-trifluoropropyl)amino]imidazo[1,2-a]pyridin-3-yl}-2-methylbenzamide

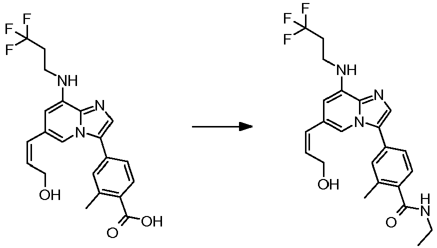

10 mg (24 µmol) 4-{6-[(1Z)-3-hydroxyprop-1-en-1-yl]-8-[(3,3,3-trifluoropropyl)amino]imidazo[1,2-a]pyridin-3-yl}-2-methylbenzoic acid which was prepared according to intermediate example 108a were transformed in analogy to example 8 using ethanamine to give after working up and purification 7.0 mg (66%) of the title compound.
¹H-NMR (CDCl₃): δ= 1.27 (3H), 2.48-2.61 (2H), 2.51 (3H), 3.51 (2H), 3.60 (2H), 4.38 (2H), 5.40 (1H), 5.88 (1H), 5.94 (1H), 6.08 (1H), 6.46 (1H), 7.35 (1H), 7.39 (1H), 7.46 (1 H), 7.51 (1 H), 7.66 (1 H) ppm.

### Example 121

### 4-{6-(3-Fluoro-4-methoxyphenoxy)-8-[(tetrahydro-2H-pyran-4-ylmethyl)amino]imidazo[1,2-a]pyridin-3-yl}-N,2-dimethylbenzamide

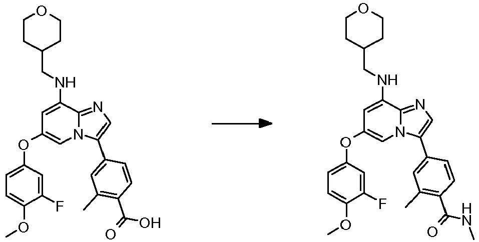

10 mg (20 µmol) 4-{6-(3-fluoro-4-methoxyphenoxy)-8-[(tetrahydro-2H-pyran-4-ylmethyl)amino]imidazo[1,2-a]pyridin-3-yl}-2-methylbenzoic acid which was prepared according to intermediate example 121 a were transformed in analogy to example 8 to give after working up and purification 8.2 mg (76%) of the title compound.
¹H-NMR (CDCl₃): δ= 1.40 (2H), 1.75 (2H), 1.94 (1H), 2.49 (3H), 3.01 (3H), 3.14 (2H), 3.39 (2H), 3.87 (3H), 3.99 (2H), 5.42 (1 H), 5.85 (1 H), 5.97 (1 H), 6.74 (1 H), 6.83 (1 H), 6.90 (1 H), 7.34 (1 H), 7.36 (1 H), 7.44 (1 H), 7.47 (1 H), 7.52 (1 H) ppm.

### Example 121 a

### 4-{6-(3-Fluoro-4-methoxyphenoxy)-8-[(tetrahydro-2H-pyran-4-ylmethyl)amino]imidazo[1,2-a]pyridin-3-yl}-2-methylbenzoic acid

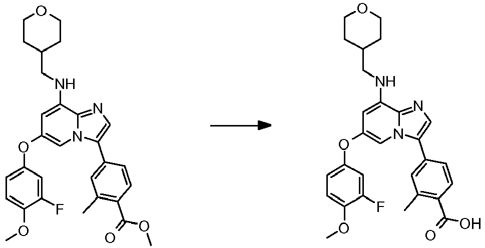

40.9 mg (79 µmol) methyl 4-{6-(3-fluoro-4-methoxyphenoxy)-8-[(3,3,3-trifluoropropyl)amino]imidazo[1,2-a]pyridin-3-yl}-2-methylbenzoate which was prepared according to intermediate example 121 b were transformed in analogy to intermediate example 8b to give after working up and purification 31.1 mg (78%) of the title compound.

### Example 121b

### Methyl 4-{6-(3-fluoro-4-methoxyphenoxy)-8-[(3,3,3-trifluoropropyl)amino]imidazo[1,2-a]pyridin-3-yl}-2-methylbenzoate

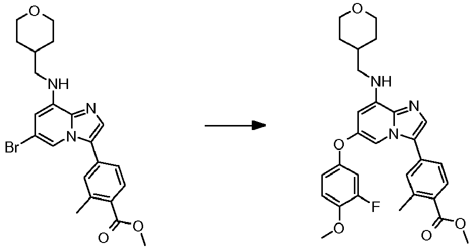

373.5 mg (815 µmol) methyl 4-[6-bromo-8-[(tetrahydro-2H-pyran-4-ylmethyl)amino]imidazo[1,2-a]pyridin-3-yl}-2-methylbenzoate which was prepared according to intermediate example 121 c were transformed in analogy to example 6-1 using 3-fluoro-4-methoxyphenol to give after working up and purification 40.9 mg (9%) of the title compound.

### Example 121c

### Methyl 4-{6-bromo-8-[(tetrahydro-2H-pyran-4-ylmethyl)amino]imidazo[1,2-a]pyridin-3-yl}-2-methylbenzoate methyl

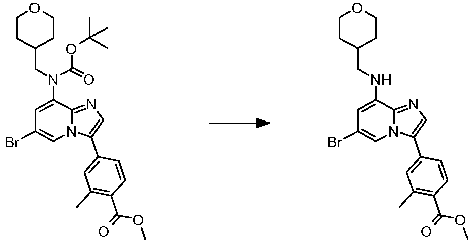

581.5 mg (1.04 mmol) methyl 4-{6-bromo-8-[(tert-butoxycarbonyl)(tetrahydro-2H-pyran-4-ylmethyl)amino]imidazo[1,2-a]pyridin-3-yl}-2-methylbenzoate which was prepared according to intermediate example 121 d were transformed in analogy to example 7 to give after working up and purification 458 mg (89%) of the title compound.

### Example 121 d

### Methyl 4-{6-bromo-8-[(tert-butoxycarbonyl)(tetrahydro-2H-pyran-4-ylmethyl)amino]imidazo[1,2-a]pyridin-3-yl}-2-methylbenzoate

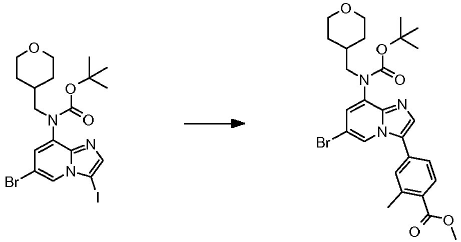

863 mg (1.61 mmol) tert-butyl (6-bromo-3-iodoimidazo[1,2-a]pyridin-8-yl)(tetrahydro-2H-pyran-4-ylmethyl)carbamate which was prepared according to intermediate example 9b were transformed in analogy to intermediate example 7b using [4-(methoxycarbonyl)-3-methylphenyl]boronic acid to give after working up and purification 689.4 mg (77%) of the title compound.

### Example 122

### N-ethyl-4-{6-(3-fluoro-4-methoxyphenoxy)-8-[(tetrahydro-2H-pyran-4-ylmethyl)amino]imidazo[1,2-a]pyridin-3-yl}-2-methylbenzamide

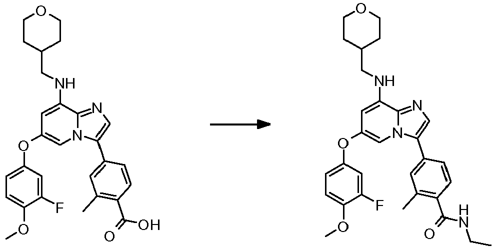

10 mg (20 µmol) 4-{6-(3-fluoro-4-methoxyphenoxy)-8-[(tetrahydro-2H-pyran-4-ylmethyl)amino]imidazo[1,2-a]pyridin-3-yl}-2-methylbenzoic acid which was prepared according to intermediate example 121 a were transformed in analogy to example 8 using ethanamine to give after working up and purification 9.1 mg (82%) of the title compound.
¹H-NMR (CDCl₃): δ= 1.26 (3H), 1.40 (2H), 1.76 (2H), 1.94 (1H), 2.49 (3H), 3.14 (2H), 3.39 (2H), 3.50 (2H), 3.87 (3H), 4.00 (2H), 5.42 (1H), 5.79 (1H), 5.97 (1H), 6.75 (1H), 6.82 (1H), 6.90 (1H), 7.34 (1H), 7.36 (1H), 7.44 (1H), 7.47 (1H), 7.52 (1H) ppm.

### Example 123

### N-cyclopropyl-4-{6-(3-fluoro-4-methoxyphenoxy)-8-[(tetrahydro-2H-pyran-4-ylmethyl)amino]imidazo[1,2-a]pyridin-3-yl}-2-methylbenzamide

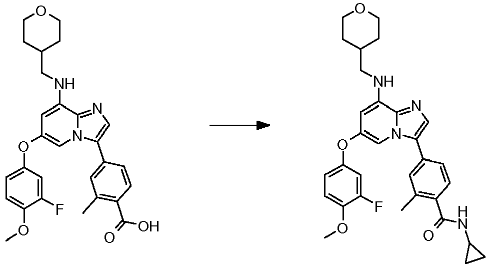

10 mg (20 µmol) 4-{6-(3-fluoro-4-methoxyphenoxy)-8-[(tetrahydro-2H-pyran-4-ylmethyl)amino]imidazo[1,2-a]pyridin-3-yl}-2-methylbenzoic acid which was prepared according to intermediate example 121 a were transformed in analogy to example 8 using cyclopropanamine to give after working up and purification 9.6 mg (85%) of the title compound.
¹H-NMR (CDCl₃): δ= 0.63 (2H), 0.89 (2H), 1.40 (2H), 1.75 (2H), 1.94 (1 H), 2.48 (3H), 2.92 (1H), 3.14 (2H), 3.39 (2H), 3.87 (3H), 3.99 (2H), 5.41 (1H), 5.94 (1H), 5.97 (1H), 6.74 (1H), 6.82 (1H), 6.90 (1H), 7.32 (1H), 7.35 (1H), 7.41 (1H), 7.46 (1H), 7.52 (1 H) ppm.

### Example 124

### N-[rel-(1S,2S)-2-fluorocyclopropyl]-4-{6-(3-fluoro-4-methoxyphenoxy)-8-[(3,3,3-trifluoropropyl)amino]imidazo[1,2-a]pyridin-3-yl}-2-methylbenzamide

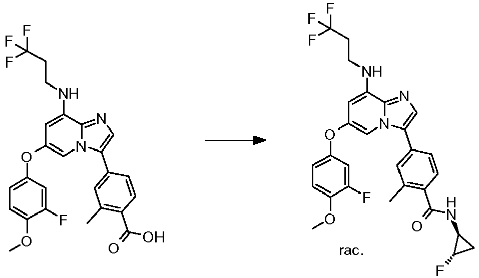

20 mg (40 µmol) 4-{6-(3-fluoro-4-methoxyphenoxy)-8-[(3,3,3-trifluoropropyl)amino]imidazo[1,2-a]pyridin-3-yl}-2-methylbenzoic acid which was prepared according to intermediate example 106a were transformed in analogy to example 8 using rel-(1S,2S)-2-fluorocyclopropanaminium chloride to give after working up and purification 11.9 mg (51%) of the title compound.
¹H-NMR (DCDCl₃): δ= 1.03 (1 H), 1.27 (1 H), 2.46-2.57 (2H), 2.51 (3H), 3.06 (1 H), 3.56 (2H), 3.87 (3H), 4.77 (1H), 5.47 (1H), 5.99 (1H), 6.01 (1H), 6.74 (1H), 6.83 (1 H), 6.91 (1 H), 7.35 (1 H), 7.37 (1 H), 7.48 (1 H), 7.51 (1 H), 7.54 (1 H) ppm.

### Example 125

### 4-{6-(3-fluoro-4-methoxyphenoxy)-8-[(3,3,3-trifluoropropyl)amino]imidazo[1,2-a]pyridin-3-yl}-2-methyl-N-[rel-(1S,2S)-2-methylcyclopropyl]benzamide

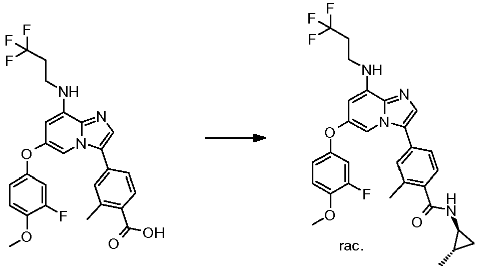

20 mg (40 µmol) 4-[6-(3-fluoro-4-methoxyphenoxy)-8-[(3,3,3-trifluoropropyl)amino]imidazo[1,2-a]pyridin-3-yl}-2-methylbenzoic acid which was prepared according to intermediate example 106a were transformed in analogy to example 8 using rel-(1R,2R)-2-methylcyclopropanamine to give after working up and purification 10.9 mg (47%) of the title compound.
¹H-NMR (CDCl₃): δ= 0.66 (1H), 0.76 (1H), 0.98 (1H), 1.16 (3H), 2.43-2.57 (2H), 2.48 (3H), 2.60 (1 H), 3.55 (2H), 3.87 (3H), 5.47 (1 H), 5.87 (1 H), 5.99 (1 H), 6.74 (1 H), 6.83 (1H), 6.91 (1H), 7.32 (1H), 7.34 (1H), 7.41 (1H), 7.50 (1H), 7.53 (1H) ppm.

### Example 126

### 4-{6-(3-hydroxypropyl)-8-[(3,3,3-trifluoropropyl)amino]imidazo[1,2-a]pyridin-3-yl}-N,2-dimethylbenzamide

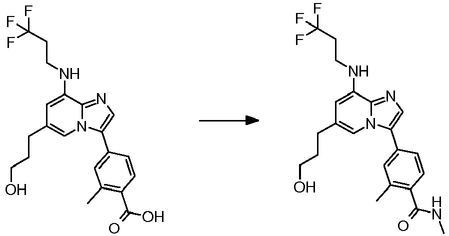

10 mg (24 µmol) 4-{6-(3-hydroxypropyl)-8-[(3,3,3-trifluoropropyl)amino] imidazo[1,2-a]pyridin-3-yl}-2-methylbenzoic acid which was prepared according to intermediate example 126a were transformed in analogy to example 8 to give after working up and purification 6.9 mg (67%) of the title compound.
¹H-NMR (CD₃OD): δ= 1.84 (2H), 2.46 (3H), 2.54-2.68 (4H), 2.79 (2H), 2.91 (3H), 3.58 (4H), 6.24 (1 H), 7.43-7.51 (4H), 7.66 (1 H) ppm.

### Example 126a

### 4-{6-(3-hydroxypropyl)-8-[(3,3,3-trifluoropropyl)amino]imidazo[1,2-a]pyridin-3-yl}-2-methylbenzoic acid

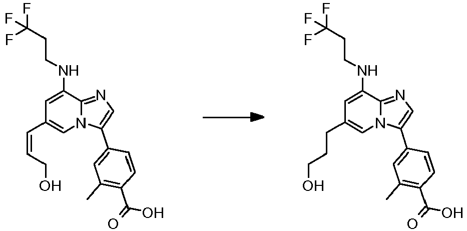

69.6 mg (166 µmol) 4-{6-[(1Z)-3-hydroxyprop-1-en-1-yl]-8-[(3,3,3-trifluoropropyl)amino]imidazo[1,2-a]pyridin-3-yl}-2-methylbenzoic acid which was prepared according to intermediate example 108a were transformed in analogy to intermediate example 99c to give after working up and purification 58.6 mg (84%) of the title compound.

### Example 127

### N-ethyl-4-{6-(3-hydroxypropyl)-8-[(3,3,3-trifluoropropyl)amino]imidazo[1,2-a]pyridin-3-yl}-2-methylbenzamide

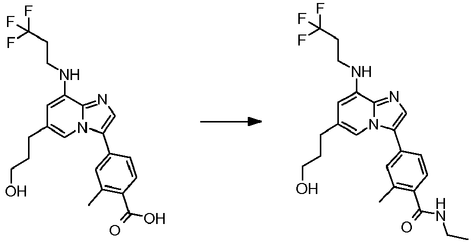

10 mg (24 µmol) 4-{6-(3-hydroxypropyl)-8-[(3,3,3-trifluoropropyl)amino] imidazo[1,2-a]pyridin-3-yl}-2-methylbenzoic acid which was prepared according to intermediate example 126a were transformed in analogy to example 8 using ethanamine to give after working up and purification 10.1 mg (95%) of the title compound.
¹H-NMR (CD₃OD): δ= 1.23 (3H), 1.84 (2H), 2.46 (3H), 2.54-2.67 (4H), 2.79 (2H), 3.40 (2H), 3.58 (4H), 6.26 (1 H), 7.43-7.49 (3H), 7.50 (1 H), 7.65 (1 H) ppm.

### Example 128

### N-cyclopropyl-4-{6-(3-hydroxypropyl)-8-[(3,3,3-trifluoropropyl)amino]imidazo[1,2-a]pyridin-3-yl}-2-methylbenzamide

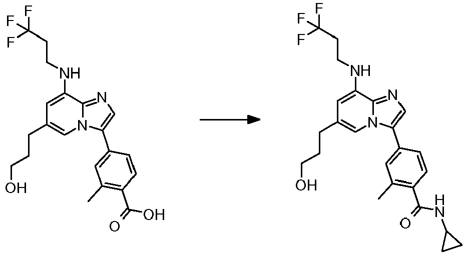

10 mg (24 µmol) 4-{6-(3-hydroxypropyl)-8-[(3,3,3-trifluoropropyl)amino]imidazo [1,2-a]pyridin-3-yl}-2-methylbenzoic acid which was prepared according to intermediate example 126a were transformed in analogy to example 8 using cyclopropanamine to give after working up and purification 9.7 mg (89%) of the title compound.
¹H-NMR (CD₃OD): δ= 0.62 (2H), 0.81 (2H), 1.84 (2H), 2.45 (3H), 2.54-2.66 (4H), 2.87 (1 H), 3.52-3.63 (4H), 6.25 (1 H), 7.42-7.48 (3H), 7.50 (1 H), 7.65 (1 H) ppm.

### Example 129

### N-cyclopropyl-4-{6-(3-fluoro-4-hydroxyphenoxy)-8-[(3,3,3-trifluoropropyl)amino]imidazo[1,2-a]pyridin-3-yl}-2-methylbenzamide

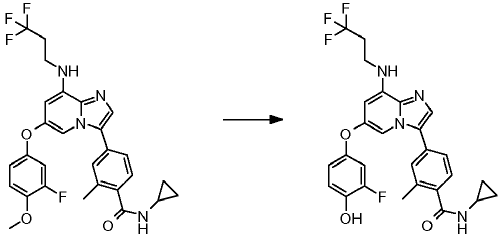

To a solution of 15.2 mg (28 µmol) N-cyclopropyl-4-{6-(3-fluoro-4-methoxyphenoxy)-8-[(3,3,3-trifluoropropyl)amino]imidazo[1,2-a]pyridin-3-yl}-2-methylbenzamide which was prepared according to example 42 in 2.4 mL dichloromethane were added 89.7 µL of a 2M boron tribromide solution in dichloromethane and the mixture was stirred at 23°C overnight. A second portion of 56 µL 2M boron tribromide solution in dichloromethane was added and stirring continued for additional 6 hours. Methanol was added and solvents were removed. The residue was purified by chromatography to give 7.4 mg (47%) of the title compound.
¹H-NMR (DMSO-d6): δ= 0.49 (2H), 0.65 (2H), 2.31 (3H), 2.61 (2H), 2.80 (1H), 3.45 (2H), 6.06 (1H), 6.49 (1H), 6.74 (1H), 6.89 (1H), 6.98 (1H), 7.31-7.47 (4H), 7.59 (1 H), 8.27 (1H), 9.62 (1 H) ppm.

### Example 130

### 4-{6-(3-Fluoro-4-hydroxyphenoxy)-8-[(3,3,3-trifluoropropyl)amino]imidazo[1,2-a]pyridin-3-yl}-N,2-dimethylbenzamide

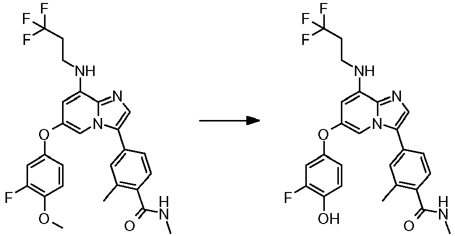

8.8 mg (17 µmol) 4-{6-(3-fluoro-4-methoxyphenoxy)-8-[(3,3,3-trifluoropropyl)amino]imidazo[1,2-a]pyridin-3-yl}-N,2-dimethylbenzamide which was prepared according to example 106 were transformed in analogy to example 129 to give after working up and purification 5.3 mg (59%) of the title compound.
¹H-NMR (DMSO-d6): δ= 2.33 (3H), 2.55-2.67 (2H), 2.72 (3H), 3.45 (2H), 6.06 (1H), 6.50 (1H), 6.75 (1H), 6.89 (1H), 6.98 (1H), 7.36-7.42 (3H), 7.44 (1H), 7.61 (1H), 8.17 (1 H), 9.62 (1 H) ppm.

### Example 131

### N-ethyl-4-{6-(3-fluoro-4-hydroxyphenoxy)-8-[(3,3,3-trifluoropropyl)amino]imidazo[1,2-a]pyridin-3-yl}-2-methylbenzamide

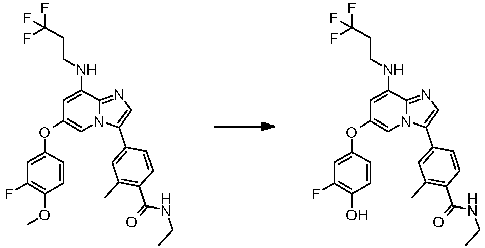

11.8 mg (22 µmol) N-ethyl-4-{6-(3-fluoro-4-methoxyphenoxy)-8-[(3,3,3-trifluoropropyl)amino]imidazo[1,2-a]pyridin-3-yl}-2-methylbenzamide which was prepared according to example 107 were transformed in analogy to example 129 to give after working up and purification 5.8 mg (48%) of the title compound. ¹H-NMR (DMSO-d6): δ= 1.08 (3H), 2.33 (3H), 2.53-2.70 (2H), 3.22 (2H), 3.45 (2H), 6.07 (1H), 6.50 (1H), 6.75 (1H), 6.89 (1H), 6.98 (1H), 7.35-7.44 (4H), 7.60 (1H), 8.23 (1 H), 9.62 (1 H) ppm.

### Example 132

### N-cyclopropyl-4-{6-(2-hydroxyphenyl)-8-[(3,3,3-trifluoropropyl)amino]imidazo[1,2-a]pyridin-3-yl}-2-methylbenzamide

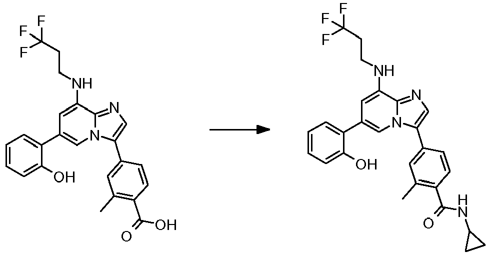

10 mg (22 µmol) 4-{6-(2-hydroxyphenyl)-8-[(3,3,3-trifluoropropyl)amino]imidazo[1,2-a]pyridin-3-yl}-2-methylbenzoic acid which was prepared according to intermediate example 132a were transformed in analogy to example 10 using cyclopropanamine to give after working up and purification 7.7 mg (67%) of the title compound.
¹H-NMR (CDCl₃): δ= 0.63 (2H), 0.87 (2H), 2.35-2.53 (2H), 2.39 (3H), 2.90 (1H), 3.55 (2H), 5.30 (1H), 5.33 (1H), 6.21 (1H), 6.30 (1H), 6.98 (1H), 7.05 (1H), 7.22-7.35 (4H), 7.40 (1 H), 7.81 (1 H) ppm.

### Example 132a

### 4-{6-(2-Hydroxyphenyl)-8-[(3,3,3-trifluoropropyl)amino]imidazo[1,2-a]pyridin-3-yl}-2-methylbenzoic acid

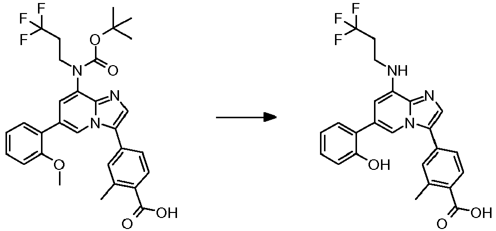

241 mg (423 µmol) 4-{8-[(tert-butoxycarbonyl)(3,3,3-trifluoropropyl)amino]-6-(2-methoxyphenyl)imidazo[1,2-a]pyridin-3-yl}-2-methylbenzoic acid which was prepared according to intermediate example 132b were transformed in analogy to example 129 to give after working up and purification 157 mg (77%) of the title compound.

### Example 132b

### 4-{8-[(tert-Butoxycarbonyl)(3,3,3-trifluoropropyl)amino]-6-(2-methoxyphenyl)imidazo[1,2-a]pyridin-3-yl}-2-methylbenzoic acid

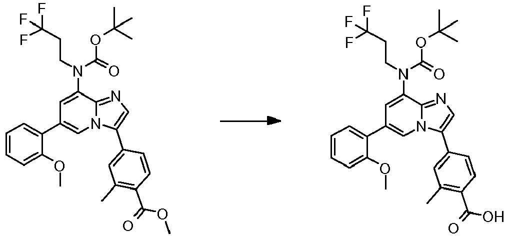

524 mg (max. 899 µmol) methyl 4-{8-[(tert-butoxycarbonyl)(3,3,3-trifluoropropyl)amino]-6-(2-methoxyphenyl)imidazo[1,2-a]pyridin-3-yl}-2-methylbenzoate which was prepared according to intermediate example 79c were transformed in analogy to intermediate example 8b to give after working up and purification 360 mg (67%) of the title compound.

### Example 133

### 4-{6-(2-Hydroxyphenyl)-8-[(3,3,3-trifluoropropyl)amino]imidazo[1,2-a]pyridin-3-yl}-N,2-dimethylbenzamide

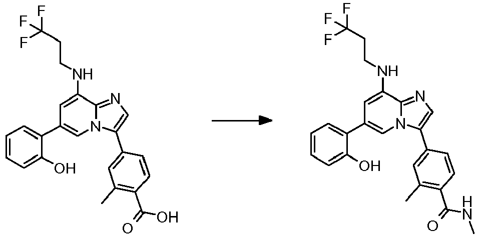

10 mg (22 µmol) 4-{6-(2-hydroxyphenyl)-8-[(3,3,3-trifluoropropyl)amino]imidazo[1,2-a]pyridin-3-yl}-2-methylbenzoic acid which was prepared according to intermediate example 132a were transformed in analogy to example 8 to give after working up and purification 6.4 mg (59%) of the title compound.
¹H-NMR (CDCl₃): δ= 2.38-2.54 (2H), 2.41 (3H), 3.00 (3H), 3.57 (2H), 5.30 (1 H), 5.34 (1 H), 6.08 (1 H), 6.30 (1 H), 6.98 (1 H), 7.04 (1 H), 7.23-7.32 (3H), 7.36 (1 H), 7.42 (1 H), 7.82 (1 H) ppm.

### Example 134

### N-ethyl-4-{6-(2-hydroxyphenyl)-8-[(3,3,3-trifluoropropyl)amino]imidazo[1,2-a]pyridin-3-yl}-2-methylbenzamide

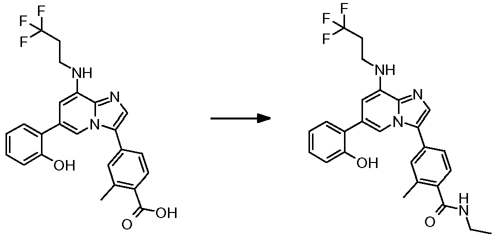

10 mg (22 µmol) 4-{6-(2-hydroxyphenyl)-8-[(3,3,3-trifluoropropyl)amino]imidazo[1,2-a]pyridin-3-yl}-2-methylbenzoic acid which was prepared according to intermediate example 132a were transformed in analogy to example 8 using ethanamine to give after working up and purification 5.2 mg (47%) of the title compound.
¹H-NMR (CDCl₃): δ= 1.25 (3H), 2.38-2.55 (2H), 2.42 (3H), 3.49 (2H), 3.57 (2H), 5.35 (1H), 5.97 (1H), 6.28 (1H), 6.99 (1H), 7.04 (1H), 7.22-7.33 (4H), 7.38 (1H), 7.45 (1 H), 7.81 (1 H) ppm.

### Example 135

### N-[rel-(1S,2S)-2-fluorocyclopropyl]-4-{6-(2-hydroxyphenyl)-8-[(3,3,3-trifluoropropyl)amino]imidazo[1,2-a]pyridin-3-yl}-2-methylbenzamide

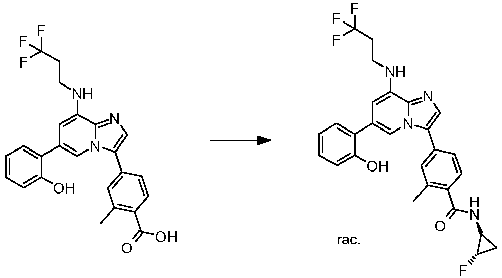

10 mg (22 µmol) 4-{6-(2-hydroxyphenyl)-8-[(3,3,3-trifluoropropyl)amino]imidazo[1,2-a]pyridin-3-yl}-2-methylbenzoic acid which was prepared according to intermediate example 132a were transformed in analogy to example 8 using rel-(1S,2S)-2-fluorocyclopropanaminium chloride to give after working up and purification 6.1 mg (52%) of the title compound.
¹H-NMR (CDCl₃): δ= 1.05 (1 H), 1.25 (1 H), 2.28-2.56 (2H), 2.45 (3H), 3.04 (1 H), 3.57 (2H), 4.75 (1H), 5.36 (1H), 6.18 (1H), 6.28 (1H), 6.95-7.11 (2H), 7.23-7.36 (4H), 7.38-7.50 (2H), 7.83 (1H) ppm.

### Example 136

### N-cyclopropyl-4-{6-(3-hydroxyphenoxy)-8-[(3,3,3-trifluoropropyl)amino]imidazo[1,2-a]pyridin-3-yl}-2-methylbenzamide

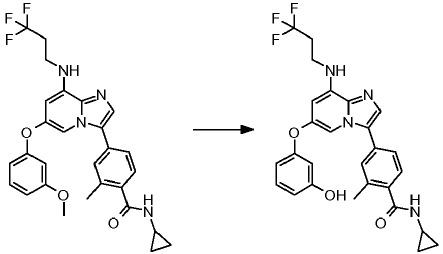

18.8 mg (36 µmol) N-cyclopropyl-4-{6-(3-methoxyphenoxy)-8-[(3,3,3-trifluoropropyl)amino]imidazo[1,2-a]pyridin-3-yl}-2-methylbenzamide which was prepared according to example 52 were transformed in analogy to example 129 to give after working up and purification 9.1 mg (47%) of the title compound. ¹H-NMR (DMSO-d6): δ= 0.49 (2H), 0.65 (2H), 2.32 (3H), 2.52-2.70 (2H), 2.79 (1H), 3.46 (2H), 6.09 (1 H), 6.39 (1 H), 6.42-6.48 (2H), 6.51 (1 H), 7.09 (1 H), 7.34 (1 H), 7.40 (1 H), 7.41 (1 H), 7.53 (1 H), 7.62 (1 H), 8.26 (1 H), 9.49 (1 H) ppm.

### Example 137

### 4-{6-(3-Hydroxyphenoxy)-8-[(3,3,3-trifluoropropyl)amino]imidazo[1,2-a]pyridin-3-yl}-2-methyl-N-(1-methylcyclopropyl)benzamide

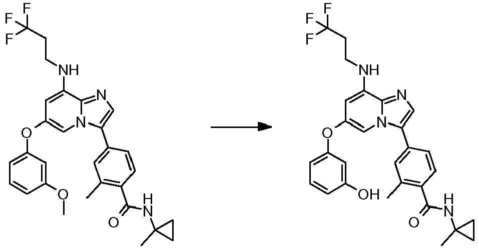

12.8 mg (24 µmol) 4-{6-(3-methoxyphenoxy)-8-[(3,3,3-trifluoropropyl)amino]imidazo[1,2-a]pyridin-3-yl}-2-methyl-N-(1-methylcyclopropyl)benzamide which was prepared according to example 54 were transformed in analogy to example 129 to give after working up and purification 5.1 mg (39%) of the title compound.
¹H-NMR (DMSO-d6): δ= 0.55 (2H), 0.68 (2H), 1.35 (3H), 2.31 (3H), 2.61 (2H), 3.45 (2H), 6.08 (1 H), 6.39 (1 H), 6.43-6.48 (2H), 6.51 (1 H), 7.09 (1 H), 7.31 (1 H), 7.38 (1 H), 7.40 (1 H), 7.52 (1 H), 7.61 (1 H), 8.41 (1 H), 9.49 (1 H) ppm.

### Example 138

### N-ethyl-4-{6-(3-hydroxyphenoxy)-8-[(3,3,3-trifluoropropyl)amino]imidazo[1,2-a]pyridin-3-yl}-2-methylbenzamide

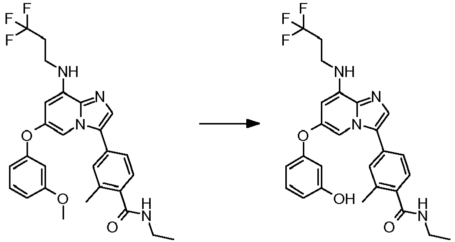

20.5 mg (40 µmol) N-ethyl-4-{6-(3-methoxyphenoxy)-8-[(3,3,3-trifluoropropyl)amino]imidazo[1,2-a]pyridin-3-yl}-2-methylbenzamide which was prepared according to example 56 were transformed in analogy to example 129 to give after working up and purification 12.1 mg (58%) of the title compound.
¹H-NMR (DMSO-d6): δ= 1.08 (3H), 2.34 (3H), 2.53-2.70 (2H), 3.22 (2H), 3.46 (2H), 6.09 (1 H), 6.40 (1 H), 6.42-6.48 (2H), 6.51 (1 H), 7.09 (1 H), 7.34-7.46 (3H), 7.54 (1 H), 7.62 (1 H), 8.22 (1 H), 9.49 (1 H) ppm.

### Example 139

### 4-{6-(3-Hydroxyphenoxy)-8-[(3,3,3-trifluoropropyl)amino]imidazo[1,2-a]pyridin-3-yl}-N,2-dimethylbenzamide

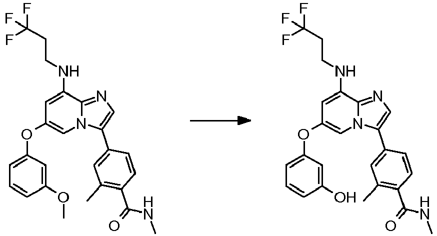

22.4 mg (45 µmol) 4-{6-(3-methoxyphenoxy)-8-[(3,3,3-trifluoropropyl)amino]imidazo[1,2-a]pyridin-3-yl}-N,2-dimethylbenzamide which was prepared according to example 55 were transformed in analogy to example 129 to give after working up and purification 6.7 mg (31%) of the title compound.
¹H-NMR (DMSO-d6): δ= 2.34 (3H), 2.53-2.68 (2H), 2.72 (3H), 3.45 (2H), 6.08 (1H), 6.39 (1 H), 6.44 (1 H), 6.46 (1 H), 6.54 (1 H), 7.09 (1 H), 7.38-7.45 (3H), 7.55 (1 H), 7.63 (1H), 8.18 (1 H), 9.51 (1 H) ppm.

### Example 140

### Methyl 2-methyl-4-{3-[3-methyl-4-(methylcarbamoyl)phenyl]-8-[(3,3,3-trifluoropropyl)amino]imidazo[1,2-a]pyridin-6-yl}benzoate

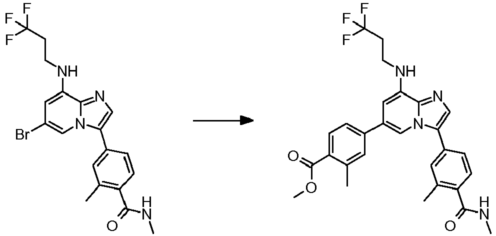

408 mg (905 µmol) 4-{6-bromo-8-[(3,3,3-trifluoropropyl)amino]imidazo[1,2-a]pyridin-3-yl}-N,2-dimethylbenzamide which was prepared according to intermediate example 140a were transformed in analogy to intermediate example 79c using [4-(methoxycarbonyl)-3-methylphenyl]boronic acid to give after working up and purification 356 mg (68%) of the title compound.
¹H-NMR (DMSO-d6): δ= 2.38 (3H), 2.56 (3H), 2.62-2.78 (2H), 2.74 (3H), 3.59 (2H), 3.80 (3H), 6.40 (1 H), 6.50 (1 H), 7.45 (1 H), 7.50-7.66 (5H), 7.87 (1 H), 7.97 (1 H), 8.22 (1 H) ppm.

### Example 140a

### 4-{6-Bromo-8-[(3,3,3-trifluoropropyl)amino]imidazo[1,2-a]pyridin-3-yl}-N,2-dimethylbenzamide

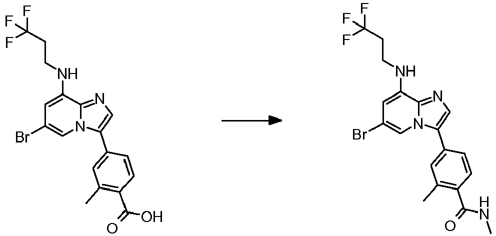

400 mg (904 µmol) 4-{6-bromo-8-[(3,3,3-trifluoropropyl)amino]imidazo[1,2-a]pyridin-3-yl}-2-methylbenzoic acid which was prepared according to intermediate example 42b were transformed in analogy to example 8 to give after working up and purification 409mg (99%) of the title compound.

### Example 141

### N-cyclopropyl-4-{6-(2-hydroxyethyl)-8-[(3,3,3-trifluoropropyl)amino]imidazo[1,2-a]pyridin-3-yl}-2-methylbenzamide

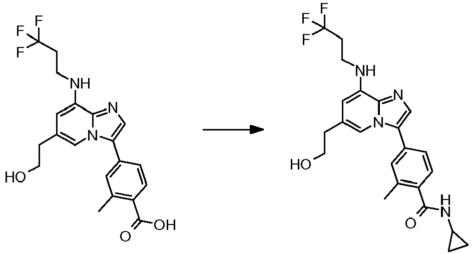

10 mg (25 µmol) 4-{6-(2-hydroxyethyl)-8-[(3,3,3-trifluoropropyl)amino]imidazo[1,2-a]pyridin-3-yl}-2-methylbenzoic acid which was prepared according to intermediate example 141 a were transformed in analogy to example 8 using cyclopropanamine to give after working up and purification 10 mg (91%) of the title compound. ¹H-NMR (CD₃OD): δ= 0.62 (2H), 0.81 (2H), 2.45 (3H), 2.60 (2H), 2.74 (2H), 2.86 (1H), 3.60 (2H), 3.78 (2H), 6.27 (1H), 7.42-7.54 (4H), 7.70 (1H), ppm.

### Example 141 a

### 4-{6-(2-Hydroxyethyl)-8-[(3,3,3-trifluoropropyl)amino]imidazo[1,2-a]pyridin-3-yl}-2-methylbenzoic acid

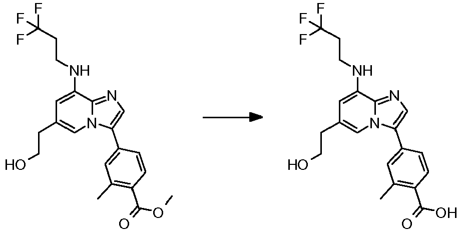

23.6 mg (56 µmol) methyl 4-{6-(2-hydroxyethyl)-8-[(3,3,3-trifluoropropyl)amino]imidazo[1,2-a]pyridin-3-yl}-2-methylbenzoate which was prepared according to intermediate example 141 b were transformed in analogy to intermediate example 8b to give after working up 21 mg (83%) of the title compound.

### Example 141 b

### Methyl 4-{6-(2-hydroxyethyl)-8-[(3,3,3-trifluoropropyl)amino]imidazo[1,2-a]pyridin-3-yl}-2-methylbenzoate (A) and methyl 4-{6-[(1RS)-1-hydroxyethyl]-8-[(3,3,3-trifluoropropyl)amino]imidazo[1,2-a]pyridin-3-yl}-2-methylbenzoate (B)

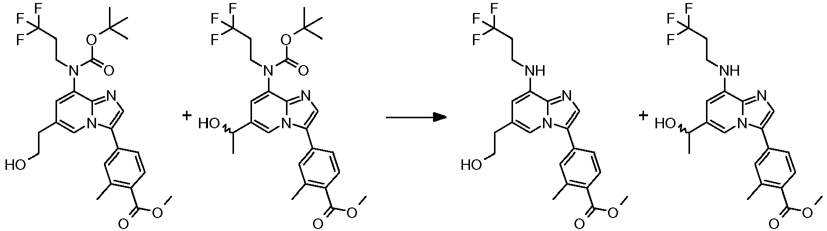

218 mg (418 µmol) of a mixture of methyl 4-{8-[(tert-butoxycarbonyl)(3,3,3-trifluoropropyl)amino]-6-(2-hydroxyethyl)imidazo[1,2-a]pyridin-3-yl}-2-methylbenzoate and methyl 4-{8-[(tert-butoxycarbonyl)(3,3,3-trifluoropropyl)amino]-6-[(1RS)-1-hydroxyethyl]imidazo[1,2-a]pyridin-3-yl}-2-methylbenzoate which was prepared according to intermediate example 141c were transformed in analogy to example 7 to give after working up and purification 23.6 mg (13%) of the title compound A and 34.9 mg (20%) of the title compound B.

### Example 141c

### Methyl 4-{8-[(tert-butoxycarbonyl)(3,3,3-trifluoropropyl)amino]-6-(2-hydroxyethyl)imidazo[1,2-a]pyridin-3-yl}-2-methylbenzoate (A) and methyl 4-{8-[(tert-butoxycarbonyl)(3,3,3-trifluoropropyl)amino]-6-[(1RS)-1-hydroxyethyl]imidazo[1,2-a]pyridin-3-yl}-2-methylbenzoate (B)

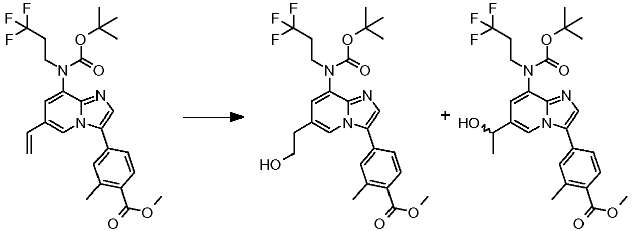

To a solution of 217 mg (431 µmol) methyl 4-{8-[(tert-butoxycarbonyl)(3,3,3-trifluoropropyl)amino]-6-vinylimidazo[1,2-a]pyridin-3-yl}-2-methylbenzoate which was prepared according to intermediate example 92c in 7.4 mL tetrahydrofuran were added 2.16 mL of a 1M boran-tetrahydrofuran solution. The mixture was stirred for two days at 23°C. After cooling to 3°C, 325 µL water and 325 µL hydrogen peroxide solution (30% in water) were added and stirring continued at 23°C for two hours. The mixture was poured into water and extracted with ethyl acetate. The combined organic layers were dried over sodiumsulfate. After filtration and removal of the solvent the crude product containing a mixture of the title compounds was used without purification.

### Example 142

### 4-{6-(2-Hydroxyethyl)-8-[(3,3,3-trifluoropropyl)amino]imidazo[1,2-a]pyridin-3-yl}-N,2-dimethylbenzamide

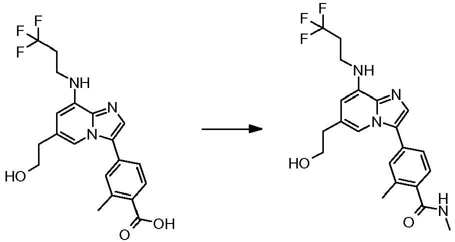

10 mg (25 µmol) 4-{6-(2-hydroxyethyl)-8-[(3,3,3-trifluoropropyl)amino]imidazo[1,2-a]pyridin-3-yl}-2-methylbenzoic acid which was prepared according to intermediate example 141 a were transformed in analogy to example 8 to give after working up and purification 8.2 mg (79%) of the title compound.
¹H-NMR (CD₃OD): δ= 2.46 (3H), 2.60 (2H), 2.74 (2H), 2.91 (3H), 3.60 (2H), 3.78 (2H), 6.27 (1H), 7.45-7.51 (4H), 7.71 (1H) ppm.

### Example 143

### N-cyclopropyl-4-{6-[(1RS)-1-hydroxyethyl]-8-[(3,3,3-trifluoropropyl)amino]imidazo[1,2-a]pyridin-3-yl}-2-methylbenzamide

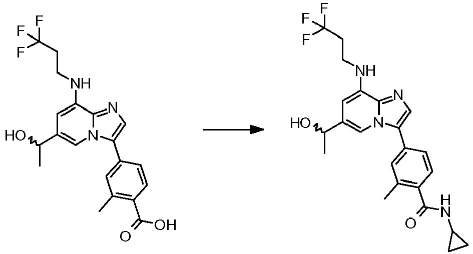

11 mg (27 µmol) 4-{6-[(1RS)-1-hydroxyethyl]-8-[(3,3,3-trifluoropropyl)amino]imidazo[1,2-a]pyridin-3-yl}-2-methylbenzoic acid which was prepared according to intermediate example 143a were transformed in analogy to example 8 using cyclopropanamine to give after working up and purification 11.7 mg (97%) of the title compound.
¹H-NMR (CD₃OD): δ= 0.62 (2H), 0.81 (2H), 1.46 (3H), 2.45 (3H), 2.52-2.69 (2H), 2.86 (1H), 3.61 (2H), 4.79 (1H), 6.32 (1H), 7.43-7.49 (3H), 7.52 (1H), 7.82 (1H) ppm.

### Example 143a

### 4-{6-[(1RS)-1-Hydroxyethyl]-8-[(3,3,3-trifluoropropyl)amino]imidazo[1,2-a]pyridin-3-yl}-2-methylbenzoic acid

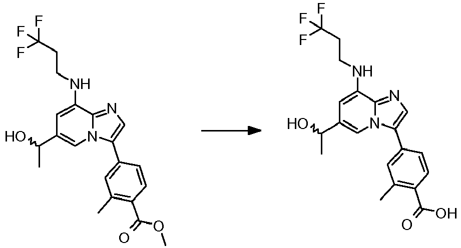

34.9 mg (83 µmol) methyl 4-{6-[(1RS)-1-hydroxyethyl]-8-[(3,3,3-trifluoropropyl)amino]imidazo[1,2-a]pyridin-3-yl}-2-methylbenzoate which was prepared according to intermediate example 141 b were transformed in analogy to intermediate example 8b to give after working up 34.1 mg of the title compound that was used without further purification.

### Example 144

### 4-{6-[(1RS)-1-Hydroxyethyl]-8-[(3,3,3-trifluoropropyl)amino]imidazo[1,2-a]pyridin-3-yl}-N,2-dimethylbenzamide

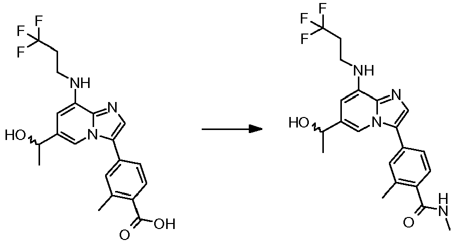

11 mg (27 µmol) 4-{6-[(1RS)-1-hydroxyethyl]-8-[(3,3,3-trifluoropropyl)amino]imidazo[1,2-a]pyridin-3-yl}-2-methylbenzoic acid which was prepared according to intermediate example 143a were transformed in analogy to example 8 to give after working up and purification 7.5 mg (63%) of the title compound.
¹H-NMR (CD₃OD): δ= 1.46 (3H), 2.46 (3H), 2.53-2.69 (2H), 2.91 (3H), 3.61 (2H), 4.80 (1 H), 6.32 (1 H), 7.44-7.55 (4H), 7.83 (1 H) ppm.

### Example 145

### N-ethyl-4-{6-[(1RS)-1-hydroxyethyl]-8-[(3,3,3-trifluoropropyl)amino]imidazo[1,2-a]pyridin-3-yl}-2-methylbenzamide

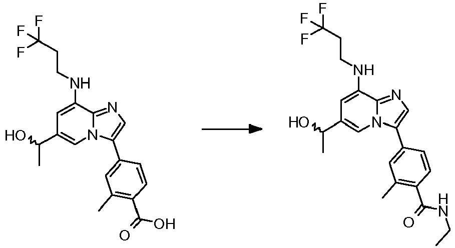

11 mg (27 µmol) 4-{6-[(1RS)-1-hydroxyethyl]-8-[(3,3,3-trifluoropropyl)amino]imidazo[1,2-a]pyridin-3-yl}-2-methylbenzoic acid which was prepared according to intermediate example 143a were transformed in analogy to example 8 using ethanamine to give after working up and purification 11.4 mg (97%) of the title compound.
¹H-NMR (CD₃OD): δ= 1.23 (3H), 1.46 (3H), 2.46 (3H), 2.52-2.69 (2H), 3.40 (2H), 3.61 (2H), 4.80 (1 H), 6.33 (1 H), 7.45-7.55 (4H), 7.83 (1 H) ppm.

### Example 146

### N-cyclopropyl-4-{6-(3-fluoro-2-methoxyphenoxy)-8-[(3,3,3-trifluoropropyl)amino]imidazo[1,2-a]pyridin-3-yl}-2-methylbenzamide

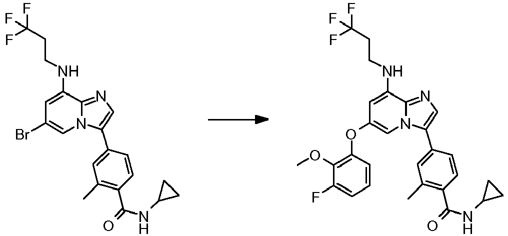

50 mg (104 µmol) 4-{6-bromo-8-[(3,3,3-trifluoropropyl)amino]imidazo[1,2-a]pyridin-3-yl}-N-cyclopropyl-2-methylbenzamide which was prepared according to intermediate example 42a were transformed in analogy to intermediate example 6-1 using 3-fluoro-2-methoxyphenol to give after working up and purification 10.0 mg (16%) of the title compound.
¹H-NMR (CDCl₃): δ= 0.62 (2H), 0.89 (2H), 2.43-2.58 (2H), 2.47 (3H), 2.91 (1H), 3.56 (2H), 3.97 (3H), 5.49 (1H), 5.92 (1H), 6.03 (1H), 6.73 (1H), 6.86-6.98 (2H), 7.31 (1 H), 7.33 (1 H), 7.40 (1 H), 7.48 (1 H), 7.53 (1 H) ppm.

### Example 147

### 4-{3-[4-(cyclopropylcarbamoyl)-3-methylphenyl]-8-[(3,3,3-trifluoropropyl)amino]imidazo[1,2-a]pyridin-6-yl}-N,2-dimethylbenzamide

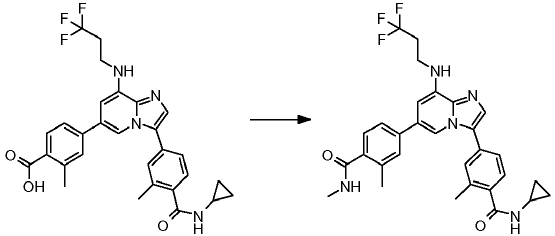

10 mg (19 µmol) 4-{3-[4-(cyclopropylcarbamoyl)-3-methylphenyl]-8-[(3,3,3-trifluoropropyl)amino]imidazo[1,2-a]pyridin-6-yl}-2-methylbenzoic acid which was prepared according to intermediate example 147a were transformed in analogy to example 8 to give after working up and purification 9.3 mg (86%) of the title compound.
¹H-NMR (CD₃OD): δ= 0.63 (2H), 0.82 (2H), 2.44 (3H), 2.46 (3H), 2.57-2.72 (2H), 2.88 (1H), 2.90 (3H), 3.68 (2H), 6.51 (1H), 7.37-7.52 (6H), 7.56 (1H), 7.90 (1H) ppm.

### Example 147a

### 4-{3-[4-(cyclopropylcarbamoyl)-3-methylphenyl]-8-[(3,3,3-trifluoropropyl)amino]imidazo[1,2-a]pyridin-6-yl}-2-methylbenzoic acid

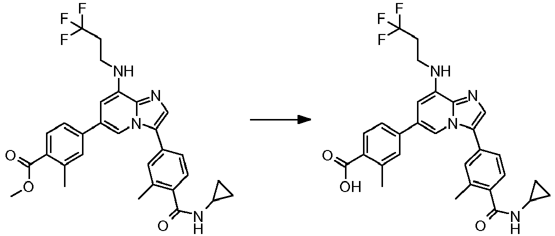

363 mg (659 µmol) methyl 4-{3-[4-(cyclopropylcarbamoyl)-3-methylphenyl]-8-[(3,3,3-trifluoropropyl)amino]imidazo[1,2-a]pyridin-6-yl}-2-methylbenzoate which was prepared according to intermediate example 147b were transformed in analogy to intermediate example 8b to give after working up and purification 265 mg (67%) of the title compound.

### Example 147b

### methyl 4-{3-[4-(cyclopropylcarbamoyl)-3-methylphenyl]-8-[(3,3,3-trifluoropropyl)amino]imidazo[1,2-a]pyridin-6-yl}-2-methylbenzoate

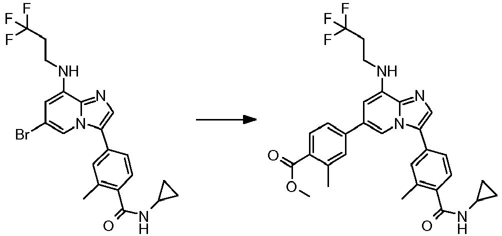

437 mg (907 µmol) 4-{6-bromo-8-[(3,3,3-trifluoropropyl)amino]imidazo[1,2-a]pyridin-3-yl}-N-cyclopropyl-2-methylbenzamide which was prepared according to intermediate example 42a were transformed in analogy to intermediate example 79c using [4-(methoxycarbonyl)-3-methylphenyl]boronic acid to give after working up and purification 397 mg (72%) of the title compound.

### Example 148

### 4-{3-[4-(cyclopropylcarbamoyl)-3-methylphenyl]-8-[(3,3,3-trifluoropropyl)amino]imidazo[1,2-a]pyridin-6-yl}-N-ethyl-2-methylbenzamide

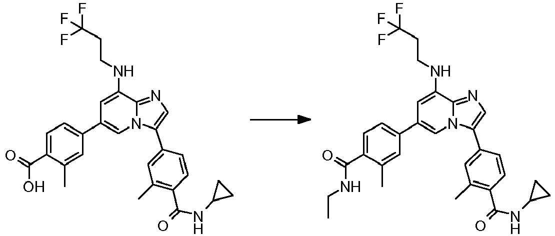

10 mg (19 µmol) 4-{3-[4-(cyclopropylcarbamoyl)-3-methylphenyl]-8-[(3,3,3-trifluoropropyl)amino]imidazo[1,2-a]pyridin-6-yl}-2-methylbenzoic acid which was prepared according to intermediate example 147a were transformed in analogy to example 8 using ethanamine to give after working up and purification 10.4 mg (94%) of the title compound.
¹H-NMR (CD₃OD): δ= 0.63 (2H), 0.82 (2H), 1.22 (3H), 2.44 (3H), 2.46 (3H), 2.55-2.73 (2H), 2.88 (1 H), 3.39 (2H), 3.68 (2H), 6.52 (1 H), 7.37-7.52 (6H), 7.56 (1 H), 7.90 (1 H) ppm.

### Example 149

### 4-{3-[4-(cyclopropylcarbamoyl)-3-methylphenyl]-8-[(3,3,3-trifluoropropyl)amino]imidazo[1,2-a]pyridin-6-yl}-2-methyl-N-(1-methylcyclopropyl)benzamide

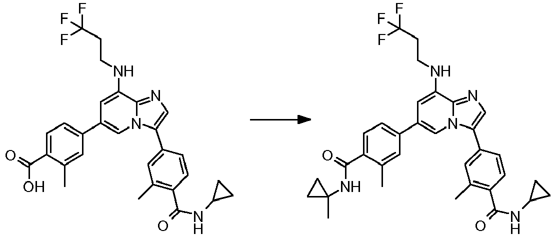

10 mg (19 µmol) -4-{3-[4-(cyclopropylcarbamoyl)-3-methylphenyl]-8-[(3,3,3 trifluoropropyl)amino]imidazo[1,2-a]pyridin-6-yl}-2-methylbenzoic acid which was prepared according to intermediate example 147a were transformed in analogy to example 8 using 1-methylcyclopropanaminium chloride to give after working up and purification 9.1 mg (83%) of the title compound.
¹H-NMR (CDCl₃): δ= 0.64 (2H), 0.76 (2H), 0.82-0.94 (4H), 1.52 (3H), 2.49 (3H), 2.51 (3H), 2.58 (2H), 2.94 (1H), 3.67 (2H), 5.51 (1H), 6.00 (1H), 6.08 (1H), 6.32 (1H), 7.28-7.43 (5H), 7.47 (1H), 7.54 (1H), 7.81 (1H) ppm.

### Example 150

### 4-(6-{4-[(1-cyanocyclopropyl)carbamoyl]-3-methylphenyl}-8-[(3,3,3-trifluoropropyl)amino]imidazo[1,2-a]pyridin-3-yl)-N-cyclopropyl-2-methylbenzamide

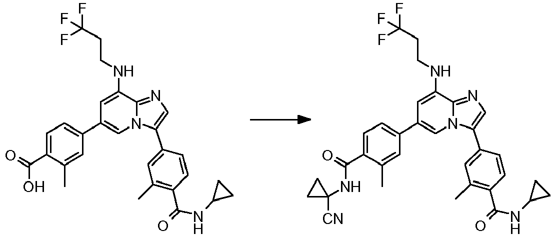

10 mg (19 µmol) 4-{3-[4-(cyclopropylcarbamoyl)-3-methylphenyl]-8-[(3,3,3-trifluoropropyl)amino]imidazo[1,2-a]pyridin-6-yl}-2-methylbenzoic acid which was prepared according to intermediate example 147a were transformed in analogy to example 8 using 1-cyanocyclopropanaminium chloride to give after working up and purification 5.3 mg (45%) of the title compound.
¹H-NMR (CD₃OD): δ= 0.62 (2H), 0.81 (2H), 1.34 (2H), 1.57 (2H), 2.45 (6H), 2.64 (2H), 2.87 (1H), 3.67 (2H), 6.51 (1H), 7.39-7.57 (7H), 7.90 (1H) ppm.

### Example 151

### 4-{3-[4-(cyclopropylcarbamoyl)-3-methylphenyl]-8-[(3,3,3-trifluoropropyl)amino]imidazo[1,2-a]pyridin-6-yl}-N-[1-(hydroxymethyl)cyclopropyl]-2-methylbenzamide

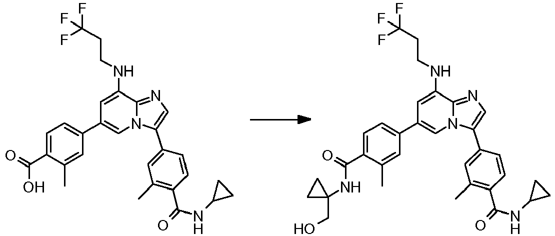

10 mg (19 µmol) 4-{3-[4-(cyclopropylcarbamoyl)-3-methylphenyl]-8-[(3,3,3-trifluoropropyl)amino]imidazo[1,2-a]pyridin-6-yl}-2-methylbenzoic acid which was prepared according to intermediate example 147a were transformed in analogy to example 8 using 1-(hydroxymethyl)cyclopropanaminium chloride to give after working up and purification 7.0 mg (59%) of the title compound.
¹H-NMR (CD₃OD): δ= 0.63 (2H), 0.82 (2H), 0.86-0.92 (4H), 2.44 (3H), 2.46 (3H), 2.56-2.73 (2H), 2.88 (1H), 3.63-3.74 (4H), 6.51 (1 H), 7.39-7.52 (6H), 7.56 (1H), 7.89 (1 H) ppm.

### Example 152

### 4-{3-[4-(cyclopropylcarbamoyl)-3-methylphenyl]-8-[(3,3,3-trifluoropropyl)amino]imidazo[1,2-a]pyridin-6-yl}-N-[rel-(1S,2S)-2-fluorocyclopropyl]-2-methylbenzamide

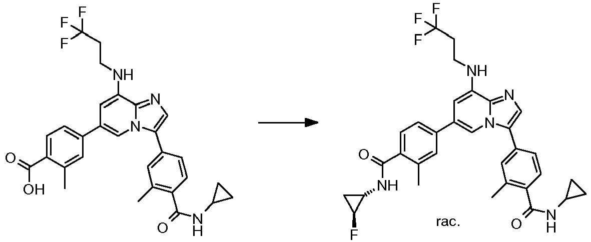

10 mg (19 µmol) 4-{3-[4-(cyclopropylcarbamoyl)-3-methylphenyl]-8-[(3,3,3-trifluoropropyl)amino]imidazo[1,2-a]pyridin-6-yl}-2-methylbenzoic acid which was prepared according to intermediate example 147a were transformed in analogy to example 8 using rel-(1S,2S)-2-fluorocyclopropanaminium chloride to give after working up and purification 6.7 mg (58%) of the title compound.
¹H-NMR (CD₃OD): δ= 0.62 (2H), 0.81 (2H), 1.05 (1 H), 1.18 (1 H), 2.44 (3H), 2.45 (3H), 2.64 (2H), 2.86 (2H), 3.67 (2H), 4.69 (1 H), 6.51 (1 H), 7.40 (1 H), 7.42 (1 H), 7.43-7.51 (4H), 7.55 (1H), 7.90 (1H) ppm.

### Example 153

### 4-{6-(4-carbamoyl-3-methylphenyl)-8-[(3,3,3-trifluoropropyl)amino]imidazo[1,2-a]pyridin-3-yl}-N-cyclopropyl-2-methylbenzamide

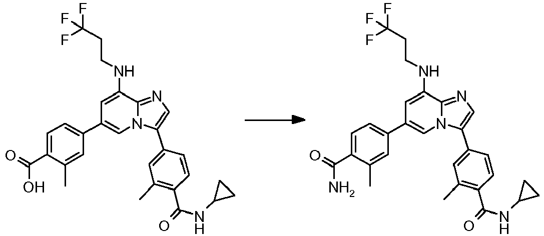

10 mg (19 µmol) 4-{3-[4-(cyclopropylcarbamoyl)-3-methylphenyl]-8-[(3,3,3-trifluoropropyl)amino]imidazo[1,2-a]pyridin-6-yl}-2-methylbenzoic acid which was prepared according to intermediate example 147a were transformed in analogy to example 8 using ammonia (0.5M in dioxane) to give after working up and purification 8.7 mg (83%) of the title compound.
¹H-NMR (CD₃OD): δ= 0.63 (2H), 0.82 (2H), 2.48 (3H), 2.49 (3H), 2.55-2.73 (2H), 2.88 (1H), 3.68 (2H), 6.52 (1H), 7.41-7.53 (6H), 7.56 (1H), 7.91 (1H) ppm.

### Example 154

### 4-{6-(4-{[rel-(1S,2S)-2-fluorocyclopropyl]carbamoyl}-3-methylphenyl)-8-[(3,3,3-trifluoropropyl)amino]imidazo[1,2-a]pyridin-3-yl}-N,2-dimethylbenzamide

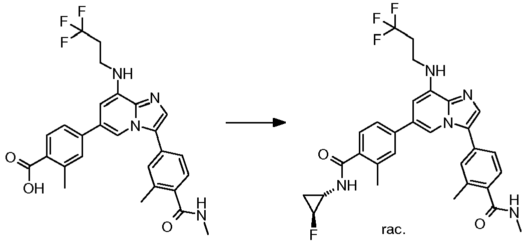

10 mg (20 µmol) 2-methyl-4-{3-[3-methyl-4-(methylcarbamoyl)phenyl]-8-[(3,3,3-trifluoropropyl)amino]imidazo[1,2-a]pyridin-6-yl}benzoic acid which was prepared according to intermediate example 154a were transformed in analogy to example 8 using rel-(1S,2S)-2-fluorocyclopropanaminium chloride to give after working up and purification 7.1 mg (61 %) of the title compound.
¹H-NMR (CDCl₃): δ= 1.04 (1 H), 1.27 (1 H), 2.52-2.64 (2H), 2.52 (6H), 3.02-3.09 (1 H), 3.03 (3H), 3.67 (2H), 4.76 (1H), 5.42 (1H), 5.90 (1H), 6.07 (1H), 6.32 (1H), 7.33 (1 H), 7.35 (1 H), 7.38-7.47 (3H), 7.50 (1 H), 7.55 (1 H), 7.83 (1 H) ppm.

### Example 154a

### 2-methyl-4-{3-[3-methyl-4-(methylcarbamoyl)phenyl]-8-[(3,3,3-trifluoropropyl)amino]imidazo[1,2-a]pyridin-6-yl}benzoic acid

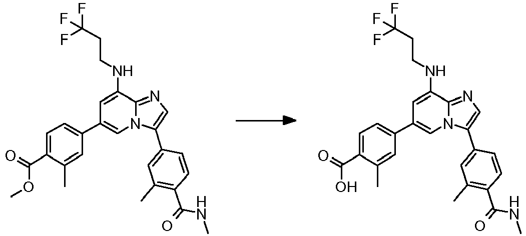

322 mg (614 µmol) methyl 2-methyl-4-{3-[3-methyl-4-(methylcarbamoyl)phenyl]-8-[(3,3,3-trifluoropropyl)amino]imidazo[1,2-a]pyridin-6-yl}benzoate which was prepared according to example 140 were transformed in analogy to intermediate example 8b to give after working up and purification 249 mg (72%) of the title compound.

### Example 155

### 4-{6-(4-{[1-(hydroxymethyl)cyclopropyl]carbamoyl}-3-methylphenyl)-8-[(3,3,3-trifluoropropyl)amino]imidazo[1,2-a]pyridin-3-yl}-N,2-dimethylbenzamide

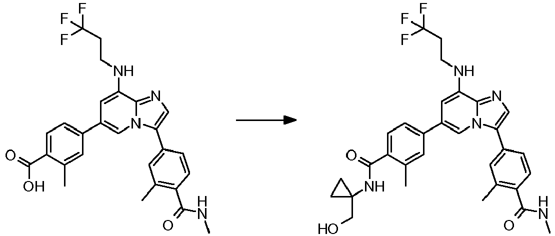

10 mg (20 µmol) 2-methyl-4-{3-[3-methyl-4-(methylcarbamoyl)phenyl]-8-[(3,3,3-trifluoropropyl)amino]imidazo[1,2-a]pyridin-6-yl}benzoic acid which was prepared according to intermediate example 154a were transformed in analogy to example 8 using 1-(hydroxymethyl)cyclopropanaminium chloride to give after working up and purification 7.5 mg (63%) of the title compound.
¹H-NMR (CD₃OD): δ= 0.87 (4H), 2.43 (3H), 2.46 (3H), 2.58-2.71 (2H), 2.91 (3H), 3.64-3.71 (4H), 6.50 (1H), 7.38-7.45 (3H), 7.50 (3H), 7.55 (1H), 7.90 (1H) ppm.

### Example 156

### 4-(6-{4-[(1-cyanocyclopropyl)carbamoyl]-3-methylphenyl}-8-[(3,3,3-trifluoropropyl)amino]imidazo[1,2-a]pyridin-3-yl)-N,2-dimethylbenzamide

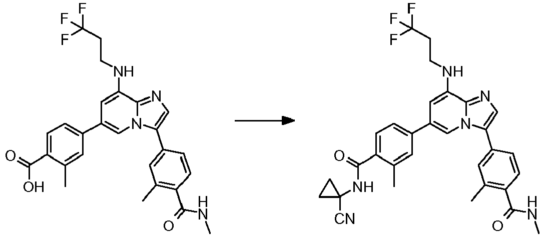

10 mg (20 µmol) 2-methyl-4-{3-[3-methyl-4-(methylcarbamoyl)phenyl]-8-[(3,3,3-trifluoropropyl)amino]imidazo[1,2-a]pyridin-6-yl}benzoic acid which was prepared according to intermediate example 154a were transformed in analogy to example 8 using 1-cyanocyclopropanaminium chloride to give after working up and purification 6.0 mg (53%) of the title compound.
¹H-NMR (CD₃OD): δ= 1.34 (2H), 1.57 (2H), 2.45 (3H), 2.46 (3H), 2.56-2.72 (2H), 2.91 (3H), 3.67 (2H), 6.50 (1H), 7.38-7.52 (6H), 7.56 (1H), 7.91 (1H) ppm.

### Example 157

### 2-methyl-N-(1-methylcyclopropyl)-4-{3-[3-methyl-4-(methylcarbamoyl)phenyl]-8-[(3,3,3-trifluoropropyl)amino]imidazo[1,2-a]pyridin-6-yl}benzamide

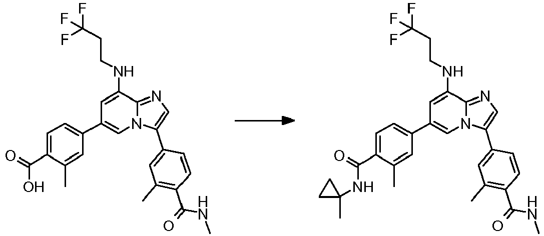

10 mg (20 µmol) 2-methyl-4-{3-[3-methyl-4-(methylcarbamoyl)phenyl]-8-[(3,3,3-trifluoropropyl)amino]imidazo[1,2-a]pyridin-6-yl}benzoic acid which was prepared according to intermediate example 154a were transformed in analogy to example 8 using 1-methylcyclopropanaminium chloride to give after working up and purification 5.2 mg (47%) of the title compound.
¹H-NMR (CDCl₃): δ= 0.76 (2H), 0.87 (2H), 1.52 (3H), 2.43-2.68 (2H), 2.49 (3H), 2.52 (3H), 3.04 (3H), 3.66 (2H), 5.82 (1H), 5.91 (1H), 6.10 (1H), 6.34 (1H), 7.28-7.43 (5H), 7.50 (1 H), 7.54 (1 H), 7.81 (1 H) ppm.

### Example 158

### 4-{6-[4-(cyclopropylcarbamoyl)-3-methylphenyl]-8-[(3,3,3-trifluoropropyl)amino]imidazo[1,2-a]pyridin-3-yl}-N,2-dimethylbenzamide

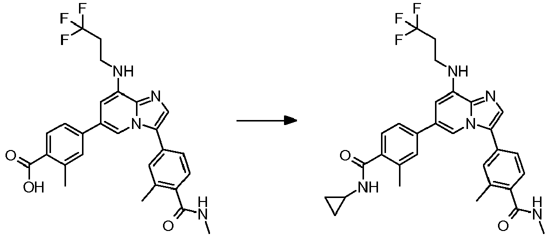

10 mg (20 µmol) 2-methyl-4-{3-[3-methyl-4-(methylcarbamoyl)phenyl]-8-[(3,3,3-trifluoropropyl)amino]imidazo[1,2-a]pyridin-6-yl}benzoic acid which was prepared according to intermediate example 154a were transformed in analogy to example 8 using cyclopropanamine to give after working up and purification 7.4 mg (65%) of the title compound.
¹H-NMR (CDCl₃): δ= 0.63 (2H), 0.89 (2H), 2.44-2.65 (2H), 2.50 (3H), 2.51 (3H), 2.91 (1 H), 3.04 (3H), 3.66 (2H), 5.43 (1 H), 5.89-6.02 (2H), 6.32 (1 H), 7.27-7.43 (5H), 7.49 (1 H), 7.54 (1 H), 7.82 (1 H) ppm.

### Example 159

### 4-{6-[4-(ethylcarbamoyl)-3-methylphenyl]-8-[(3,3,3-trifluoropropyl)amino]imidazo[1,2-a]pyridin-3-yl}-N,2-dimethylbenzamide

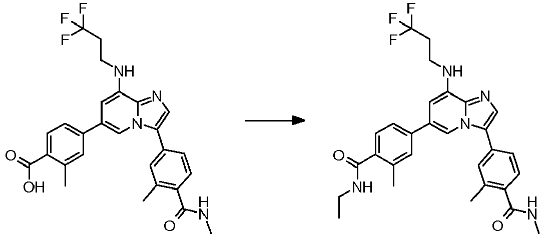

10 mg (20 µmol) 2-methyl-4-{3-[3-methyl-4-(methylcarbamoyl)phenyl]-8-[(3,3,3-trifluoropropyl)amino]imidazo[1,2-a]pyridin-6-yl}benzoic acid which was prepared according to intermediate example 154a were transformed in analogy to example 8 using ethanamine to give after working up and purification 7.6 mg (69%) of the title compound.
¹H-NMR (CDCl₃): δ= 1.26 (3H), 2.49-2.65 (2H), 2.51 (6H), 3.04 (3H), 3.50 (2H), 3.67 (2H), 5.44 (1 H), 5.81 (1 H), 5.94 (1 H), 6.34 (1 H), 7.30-7.45 (5H), 7.49 (1 H), 7.55 (1 H), 7.83 (1 H) ppm.

### Example 160

### 4-{6-(4-carbamoyl-3-methylphenyl)-8-[(3,3,3-trifluoropropyl)amino]imidazo[1,2-a]pyridin-3-yl}-N,2-dimethylbenzamide

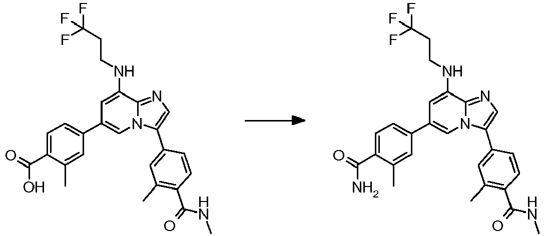

10 mg (20 µmol) 2-methyl-4-{3-[3-methyl-4-(methylcarbamoyl)phenyl]-8-[(3,3,3-trifluoropropyl)amino]imidazo[1,2-a]pyridin-6-yl}benzoic acid which was prepared according to intermediate example 154a were transformed in analogy to example 8 using ammonia (0.5M in dioxane) to give after working up and purification 6.3 mg (60%) of the title compound.
¹H-NMR (CD₃OD): δ= 2.46 (3H), 2.48 (3H), 2.55-2.72 (2H), 2.91 (3H), 3.67 (2H), 6.51 (1H), 7.41-7.52 (6H), 7.56 (1H), 7.91 (1 H) ppm.

### Example 161

### N-cyclopropyl-4-{8-[(2,2-difluoroethyl)amino]-6-(3-fluorophenoxy)imidazo[1,2-a]pyridin-3-yl}-2-methylbenzamide

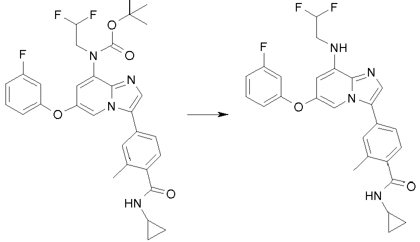

To a solution of 174.18 mg (0.3 mmol) tert-butyl {3-[4-(cyclopropylcarbamoyl)-3-methylphenyl]-6-(3-fluorophenoxy)imidazo[1,2-a]pyridin-8-yl}(2,2-difluoroethyl)carbamate in 1.0 mL DCM were added 3 mL TFA and 200 µL water and the mixture was stirred for 1 h at overnight.
After evaporation, the residue was purified by HPLC to yield 36 mg (24 %) of the title compound. UPLC MS: RT = 1.18 min; m/z (ES+) 481.5 [MH+]; required MW = 480.5. 1 H-NMR (300 MHz ,DMSO-d6), δ [ppm]= 0.43-0.54 (2H), 0.60-0.70 (2H), 2.33 (3H), 2.79 (1H), 3.60-3.81 (3H), 6.32 (1H), 6.66 (1H), 6.81-6.94 (3H), 7.29-7.40 (2H), 7.40-7.50 (2H), 7.63-7.75 (2H), 8.28 (1H).

### Example 161 a

### tert-butyl {3-[4-(cyclopropylcarbamoyl)-3-methylphenyl]-6-(3-fluorophenoxy)imidazo[1,2-a]pyridin-8-yl}(2,2-difluoroethyl)carbamate

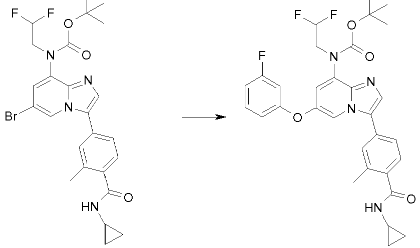

To a solution of 164.83 mg (0.3 mmol) tert-butyl {6-bromo-3-[4-(cyclopropylcarbamoyl)-3-methylphenyl]imidazo[1,2-a]pyridin-8-yl}(2,2-difluoroethyl)carbamate in 4.0 mL dioxane were added 782 mg (2.4 mmol) cesium carbonate, 11.88 mg (0.12 mmol) copper(I) chloride and 12.37 mg (0.12 mmol) dimethylglycine and the mixture was stirred for 1 h at 160°C. The solvent was removed in vaccuo and the residue was used in the next step without further purification.
UPLC MS: RT = 1.37 min; m/z (ES+) 581.6 [MH+]; required MW = 580.6.

### Example 161b

### tert-butyl {6-bromo-3-[4-(cyclopropylcarbamoyl)-3-methylphenyl]imidazo[1,2-a]pyridin-8-yl}(2,2-difluoroethyl)carbamate

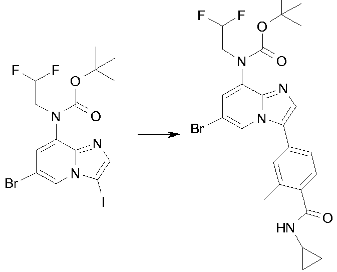

To a solution of 3.5 g (6.97 mmol) tert-butyl (6-bromo-3-iodoimidazo[1,2-a]pyridin-8-yl)(2,2-difluoroethyl)carbamate in 100 mL THF were added 2.29 g (10.46 mmol) [4-(cyclopropylcarbamoyl)-3-methylphenyl]boronic acid, 0.569 g (0.697 mmol) Pd(dppf)Cl₂ and 20.91 mL (20.91 mmol, 1M aqueous solution) potassium carbonate and the mixture was stirred for 2 h at 55°C to give, after working up and purification, 2.41 g (62.9 %) of the title compound.
UPLC MS: RT = 1.28 min; m/z (ES+) 550.46 [MH+]; required MW = 549.4. 1 H-NMR (300 MHz ,DMSO-d6), δ [ppm]= 0.46-0.55 (2H), 0.62-0.71 (2H), 1.33 (10H), 2.37 (3H), 2.76-2.87 (1H), 4.17 (2H), 7.38-7.45 (2H), 7.48-7.57 (2H), 7.79 (1H), 8.34 (1 H), 8.56 (1 H).

### Example 161c

### tert-butyl (6-bromo-3-iodoimidazo[1,2-a]pyridin-8-yl)(2,2-difluoroethyl)carbamate

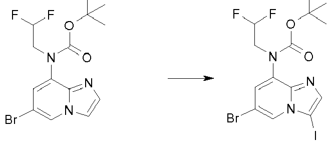

To a solution of 5.9 g (15.68 mmol) tert-butyl (6-bromoimidazo[1,2-a]pyridin-8-yl)(2,2-difluoroethyl)carbamate in 80 mL DMF were added 17.64 g (78.41 mmol) 1-iodopyrrolidine-2,5-dione and the mixture was stirred for 2 h at 40°C to give, after working up and purification, 5.91 g (80 %) of the title compound.
UPLC MS: RT = 1.39 min; m/z (ES+) 503.1 [MH+]; required MW = 502.1. 1H-NMR (300 MHz ,DMSO-d6), δ [ppm]= 1.29 (10H), 4.12 (2H), 7.45 (1H), 7.72 (1H), 8.43 (1H).

### Example 161 d

### tert-butyl (6-bromoimidazo[1,2-a]pyridin-8-yl)(2,2-difluoroethyl)carbamate

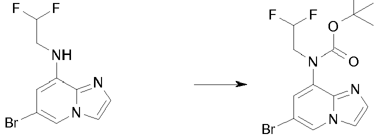

To a solution of 5.39 g (19.52 mmol) 6-bromo-N-(2,2-difluoroethyl)imidazo[1,2-a]pyridin-8-amine in 250 mL THF were added 19.76 g (27 mL, 195.23 mmol) TEA, 0.48 g (3.9 mmol) DIPEA and a solution of 29.82 g (136.66 mmol) di-tert-butyl dicarbonate in 50 mL THF and the mixture was stirred for 48 h at 40°C to give, after working up and purification, 7.42 g (94.2 %) of the title compound.
UPLC MS: RT = 1.20 min; m/z (ES+) 377.2 [MH+]; required MW = 376.2. 1 H-NMR (300 MHz ,DMSO-d6), δ [ppm]= 1.22-1.40 (10H), 4.14 (2H), 7.31 (1H), 7.57 (1H), 7.94 (1 H), 8.86 (1 H).

### Example 161e

### 6-bromo-N-(2,2-difluoroethyl)imidazo[1,2-a]pyridin-8-amine

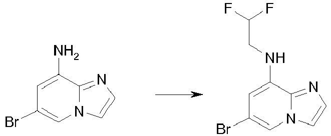

To a solution of 8.00 g (100 mmol) freshly prepared difluoroacetaldehyde in 560 mL DCM was added 5.30 g (25 mmol) 6-bromoimidazo[1,2-a]pyridin-8-amine, 26.49 g (125 mmol) sodium triacetoxy borohydride and 28.5 g (18.56 mL, 250 mmol) TFA and the mixture was stirred for 72 h at rt to give, after working up and purification, 4.0 g (58 %) of the title compound.
UPLC MS: RT = 0.71 min; m/z (ES+) 277.1 [MH+]; required MW = 276.1. 1 H-NMR (300 MHz ,DMSO-d6), δ [ppm]= 3.69 (2H), 6.36 (1 H), 6.54 (1 H), 7.41 (1 H), 7.77 (1 H), 8.10 (1H).

### Example 161 f

### difluoroacetaldehyde

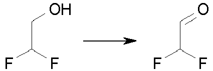

To a solution of 13.96 g (110 mmol) oxalyl chloride in 280 mL DCM was added dropwise at -78°C 52.28 mL (220 mmol) DMSO. After stirring for 2 min, 8.21 g (100 mmol) 2,2-difluoroethanol in 283 mL DCM were slowly added and the mixture was stirred for 1 h at -78°C. 30.35 g (300 mmol) TEA were added and the mixture was left to come to rt over 90 min to yield the title compound in solution, which was used without further work-up in the next step.

### Example 162

### N-cyclopropyl-4-{8-[(2,2-difluoroethyl)amino]-6-(4-methoxyphenoxy)imidazo[1,2-a]pyridin-3-yl}-2-methylbenzamide

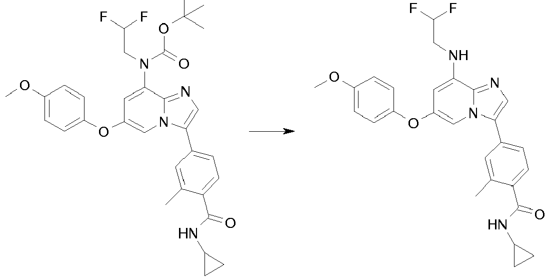

178 mg (300 µmol) tert-butyl {3-[4-(cyclopropylcarbamoyl)-3-methylphenyl]-6-(4-methoxyphenoxy)imidazo[1,2-a]pyridin-8-yl}(2,2-difluoroethyl)carbamate which was prepared according to intermediate example 162a were transformed in analogy to example 161 using TFA to give after working up and purification 6.3 mg (3%) of the title compound.
UPLC MS: RT = 1.11 min; m/z (ES+) 493.5 [MH+]; required MW = 492.5. 1H-NMR (300 MHz ,DMSO-d6), δ [ppm]= 0.44-0.54 (2H), 0.61-0.69 (2H), 2.30 (3H), 2.79 (1H), 3.65 (3H), 3.67-3.71 (3H), 6.49 (1H), 6.52-6.59 (1H), 6.75-6.80 (2H), 6.87-6.94 (2H), 7.00-7.06 (2H), 7.32-7.42 (2H), 7.74 (1H), 8.29 (1H)

### Example 162a

### tert-butyl {3-[4-(cyclopropylcarbamoyl)-3-methylphenyl]-6-(4-methoxyphenoxy)imidazo[1,2-a]pyridin-8-yl}(2,2-difluoroethyl)carbamate

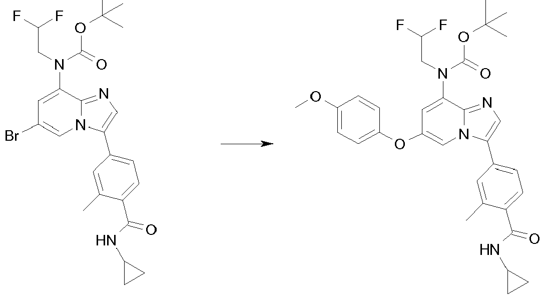

165 mg (300 µmol) tert-butyl {6-bromo-3-[4-(cyclopropylcarbamoyl)-3-methylphenyl]imidazo[1,2-a]pyridin-8-yl}(2,2-difluoroethyl)carbamate which was prepared according to intermediate example 161 b were transformed in analogy to example 161 a using 4-methoxyphenol to give after evaporation the crude title compound which was used without purification in the next step.
UPLC MS: RT = 1.34 min; m/z (ES+) 593.6 [MH+]; required MW = 592.6.

### Example 163

### N-cyclopropyl-4-{8-[(2,2-difluoroethyl)amino]-6-(2,3-difluorophenoxy)imidazo[1,2-a]pyridin-3-yl}-2-methylbenzamide

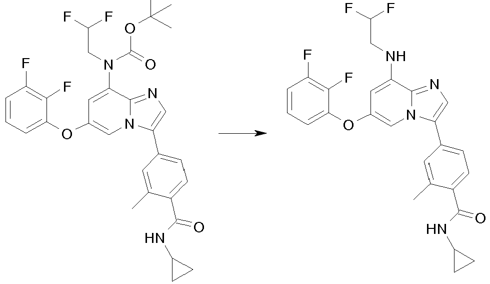

179 mg (300 µmol) tert-butyl {3-[4-(cyclopropylcarbamoyl)-3-methylphenyl]-6-(2,3-difluorophenoxy)imidazo[1,2-a]pyridin-8-yl}(2,2-difluoroethyl)carbamate which was prepared according to intermediate example 163a were transformed in analogy to example 161 using TFA to give after working up and purification 17.9 mg (11 %) of the title compound.
UPLC MS: RT = 1.25 min; m/z (ES+) 499.5 [MH+]; required MW = 498.5. 1 H-NMR (300 MHz ,DMSO-d6), δ [ppm]= 0.44-0.54 (2H), 0.60-0.70 (2H), 2.33 (3H), 2.79 (1H), 3.63-3.81 (3H), 6.39-6.44 (1H), 6.67-6.74 (1H), 6.93 (1H), 7.05-7.21 (2H), 7.32-7.39 (1H), 7.40-7.47 (2H), 7.71 (2H), 8.29 (1H)

### Example 163a

### tert-butyl {3-[4-(cyclopropylcarbamoyl)-3-methylphenyl]-6-(2,3-difluorophenoxy)imidazo[1,2-a]pyridin-8-yl}(2,2-difluoroethyl)carbamate

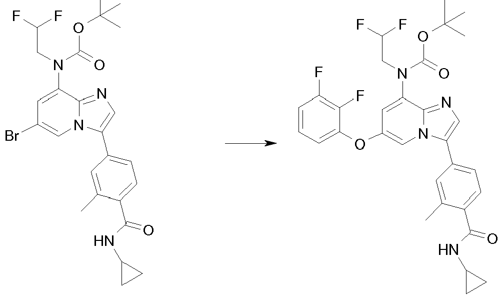

165 mg (300 µmol) tert-butyl {6-bromo-3-[4-(cyclopropylcarbamoyl)-3-methylphenyl]imidazo[1,2-a]pyridin-8-yl}(2,2-difluoroethyl)carbamate which was prepared according to intermediate example 161 b were transformed in analogy to example 161a using 2,3-difluorophenol to give after evaporation the crude title compound which was used without purification in the next step.
UPLC MS: RT = 1.37 min; m/z (ES+) 599.6 [MH+]; required MW = 598.6.

### Example 164

### N-cyclopropyl-4-{8-[(2,2-difluoroethyl)amino]-6-(5-fluoro-2-methylphenoxy)imidazo[1,2-a]pyridin-3-yl}-2-methylbenzamide

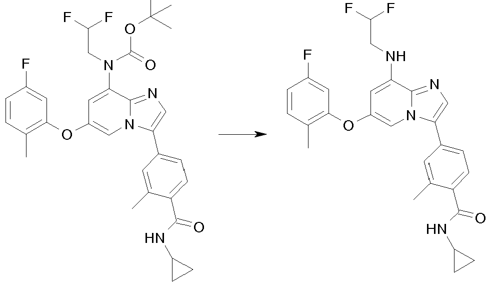

179 mg (300 µmol) tert-butyl {3-[4-(cyclopropylcarbamoyl)-3-methylphenyl]-6-(5-fluoro-2-methylphenoxy)imidazo[1,2-a]pyridin-8-yl}(2,2-difluoroethyl)carbamate which was prepared according to intermediate example 164a were transformed in analogy to example 161 using TFA to give after working up and purification 19.6 mg (13 %) of the title compound.
UPLC MS: RT = 1.30 min; m/z (ES+) 495.5 [MH+]; required MW = 494.5. 1 H-NMR (300 MHz ,DMSO-d6), δ [ppm]= 0.44-0.52 (2H), 0.60-0.69 (2H), 2.20 (3H), 2.31 (3H), 2.79 (1H), 3.71 (3H), 6.30-6.35 (1H), 6.65 (1H), 6.74 (1H), 6.84 (1H), 7.23-7.31 (1H), 7.32-7.37 (1H), 7.37-7.44 (2H), 7.50 (1H), 7.67 (1H), 8.28 (1H).

### Example 164a

### tert-butyl {3-[4-(cyclopropylcarbamoyl)-3-methylphenyl]-6-(5-fluoro-2-methylphenoxy)imidazo[1,2-a]pyridin-8-yl}(2,2-difluoroethyl)carbamate

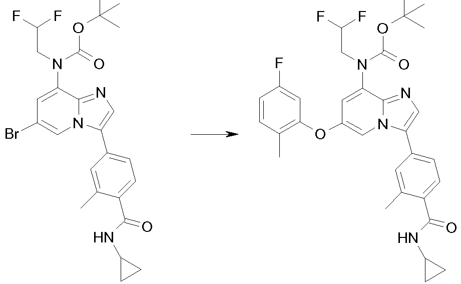

165 mg (300 µmol) tert-butyl {6-bromo-3-[4-(cyclopropylcarbamoyl)-3-methylphenyl]imidazo[1,2-a]pyridin-8-yl}(2,2-difluoroethyl)carbamate which was prepared according to intermediate example 161 b were transformed in analogy to example 161a using 5-fluoro-2-methylphenol to give after evaporation the crude title compound which was used without purification in the next step.
UPLC MS: RT = 1.42 min; m/z (ES+) 595.6 [MH+]; required MW = 594.6.

### Example 165

### N-cyclopropyl-4-{8-[(2,2-difluoroethyl)amino]-6-(2,5-difluorophenoxy)imidazo[1,2-a]pyridin-3-yl}-2-methylbenzamide

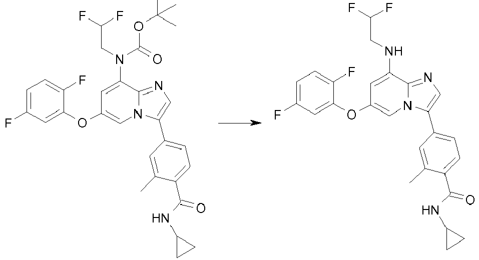

179 mg (300 µmol) tert-butyl {3-[4-(cyclopropylcarbamoyl)-3-methylphenyl]-6-(5-fluoro-2-methylphenoxy)imidazo[1,2-a]pyridin-8-yl}(2,2-difluoroethyl)carbamate which was prepared according to intermediate example 165a were transformed in analogy to example 161 using TFA to give after working up and purification 11.6 mg (7 %) of the title compound.
UPLC MS: RT = 1.24 min; m/z (ES+) 499.5 [MH+]; required MW = 498.5. 1 H-NMR (300 MHz ,DMSO-d6), δ [ppm]= 0.44-0.53 (2H), 0.60-0.69 (2H), 2.33 (3H), 2.79 (1H), 3.63-3.81 (3H), 6.40 (1H), 6.66-6.75 (1H), 6.91-7.07 (2H), 7.33-7.48 (4H), 7.67-7.76 (2H), 8.29 (1H).

### Example 165a

### tert-butyl {3-[4-(cyclopropylcarbamoyl)-3-methylphenyl]-6-(5-fluoro-2-methylphenoxy)imidazo[1,2-a]pyridin-8-yl}(2,2-difluoroethyl)carbamate

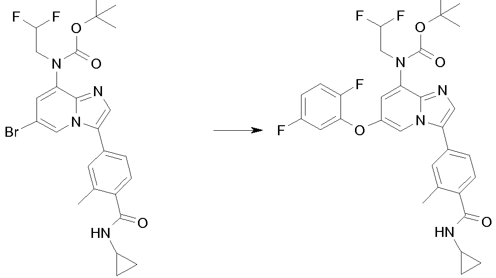

165 mg (300 µmol) tert-butyl {6-bromo-3-[4-(cyclopropylcarbamoyl)-3-methylphenyl]imidazo[1,2-a]pyridin-8-yl}(2,2-difluoroethyl)carbamate which was prepared according to intermediate example 161b were transformed in analogy to example 161a using 2,5-difluorophenol to give after evaporation the crude title compound which was used without purification in the next step.
UPLC MS: RT = 1.36 min; m/z (ES+) 599.6 [MH+]; required MW = 598.6.

### Example 166

### N-cyclopropyl-4-{8-[(2,2-difluoroethyl)amino]-6-(2,5-difluorophenoxy)imidazo[1,2-a]pyridin-3-yl}-2-methylbenzamide

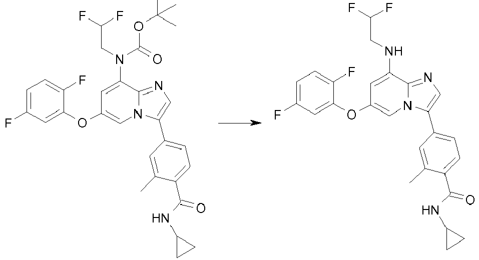

179 mg (300 µmol) tert-butyl {3-[4-(cyclopropylcarbamoyl)-3-methylphenyl]-6-(3-fluoro-2-methylphenoxy)imidazo[1,2-a]pyridin-8-yl}(2,2-difluoroethyl)carbamate which was prepared according to intermediate example 166a were transformed in analogy to example 161 using TFA to give after working up and purification 44.7 mg (24 %) of the title compound.
UPLC MS: RT = 1.31 min; m/z (ES+) 495.5 [MH+]; required MW = 494.5. 1H-NMR (300 MHz ,DMSO-d6), δ [ppm]= 0.43-0.53 (2H), 0.65 (2H), 2.15 (3H), 2.31 (3H), 2.79 (1H), 3.72 (3H), 6.30-6.37 (1H), 6.64 (1H), 6.74 (1H), 6.87-6.97 (1H), 7.14 (1H), 7.30-7.43 (3H), 7.48 (1H), 7.66 (1H), 8.28 (1H).

### Example 166a

### tert-butyl {3-[4-(cyclopropylcarbamoyl)-3-methylphenyl]-6-(3-fluoro-2-methylphenoxy)imidazo[1,2-a]pyridin-8-yl}(2,2-difluoroethyl)carbamate

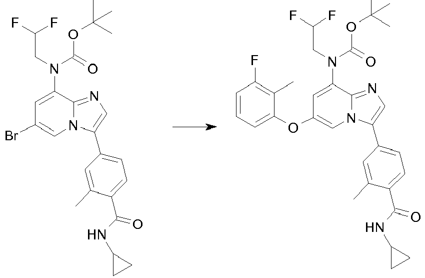

165 mg (300 µmol) tert-butyl {6-bromo-3-[4-(cyclopropylcarbamoyl)-3-methylphenyl]imidazo[1,2-a]pyridin-8-yl}(2,2-difluoroethyl)carbamate which was prepared according to intermediate example 161 b were transformed in analogy to example 161a using 3-fluoro-2-methylphenol to give after evaporation the crude title compound which was used without purification in the next step.
UPLC MS: RT = 1.43 min; m/z (ES+) 595.6 [MH+]; required MW = 594.6.

### Example 167

### N-cyclopropyl-4-{8-[(2,2-difluoroethyl)amino]-6-(3-fluoro-4-methoxyphenoxy)imidazo[1,2-a]pyridin-3-yl}-2-methylbenzamide

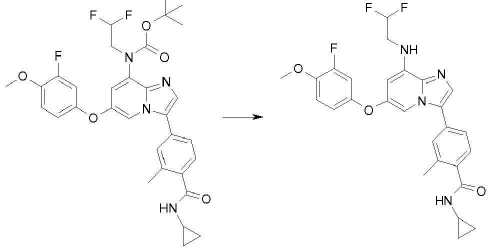

179 mg (300 µmol) N-cyclopropyl-4-{8-[(2,2-difluoroethyl)amino]-6-(3-fluoro-4-methoxyphenoxy)imidazo[1,2-a]pyridin-3-yl}-2-methylbenzamide which was prepared according to intermediate example 167a were transformed in analogy to example 161 using TFA to give after working up and purification 10.8 mg (4 %) of the title compound.
UPLC MS: RT = 1.22 min; m/z (ES+) 511.5 [MH+]; required MW = 510.5. 1H-NMR (300 MHz ,DMSO-d6), δ [ppm]= 0.45-0.53 (2H), 0.62-0.69 (2H), 2.32 (3H), 2.76-2.83 (1H), 3.66 (3H), 3.68-3.74 (3H), 6.36 (1 H), 6.60-6.63 (1 H), 6.85-6.91 (1 H), 7.03-7.14 (2H), 7.33-7.39 (1H), 7.40-7.45 (2H), 7.54 (1H), 7.72 (1H), 8.28 (1H).

### Example 167a

### N-cyclopropyl-4-{8-[(2,2-difluoroethyl)amino]-6-(3-fluoro-4-methoxyphenoxy)imidazo[1,2-a]pyridin-3-yl}-2-methylbenzamide

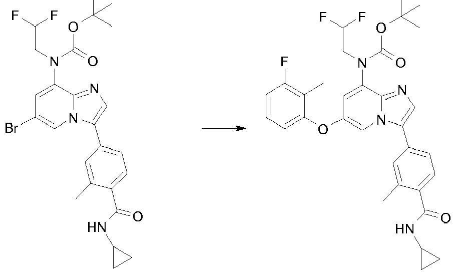

165 mg (300 µmol) tert-butyl {6-bromo-3-[4-(cyclopropylcarbamoyl)-3-methylphenyl]imidazo[1,2-a]pyridin-8-yl}(2,2-difluoroethyl)carbamate which was prepared according to intermediate example 161b were transformed in analogy to example 161a using 3-fluoro-4-methoxyphenol to give after evaporation the crude title compound which was used without purification in the next step.
UPLC MS: RT = 1.34 min; m/z (ES+) 611.6 [MH+]; required MW = 610.6.

### Example 168

### tert-butyl {3-[4-(cyclopropylcarbamoyl)-3-methylphenyl]-6-(2-hydroxybenzoyl)imidazo[1,2-a]pyridin-8-yl}(2,2-difluoroethyl)carbamate

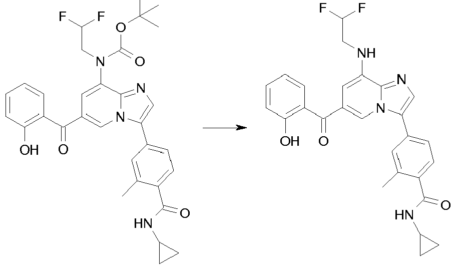

179 mg (300 µmol) tert-butyl {3-[4-(cyclopropylcarbamoyl)-3-methylphenyl]-6-(2-hydroxybenzoyl)imidazo[1,2-a]pyridin-8-yl}(2,2-difluoroethyl)carbamate which was prepared according to intermediate example 168a were transformed in analogy to example 161 using TFA to give after working up and purification 7.2 mg (4 %) of the title compound.
UPLC MS: RT = 1.23 min; m/z (ES+) 491.5 [MH+]; required MW = 490.5. 1H-NMR (300 MHz ,DMSO-d6), δ [ppm]= 0.43-0.54 (2H), 0.59-0.69 (2H), 2.36 (3H), 2.74-2.86 (1H), 3.80 (3H), 6.72 (1H), 6.83 (1H), 7.24 (2H), 7.29 (1H), 7.40-7.48 (3H), 7.50-7.56 (2H), 7.78 (1H), 8.34 (1H), 8.51 (1H)

### Example 168a

### tert-butyl {3-[4-(cyclopropylcarbamoyl)-3-methylphenyl]-6-(2-hydroxybenzoyl)imidazo[1,2-a]pyridin-8-yl}(2,2-difluoroethyl)carbamate

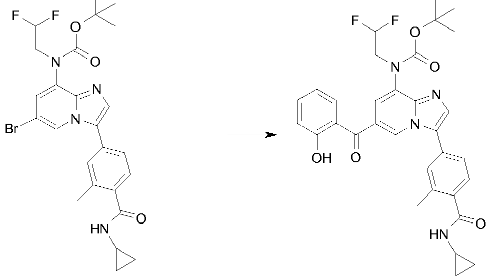

To a suspension of 240 mg (437 µmol) tert-butyl {6-bromo-3-[4-(cyclopropylcarbamoyl)-3-methylphenyl]imidazo[1,2-a]pyridin-8-yl}(2,2-difluoroethyl)carbamate which was prepared according to intermediate example 161b in 10 mL toluene were added 3 mg (7 µmol) butyldi-1-adamantylphosphine, 1 mg (4.37 µmol) palladium(II) acetate, 90 mg (655 µmol) 2-hydroxyphenylboronic acid and 70 µL (437 µmol) N,N,N',N'-tetramethylenediamine and the mixture was heated in an autoclave at 100°C at 12 bar carbon monoxide pressure for 22h.The oranic solvent was removed in vaccuo, dissolved in ethyl acetate, washed with 1 N NaOH and water, dried and evaporated to yield 190 mg (73 %) of the crude title compound which was used in the next step without further purification.
UPLC MS: RT = 1.34 min; m/z (ES+) 611.6 [MH+]; required MW = 610.6. 1 H-NMR (300 MHz ,DMSO-d6), δ [ppm]= 0.45-0.54 (2H), 0.61-0.69 (2H), 1.29-1.43 (9H), 2.37 (3H), 2.80 (1 H), 4.14-4.30 (2H), 7.23-7.31 (3H), 7.45 (3H), 7.59 (1 H), 7.74 (1 H), 7.93 (1 H), 8.36 (1 H), 9.07 (1 H)

### Example 169

### N-cyclopropyl-4-{6-(3-fluorophenoxy)-8-[(2-methoxyethyl)amino]imidazo[1,2-a]pyridin-3-yl}-2-methylbenzamide

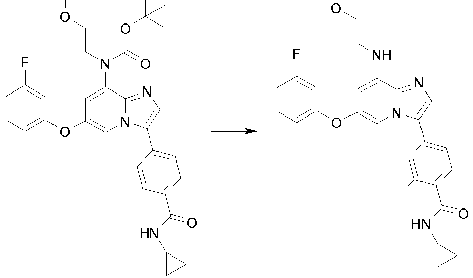

163.04 mg (0.284 mmol) tert-butyl {3-[4-(cyclopropylcarbamoyl)-3-methylphenyl]-6-(3-fluorophenoxy)imidazo[1,2-a]pyridin-8-yl}(2-methoxyethyl)carbamate which was prepared according to intermediate example 169a were transformed in analogy to example 161 using TFA to give after working up and purification 14.7 mg (10 %) of the title compound.
UPLC MS: RT = 1.12 min; m/z (ES+) 475.5 [MH+]; required MW = 474.5.

### Example 169a

### tert-butyl {3-[4-(cyclopropylcarbamoyl)-3-methylphenyl]-6-(3-fluorophenoxy)imidazo[1,2-a]pyridin-8-yl}(2-methoxyethyl)carbamate

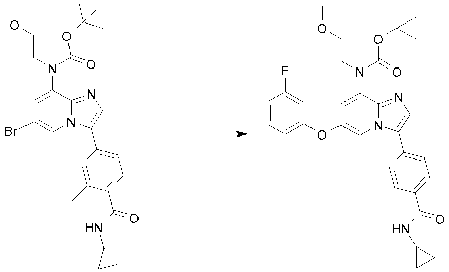

163.04 mg (0.3 mmol) tert-butyl {6-bromo-3-[4-(cyclopropylcarbamoyl)-3-methylphenyl]imidazo[1,2-a]pyridin-8-yl}(2,2-difluoroethyl)carbamate which was prepared according to intermediate example 169b were transformed in analogy to example 161a using 3-fluorophenol to give after evaporation the crude title compound which was used without purification in the next step.
UPLC MS: RT = 1.22 min; m/z (ES+) 575.7 [MH+]; required MW = 574.7.

### Example 169b

### tert-butyl {6-bromo-3-[4-(cyclopropylcarbamoyl)-3-methylphenyl]imidazo[1,2-a]pyridin-8-yl}(2-methoxyethyl)carbamate

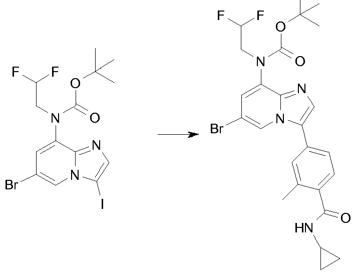

4.76 g (9.59 mmol) tert-butyl (6-bromo-3-iodoimidazo[1,2-a]pyridin-8-yl)(2-methoxyethyl)carbamate which was prepared according to intermediate example 169c were transformed in analogy to example 161 b using [4-(cyclopropylcarbamoyl)-3-methylphenyl]boronic acid to give, after working up and purification, 3.96 g (73.7 %) of the title compound.
UPLC MS: RT = 1.17 min; m/z (ES+) 544.5 [MH+]; required MW = 543.5. 1H-NMR (300 MHz ,DMSO-d6), δ [ppm]= 0.46-0.54 (2H), 0.62-0.71 (2H), 1.31 (9H), 2.37 (3H), 2.82 (1H), 3.15 (3H), 3.42 (2H), 3.85 (2H), 7.34 (1H), 7.39-7.44 (1H), 7.49-7.55 (2H), 7.76 (1H), 8.33 (1H), 8.52 (1H).

### Example 169c

### tert-butyl (6-bromo-3-iodoimidazo[1,2-a]pyridin-8-yl)(2-methoxyethyl)carbamate

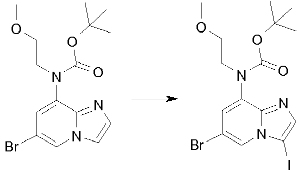

9.61 g (25.96 mmol) tert-butyl (6-bromoimidazo[1,2-a]pyridin-8-yl)(2-methoxyethyl)carbamate which was prepared according to intermediate example 169d were transformed in analogy to example 161 c using 1-iodopyrrolidine-2,5-dione to give, after working up and purification, 9.62 g (74.7 %) of the title compound.
UPLC MS: RT = 1.34 min; m/z (ES+) 497.1 [MH+]; required MW = 496.1. 1H-NMR (300 MHz ,DMSO-d6), δ [ppm]= 1.27 (9H), 3.11 (3H), 3.39 (2H), 3.80 (2H), 7.38 (1 H), 7.70 (1 H), 8.40 (1 H).

### Example 169d

### tert-butyl (6-bromoimidazo[1,2-a]pyridin-8-yl)(2-methoxyethyl)carbamate

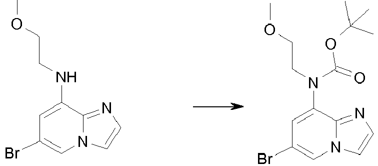

7.3 g (27.02 mmol) 6-bromo-N-(2-methoxyethyl)imidazo[1,2-a]pyridin-8-amine which was prepared according to intermediate example 169e were transformed in analogy to example 161d using di-tert-butyl dicarbonate to give, after working up and purification, 9.62 g (90.9 %) of the title compound.
UPLCMS: RT = 1.13 min; m/z (ES+) 371.2 [MH+]; required MW = 370.2. 1 H-NMR (300 MHz ,DMSO-d6), δ [ppm]= 1.27 (9H), 3.12 (3H), 3.39 (2H), 3.82 (2H), 7.24 (1 H), 7.55 (1 H), 7.91 (1 H), 8.81 (1 H).

### Example 169e

### 6-bromo-N-(2-methoxyethyl)imidazo[1,2-a]pyridin-8-amine

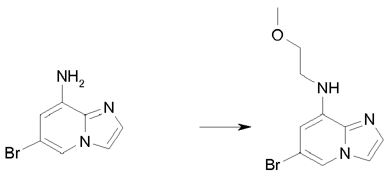

8.2 g (38.72 mmol) 6-bromoimidazo[1,2-a]pyridin-8-amine were transformed in analogy to example 161e using methoxyacetaldehyde which was prepared according to intermediate example 169f to give, after working up and purification, 7.55 g (72.2 %) of the title compound.
UPLC MS: RT = 0.69 min; m/z (ES+) 271.1 [MH+]; required MW = 270.1. 1H-NMR (300 MHz ,DMSO-d6), δ [ppm]= 3.25 (3H), 3.32-3.38 (2H), 3.46-3.56 (2H), 6.08 (1H), 6.18 (1 H), 7.38 (1 H), 7.75 (1 H), 8.03 (1 H).

### Example 169f

### methoxyacetaldehyde

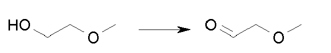

15.2 g (200 mmol) 2-methoxyethanol were transformed in analogy to example 161f using 2-methoxyethanol to give the title compound in solution, which was used without further work-up in the next step.

### Example 170

### N-cyclopropyl-4-{8-[(2,2-difluoroethyl)amino]-6-(4-methoxyphenoxy)imidazo[1,2-a]pyridin-3-yl}-2-methylbenzamide

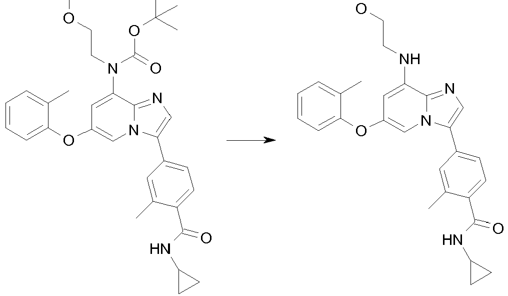

163 mg (286 µmol) tert-butyl {3-[4-(cyclopropylcarbamoyl)-3-methylphenyl]-6-(2-methylphenoxy)imidazo[1,2-a]pyridin-8-yl}(2-methoxyethyl)carbamate which was prepared according to intermediate example 170a were transformed in analogy to example 161 using TFA to give after working up and purification 20.2 mg (15 %) of the title compound.
UPLC MS: RT = 1.11 min; m/z (ES+) 471.6 [MH+]; required MW = 470.6. 1 H-NMR (300 MHz ,DMSO-d6), δ [ppm]= 0.44-0.52 (2H), 0.59-0.69 (2H), 2.21 (3H), 2.28 (3H), 2.79 (1H), 3.25 (3H), 3.32-3.39 (2H), 3.50-3.58 (2H), 6.07-6.16 (2H), 6.92 (1H), 6.98-7.06 (1 H), 7.13 (1 H), 7.23-7.30 (2H), 7.30-7.38 (3H), 7.58 (1 H), 8.25 (1 H)

### Example 170a

### tert-butyl {3-[4-(cyclopropylcarbamoyl)-3-methylphenyl]-6-(2-methylphenoxy)imidazo[1,2-a]pyridin-8-yl}(2-methoxyethyl)carbamate

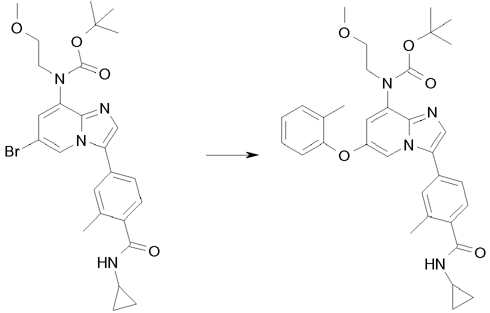

163 mg (300 µmol) tert-butyl {6-bromo-3-[4-(cyclopropylcarbamoyl)-3-methylphenyl]imidazo[1,2-a]pyridin-8-yl}(2-methoxyethyl)carbamate which was prepared according to intermediate example 169b were transformed in analogy to example 161 a using 2-methylphenol to give after evaporation the crude title compound which was used without purification in the next step.
UPLC MS: RT = 1.22 min; m/z (ES+) 571.7 [MH+]; required MW = 570.7.

### Example 171

### N-cyclopropyl-4-{6-(3,4-difluorophenoxy)-8-[(2-methoxyethyl)amino]imidazo[1,2-a]pyridin-3-yl}-2-methylbenzamide

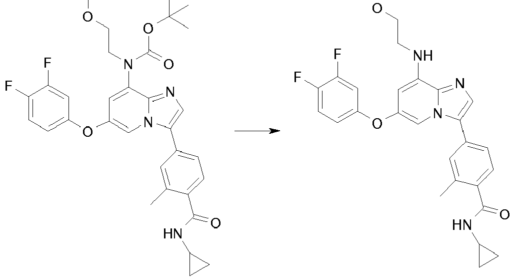

163 mg (275 µmol) tert-butyl {3-[4-(cyclopropylcarbamoyl)-3-methylphenyl]-6-(3,4-difluorophenoxy)imidazo[1,2-a]pyridin-8-yl}(2-methoxyethyl)carbamate which was prepared according to intermediate example 171 a were transformed in analogy to example 161 using TFA to give after working up and purification 8.5 mg (6 %) of the title compound.
UPLC MS: RT = 1.14 min; m/z (ES+) 493.5 [MH+]; required MW = 492.5. 1H-NMR (300 MHz ,DMSO-d6), δ [ppm]= 0.44-0.53 (2H), 0.60-0.70 (2H), 2.33 (3H), 2.80 (1H), 3.24 (3H), 3.32-3.39 (2H), 3.48-3.55 (2H), 6.07 (1 H), 6.15-6.24 (1 H), 6.84-6.92 (1 H), 7.21 (1H), 7.31-7.40 (2H), 7.40-7.47 (2H), 7.57-7.66 (2H), 8.26 (1H)

### Example 171 a

### tert-butyl {3-[4-(cyclopropylcarbamoyl)-3-methylphenyl]-6-(3,4-difluorophenoxy)imidazo[1,2-a]pyridin-8-yl}(2-methoxyethyl)carbamate

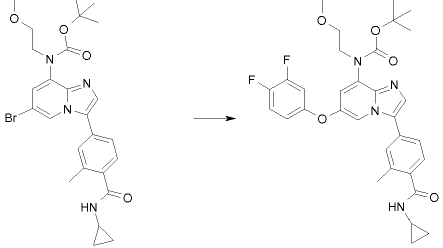

163 mg (300 µmol) tert-butyl {6-bromo-3-[4-(cyclopropylcarbamoyl)-3-methylphenyl]imidazo[1,2-a]pyridin-8-yl}(2-methoxyethyl)carbamate which was prepared according to intermediate example 169b were transformed in analogy to example 161a using 3,4-difluorophenol to give after evaporation the crude title compound which was used without purification in the next step.
UPLC MS: RT = 1.24 min; m/z (ES+) 593.7 [MH+]; required MW = 592.7.

### Example 172

### N-cyclopropyl-4-{6-(3,4-difluorophenoxy)-8-[(2-methoxyethyl)amino]imidazo[1,2-a]pyridin-3-yl}-2-methylbenzamide

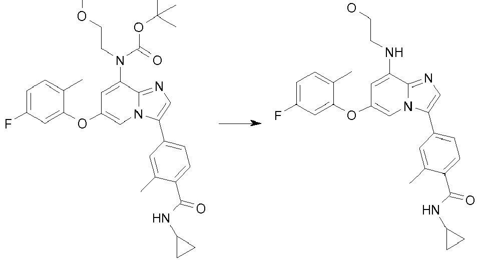

163 mg (277 µmol) tert-butyl {3-[4-(cyclopropylcarbamoyl)-3-methylphenyl]-6-(5-fluoro-2-methylphenoxy)imidazo[1,2-a]pyridin-8-yl}(2-methoxyethyl)carbamate which was prepared according to intermediate example 172a were transformed in analogy to example 161 using TFA to give after working up and purification 16.9 mg (12 %) of the title compound.
UPLC MS: RT = 1.15 min; m/z (ES+) 489.6 [MH+]; required MW = 488.6. 1H-NMR (300 MHz ,DMSO-d6), δ [ppm]= 0.46-0.52 (2H), 0.61-0.68 (2H), 2.20 (3H), 2.31 (3H), 2.79 (1H), 3.24 (3H), 3.36 (2H), 3.48-3.56 (2H), 6.08 (1H), 6.17 (1H), 6.75 (1H), 6.79-6.88 (1 H), 7.27 (1 H), 7.33-7.42 (3H), 7.45 (1 H), 7.60 (1 H), 8.26 (1 H).

### Example 172a

### tert-butyl {3-[4-(cyclopropylcarbamoyl)-3-methylphenyl]-6-(5-fluoro-2-methylphenoxy)imidazo[1,2-a]pyridin-8-yl}(2-methoxyethyl)carbamate

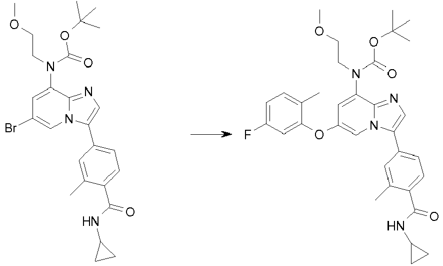

163 mg (300 µmol) tert-butyl {6-bromo-3-[4-(cyclopropylcarbamoyl)-3-methylphenyl]imidazo[1,2-a]pyridin-8-yl}(2-methoxyethyl)carbamate which was prepared according to intermediate example 169b were transformed in analogy to example 161a using 5-fluoro-2-methylphenol to give after evaporation the crude title compound which was used without purification in the next step.
UPLC MS: RT = 1.26 min; m/z (ES+) 589.7 [MH+]; required MW = 588.7.

### Example 173

### N-cyclopropyl-4-{6-(2,3-difluorophenoxy)-8-[(2-methoxyethyl)amino]imidazo[1,2-a]pyridin-3-yl}-2-methylbenzamide

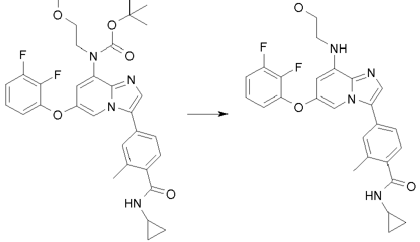

163 mg (275 µmol) tert-butyl {3-[4-(cyclopropylcarbamoyl)-3-methylphenyl]-6-(2,3-difluorophenoxy)imidazo[1,2-a]pyridin-8-yl}(2-methoxyethyl)carbamate which was prepared according to intermediate example 173a were transformed in analogy to example 161 using TFA to give after working up and purification 10.6 mg (7.5 %) of the title compound.
UPLC MS: RT = 1.14 min; m/z (ES+) 493.5 [MH+]; required MW = 492.5. 1H-NMR (300 MHz ,DMSO-d6), δ [ppm]= 0.45-0.52 (2H), 0.61-0.69 (2H), 2.33 (3H), 2.79 (1H), 3.24 (3H), 3.36 (2H), 3.48-3.55 (2H), 6.18 (1H), 6.24 (1H), 6.95 (1H), 7.06-7.20 (2H), 7.32-7.38 (1H), 7.39-7.45 (2H), 7.63-7.68 (2H), 8.27 (1H)

### Example 173a

### tert-butyl {3-[4-(cyclopropylcarbamoyl)-3-methylphenyl]-6-(2,3-difluorophenoxy)imidazo[1,2-a]pyridin-8-yl}(2-methoxyethyl)carbamate

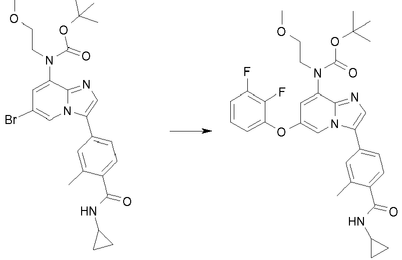

163 mg (300 µmol) tert-butyl {6-bromo-3-[4-(cyclopropylcarbamoyl)-3-methylphenyl]imidazo[1,2-a]pyridin-8-yl}(2-methoxyethyl)carbamate which was prepared according to intermediate example 169b were transformed in analogy to example 161a using 2,3-difluorophenol to give after evaporation the crude title compound which was used without purification in the next step.
UPLC MS: RT = 1.24 min; m/z (ES+) 593.7 [MH+]; required MW = 592.7.

### Example 174

### N-cyclopropyl-4-{6-(5-fluoro-2-hydroxybenzoyl)-8-[(2-methoxyethyl)amino]imidazo[1,2-a]pyridin-3-yl}-2-methylbenzamide

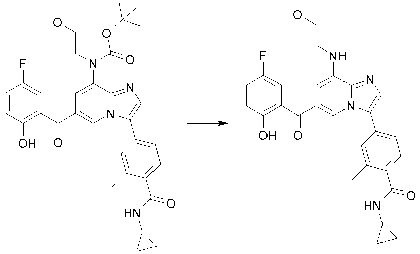

260 mg (431 µmol) tert-butyl {3-[4-(cyclopropylcarbamoyl)-3-methylphenyl]-6-(5-fluoro-2-hydroxybenzoyl)imidazo[1,2-a]pyridin-8-yl}(2-methoxyethyl)carbamate which was prepared according to intermediate example 174a were transformed in analogy to example 161 using TFA to give after working up and purification 184 mg (85 %) of the title compound.
UPLC MS: RT = 1.18 min; m/z (ES+) 503.6 [MH+]; required MW = 502.6. 1 H-NMR (300 MHz ,DMSO-d6), δ [ppm]= 0.46-0.53 (2H), 0.61-0.69 (2H), 2.36 (3H), 2.80 (1H), 3.26-3.29 (3H), 3.42-3.50 (2H), 3.56-3.63 (2H), 6.23 (1 H), 6.62-6.67 (1 H), 7.23-7.33 (3H), 7.41-7.47 (1H), 7.48-7.53 (2H), 7.73 (1H), 8.32 (1H), 8.47 (1H)

### Example 174a

### tert-butyl {3-[4-(cyclopropylcarbamoyl)-3-methylphenyl]-6-(5-fluoro-2-hydroxybenzoyl)imidazo[1,2-a]pyridin-8-yl}(2-methoxyethyl)carbamate

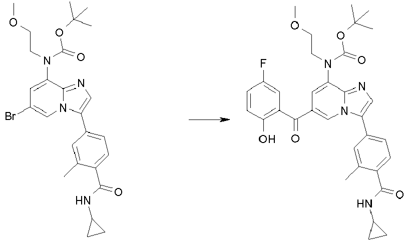

300 mg (552 µmol) tert-butyl {6-bromo-3-[4-(cyclopropylcarbamoyl)-3-methylphenyl]imidazo[1,2-a]pyridin-8-yl}(2-methoxyethyl)carbamate which was prepared according to intermediate example 169b were transformed in analogy to example 168a using (5-fluoro-2-hydroxyphenyl)boronic acid to give after working up 260 mg (78 %) of the title compound.
UPLC MS: RT = 1.28 min; m/z (ES+) 603.7 [MH+]; required MW = 602.7.

### Example 175

### N-cyclopropyl-4-{6-(5-fluoro-2-hydroxyphenoxy)-8-[(3,3,3-trifluoropropyl)amino]imidazo[1,2-a]pyridin-3-yl}-2-methylbenzamide

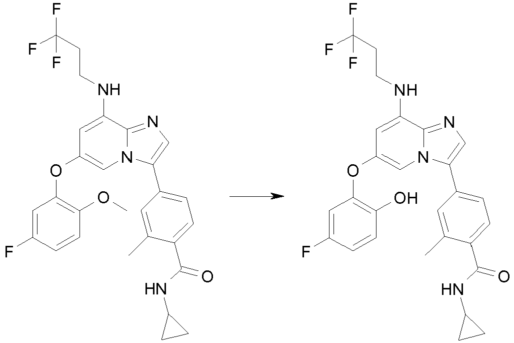

17 mg (31 µmol) N-cyclopropyl-4-{6-(5-fluoro-2-methoxyphenoxy)-8-[(3,3,3-trifluoropropyl)amino]imidazo[1,2-a]pyridin-3-yl}-2-methylbenzamide which was prepared according to intermediate example 175a were transformed in analogy to example 129 using boron tribromide to give after working up and purification 1.7 mg (10 %) of the title compound.
UPLC MS: RT = 1.08 min; m/z (ES+) 529.5 [MH+]; required MW = 528.5.5. 1 H-NMR (400 MHz ,DMSO-d6), Shift [ppm]= 0.46-0.52 (2H), 0.62-0.68 (2H), 2.29 (3H), 2.57-2.69 (2H), 2.79 (1H), 3.47 (2H), 6.11 (1H), 6.47-6.53 (1H), 6.77-6.85 (1H), 6.87-6.95 (2H), 7.29-7.38 (3H), 7.55-7.63 (1 H), 8.27 (1 H), 9.50 (1 H)

### Example 175a

### N-cyclopropyl-4-{6-(5-fluoro-2-methoxyphenoxy)-8-[(3,3,3-trifluoropropyl)amino]imidazo[1,2-a]pyridin-3-yl}-2-methylbenzamide

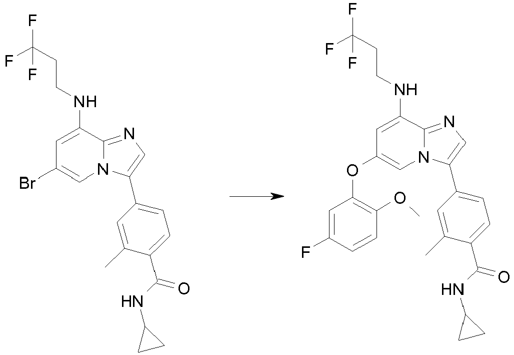

100 mg (208 µmol) 4-{6-bromo-8-[(3,3,3-trifluoropropyl)amino]imidazo[1,2-a]pyridin-3-yl}-N-cyclopropyl-2-methylbenzamide which was prepared according to intermediate example 6-1 were transformed in analogy to example 6-1 using 5-fluoro-2-methoxyphenol to give after working up the crude title compound which was used without further purification I the next step.

### Example 176

### N-cyclopropyl-4-{6-(3-fluoro-2-hydroxyphenoxy)-8-[(3,3,3-trifluoropropyl)amino]imidazo[1,2-a]pyridin-3-yl}-2-methylbenzamide

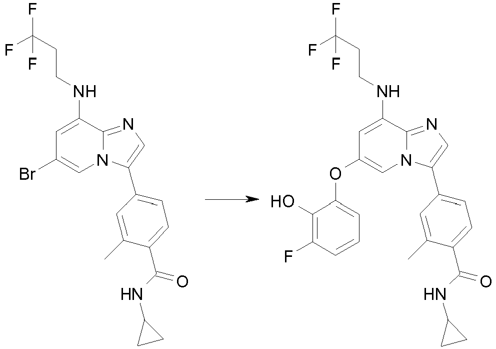

N-cyclopropyl-4-{6-(3-fluoro-2-hydroxyphenoxy)-8-[(3,3,3-trifluoropropyl)amino]imidazo[1,2-a]pyridin-3-yl}-2-methylbenzamide was prepared in analogy to example 175 and 175a using 3-fluoro-2-methoxyphenol to give after working up and purification 2.3 mg (6 %) of the title compound.
UPLC MS: RT = 1.08 min; m/z (ES+) 529.5 [MH+]; required MW = 528.5.5. 1 H-NMR (400 MHz ,DMSO-d6), Shift [ppm]= 0.45-0.52 (2H), 0.61-0.69 (2H), 2.28 (3H), 2.57-2.69 (2H), 2.79 (1H), 3.47 (2H), 6.11 (1H), 6.42-6.54 (1H), 6.70-6.86 (1H), 6.86-6.93 (2H), 7.29-7.38 (3H), 7.57-7.61 (1 H), 8.27 (1 H), 9.50 (1 H)

### Example 177

### N-cyclopropyl-4-{6-[(5-fluoropyridin-3-yl)oxy]-8-[(3,3,3-trifluoropropyl)amino]imidazo[1,2-a]pyridin-3-yl}-2-methylbenzamide

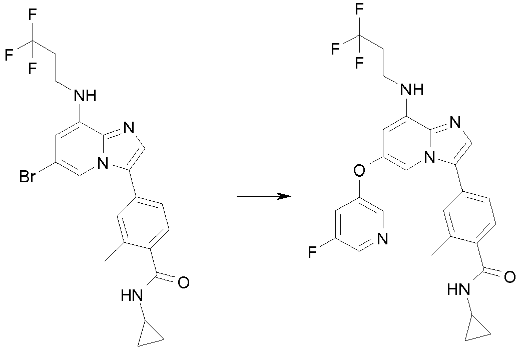

96 mg (200 µmol) 4-{6-bromo-8-[(3,3,3-trifluoropropyl)amino]imidazo[1,2-a]pyridin-3-yl}-N-cyclopropyl-2-methylbenzamide which was prepared according to intermediate example 6-1 were transformed in analogy to example 6-1 using 5-fluoropyridin-3-ol to give after working up and purification 9 mg (9 %) of the title compound.
UPLC MS: RT = 1.13 min; m/z (ES+) 514.5 [MH+]; required MW = 513.5.5. 1H-NMR (300 MHz ,DMSO-d6), Shift [ppm]= 0.49 (2H), 0.65 (2H), 2.33 (3H), 2.56-2.68 (2H), 2.79 (1 H), 3.42-3.51 (2H), 6.16 (1 H), 6.59 (1 H), 7.32-7.39 (1 H), 7.40-7.55 (3H), 7.65 (1H), 7.74 (1H), 8.23-8.32 (3H)

### Example 178

### N-cyclopropyl-4-{6-[(5-fluoro-6-methoxypyridin-3-yl)oxy]-8-[(3,3,3-trifluoropropyl)amino]imidazo[1,2-a]pyridin-3-yl}-2-methylbenzamide

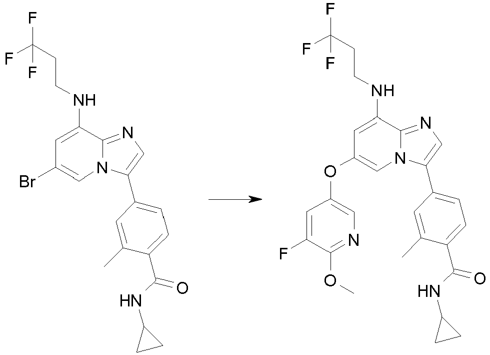

96 mg (200 µmol) 4-{6-bromo-8-[(3,3,3-trifluoropropyl)amino]imidazo[1,2-a]pyridin-3-yl}-N-cyclopropyl-2-methylbenzamide which was prepared according to intermediate example 6-1 were transformed in analogy to example 6-1 using 5-fluoro-6-methoxypyridin-3-ol to give after working up and purification 15 mg (15 %) of the title compound.
UPLC MS: RT = 1.21 min; m/z (ES+) 544.5 [MH+]; required MW = 543.5.5. 1 H-NMR (300 MHz ,DMSO-d6), Shift [ppm]= 0.44-0.53 (2H), 0.60-0.69 (2H), 2.32 (3H), 2.56-2.71 (2H), 2.79 (1 H), 3.45 (2H), 3.88 (3H), 6.12 (1 H), 6.57 (1 H), 7.32-7.38 (1 H), 7.39-7.46 (2H), 7.56 (1H), 7.62 (1H), 7.70 (1H), 7.87 (1H), 8.29 (1H)

### Pharmaceutical compositions of the compounds of the invention

This invention also relates to pharmaceutical compositions containing one or more compounds of the present invention. These compositions can be utilised to achieve the desired pharmacological effect by administration to a patient in need thereof. A patient, for the purpose of this invention, is a mammal, including a human, in need of treatment for the particular condition or disease. Therefore, the present invention includes pharmaceutical compositions that are comprised of a pharmaceutically acceptable carrier and a pharmaceutically effective amount of a compound, or salt thereof, of the present invention. A pharmaceutically acceptable carrier is preferably a carrier that is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is preferably that amount which produces a result or exerts an influence on the particular condition being treated. The compounds of the present invention can be administered with pharmaceutically-acceptable carriers well known in the art using any effective conventional dosage unit forms, including immediate, slow and timed release preparations, orally, parenterally, topically, nasally, ophthalmically, optically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatine type containing, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatine, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, colouring agents, and flavouring agents such as peppermint, oil of wintergreen, or cherry flavouring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavouring and colouring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived form fatty acids and hexitol anhydrides, for example, sorbitan monooleate, (4) condensation products of said partial esters with ethylene oxide, for example, polyoxyethylene sorbitan monooleate. The emulsions may also contain sweetening and flavouring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more colouring agents; one or more flavouring agents; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavouring and colouring agents.

The compounds of this invention may also be administered parenterally, that is, subcutaneously, intravenously, intraocularly, intrasynovially, intramuscularly, or interperitoneally, as injectable dosages of the compound in preferably a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates ; anionic detergents, for example, alkyl, aryl, and olefin sulfonates, alkyl, olefin, ether, and monoglyceride sulfates, and sulfosuccinates ; non-ionic detergents, for example, fatty amine oxides, fatty acid alkanolamides, and poly(oxyethylene-oxypropylene)s or ethylene oxide or propylene oxide copolymers ; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimise or eliminate irritation at the site of injection, such compositions may contain a non-ionic surfactant having a hydrophile-lipophile balance (HLB) preferably of from about 12 to about 17. The quantity of surfactant in such formulation preferably ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia ; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Another formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art (see, e.g., US Patent No. 5,023,252, issued June 11, 1991, incorporated herein by reference). Such patches may be constructed for continuous, pulsatile, or on demand delivery of pharmaceutical agents.

Controlled release formulations for parenteral administration include liposomal, polymeric microsphere and polymeric gel formulations that are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions of the invention can also contain other conventional pharmaceutically acceptable compounding ingredients, generally referred to as carriers or diluents, as necessary or desired. Conventional procedures for preparing such compositions in appropriate dosage forms can be utilized. Such ingredients and procedures include those described in the following references, each of which is incorporated herein by reference: Powell, M.F. et al., "Compendium of Excipients for Parenteral Formulations" PDA Journal of Pharmaceutical Science & Technology 1998, 52(5), 238-311 ; Strickley, R.G "Parenteral Formulations of Small Molecule Therapeutics Marketed in the United States (1999)-Part-1" PDA Journal of Pharmaceutical Science & Technology 1999, 53(6), 324-349 ; and Nema, S. et al., "Excipients and Their Use in Injectable Products" PDA Journal of Pharmaceutical Science & Technology 1997, 51(4), 166-171.

Commonly used pharmaceutical ingredients that can be used as appropriate to formulate the composition for its intended route of administration include:
**acidifying agents** (examples include but are not limited to acetic acid, citric acid, fumaric acid, hydrochloric acid, nitric acid) ;
**alkalinizing agents** (examples include but are not limited to ammonia solution, ammonium carbonate, diethanolamine, monoethanolamine, potassium hydroxide, sodium borate, sodium carbonate, sodium hydroxide, triethanolamine, trolamine) ;
**adsorbents** (examples include but are not limited to powdered cellulose and activated charcoal) ;
**aerosol propellants** (examples include but are not limited to carbon dioxide, CCl₂F₂, F₂ClC-CClF₂ and CClF₃)
**air displacement agents** (examples include but are not limited to nitrogen and argon) ;
**antifungal preservatives** (examples include but are not limited to benzoic acid, butylparaben, ethylparaben, methylparaben, propylparaben, sodium benzoate) ;
**antimicrobial preservatives** (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal) ;
**antioxidants** (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite) ;
**binding materials** (examples include but are not limited to block polymers, natural and synthetic rubber, polyacrylates, polyurethanes, silicones, polysiloxanes and styrene-butadiene copolymers) ;
**buffering agents** (examples include but are not limited to potassium metaphosphate, dipotassium phosphate, sodium acetate, sodium citrate anhydrous and sodium citrate dihydrate)
**carrying agents** (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection)
**chelating agents** (examples include but are not limited to edetate disodium and edetic acid)
**colourants** (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red) ;
**clarifying agents** (examples include but are not limited to bentonite) ;
**emulsifying agents** (examples include but are not limited to acacia, cetomacrogol, cetyl alcohol, glyceryl monostearate, lecithin, sorbitan monooleate, polyoxyethylene 50 monostearate) ;
**encapsulating agents** (examples include but are not limited to gelatin and cellulose acetate phthalate)
**flavourants** (examples include but are not limited to anise oil, cinnamon oil, cocoa, menthol, orange oil, peppermint oil and vanillin) ;
**humectants** (examples include but are not limited to glycerol, propylene glycol and sorbitol) ;
**levigating agents** (examples include but are not limited to mineral oil and glycerin) ;
**oils** (examples include but are not limited to arachis oil, mineral oil, olive oil, peanut oil, sesame oil and vegetable oil) ;
**ointment bases** (examples include but are not limited to lanolin, hydrophilic ointment, polyethylene glycol ointment, petrolatum, hydrophilic petrolatum, white ointment, yellow ointment, and rose water ointment) ;
**penetration enhancers (transdermal delivery)** (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)
**plasticizers** (examples include but are not limited to diethyl phthalate and glycerol) ;
**solvents** (examples include but are not limited to ethanol, corn oil, cottonseed oil, glycerol, isopropanol, mineral oil, oleic acid, peanut oil, purified water, water for injection, sterile water for injection and sterile water for irrigation) ;
**stiffening agents** (examples include but are not limited to cetyl alcohol, cetyl esters wax, microcrystalline wax, paraffin, stearyl alcohol, white wax and yellow wax) ;
**suppository bases** (examples include but are not limited to cocoa butter and polyethylene glycols (mixtures)) ;
**surfactants** (examples include but are not limited to benzalkonium chloride, nonoxynol 10, oxtoxynol 9, polysorbate 80, sodium lauryl sulfate and sorbitan mono-palmitate) ;
**suspending agents** (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum) ;
**sweetening agents** (examples include but are not limited to aspartame, dextrose, glycerol, mannitol, propylene glycol, saccharin sodium, sorbitol and sucrose) ;
**tablet anti-adherents** (examples include but are not limited to magnesium stearate and talc) ;
**tablet binders** (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch) ;
**tablet and capsule diluents** (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch) ;
**tablet coating agents** (examples include but are not limited to liquid glucose, hydroxyethyl cellulose, hydroxypropyl cellulose, hydroxypropyl methylcellulose, methylcellulose, ethylcellulose, cellulose acetate phthalate and shellac) ;
**tablet direct compression excipients** (examples include but are not limited to dibasic calcium phosphate) ;
**tablet disintegrants** (examples include but are not limited to alginic acid, carboxymethylcellulose calcium, microcrystalline cellulose, polacrillin potassium, cross-linked polyvinylpyrrolidone, sodium alginate, sodium starch glycollate and starch) ;
**tablet glidants** (examples include but are not limited to colloidal silica, corn starch and talc) ;
**tablet lubricants** (examples include but are not limited to calcium stearate, magnesium stearate, mineral oil, stearic acid and zinc stearate) ;
**tablet/capsule opaquants** (examples include but are not limited to titanium dioxide) ;
tablet **polishing agents** (examples include but are not limited to carnuba wax and white wax) ;
**thickening agents** (examples include but are not limited to beeswax, cetyl alcohol and paraffin) ;
**tonicity agents** (examples include but are not limited to dextrose and sodium chloride) ;
**viscosity increasing agents** (examples include but are not limited to alginic acid, bentonite, carbomers, carboxymethylcellulose sodium, methylcellulose, polyvinyl pyrrolidone, sodium alginate and tragacanth) ; and
**wetting agents** (examples include but are not limited to heptadecaethylene oxycetanol, lecithins, sorbitol monooleate, polyoxyethylene sorbitol monooleate, and polyoxyethylene stearate).

Pharmaceutical compositions according to the present invention can be illustrated as follows:
Sterile IV Solution: A 5 mg/mL solution of the desired compound of this invention can be made using sterile, injectable water, and the pH is adjusted if necessary. The solution is diluted for administration to 1 - 2 mg/mL with sterile 5% dextrose and is administered as an IV infusion over about 60 minutes.
Lyophilised powder for IV administration: A sterile preparation can be prepared with (i) 100 - 1000 mg of the desired compound of this invention as a lyophilised powder, (ii) 32- 327 mg/mL sodium citrate, and (iii) 300 - 3000 mg Dextran 40. The formulation is reconstituted with sterile, injectable saline or dextrose 5% to a concentration of 10 to 20 mg/mL, which is further diluted with saline or dextrose 5% to 0.2 - 0.4 mg/mL, and is administered either IV bolus or by IV infusion over 15 - 60 minutes.
Intramuscular suspension: The following solution or suspension can be prepared, for intramuscular injection:
   50 mg/mL of the desired, water-insoluble compound of this invention
   5 mg/mL sodium carboxymethylcellulose
   4 mg/mL TWEEN 80
   9 mg/mL sodium chloride
   9 mg/mL benzyl alcohol
Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.
Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.
Tablets: A large number of tablets are prepared by conventional procedures so that the dosage unit is 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.
Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

### Combination therapies

The compounds of this invention can be administered as the sole pharmaceutical agent or in combination with one or more other pharmaceutical agents where the combination causes no unacceptable adverse effects. The present invention relates also to such combinations. For example, the compounds of this invention can be combined with known anti-hyper-proliferative or other indication agents, and the like, as well as with admixtures and combinations thereof. Other indication agents include, but are not limited to, anti-angiogenic agents, mitotic inhibitors, alkylating agents, anti-metabolites, DNA-intercalating antibiotics, growth factor inhibitors, cell cycle inhibitors, enzyme inhibitors, toposisomerase inhibitors, biological response modifiers, or anti-hormones.

The additional pharmaceutical agent can be 1311-chTNT, abarelix, abiraterone, aclarubicin, aldesleukin, alemtuzumab, alitretinoin, altretamine, aminoglutethimide, amrubicin, amsacrine, anastrozole, arglabin, arsenic trioxide, asparaginase, azacitidine, basiliximab, BAY 80-6946, BAY 1000394, BAY 86-9766 (RDEA 119), belotecan, bendamustine, bevacizumab, bexarotene, bicalutamide, bisantrene, bleomycin, bortezomib, buserelin, busulfan, cabazitaxel, calcium folinate, calcium levofolinate, capecitabine, carboplatin, carmofur, carmustine, catumaxomab, celecoxib, celmoleukin, cetuximab, chlorambucil, chlormadinone, chlormethine, cisplatin, cladribine, clodronic acid, clofarabine, crisantaspase, cyclophosphamide, cyproterone, cytarabine, dacarbazine, dactinomycin, darbepoetin alfa, dasatinib, daunorubicin, decitabine, degarelix, denileukin diftitox, denosumab, deslorelin, dibrospidium chloride, docetaxel, doxifluridine, doxorubicin, doxorubicin + estrone, eculizumab, edrecolomab, elliptinium acetate, eltrombopag, endostatin, enocitabine, epirubicin, epitiostanol, epoetin alfa, epoetin beta, eptaplatin, eribulin, erlotinib, estradiol, estramustine, etoposide, everolimus, exemestane, fadrozole, filgrastim, fludarabine, fluorouracil, flutamide, formestane, fotemustine, fulvestrant, gallium nitrate, ganirelix, gefitinib, gemcitabine, gemtuzumab, glutoxim, goserelin, histamine dihydrochloride, histrelin, hydroxycarbamide, I-125 seeds, ibandronic acid, ibritumomab tiuxetan, idarubicin, ifosfamide, imatinib, imiquimod, improsulfan, interferon alfa, interferon beta, interferon gamma, ipilimumab, irinotecan, ixabepilone, lanreotide, lapatinib, lenalidomide, lenograstim, lentinan, letrozole, leuprorelin, levamisole, lisuride, lobaplatin, lomustine, lonidamine, masoprocol, medroxyprogesterone, megestrol, melphalan, mepitiostane, mercaptopurine, methotrexate, methoxsalen, Methyl aminolevulinate, methyltestosterone, mifamurtide, miltefosine, miriplatin, mitobronitol, mitoguazone, mitolactol, mitomycin, mitotane, mitoxantrone, nedaplatin, nelarabine, nilotinib, nilutamide, nimotuzumab, nimustine, nitracrine, ofatumumab, omeprazole, oprelvekin, oxaliplatin, p53 gene therapy, paclitaxel, palifermin, palladium-103 seed, pamidronic acid, panitumumab, pazopanib, pegaspargase, PEG-epoetin beta (methoxy PEG-epoetin beta), pegfilgrastim, peginterferon alfa-2b, pemetrexed, pentazocine, pentostatin, peplomycin, perfosfamide, picibanil, pirarubicin, plerixafor, plicamycin, poliglusam, polyestradiol phosphate, polysaccharide-K, porfimer sodium, pralatrexate, prednimustine, procarbazine, quinagolide, raloxifene, raltitrexed, ranimustine, razoxane, regorafenib, risedronic acid, rituximab, romidepsin, romiplostim, sargramostim, sipuleucel-T, sizofiran, sobuzoxane, sodium glycididazole, sorafenib, streptozocin, sunitinib, talaporfin, tamibarotene, tamoxifen, tasonermin, teceleukin, tegafur, tegafur + gimeracil + oteracil, temoporfin, temozolomide, temsirolimus, teniposide, testosterone, tetrofosmin, thalidomide, thiotepa, thymalfasin, tioguanine, tocilizumab, topotecan, toremifene, tositumomab, trabectedin, trastuzumab, treosulfan, tretinoin, trilostane, triptorelin, trofosfamide, tryptophan, ubenimex, valrubicin, vandetanib, vapreotide, vemurafenib, vinblastine, vincristine, vindesine, vinflunine, vinorelbine, vorinostat, vorozole, yttrium-90 glass microspheres, zinostatin, zinostatin stimalamer, zoledronic acid, zorubicin.

Preferably, the additional pharmaceutical agent is selected from: afinitor, aldesleukin, alendronic acid, alfaferone, alitretinoin, allopurinol, aloprim, aloxi, altretamine, aminoglutethimide, amifostine, amrubicin, amsacrine, anastrozole, anzmet, aranesp, arglabin, arsenic trioxide, aromasin, 5-azacytidine, azathioprine, BCG or tice BCG, bestatin, betamethasone acetate, betamethasone sodium phosphate, bexarotene, bleomycin sulfate, broxuridine , bortezomib, busulfan, calcitonin, campath, capecitabine, carboplatin, casodex, cefesone, celmoleukin, cerubidine, chlorambucil, cisplatin, cladribine, cladribine, clodronic acid, cyclophosphamide, cytarabine, dacarbazine, dactinomycin, DaunoXome, decadron, decadron phosphate, delestrogen, denileukin diftitox, depo-medrol, deslorelin, dexrazoxane, diethylstilbestrol, diflucan, docetaxel, doxifluridine, doxorubicin, dronabinol, DW-166HC, eligard, elitek, ellence, emend, epirubicin, epoetin alfa, epogen, eptaplatin, ergamisol, estrace, estradiol, estramustine phosphate sodium, ethinyl estradiol, ethyol, etidronic acid, etopophos, etoposide, fadrozole, farston, filgrastim, finasteride, fligrastim, floxuridine, fluconazole, fludarabine, 5-fluorodeoxyuridine monophosphate, 5-fluorouracil (5-FU), fluoxymesterone, flutamide, formestane, fosteabine, fotemustine, fulvestrant, gammagard, gemcitabine, gemtuzumab, gleevec, gliadel, goserelin, granisetron HCl, histrelin, hycamtin, hydrocortone, eyrthro-hydroxynonyladenine, hydroxyurea, ibritumomab tiuxetan, idarubicin, ifosfamide, interferon alpha, interferon-alpha 2, interferon alfa-2A, interferon alfa-2B, interferon alfa-n1, interferon alfa-n3, interferon beta, interferon gamma-1a, interleukin-2, intron A, iressa, irinotecan, kytril, lentinan sulfate, letrozole, leucovorin, leuprolide, leuprolide acetate, levamisole, levofolinic acid calcium salt, levothroid, levoxyl, lomustine, lonidamine, marinol, mechlorethamine, mecobalamin, medroxyprogesterone acetate, megestrol acetate, melphalan, menest, 6-mercaptopurine, Mesna, methotrexate, metvix, miltefosine, minocycline, mitomycin C, mitotane, mitoxantrone, Modrenal, Myocet, nedaplatin, neulasta, neumega, neupogen, nilutamide, nolvadex, NSC-631570, OCT-43, octreotide, ondansetron HCl, orapred, oxaliplatin, paclitaxel, pediapred, pegaspargase, Pegasys, pentostatin, picibanil, pilocarpine HCl, pirarubicin, plicamycin, porfimer sodium, prednimustine, prednisolone, prednisone, premarin, procarbazine, procrit, raltitrexed, RDEA 119, rebif, rhenium-186 etidronate, rituximab, roferon-A, romurtide, salagen, sandostatin, sargramostim, semustine, sizofiran, sobuzoxane, solu-medrol, sparfosic acid, stem-cell therapy, streptozocin, strontium-89 chloride, synthroid, tamoxifen, tamsulosin, tasonermin, tastolactone, taxotere, teceleukin, temozolomide, teniposide, testosterone propionate, testred, thioguanine, thiotepa, thyrotropin, tiludronic acid, topotecan, toremifene, tositumomab, trastuzumab, treosulfan, tretinoin, trexall, trimethylmelamine, trimetrexate, triptorelin acetate, triptorelin pamoate, UFT, uridine, valrubicin, vesnarinone, vinblastine, vincristine, vindesine, vinorelbine, virulizin, zinecard, zinostatin stimalamer, zofran, ABI-007, acolbifene, actimmune, affinitak, aminopterin, arzoxifene, asoprisnil, atamestane, atrasentan, sorafenib (BAY 43-9006), avastin, CCI-779, CDC-501, celebrex, cetuximab, crisnatol, cyproterone acetate, decitabine, DN-101, doxorubicin-MTC, DSLIM, dutasteride, edotecarin, eflornithine, exatecan, fenretinide, histamine dihydrochloride, histrelin hydrogel implant, holmium-166 DOTMP, ibandronic acid, interferon gamma, intron-PEG, ixabepilone, keyhole limpet hemocyanin, L-651582, lanreotide, lasofoxifene, libra, lonafarnib, miproxifene, minodronate, MS-209, liposomal MTP-PE, MX-6, nafarelin, nemorubicin, neovastat, nolatrexed, oblimersen, onco-TCS, osidem, paclitaxel polyglutamate, pamidronate disodium, PN-401, QS-21, quazepam, R-1549, raloxifene, ranpirnase, 13-cis -retinoic acid, satraplatin, seocalcitol, T-138067, tarceva, taxoprexin, thymosin alpha 1, tiazofurine, tipifarnib, tirapazamine, TLK-286, toremifene, TransMID-107R, valspodar, vapreotide, vatalanib, verteporfin, vinflunine, Z-100, and zoledronic acid or combinations thereof. Optional anti-hyper-proliferative agents which can be added to the composition include but are not limited to compounds listed on the cancer chemotherapy drug regimens in the 11th Edition of the Merck Index, (1996), which is hereby incorporated by reference, such as asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, epothilone, an epothilone derivative, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), which is hereby incorporated by reference, such as aminoglutethimide, L-asparaginase, azathioprine, 5-azacytidine cladribine, busulfan, diethylstilbestrol, 2',2'-difluorodeoxycytidine, docetaxel, erythrohydroxynonyl adenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other anti-hyper-proliferative agents suitable for use with the composition of the invention include but are not limited to other anti-cancer agents such as epothilone and its derivatives, irinotecan, raloxifen and topotecan.

The compounds of the invention may also be administered in combination with protein therapeutics. Such protein therapeutics suitable for the treatment of cancer or other angiogenic disorders and for use with the compositions of the invention include, but are not limited to, an interferon (e.g., interferon .alpha., .beta., or .gamma.) supraagonistic monoclonal antibodies, Tuebingen, TRP-1 protein vaccine, Colostrinin, anti-FAP antibody, YH-16, gemtuzumab, infliximab, cetuximab, trastuzumab, denileukin diftitox, rituximab, thymosin alpha 1, bevacizumab, mecasermin, mecasermin rinfabate, oprelvekin, natalizumab, rhMBL, MFE-CP1 + ZD-2767-P, ABT-828, ErbB2-specific immunotoxin, SGN-35, MT-103, rinfabate, AS-1402, B43-genistein, L-19 based radioimmunotherapeutics, AC-9301, NY-ESO-1 vaccine, IMC-1C11, CT-322, rhCC10, r(m)CRP, MORAb-009, aviscumine, MDX-1307, Her-2 vaccine, APC-8024, NGR-hTNF, rhH1.3, IGN-311, Endostatin, volociximab, PRO-1762, lexatumumab, SGN-40, pertuzumab, EMD-273063, L19-IL-2 fusion protein, PRX-321, CNTO-328, MDX-214, tigapotide, CAT-3888, labetuzumab, alpha-particle-emitting radioisotope-llinked lintuzumab, EM-1421, HyperAcute vaccine, tucotuzumab celmoleukin, galiximab, HPV-16-E7, Javelin - prostate cancer, Javelin - melanoma, NY-ESO-1 vaccine, EGF vaccine, CYT-004-MelQbG10, WT1 peptide, oregovomab, ofatumumab, zalutumumab, cintredekin besudotox, WX-G250, Albuferon, aflibercept, denosumab, vaccine, CTP-37, efungumab, or 131I-chTNT-1/B. Monoclonal antibodies useful as the protein therapeutic include, but are not limited to, muromonab-CD3, abciximab, edrecolomab, daclizumab, gentuzumab, alemtuzumab, ibritumomab, cetuximab, bevicizumab, efalizumab, adalimumab, omalizumab, muromomab-CD3, rituximab, daclizumab, trastuzumab, palivizumab, basiliximab, and infliximab.

The compounds of the invention may also be combined with biological therapeutic agents, such as antibodies (e.g. avastin, rituxan, erbitux, herceptin), or recombinant proteins.

The compounds of the invention may also be in combination with antiangiogenesis agents, such as, for example, with avastin, axitinib, DAST, recentin, sorafenib or sunitinib. Combinations with inhibitors of proteasomes or mTOR inhibitors, or anti-hormones or steroidal metabolic enzyme inhibitors are also possible.

Generally, the use of cytotoxic and/or cytostatic agents in combination with a compound or composition of the present invention will serve to:
(1) yield better efficacy in reducing the growth of a tumour or even eliminate the tumour as compared to administration of either agent alone,
(2) provide for the administration of lesser amounts of the administered chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumour progression, and/or
(8) yield efficacy and tolerability results at least as good as those of the agents used alone, compared to known instances where other cancer agent combinations produce antagonistic effects.

### Methods of Sensitizing Cells to Radiation

In a distinct embodiment of the present invention, a compound of the present invention may be used to sensitize a cell to radiation. That is, treatment of a cell with a compound of the present invention prior to radiation treatment of the cell renders the cell more susceptible to DNA damage and cell death than the cell would be in the absence of any treatment with a compound of the invention. In one aspect, the cell is treated with at least one compound of the invention.
Thus, the present invention also provides a method of killing a cell, wherein a cell is administered one or more compounds of the invention in combination with conventional radiation therapy.

The present invention also provides a method of rendering a cell more susceptible to cell death, wherein the cell is treated one or more compounds of the invention prior to the treatment of the cell to cause or induce cell death. In one aspect, after the cell is treated with one or more compounds of the invention, the cell is treated with at least one compound, or at least one method, or a combination thereof, in order to cause DNA damage for the purpose of inhibiting the function of the normal cell or killing the cell.

In one embodiment, a cell is killed by treating the cell with at least one DNA damaging agent. That is, after treating a cell with one or more compounds of the invention to sensitize the cell to cell death, the cell is treated with at least one DNA damaging agent to kill the cell. DNA damaging agents useful in the present invention include, but are not limited to, chemotherapeutic agents (*e.g.,* cisplatinum), ionizing radiation (X-rays, ultraviolet radiation), carcinogenic agents, and mutagenic agents.

In another embodiment, a cell is killed by treating the cell with at least one method to cause or induce DNA damage. Such methods include, but are not limited to, activation of a cell signalling pathway that results in DNA damage when the pathway is activated, inhibiting of a cell signalling pathway that results in DNA damage when the pathway is inhibited, and inducing a biochemical change in a cell, wherein the change results in DNA damage. By way of a non-limiting example, a DNA repair pathway in a cell can be inhibited, thereby preventing the repair of DNA damage and resulting in an abnormal accumulation of DNA damage in a cell.

In one aspect of the invention, a compound of the invention is administered to a cell prior to the radiation or other induction of DNA damage in the cell. In another aspect of the invention, a compound of the invention is administered to a cell concomitantly with the radiation or other induction of DNA damage in the cell. In yet another aspect of the invention, a compound of the invention is administered to a cell immediately after radiation or other induction of DNA damage in the cell has begun.

In another aspect, the cell is in vitro. In another embodiment, the cell is in vivo.

As mentioned supra, the compounds of the present invention have surprisingly been found to effectively inhibit Mps-1 and may therefore be used for the treatment or prophylaxis of diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by Mps-1, such as, for example, haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

In accordance with another aspect therefore, the present invention covers a compound of general formula I, or a stereoisomer, a tautomer, an N-oxide, a hydrate, a solvate, or a salt thereof, particularly a pharmaceutically acceptable salt thereof, or a mixture of same, as described and defined herein, for use in the treatment or prophylaxis of a disease, as mentioned supra.

Another particular aspect of the present invention is therefore the use of a compound of general formula I described supra for manufacturing a pharmaceutical composition for the treatment or prophylaxis of a disease.

The diseases referred to in the two preceding paragraphs are diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by Mps-1, such as, for example, haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

The term "inappropriate" within the context of the present invention, in particular in the context of "inappropriate cellular immune responses, or inappropriate cellular inflammatory responses", as used herein, is to be understood as preferably meaning a response which is less than, or greater than normal, and which is associated with, responsible for, or results in, the pathology of said diseases.

Preferably, the use is in the treatment or prophylaxis of diseases, wherein the diseases are haemotological tumours, solid tumours and/or metastases thereof.

### Method of treating hyper-proliferative disorders

The present invention relates to a method for using the compounds of the present invention and compositions thereof, to treat mammalian hyper-proliferative disorders. Compounds can be utilized to inhibit, block, reduce, decrease, etc., cell proliferation and/or cell division, and/or produce apoptosis. This method comprises administering to a mammal in need thereof, including a human, an amount of a compound of this invention, or a pharmaceutically acceptable salt, isomer, polymorph, metabolite, hydrate, solvate or ester thereof; etc. which is effective to treat the disorder. Hyper-proliferative disorders include but are not limited, e.g., psoriasis, keloids, and other hyperplasias affecting the skin, benign prostate hyperplasia (BPH), solid tumours, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukaemias.
Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumour.

Tumours of the male reproductive organs include, but are not limited to prostate and testicular cancer. Tumours of the female reproductive organs include, but are not limited to endometrial, cervical, ovarian, vaginal, and vulvar cancer, as well as sarcoma of the uterus.

Tumours of the digestive tract include, but are not limited to anal, colon, colorectal, oesophageal, gallbladder, gastric, pancreatic, rectal, small-intestine, and salivary gland cancers.

Tumours of the urinary tract include, but are not limited to bladder, penile, kidney, renal pelvis, ureter, urethral and human papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer and squamous cell. Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this document is used conventionally, *e.g.*, the management or care of a subject for the purpose of combating, alleviating, reducing, relieving, improving the condition of, *etc.,* of a disease or disorder, such as a carcinoma.

### Methods of treating kinase disorders

The present invention also provides methods for the treatment of disorders associated with aberrant mitogen extracellular kinase activity, including, but not limited to stroke, heart failure, hepatomegaly, cardiomegaly, diabetes, Alzheimer's disease, cystic fibrosis, symptoms of xenograft rejections, septic shock or asthma. Effective amounts of compounds of the present invention can be used to treat such disorders, including those diseases (*e.g.,* cancer) mentioned in the Background section above. Nonetheless, such cancers and other diseases can be treated with compounds of the present invention, regardless of the mechanism of action and/or the relationship between the kinase and the disorder.

The phrase "aberrant kinase activity" or "aberrant tyrosine kinase activity," includes any abnormal expression or activity of the gene encoding the kinase or of the polypeptide it encodes. Examples of such aberrant activity, include, but are not limited to, over-expression of the gene or polypeptide ; gene amplification ; mutations which produce constitutively-active or hyperactive kinase activity ; gene mutations, deletions, substitutions, additions, etc.

The present invention also provides for methods of inhibiting a kinase activity, especially of mitogen extracellular kinase, comprising administering an effective amount of a compound of the present invention, including salts, polymorphs, metabolites, hydrates, solvates, prodrugs (*e.g*.: esters) thereof, and diastereoisomeric forms thereof. Kinase activity can be inhibited in cells (*e.g., in vitro*)*,* or in the cells of a mammalian subject, especially a human patient in need of treatment.

### Methods of treating angiogenic disorders

The present invention also provides methods of treating disorders and diseases associated with excessive and/or abnormal angiogenesis.
Inappropriate and ectopic expression of angiogenesis can be deleterious to an organism. A number of pathological conditions are associated with the growth of extraneous blood vessels. These include, e.g., diabetic retinopathy, ischemic retinal-vein occlusion, and retinopathy of prematurity [Aiello et al. New Engl. J. Med. 1994, 331, 1480 ; Peer et al. Lab. Invest. 1995, 72, 638], age-related macular degeneration [AMD ; see, Lopez et al. Invest. Opththalmol. Vis. Sci. 1996, 37, 855], neovascular glaucoma, psoriasis, retrolental fibroplasias, angiofibroma, inflammation, rheumatoid arthritis (RA), restenosis, in-stent restenosis, vascular graft restenosis, etc. In addition, the increased blood supply associated with cancerous and neoplastic tissue, encourages growth, leading to rapid tumour enlargement and metastasis. Moreover, the growth of new blood and lymph vessels in a tumour provides an escape route for renegade cells, encouraging metastasis and the consequence spread of the cancer. Thus, compounds of the present invention can be utilized to treat and/or prevent any of the aforementioned angiogenesis disorders, e.g., by inhibiting and/or reducing blood vessel formation ; by inhibiting, blocking, reducing, decreasing, etc. endothelial cell proliferation or other types involved in angiogenesis, as well as causing cell death or apoptosis of such cell types.

### Dose and administration

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.
The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

Preferably, the diseases of said method are haematological tumours, solid tumour and/or metastases thereof.

The compounds of the present invention can be used in particular in therapy and prevention, i.e. prophylaxis, of tumour growth and metastases, especially in solid tumours of all indications and stages with or without pre-treatment of the tumour growth.

Methods of testing for a particular pharmacological or pharmaceutical property are well known to persons skilled in the art.

The example testing experiments described herein serve to illustrate the present invention and the invention is not limited to the examples given.

### Biological assay: Proliferation Assay

Cultivated tumour cells (MCF7, hormone dependent human mammary carcinoma cells, ATCC HTB22; NCI-H460, human non-small cell lung carcinoma cells, ATCC HTB-177; DU 145, hormone-independent human prostate carcinoma cells, ATCC HTB-81; HeLa-MaTu, human cervical carcinoma cells, EPO-GmbH, Berlin; HeLa-MaTu-ADR, multidrug-resistant human cervical carcinoma cells, EPO-GmbH, Berlin; HeLa human cervical tumour cells, ATCC CCL-2; B16F10 mouse melanoma cells, ATCC CRL-6475) were plated at a density of 5000 cells/well (MCF7, DU145, HeLa-MaTu-ADR), 3000 cells/well (NCI-H460, HeLa-MaTu, HeLa), or 1000 cells/well (B16F10) in a 96-well multititer plate in 200 µL of their respective growth medium supplemented 10% fetal calf serum. After 24 hours, the cells of one plate (zero-point plate) were stained with crystal violet (see below), while the medium of the other plates was replaced by fresh culture medium (200 µl), to which the test substances were added in various concentrations (0 µM, as well as in the range of 0.01-30 µM; the final concentration of the solvent dimethyl sulfoxide was 0.5%). The cells were incubated for 4 days in the presence of test substances. Cell proliferation was determined by staining the cells with crystal violet: the cells were fixed by adding 20 µl/measuring point of an 11% glutaric aldehyde solution for 15 minutes at room temperature. After three washing cycles of the fixed cells with water, the plates were dried at room temperature. The cells were stained by adding 100 µl/measuring point of a 0.1 % crystal violet solution (pH 3.0). After three washing cycles of the stained cells with water, the plates were dried at room temperature. The dye was dissolved by adding 100 µl/measuring point of a 10% acetic acid solution. The extinction was determined by photometry at a wavelength of 595 nm. The change of cell number, in percent, was calculated by normalization of the measured values to the extinction values of the zero-point plate (=0%) and the extinction of the untreated (0 µm) cells (=100%). The IC50 values were determined by means of a 4 parameter fit using the company's own software.

### Mps-1 kinase assay

The human kinase Mps-1 phosphorylates a biotinylated substrate peptide. Detection of the phosphorylated product is achieved by time-resolved fluorescence resonance energy transfer (TR-FRET) from Europium-labelled anti-phospho-Serine/Threonine antibody as donor to streptavidin labelled with cross-linked allophycocyanin (SA-XLent) as acceptor. Compounds are tested for their inhibition of the kinase activity.
N-terminally GST-tagged human full length recombinant Mps-1 kinase (purchased from Invitrogen, Karslruhe, Germany, cat. no PV4071) was used. As substrate for the kinase reaction a biotinylated peptide of the amino-acid sequence PWDPDDADITEILG (C-terminus in amide form, purchased from Biosynthan GmbH, Berlin) was used.

For the assay 50 nL of a 100-fold concentrated solution of the test compound in DMSO was pipetted into a black low volume 384well microtiter plate (Greiner Bio-One, Frickenhausen, Germany), 2 µl of a solution of Mps-1 in assay buffer [0.1 mM sodium-ortho-vanadate, 10 mM MgCl₂, 2 mM DTT, 25 mM Hepes pH 7.7, 0.05% BSA, 0.001 % Pluronic F-127] were added and the mixture was incubated for 15 min at 22°C to allow pre-binding of the test compounds to Mps-1 before the start of the kinase reaction. Then the kinase reaction was started by the addition of 3 µl of a solution of 16.7 adenosine-tri-phosphate (ATP, 16.7 µM => final conc. in the 5 µl assay volume is 10 µM) and peptide substrate (1.67 µM => final conc. in the 5 µl assay volume is 1 µM) in assay buffer and the resulting mixture was incubated for a reaction time of 60 min at 22°C. The concentration of Mps-1 in the assay was adjusted to the activity of the enzyme lot and was chosen appropriate to have the assay in the linear range, typical enzyme concentrations were in the range of about 1 nM (final conc. in the 5 µl assay volume). The reaction was stopped by the addition of 3 µl of a solution of HTRF detection reagents (100 mM Hepes pH 7.4, 0.1% BSA, 40 mM EDTA, 140 nM Streptavidin-XLent [# 61GSTXLB, Fa. Cis Biointernational, Marcoule, France], 1.5 nM anti-phospho(Ser/Thr)-Europium-antibody [#AD0180, PerkinElmer LAS, Rodgau-Jügesheim, Germany].

The resulting mixture was incubated 1 h at 22°C to allow the binding of the phosphorylated peptide to the anti-phospho(Ser/Thr)-Europium-antibody. Subsequently the amount of phosphorylated substrate was evaluated by measurement of the resonance energy transfer from the Europium-labelled anti-phospho(Ser/Thr) antibody to the Streptavidin-XLent. Therefore, the fluorescence emissions at 620 nm and 665 nm after excitation at 350 nm was measured in a Viewlux TR-FRET reader (PerkinElmer LAS, Rodgau-Jügesheim, Germany). The "blank-corrected normalized ratio" (a Viewlux specific readout, similar to the traditional ratio of the emissions at 665 nm and at 622 nm, in which blank and Eu-donor crosstalk are subtracted from the 665 nm signal before the ratio is calculated) was taken as the measure for the amount of phosphorylated substrate. The data were normalised (enzyme reaction without inhibitor = 0 % inhibition, all other assay components but no enzyme = 100 % inhibition). Test compounds were tested on the same microtiter plate at 10 different concentrations in the range of 20 µM to 1 nM (20 µM, 6.7 µM, 2.2 µM, 0.74 µM, 0.25 µM, 82 nM, 27 nM, 9.2 nM, 3.1 nM and 1 nM, dilution series prepared before the assay at the level of the 100fold conc. stock solutions by serial 1:3 dilutions) in duplicate values for each concentration and IC₅₀ values were calculated by a 4 parameter fit using an in-house software.

It was surprisingly found that the inhibitory activity of compounds of general formula I can be positively influenced by R³ being an aryl-X- or heteroaryl-X- group. Therefore, compounds of general formula I, *supra,* in which R³ represents an aryl-X- or heteroaryl-X- group (X being selected from O, S, S(=O), S(=O)₂, NR, CR'R") are preferred.

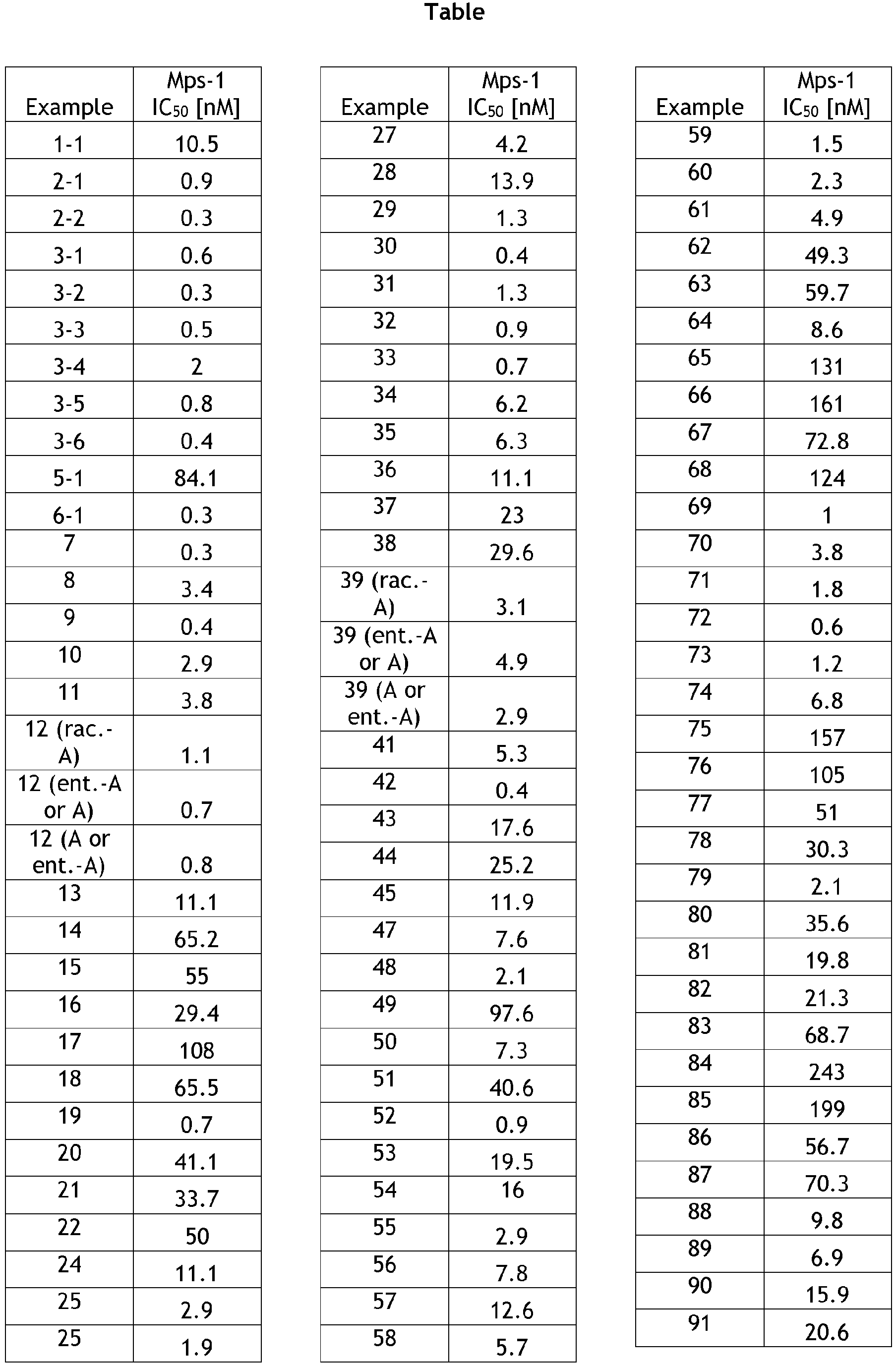

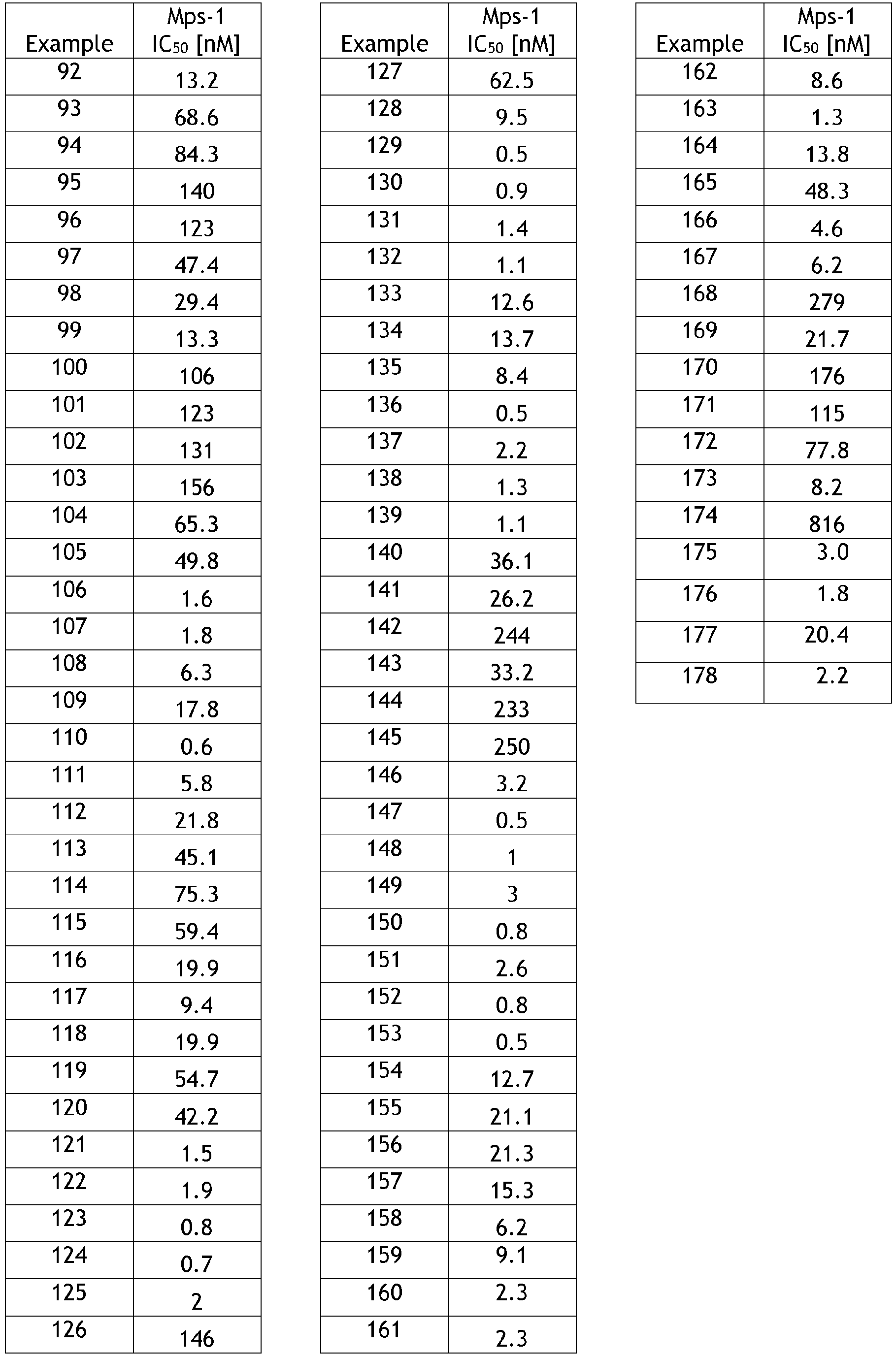

### Spindle Assembly Checkpoint Assay

The spindle assembly checkpoint assures the proper segregation of chromosomes during mitosis. Upon entry into mitosis, chromosomes begin to condensate which is accompanied by the phosphorylation of histone H3 on serine 10. Dephosphorylation of histone H3 on serine 10 begins in anaphase and ends at early telophase. Accordingly, phosphorylation of histone H3 on serine 10 can be utilized as a marker of cells in mitosis. Nocodazole is a microtubule destabilizing substance. Thus, nocodazole interferes with microtubule dynamics and mobilises the spindle assembly checkpoint. The cells arrest in mitosis at G2/M transition and exhibit phosphorylated histone H3 on serine 10. An inhibition of the spindle assembly checkpoint by Mps-1 inhibitors overrides the mitotic blockage in the presence of nocodazole, and the cells complete mitosis prematurely. This alteration is detected by the decrease of cells with phosphorylation of histone H3 on serine 10. This decline is used as a marker to determine the capability of compounds of the present invention to induce a mitotic breakthrough.

Cultivated cells of the human cervical tumour cell line HeLa (ATCC CCL-2) were plated at a density of 2500 cells/well in a 384-well microtiter plate in 20 µl Dulbeco's Medium (w/o phenol red, w/o sodium pyruvate, w 1000 mg/ml glucose, w pyridoxine) supplemented with 1% (v/v) glutamine, 1% (v/v) penicillin, 1% (v/v) streptomycin and 10% (v/v) fetal calf serum. After incubation overnight at 37°C, 10 µl/well nocodazole at a final concentration of 0.1 µg/ml were added to cells. After 24 h incubation, cells were arrested at G2/M phase of the cell cycle progression. Test compounds solubilised in dimethyl sulfoxide (DMSO) were added at various concentrations (0 µM, as well as in the range of 0.005 µM - 10 µM; the final concentration of the solvent DMSO was 0.5% (v/v)). Cells were incubated for 4 h at 37°C in the presence of test compounds. Thereafter, cells were fixed in 4% (v/v) paraformaldehyde in phosphate buffered saline (PBS) at 4°C overnight then permeabilised in 0.1% (v/v) Triton X™ 100 in PBS at room temperature for 20 min and blocked in 0.5% (v/v) bovine serum albumin (BSA) in PBS at room temperature for 15 min. After washing with PBS, 20 µl/well antibody solution (anti-phospho-histone H3 clone 3H10, FITC; Upstate, Cat# 16-222; 1:200 dilution) was added to cells, which were incubated for 2 h at room temperature. Afterwards, cells were washed with PBS and 20 µl/well HOECHST 33342 dye solution (5 µg/ml) was added to cells and cells were incubated 12 min at room temperature in the dark. Cells were washed twice with PBS then covered with PBS and stored at 4°C until analysis. Images were acquired with a Perkin Elmer OPERA™ High-Content Analysis reader. Images were analyzed with image analysis software MetaXpress™ from Molecular devices utilizing the Cell Cycle application module. In this assay both labels HOECHST 33342 and phosphorylated Histone H3 on serine 10 were measured. HOECHST 33342 labels DNA and is used to count cell number. The staining of phosphorylated Histone H3 on serine 10 determines the number of mitotic cells. Inhibition of Mps-1 decreases the number of mitotic cells in the presence of nocodazole indicating an inappropriate mitotic progression. The raw assay data were further analysed by four parameter logistic regression analysis to determine the IC₅₀ value for each tested compound.

It will be apparent to persons skilled in the art that assays for other Mps kinases may be performed in analogy using the appropriate reagents.

Thus the compounds of the present invention effectively inhibit one or more Mps-1 kinases and are therefore suitable for the treatment or prophylaxis of diseases of uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by Mps-1, more particularly in which the diseases of uncontrolled cell growth, proliferation and/or survival, inappropriate cellular immune responses, or inappropriate cellular inflammatory responses are haemotological tumours, solid tumours and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

### Investigation of in vitro metabolic stability in rat hepatocytes (including calculation of hepatic in vivo blood clearance (CL))

Hepatocytes from Han Wistar rats were isolated via a 2-step perfusion method. After perfusion, the liver was carefully removed from the rat: the liver capsule was opened and the hepatocytes were gently shaken out into a Petri dish with ice-cold WME. The resulting cell suspension was filtered through sterile gaze in 50 ml falcon tubes and centrifuged at 50 × g for 3 min at room temperature. The cell pellet was resuspended in 30 ml WME and centrifuged through a Percoll^{®} gradient for 2 times at 100 × g. The hepatocytes were washed again with Williams' medium E (WME) and resuspended in medium containing 5% FCS. Cell viability was determined by trypan blue exclusion.
For the metabolic stability assay liver cells were distributed in WME containing 5% FCS to glas vials at a density of 1.0 × 10⁶ vital cells/ml. The test compound was added to a final concentration of 1 µM. During incubation, the hepatocyte suspensions were continuously shaken and aliquots were taken at 2, 8, 16, 30, 45 and 90 min, to which equal volumes of cold methanol were immediately added. Samples were frozen at -20° C over night, after subsequently centrifuged for 15 minutes at 3000 rpm and the supernatant was analyzed with an Agilent 1200 HPLC-system with LCMS/MS detection.

The half-life of a test compound was determined from the concentration-time plot. From the half-life the intrinsic clearances were calculated. Together with the additional parameters liver blood flow, amount of liver cells *in vivo* and *in vitro.* The hepatic *in vivo* blood clearance (CL) and the maximal oral bioavailability (Fₘₐₓ) was calculated. The following parameter values were used: Liver blood flow - 4.2 L/h/kg rat; specific liver weight - 32 g/kg rat body weight; liver cells *in vivo-* 1.1 x 10⁸ cells/g liver, liver cells *in vitro -* 0.5 x 10⁶/ml.

It was surprisingly found that the metabolic stability of compounds of general formula I can be positively influenced by at least one of the groups R^{4b} and R^{4c} being different from a hydrogen atom.

It was surprisingly found that the metabolic stability of compounds of general formula I can be positively influenced by R⁵ being a 1,1,1-trifluoroethyl group. Compounds of formula I with R⁵ being a 1,1,1-trifluoroethyl group are therefore preferred.

## Claims

1. A compound of general formula I : 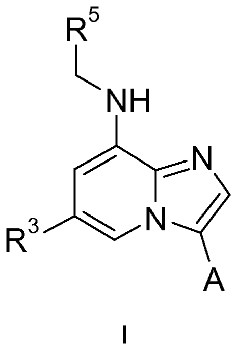 in which :
A represents a 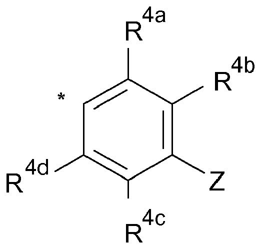 group ;
wherein * indicates the point of attachment of said groups with the rest of the molecule ;
Z represents a -C(=O)N(H)R¹ or a -C(=S)N(H)R¹ group ;
R¹ represents a C₁-C₃-alkyl- or a cyclopropyl- group ;
wherein said cyclopropyl- group is optionally substituted, identically or differently, with 1, 2 or 3 groups selected from: halogen, -OH, -CN, C₁-C₃-alkyl- ;
wherein said C₁-C₃-alkyl- group is optionally substituted, identically or differently, with 1, 2 or 3 groups selected from: halogen, -OH, -CN, C₁-C₃-alkoxy-;
R³ represents a hydrogen atom or a -CN, C₁-C₆-alkyl-, -(CH₂)m-C₂-C₆-alkenyl, -(CH₂)ₘ-C₂-C₆-alkynyl, aryl-, heteroaryl-, aryl-X- group ;
wherein said C₁-C₆-alkyl-, -(CH₂)ₘ-C₂-C₆-alkenyl, -(CH₂)ₘ-C₂-C₆-alkynyl, aryl- aryl-X- or heteroaryl- group is optionally substituted, identically or differently, with 1, 2 or 3 R⁷ groups;
R^{4a}, R^{4b}, R^{4d} represent a hydrogen atom ;
R^{4c} represents a halogen atom, or a -CN, -OH, C₁-C₃-alkyl-, C₁-C₃-alkoxy-, halo-C₁-C₃-alkyl-, halo-C₁-C₃-alkoxy-, R^{6a}(R^{6b})N-C₁-C₃-alkyl-, HO-C₁-C₃-alkyl-, NC-C₁-C₃-alkyl-, C₁-C₃-alkoxy-C₁-C₃-alkyl-, halo-C₁-C₃-alkoxy-C₁-C₃-alkyl- group ;
R⁵ represents a hydrogen atom, or a C₁-C₆-alkyl-, (CH₂)ₙ-C₂-C₆-alkenyl, -(CH₂)ₙ-C₂-C₆-alkynyl, -(CH₂)ₘ-C₃-C₆-cycloalkyl, -(CH₂)ₘ-(3- to 7-membered heterocycloalkyl), aryl-C₁-C₆-alkyl-, heteroaryl-C₁-C₆-alkyl-, halo-C₁-C₆-alkyl-, R^{6a}(R^{6b})N-C₁-C₆-alkyl-, HO-C₁-C₆-alkyl-, -C₁-C₆-alkyl-CN, C₁-C₆-alkoxy-C₁-C₆-alkyl-, halo-C₁-C₆-alkoxy-C₁-C₆-alkyl-, C₃-C₆-cycloalkyl-, 3- to 7-membered heterocycloalkyl-, C₄-C₈-cycloalkenyl-, aryl- or heteroaryl-group ;
wherein said C₁-C₆-alkyl-, -(CH₂)ₙ-C₂-C₆-alkenyl, -(CH₂)ₙ-C₂-C₆-alkynyl, -(CH₂)ₘ-C₃-C₆-cycloalkyl, -(CH₂)ₘ-(3- to 7-membered heterocycloalkyl), aryl-C₁-C₆-alkyl-, heteroaryl-C₁-C₆-alkyl-, halo-C₁-C₆-alkyl-, R^{6a}(R^{6b})N-C₁-C₆-alkyl-, HO-C₁-C₆-alkyl-, -C₁-C₆-alkyl-CN, C₁-C₆-alkoxy-C₁-C₆-alkyl-, halo-C₁-C₆-alkoxy-C₁-C₆-alkyl-, C₃-C₆-cycloalkyl-, 3- to 7-membered heterocycloalkyl-, C₄-C₈-cycloalkenyl-, aryl- or heteroaryl-group is optionally substituted, identically or differently, with 1, 2, 3, 4 or 5 R⁸ groups ;
R⁶, R^{6a}, R^{6b}, R^{6c} represent, independently from each other, a hydrogen atom, or a C₁-C₆-alkyl-, C₃-C₆-cycloalkyl- or aryl-C₁-C₆-alkyl- group ;
wherein said C₃-C₆-cycloalkyl- group is optionally substituted, identically or differently with 1 or 2 groups selected from: halogen, -OH, -CN, C₁-C₆-alkyl-, HO-C₁-C₆-alkyl- ;
R⁷ represents a halogen atom or a HO-, C₁-C₆-alkoxy-, C₁-C₆-alkyl-, halo-C₁-C₆-alkyl-, halo-C₁-C₆-alkoxy-, HO-C₁-C₆-alkyl-, -C(=O)N(H)R^{ba}, -N(R^{6a})R^{6b}, -O(C=O)OR⁶ or -OR⁶ group ;
R⁸ represents a hydrogen or halogen atom or a -CN, C₁-C₆-alkoxy-, C₁-C₆-alkyl-, halo-C₁-C₆-alkyl-, R^{6a}(R^{6b})N-C₁-C₆-alkyl-, HO-C₁-C₆-alkyl-, C₁-C₆-alkoxy-C₁-C₆-alkyl-, halo-C₁-C₆-alkoxy-C₁-C₆-alkyl-, C₂-C₆-alkenyl-, C₂-C₆-alkynyl-, 3- to 7-membered heterocycloalkyl-, aryl-, heteroaryl-, -C(=O)R⁶, -C(=O)N(H)R^{6a}, -C(=O)N(R^{6a})R^{6b}, -C(=O)O-R⁶, -N(R^{6a})R^{6b}, -NO₂, -N(H)C(=O)R⁶, -N(R^{6c})C(=O)R⁶, -N(H)C(=O)N(R^{6a})R^{6b}, -N(R^{6c})C(=O)N(R^{6a})R^{6b}, -N(H)C(=O)OR⁶, -N(R^{6c})C(=O)OR⁶, -N(H)S(=O)R⁶, -N(R^{6c})S(=O)R⁶, -N(H)S(=O)₂R⁶, -N(R^{6c})S(=O)₂R⁶, -N=S(=O)(R^{6a})R^{6b}, -OR⁶, -O(C=O)R⁶, -O(C=O)N(R^{6a})R^{6b}, -O(C=O)OR⁶, -SR⁶, -S(=O)R⁶, - S(=O)N(H)R⁶, -S(=O)N(R^{6a})R^{6b}, -S(=O₂)R⁶, -S(=O)₂N(H)R⁶, -S(=O)₂N(R^{6a})R^{6b}, -S(=O)(=NR^{6c})R⁶ or -S(=O)₂-(3- to 7-membered heterocycloalkyl) group ; wherein said 3- to 7-membered heterocycloalkyl- or heteroaryl- group, is optionally substituted, identically or differently, with 1, 2, 3 or 4 C₁-C₆-alkyl- groups ;
m is an integer of 0, 1, 2, 3, 4, 5 or 6 ;
n is an integer of 0, 1, 2, 3, 4 or 5 ; and
X is S, S(=O), S(=O)₂, O, NR⁶, C(=O) or CR^{6a}R^{6b} ;
or a stereoisomer, a tautomer, an N-oxide, a hydrate, a solvate, or a salt thereof, or a mixture of same.

2. A compound according to claim 1, wherein :
A represents 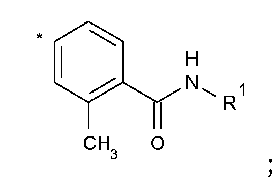 wherein * indicates the point of attachment of said group with the rest of the molecule ;
R¹ represents a methyl- group or a cyclopropyl- group ; wherein said cyclopropyl- group is optionally substituted with one fluorine atom;
R³ represents a pyridyl-, phenyl-, phenyl-O- or phenyl-S- group ; wherein the phenyl- group is either substituted with a methyl- group and a -C(=O)N(H)R^{6a} group, or with a HO-CH₂- group;
wherein the phenyl-O- group is optionally substituted, identically or differently, with 1 or 2 groups selected from: -F, H₃C-O-, HO-, H₃C-; wherein the phenyl-S- group is optionally substituted, identically or differently, with 1 or 2 groups selected from: -F, H₃C-O-, HO-, H₃C-;
R⁵ represents a group selected from: 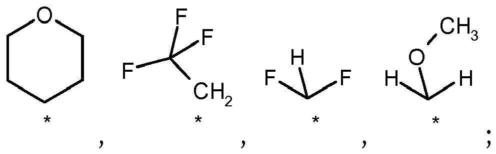 wherein * indicates the point of attachment of said groups with the rest of the molecule
R^{6a} represents a hydrogen atom or methyl- group or a C₃-C₆-cyclpropyl- group;
wherein said C₃-C₆-cyclpropyl- group is optionally substituted with one -CN group ;
or a stereoisomer, a tautomer, an N-oxide, a hydrate, a solvate, or a salt thereof, or a mixture of same.

3. A compound according to claim 1, which is selected from the group consisting of:
N-cyclopropyl-4-{8-[(2-methylpropyl)amino]imidazo[1,2-a]pyridin-3-yl}benzamide,
N-cyclopropyl-4-{6-[2-(hydroxymethyl)phenyl]-8-[(3,3,3-trifluoropropyl)amino]imidazo[1,2-a]pyridin-3-yl}-2-methylbenzamide,
N-cyclopropyl-2-methyl-4-{6-(pyridin-4-yl)-8-[(3,3,3-trifluoropropyl)amino]imidazo[1,2-a]pyridin-3-yl}benzamide,
N-cyclopropyl-4-{6-[(3-fluoro-5-methylphenyl)sulfanyl]-8-[(3,3,3-trifluoropropyl)amino]imidazo[1,2-a]pyridin-3-yl}-2-methylbenzamide,
N-cyclopropyl-4-{6-[(2,3-difluorophenyl)sulfanyl]-8-[(3,3,3-trifluoropropyl)amino]imidazo[1,2-a]pyridin-3-yl}-2-methylbenzamide,
N-cyclopropyl-2-methyl-4-{6-(phenylsulfanyl)-8-[(3,3,3-trifluoropropyl)amino]imidazo[1,2-a]pyridin-3-yl}benzamide,
4-{6-[(2-aminophenyl)sulfanyl]-8-[(3,3,3-trifluoropropyl)amino]imidazo[1,2-a]pyridin-3-yl}-N-cyclopropyl-2-methylbenzamide,
N-cyclopropyl-4-{6-[(3-fluorophenyl)sulfanyl]-8-[(3,3,3-trifluoropropyl)amino]imidazo[1,2-a]pyridin-3-yl}-2-methylbenzamide,
N-cyclopropyl-4-{6-[(2-hydroxyphenyl)sulfanyl]-8-[(3,3,3-trifluoropropyl)amino]imidazo[1,2-a]pyridin-3-yl}-2-methylbenzamide,
N-cyclopropyl-2-methyl-4-{6-(methylsulfonyl)-8-[(3,3,3-trifluoropropyl)amino]imidazo[1,2-a]pyridin-3-yl}benzamide,
N-cyclopropyl-4-{6-(2-fluoro-4-methoxyphenoxy)-8-[(3,3,3-trifluoropropyl)amino]imidazo[1,2-a]pyridin-3-yl}-2-methylbenzamide,
N-cyclopropyl-4-{6-(3-fluorophenoxy)-8-[(3,3,3-trifluoropropyl)amino]imidazo[1,2-a]pyridin-3-yl}-2-methylbenzamide,
4-{6-(3-fluorophenoxy)-8-[(3,3,3-trifluoropropyl)amino]imidazo[1,2-a]pyridin-3-yl}-N,2-dimethylbenzamide,
4,4'-{8-[(tetrahydro-2H-pyran-4-ylmethyl)amino]imidazo[1,2-a]pyridine-3,6-diyl}bis(N-cyclopropyl-2-methylbenzamide),
N-ethyl-4-{6-(3-fluorophenoxy)-8-[(3,3,3-trifluoropropyl)amino]imidazo[1,2-a]pyridin-3-yl}-2-methylbenzamide,
4-{6-(3-fluorophenoxy)-8-[(3,3,3-trifluoropropyl)amino]imidazo[1,2-a]pyridin-3-yl}-2-methyl-N-(1-methylcyclopropyl)benzamide,
N-[rel-(1S,2S)-2-fluorocyclopropyl]-4-{6-(3-fluorophenoxy)-8-[(3,3,3-trifluoropropyl)amino]imidazo[1,2-a]pyridin-3-yl}-2-methylbenzamide,
N-{[(1R,2R) or (1S,2S)]-2-fluorocyclopropyl}-4-{6-(3-fluorophenoxy)-8-[(3,3,3-trifluoropropyl)amino]imidazo[1,2-a]pyridin-3-yl}-2-methylbenzamide,
N-{[(1S,2S) or (1R,2R)]-2-fluorocyclopropyl}-4-{6-(3-fluorophenoxy)-8-[(3,3,3-trifluoropropyl)amino]imidazo[1,2-a]pyridin-3-yl}-2-methylbenzamide,
N-(1-cyanocyclopropyl)-4-{6-(3-fluorophenoxy)-8-[(3,3,3-trifluoropropyl)amino]imidazo[1,2-a]pyridin-3-yl}-2-methylbenzamide,
4-{6-ethynyl-8-[(3,3,3-trifluoropropyl)amino]imidazo[1,2-a]pyridin-3-yl}-N,2-dimethylbenzamide,
N-ethyl-4-{6-ethynyl-8-[(3,3,3-trifluoropropyl)amino]imidazo[1,2-a]pyridin-3-yl}-2-methylbenzamide,
4-{6-ethynyl-8-[(3,3,3-trifluoropropyl)amino]imidazo[1,2-a]pyridin-3-yl}-N-[rel-(1R,2R)-2-fluorocyclopropyl]-2-methylbenzamide,
4-{6-ethynyl-8-[(3,3,3-trifluoropropyl)amino]imidazo[1,2-a]pyridin-3-yl}-2-methyl-N-(1-methylcyclopropyl)benzamide,
N-(1-cyanocyclopropyl)-4-{6-ethynyl-8-[(3,3,3-trifluoropropyl)amino]imidazo[1,2-a]pyridin-3-yl}-2-methylbenzamide,
N-cyclopropyl-4-{6-(5-fluoro-2-methylphenoxy)-8-[(3,3,3-trifluoropropyl)amino]imidazo[1,2-a]pyridin-3-yl}-2-methylbenzamide,
4-{6-(3-hydroxyprop-1-yn-1-yl)-8-[(3,3,3-trifluoropropyl)amino]imidazo[1,2-a]pyridin-3-yl}-N,2-dimethylbenzamide,
N-ethyl-4-{6-(3-hydroxyprop-1-yn-1-yl)-8-[(3,3,3-trifluoropropyl)amino]imidazo[1,2-a]pyridin-3-yl}-2-methylbenzamide,
4-{6-(3-hydroxyprop-1-yn-1-yl)-8-[(3,3,3-trifluoropropyl)amino]imidazo[1,2-a]pyridin-3-yl}-2-methyl-N-(1-methylcyclopropyl)benzamide,
N-(1-cyanocyclopropyl)-4-{6-(3-hydroxyprop-1-yn-1-yl)-8-[(3,3,3-trifluoropropyl)amino]imidazo[1,2-a]pyridin-3-yl}-2-methylbenzamide,
N-cyclobutyl-4-{6-(3-fluorophenoxy)-8-[(3,3,3-trifluoropropyl)amino]imidazo[1,2-a]pyridin-3-yl}-2-methylbenzamide,
4-{6-(5-fluoro-2-methylphenoxy)-8-[(3,3,3-trifluoropropyl)amino]imidazo[1,2-a]pyridin-3-yl}-N,2-dimethylbenzamide,
N-ethyl-4-{6-(5-fluoro-2-methylphenoxy)-8-[(3,3,3-trifluoropropyl)amino]imidazo[1,2-a]pyridin-3-yl}-2-methylbenzamide,
4-{6-(5-fluoro-2-methylphenoxy)-8-[(3,3,3-trifluoropropyl)amino]imidazo[1,2-a]pyridin-3-yl}-2-methyl-N-(1-methylcyclopropyl)benzamide,
N-(1-cyanocyclopropyl)-4-{6-(5-fluoro-2-methylphenoxy)-8-[(3,3,3-trifluoropropyl)amino]imidazo[1,2-a]pyridin-3-yl}-2-methylbenzamide,
N-[rel-(1S,2S)-2-fluorocyclopropyl]-4-{6-(5-fluoro-2-methylphenoxy)-8-[(3,3,3-trifluoropropyl)amino]imidazo[1,2-a]pyridin-3-yl}-2-methylbenzamide,
N-cyclopropyl-4-{6-(2,3-difluorophenoxy)-8-[(3,3,3-trifluoropropyl)amino]imidazo[1,2-a]pyridin-3-yl}-2-methylbenzamide,
4-{6-(3-chlorophenoxy)-8-[(3,3,3-trifluoropropyl)amino]imidazo[1,2-a]pyridin-3-yl}-N-cyclopropyl-2-methylbenzamide,
4,4'-{8-[(3,3,3-trifluoropropyl)amino]imidazo[1,2-a]pyridine-3,6-diyl}bis(N-cyclopropyl-2-methylbenzamide),
N-cyclopropyl-2-methyl-4-{6-phenoxy-8-[(3,3,3-trifluoropropyl)amino]imidazo[1,2-a]pyridin-3-yl}benzamide,
4-{6-(3-chlorophenoxy)-8-[(3,3,3-trifluoropropyl)amino]imidazo[1,2-a]pyridin-3-yl}-N,2-dimethylbenzamide,
4-{6-(3-chlorophenoxy)-8-[(3,3,3-trifluoropropyl)amino]imidazo[1,2-a]pyridin-3-yl}-N-ethyl-2-methylbenzamide,
4-{6-(3-chlorophenoxy)-8-[(3,3,3-trifluoropropyl)amino]imidazo[1,2-a]pyridin-3-yl}-2-methyl-N-(1-methylcyclopropyl)benzamide,
4-{6-(3-chlorophenoxy)-8-[(3,3,3-trifluoropropyl)amino]imidazo[1,2-a]pyridin-3-yl}-N-(1-cyanocyclopropyl)-2-methylbenzamide,
4-{6-(3-fluorophenoxy)-8-[(3,3,3-trifluoropropyl)amino]imidazo[1,2-a]pyridin-3-yl}-2-methyl-N-(1-methylcyclobutyl)benzamide,
4-{6-(3-chlorophenoxy)-8-[(3,3,3-trifluoropropyl)amino]imidazo[1,2-a]pyridin-3-yl}-N-[rel-(1R,2R)-2-fluorocyclopropyl]-2-methylbenzamide,
4-{6-(3-chlorophenoxy)-8-[(3,3,3-trifluoropropyl)amino]imidazo[1,2-a]pyridin-3-yl}-N-{[(1S,2S) or (1R,2R)]-2-fluorocyclopropyl}-2-methylbenzamide,
4-{6-(3-chlorophenoxy)-8-[(3,3,3-trifluoropropyl)amino]imidazo[1,2-a]pyridin-3-yl}-N-{[(1R,2R) or (1S,2S)]-2-fluorocyclopropyl}-2-methylbenzamide,
N-(2,6-diethylphenyl)-4-{6-(3-fluorophenoxy)-8-[(3,3,3-trifluoropropyl)amino]imidazo[1,2-a]pyridin-3-yl}-2-methylbenzamide,
4,4'-{8-[methyl(3,3,3-trifluoropropyl)amino]imidazo[1,2-a]pyridine-3,6-diyl}bis(N,2-dimethylbenzamide),
N-cyclopropyl-4-{6-(3-fluoro-4-methoxyphenoxy)-8-[(3,3,3-trifluoropropyl)amino]imidazo[1,2-a]pyridin-3-yl}-2-methylbenzamide,
4,4'-{8-[methyl(3,3,3-trifluoropropyl)amino]imidazo[1,2-a]pyridine-3,6-diyl}bis[N-(1-cyanocyclopropyl)-2-methylbenzamide],
4,4'-{8-[methyl(3,3,3-trifluoropropyl)amino]imidazo[1,2-a]pyridine-3,6-diyl}bis[2-methyl-N-(1-methylcyclopropyl)benzamide],
4,4'-{8-[methyl(3,3,3-trifluoropropyl)amino]imidazo[1,2-a]pyridine-3,6-diyl}bis(N-ethyl-2-methylbenzamide),
N-(2,6-diethylphenyl)-4-{6-(5-fluoro-2-methylphenoxy)-8-[(3,3,3-trifluoropropyl)amino]imidazo[1,2-a]pyridin-3-yl}-2-methylbenzamide,
N-cyclobutyl-4-{6-(5-fluoro-2-methylphenoxy)-8-[(3,3,3-trifluoropropyl)amino]imidazo[1,2-a]pyridin-3-yl}-2-methylbenzamide,
rel-4-{6-(3-fluorophenoxy)-8-[(3,3,3-trifluoropropyl)amino]imidazo[1,2-a]pyridin-3-yl}-2-methyl-N-[(1R,2R)-2-methylcyclopropyl]benzamide,
N-[1,1'-bi(cyclopropyl)-1-yl]-4-{6-(3-fluorophenoxy)-8-[(3,3,3-trifluoropropyl)amino]imidazo[1,2-a]pyridin-3-yl}-2-methylbenzamide,
4-{6-(3-fluorophenoxy)-8-[(3,3,3-trifluoropropyl)amino]imidazo[1,2-a]pyridin-3-yl}-N-[1-(hydroxymethyl)cyclopropyl]-2-methylbenzamide,
N-(1-cyanocyclobutyl)-4-{6-(3-fluorophenoxy)-8-[(3,3,3-trifluoropropyl)amino]imidazo[1,2-a]pyridin-3-yl}-2-methylbenzamide,
N-cyclopropyl-4-{6-(3-methoxyphenoxy)-8-[(3,3,3-trifluoropropyl)amino]imidazo[1,2-a]pyridin-3-yl}-2-methylbenzamide,
N-(1-cyanocyclopropyl)-4-{6-(3-methoxyphenoxy)-8-[(3,3,3-trifluoropropyl)amino]imidazo[1,2-a]pyridin-3-yl}-2-methylbenzamide,
4-{6-(3-methoxyphenoxy)-8-[(3,3,3-trifluoropropyl)amino]imidazo[1,2-a]pyridin-3-yl}-2-methyl-N-(1-methylcyclopropyl)benzamide,
4-{6-(3-methoxyphenoxy)-8-[(3,3,3-trifluoropropyl)amino]imidazo[1,2-a]pyridin-3-yl}-N,2-dimethylbenzamide,
N-ethyl-4-{6-(3-methoxyphenoxy)-8-[(3,3,3-trifluoropropyl)amino]imidazo[1,2-a]pyridin-3-yl}-2-methylbenzamide,
N-(1-cyanocyclopropyl)-2-methyl-4-{6-phenoxy-8-[(3,3,3-trifluoropropyl)amino]imidazo[1,2-a]pyridin-3-yl}benzamide,
2-methyl-N-(1-methylcyclopropyl)-4-{6-phenoxy-8-[(3,3,3-trifluoropropyl)amino]imidazo[1,2-a]pyridin-3-yl}benzamide,
N,2-dimethyl-4-{6-phenoxy-8-[(3,3,3-trifluoropropyl)amino]imidazo[1,2-a]pyridin-3-yl}benzamide,
N-ethyl-2-methyl-4-{6-phenoxy-8-[(3,3,3-trifluoropropyl)amino]imidazo[1,2-a]pyridin-3-yl}benzamide,
rel-4-{6-(5-fluoro-2-methylphenoxy)-8-[(3,3,3-trifluoropropyl)amino]imidazo[1,2-a]pyridin-3-yl}-2-methyl-N-[(1R,2R)-2-methylcyclopropyl]benzamide,
N-[1,1'-bi(cyclopropyl)-1-yl]-4-{6-(5-fluoro-2-methylphenoxy)-8-[(3,3,3-trifluoropropyl)amino]imidazo[1,2-a]pyridin-3-yl}-2-methylbenzamide,
N-(1-cyanocyclobutyl)-4-{6-(5-fluoro-2-methylphenoxy)-8-[(3,3,3-trifluoropropyl)amino]imidazo[1,2-a]pyridin-3-yl}-2-methylbenzamide,
N-cyclopropyl-2-methyl-4-{6-[4-(trifluoromethoxy)phenoxy]-8-[(3,3,3-trifluoropropyl)amino]imidazo[1,2-a]pyridin-3-yl}benzamide,
N-(1-cyanocyclopropyl)-2-methyl-4-{6-[4-(trifluoromethoxy)phenoxy]-8-[(3,3,3-trifluoropropyl)amino]imidazo[1,2-a]pyridin-3-yl}benzamide,
2-methyl-N-(1-methylcyclopropyl)-4-{6-[4-(trifluoromethoxy)phenoxy]-8-[(3,3,3-trifluoropropyl)amino]imidazo[1,2-a]pyridin-3-yl}benzamide,
N,2-dimethyl-4-{6-[4-(trifluoromethoxy)phenoxy]-8-[(3,3,3-trifluoropropyl)amino]imidazo[1,2-a]pyridin-3-yl}benzamide,
N-ethyl-2-methyl-4-{6-[4-(trifluoromethoxy)phenoxy]-8-[(3,3,3-trifluoropropyl)amino]imidazo[1,2-a]pyridin-3-yl}benzamide,
N-cyclopropyl-4-{6-(2-fluoro-4-methoxyphenoxy)-8-[(3,3,3-trifluoropropyl)amino]imidazo[1,2-a]pyridin-3-yl}-2-methylbenzamide,
N-(1-cyanocyclopropyl)-4-{6-(2,3-difluorophenoxy)-8-[(3,3,3-trifluoropropyl)amino]imidazo[1,2-a]pyridin-3-yl}-2-methylbenzamide,
4-{6-(2,3-difluorophenoxy)-8-[(3,3,3-trifluoropropyl)amino]imidazo[1,2-a]pyridin-3-yl}-2-methyl-N-(1-methylcyclopropyl)benzamide,
4-{6-(2,3-difluorophenoxy)-8-[(3,3,3-trifluoropropyl)amino]imidazo[1,2-a]pyridin-3-yl}-N,2-dimethylbenzamide,
4-{6-(2,3-difluorophenoxy)-8-[(3,3,3-trifluoropropyl)amino]imidazo[1,2-a]pyridin-3-yl}-N-ethyl-2-methylbenzamide,
N-cyclopropyl-2-methyl-4-{6-[3-(trifluoromethoxy)phenoxy]-8-[(3,3,3-trifluoropropyl)amino]imidazo[1,2-a]pyridin-3-yl}benzamide,
N-(1-cyanocyclopropyl)-2-methyl-4-{6-[3-(trifluoromethoxy)phenoxy]-8-[(3,3,3-trifluoropropyl)amino]imidazo[1,2-a]pyridin-3-yl}benzamide,
2-methyl-N-(1-methylcyclopropyl)-4-{6-[3-(trifluoromethoxy)phenoxy]-8-[(3,3,3-trifluoropropyl)amino]imidazo[1,2-a]pyridin-3-yl}benzamide,
N-ethyl-2-methyl-4-{6-[3-(trifluoromethoxy)phenoxy]-8-[(3,3,3-trifluoropropyl)amino]imidazo[1,2-a]pyridin-3-yl}benzamide,
N,2-dimethyl-4-{6-[3-(trifluoromethoxy)phenoxy]-8-[(3,3,3-trifluoropropyl)amino]imidazo[1,2-a]pyridin-3-yl}benzamide,
N-cyclopropyl-4-{6-(2-methoxyphenyl)-8-[(3,3,3-trifluoropropyl)amino]imidazo[1,2-a]pyridin-3-yl}-2-methylbenzamide,
4-{6-(2-methoxyphenyl)-8-[(3,3,3-trifluoropropyl)amino]imidazo[1,2-a]pyridin-3-yl}-2-methyl-N-(1-methylcyclopropyl)benzamide,
4-{6-(2-methoxyphenyl)-8-[(3,3,3-trifluoropropyl)amino]imidazo[1,2-a]pyridin-3-yl}-N,2-dimethylbenzamide,
N-ethyl-4-{6-(2-methoxyphenyl)-8-[(3,3,3-trifluoropropyl)amino]imidazo[1,2-a]pyridin-3-yl}-2-methylbenzamide,
N-cyclopropyl-4-{6-[3-fluoro-4-(trifluoromethoxy)phenoxy]-8-[(3,3,3-trifluoropropyl)amino]imidazo[1,2-a]pyridin-3-yl}-2-methylbenzamide,
N-(1-cyanocyclopropyl)-4-{6-[3-fluoro-4-(trifluoromethoxy)phenoxy]-8-[(3,3,3-trifluoropropyl)amino]imidazo[1,2-a]pyridin-3-yl}-2-methylbenzamide, 4-{6-[3-fluoro-4-(trifluoromethoxy)phenoxy]-8-[(3,3,3-trifluoropropyl)amino]imidazo[1,2-a]pyridin-3-yl}-2-methyl-N-(1-methylcyclopropyl)benzamide,
4-{6-[3-fluoro-4-(trifluoromethoxy)phenoxy]-8-[(3,3,3-trifluoropropyl)amino]imidazo[1,2-a]pyridin-3-yl}-N,2-dimethylbenzamide,
N-ethyl-4-{6-[3-fluoro-4-(trifluoromethoxy)phenoxy]-8-[(3,3,3-trifluoropropyl)amino]imidazo[1,2-a]pyridin-3-yl}-2-methylbenzamide,
N,2-dimethyl-4-{6-(pyridin-4-yl)-8-[(3,3,3-trifluoropropyl)amino]imidazo[1,2-a]pyridin-3-yl}benzamide,
N-ethyl-2-methyl-4-{6-(pyridin-4-yl)-8-[(3,3,3-trifluoropropyl)amino]imidazo[1,2-a]pyridin-3-yl}benzamide,
2-methyl-N-(1-methylcyclopropyl)-4-{6-(pyridin-4-yl)-8-[(3,3,3-trifluoropropyl)amino]imidazo[1,2-a]pyridin-3-yl}benzamide,
N-(1-cyanocyclopropyl)-2-methyl-4-{6-(pyridin-4-yl)-8-[(3,3,3-trifluoropropyl)amino]imidazo[1,2-a]pyridin-3-yl}benzamide,
N-cyclopropyl-2-methyl-4-{8-[(3,3,3-trifluoropropyl)amino]-6-vinylimidazo[1,2-a]pyridin-3-yl}benzamide,
N,2-dimethyl-4-{8-[(3,3,3-trifluoropropyl)amino]-6-vinylimidazo[1,2-a]pyridin-3-yl}benzamide,
N-ethyl-2-methyl-4-{8-[(3,3,3-trifluoropropyl)amino]-6-vinylimidazo[1,2-a]pyridin-3-yl}benzamide,
2-methyl-N-(1-methylcyclopropyl)-4-{8-[(3,3,3-trifluoropropyl)amino]-6-vinylimidazo[1,2-a]pyridin-3-yl}benzamide,
N-(1-cyanocyclopropyl)-2-methyl-4-{8-[(3,3,3-trifluoropropyl)amino]-6-vinylimidazo[1,2-a]pyridin-3-yl}benzamide,
N-[rel-(1S,2S)-2-fluorocyclopropyl]-2-methyl-4-{8-[(3,3,3-trifluoropropyl)amino]-6-vinylimidazo[1,2-a]pyridin-3-yl}benzamide,
2-methyl-N-[rel-(1S,2S)-2-methylcyclopropyl]-4-{8-[(3,3,3-trifluoropropyl)amino]-6-vinylimidazo[1,2-a]pyridin-3-yl}benzamide,
N-cyclopropyl-4-{6-ethyl-8-[(3,3,3-trifluoropropyl)amino]imidazo[1,2-a]pyridin-3-yl}-2-methylbenzamide,
4-{6-ethyl-8-[(3,3,3-trifluoropropyl)amino]imidazo[1,2-a]pyridin-3-yl}-N,2-dimethylbenzamide,
N-ethyl-4-{6-ethyl-8-[(3,3,3-trifluoropropyl)amino]imidazo[1,2-a]pyridin-3-yl}-2-methylbenzamide,
4-{6-ethyl-8-[(3,3,3-trifluoropropyl)amino]imidazo[1,2-a]pyridin-3-yl}-2-methyl-N-(1-methylcyclopropyl)benzamide,
N-(1-cyanocyclopropyl)-4-{6-ethyl-8-[(3,3,3-trifluoropropyl)amino]imidazo[1,2-a]pyridin-3-yl}-2-methylbenzamide,
4-{6-ethyl-8-[(3,3,3-trifluoropropyl)amino]imidazo[1,2-a]pyridin-3-yl}-N-[rel-(1S,2S)-2-fluorocyclopropyl]-2-methylbenzamide,
4-{6-ethyl-8-[(3,3,3-trifluoropropyl)amino]imidazo[1,2-a]pyridin-3-yl}-2-methyl-N-[rel-(1S,2S)-2-methylcyclopropyl]benzamide,
4-{6-(3-fluoro-4-methoxyphenoxy)-8-[(3,3,3-trifluoropropyl)amino]imidazo[1,2-a]pyridin-3-yl}-N,2-dimethylbenzamide,
N-ethyl-4-{6-(3-fluoro-4-methoxyphenoxy)-8-[(3,3,3-trifluoropropyl)amino]imidazo[1,2-a]pyridin-3-yl}-2-methylbenzamide,
N-cyclopropyl-4-{6-[(1Z)-3-hydroxyprop-1-en-1-yl]-8-[(3,3,3-trifluoropropyl)amino]imidazo[1,2-a]pyridin-3-yl}-2-methylbenzamide,
4-{6-[4-(benzyloxy)phenoxy]-8-[(3,3,3-trifluoropropyl)amino]imidazo[1,2-a]pyridin-3-yl}-N-cyclopropyl-2-methylbenzamide,
N-cyclopropyl-4-{6-(4-hydroxyphenoxy)-8-[(3,3,3-trifluoropropyl)amino]imidazo[1,2-a]pyridin-3-yl}-2-methylbenzamide,
N-cyclopropyl-2-methyl-4-{6-[(1E)-prop-1-en-1-yl]-8-[(3,3,3-trifluoropropyl)amino]imidazo[1,2-a]pyridin-3-yl}benzamide,
N,2-dimethyl-4-{6-[(1E)-prop-1-en-1-yl]-8-[(3,3,3-trifluoropropyl)amino]imidazo[1,2-a]pyridin-3-yl}benzamide,
N-ethyl-2-methyl-4-{6-[(1E)-prop-1-en-1-yl]-8-[(3,3,3-trifluoropropyl)amino]imidazo[1,2-a]pyridin-3-yl}benzamide,
2-methyl-N-(1-methylcyclopropyl)-4-{6-[(1E)-prop-1-en-1-yl]-8-[(3,3,3-trifluoropropyl)amino]imidazo[1,2-a]pyridin-3-yl}benzamide,
N-(1-cyanocyclopropyl)-2-methyl-4-{6-[(1E)-prop-1-en-1-yl]-8-[(3,3,3-trifluoropropyl)amino]imidazo[1,2-a]pyridin-3-yl}benzamide,
N-[rel-(1S,2S)-2-fluorocyclopropyl]-2-methyl-4-{6-[(1E)-prop-1-en-1-yl]-8-[(3,3,3-trifluoropropyl)amino]imidazo[1,2-a]pyridin-3-yl}benzamide,
2-methyl-N-[rel-(1S,2S)-2-methylcyclopropyl]-4-{6-[(1E)-prop-1-en-1-yl]-8-[(3,3,3-trifluoropropyl)amino]imidazo[1,2-a]pyridin-3-yl}benzamide,
N-[rel-(1R,2R)-2-fluorocyclopropyl]-4-{6-[(1Z)-3-hydroxyprop-1-en-1-yl]-8-[(3,3,3-trifluoropropyl)amino]imidazo[1,2-a]pyridin-3-yl}-2-methylbenzamide,
4-{6-[(1Z)-3-hydroxyprop-1-en-1-yl]-8-[(3,3,3-trifluoropropyl)amino]imidazo[1,2-a]pyridin-3-yl}-N,2-dimethylbenzamide,
N-ethyl-4-{6-[(1Z)-3-hydroxyprop-1-en-1-yl]-8-[(3,3,3-trifluoropropyl)amino]imidazo[1,2-a]pyridin-3-yl}-2-methylbenzamide,
4-{6-(3-fluoro-4-methoxyphenoxy)-8-[(tetrahydro-2H-pyran-4-ylmethyl)amino]imidazo[1,2-a]pyridin-3-yl}-N,2-dimethylbenzamide,
N-ethyl-4-{6-(3-fluoro-4-methoxyphenoxy)-8-[(tetrahydro-2H-pyran-4-ylmethyl)amino]imidazo[1,2-a]pyridin-3-yl}-2-methylbenzamide,
N-cyclopropyl-4-{6-(3-fluoro-4-methoxyphenoxy)-8-[(tetrahydro-2H-pyran-4-ylmethyl)amino]imidazo[1,2-a]pyridin-3-yl}-2-methylbenzamide,
N-[rel-(1S,2S)-2-fluorocyclopropyl]-4-{6-(3-fluoro-4-methoxyphenoxy)-8-[(3,3,3-trifluoropropyl)amino]imidazo[1,2-a]pyridin-3-yl}-2-methylbenzamide,
4-{6-(3-fluoro-4-methoxyphenoxy)-8-[(3,3,3-trifluoropropyl)amino]imidazo[1,2-a]pyridin-3-yl}-2-methyl-N-[rel-(1S,2S)-2-methylcyclopropyl]benzamide,
4-{6-(3-hydroxypropyl)-8-[(3,3,3-trifluoropropyl)amino]imidazo[1,2-a]pyridin-3-yl}-N,2-dimethylbenzamide,
N-ethyl-4-{6-(3-hydroxypropyl)-8-[(3,3,3-trifluoropropyl)amino]imidazo[1,2-a]pyridin-3-yl}-2-methylbenzamide,
N-cyclopropyl-4-{6-(3-hydroxypropyl)-8-[(3,3,3-trifluoropropyl)amino]imidazo[1,2-a]pyridin-3-yl}-2-methylbenzamide,
N-cyclopropyl-4-{6-(3-fluoro-4-hydroxyphenoxy)-8-[(3,3,3-trifluoropropyl)amino]imidazo[1,2-a]pyridin-3-yl}-2-methylbenzamide,
4-{6-(3-fluoro-4-hydroxyphenoxy)-8-[(3,3,3-trifluoropropyl)amino]imidazo[1,2-a]pyridin-3-yl}-N,2-dimethylbenzamide,
N-ethyl-4-{6-(3-fluoro-4-hydroxyphenoxy)-8-[(3,3,3-trifluoropropyl)amino]imidazo[1,2-a]pyridin-3-yl}-2-methylbenzamide,
N-cyclopropyl-4-{6-(2-hydroxyphenyl)-8-[(3,3,3-trifluoropropyl)amino]imidazo[1,2-a]pyridin-3-yl}-2-methylbenzamide,
4-{6-(2-hydroxyphenyl)-8-[(3,3,3-trifluoropropyl)amino]imidazo[1,2-a]pyridin-3-yl}-N,2-dimethylbenzamide,
N-ethyl-4-{6-(2-hydroxyphenyl)-8-[(3,3,3-trifluoropropyl)amino]imidazo[1,2-a]pyridin-3-yl}-2-methylbenzamide,
N-[rel-(1S,2S)-2-fluorocyclopropyl]-4-{6-(2-hydroxyphenyl)-8-[(3,3,3-trifluoropropyl)amino]imidazo[1,2-a]pyridin-3-yl}-2-methylbenzamide,
N-cyclopropyl-4-{6-(3-hydroxyphenoxy)-8-[(3,3,3-trifluoropropyl)amino]imidazo[1,2-a]pyridin-3-yl}-2-methylbenzamide,
4-{6-(3-hydroxyphenoxy)-8-[(3,3,3-trifluoropropyl)amino]imidazo[1,2-a]pyridin-3-yl}-2-methyl-N-(1-methylcyclopropyl)benzamide,
N-ethyl-4-{6-(3-hydroxyphenoxy)-8-[(3,3,3-trifluoropropyl)amino]imidazo[1,2-a]pyridin-3-yl}-2-methylbenzamide,
4-{6-(3-hydroxyphenoxy)-8-[(3,3,3-trifluoropropyl)amino]imidazo[1,2-a]pyridin-3-yl}-N,2-dimethylbenzamide,
methyl 2-methyl-4-{3-[3-methyl-4-(methylcarbamoyl)phenyl]-8-[(3,3,3-trifluoropropyl)amino]imidazo[1,2-a]pyridin-6-yl}benzoate,
N-cyclopropyl-4-{6-(2-hydroxyethyl)-8-[(3,3,3-trifluoropropyl)amino]imidazo[1,2-a]pyridin-3-yl}-2-methylbenzamide,
4-{6-(2-hydroxyethyl)-8-[(3,3,3-trifluoropropyl)amino]imidazo[1,2-a]pyridin-3-yl}-N,2-dimethylbenzamide,
N-cyclopropyl-4-{6-[(1RS)-1-hydroxyethyl]-8-[(3,3,3-trifluoropropyl)amino]imidazo[1,2-a]pyridin-3-yl}-2-methylbenzamide,
4-{6-[(1RS)-1-hydroxyethyl]-8-[(3,3,3-trifluoropropyl)amino]imidazo[1,2-a]pyridin-3-yl}-N,2-dimethylbenzamide,
N-ethyl-4-{6-[(1RS)-1-hydroxyethyl]-8-[(3,3,3-trifluoropropyl)amino]imidazo[1,2-a]pyridin-3-yl}-2-methylbenzamide,
N-cyclopropyl-4-{6-(3-fluoro-2-methoxyphenoxy)-8-[(3,3,3-trifluoropropyl)amino]imidazo[1,2-a]pyridin-3-yl}-2-methylbenzamide,
4-{3-[4-(cyclopropylcarbamoyl)-3-methylphenyl]-8-[(3,3,3-trifluoropropyl)amino]imidazo[1,2-a]pyridin-6-yl}-N,2-dimethylbenzamide,
4-{3-[4-(cyclopropylcarbamoyl)-3-methylphenyl]-8-[(3,3,3-trifluoropropyl)amino]imidazo[1,2-a]pyridin-6-yl}-N-ethyl-2-methylbenzamide,
4-{3-[4-(cyclopropylcarbamoyl)-3-methylphenyl]-8-[(3,3,3-trifluoropropyl)amino]imidazo[1,2-a]pyridin-6-yl}-2-methyl-N-(1-methylcyclopropyl)benzamide,
4-(6-{4-[(1-cyanocyclopropyl)carbamoyl]-3-methylphenyl}-8-[(3,3,3-trifluoropropyl)amino]imidazo[1,2-a]pyridin-3-yl)-N-cyclopropyl-2-methylbenzamide,
4-{3-[4-(cyclopropylcarbamoyl)-3-methylphenyl]-8-[(3,3,3-trifluoropropyl)amino]imidazo[1,2-a]pyridin-6-yl}-N-[1-(hydroxymethyl)cyclopropyl]-2-methylbenzamide,
4-{3-[4-(cyclopropylcarbamoyl)-3-methylphenyl]-8-[(3,3,3-trifluoropropyl)amino]imidazo[1,2-a]pyridin-6-yl}-N-[rel-(1S,2S)-2-fluorocyclopropyl]-2-methylbenzamide,
4-{6-(4-carbamoyl-3-methylphenyl)-8-[(3,3,3-trifluoropropyl)amino]imidazo[1,2-a]pyridin-3-yl}-N-cyclopropyl-2-methylbenzamide,
4-{6-(4-{[rel-(1S,2S)-2-fluorocyclopropyl]carbamoyl}-3-methylphenyl)-8-[(3,3,3-trifluoropropyl)amino]imidazo[1,2-a]pyridin-3-yl}-N,2-dimethylbenzamide,
4-{6-(4-{[1-(hydroxymethyl)cyclopropyl]carbamoyl}-3-methylphenyl)-8-[(3,3,3-trifluoropropyl)amino]imidazo[1,2-a]pyridin-3-yl}-N,2-dimethylbenzamide,
4-(6-{4-[(1-cyanocyclopropyl)carbamoyl]-3-methylphenyl}-8-[(3,3,3-trifluoropropyl)amino]imidazo[1,2-a]pyridin-3-yl)-N,2-dimethylbenzamide,
2-methyl-N-(1-methylcyclopropyl)-4-{3-[3-methyl-4-(methylcarbamoyl)phenyl]-8-[(3,3,3-trifluoropropyl)amino]imidazo[1,2-a]pyridin-6-yl}benzamide,
4-{6-[4-(cyclopropylcarbamoyl)-3-methylphenyl]-8-[(3,3,3-trifluoropropyl)amino]imidazo[1,2-a]pyridin-3-yl}-N,2-dimethylbenzamide,
4-{6-[4-(ethylcarbamoyl)-3-methylphenyl]-8-[(3,3,3-trifluoropropyl)amino]imidazo[1,2-a]pyridin-3-yl}-N,2-dimethylbenzamide,
4-{6-(4-carbamoyl-3-methylphenyl)-8-[(3,3,3-trifluoropropyl)amino]imidazo[1,2-a]pyridin-3-yl}-N,2-dimethylbenzamide,
N-cyclopropyl-4-{8-[(2,2-difluoroethyl)amino]-6-(3-fluorophenoxy)imidazo[1,2-a]pyridin-3-yl}-2-methylbenzamide,
N-cyclopropyl-4-{8-[(2,2-difluoroethyl)amino]-6-(4-methoxyphenoxy)imidazo[1,2-a]pyridin-3-yl}-2-methylbenzamide,
N-cyclopropyl-4-{8-[(2,2-difluoroethyl)amino]-6-(2,3-difluorophenoxy)imidazo[1,2-a]pyridin-3-yl}-2-methylbenzamide,
N-cyclopropyl-4-{8-[(2,2-difluoroethyl)amino]-6-(5-fluoro-2-methylphenoxy)imidazo[1,2-a]pyridin-3-yl}-2-methylbenzamide,
N-cyclopropyl-4-{8-[(2,2-difluoroethyl)amino]-6-(2,5-difluorophenoxy)imidazo[1,2-a]pyridin-3-yl}-2-methylbenzamide,
N-cyclopropyl-4-{8-[(2,2-difluoroethyl)amino]-6-(3-fluoro-2-methylphenoxy)imidazo[1,2-a]pyridin-3-yl}-2-methylbenzamide,
N-cyclopropyl-4-{8-[(2,2-difluoroethyl)amino]-6-(3-fluoro-4-methoxyphenoxy)imidazo[1,2-a]pyridin-3-yl}-2-methylbenzamide,
N-cyclopropyl-4-{8-[(2,2-difluoroethyl)amino]-6-(2-hydroxybenzoyl)imidazo[1,2-a]pyridin-3-yl}-2-methylbenzamide,
N-cyclopropyl-4-{6-(3-fluorophenoxy)-8-[(2-methoxyethyl)amino]imidazo[1,2-a]pyridin-3-yl}-2-methylbenzamide,
N-cyclopropyl-4-{8-[(2-methoxyethyl)amino]-6-(2-methylphenoxy)imidazo[1,2-a]pyridin-3-yl}-2-methylbenzamide,
N-cyclopropyl-4-{6-(3,4-difluorophenoxy)-8-[(2-methoxyethyl)amino]imidazo[1,2-a]pyridin-3-yl}-2-methylbenzamide,
N-cyclopropyl-4-{6-(5-fluoro-2-methylphenoxy)-8-[(2-methoxyethyl)amino]imidazo[1,2-a]pyridin-3-yl}-2-methylbenzamide,
N-cyclopropyl-4-{6-(2,3-difluorophenoxy)-8-[(2-methoxyethyl)amino]imidazo[1,2-a]pyridin-3-yl}-2-methylbenzamide,
N-cyclopropyl-4-{6-(5-fluoro-2-hydroxybenzoyl)-8-[(2-methoxyethyl)amino]imidazo[1,2-a]pyridin-3-yl}-2-methylbenzamide,
N-cyclopropyl-4-{8-[(2,2-difluoroethyl)amino]-6-(3-fluorophenoxy)imidazo[1,2-a]pyridin-3-yl}-2-methylbenzamide,
N-cyclopropyl-4-{6-(5-fluoro-2-hydroxyphenoxy)-8-[(3,3,3-trifluoropropyl)amino]imidazo[1,2-a]pyridin-3-yl}-2-methylbenzamide,
N-cyclopropyl-4-{6-(3-fluoro-2-hydroxyphenoxy)-8-[(3,3,3-trifluoropropyl)amino]imidazo[1,2-a]pyridin-3-yl}-2-methylbenzamide,
N-cyclopropyl-4-{6-[(5-fluoropyridin-3-yl)oxy]-8-[(3,3,3-trifluoropropyl)amino]imidazo[1,2-a]pyridin-3-yl}-2-methylbenzamide,
N-cyclopropyl-4-{6-[(5-fluoro-6-methoxypyridin-3-yl)oxy]-8-[(3,3,3-trifluoropropyl)amino]imidazo[1,2-a]pyridin-3-yl}-2-methylbenzamide,
or a stereoisomer, a tautomer, an N-oxide, a hydrate, a solvate, or a salt thereof, or a mixture of same.

4. A method of preparing a compound according to any one of claims 1 to 3, said method comprising the step :
- allowing an intermediate compound of general formula **IV** : 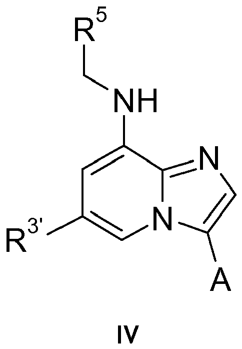 in which
R⁵ and A are as defined for general formula **I** in any one of claims 1 to **3**; and R^{3'} is a halogen atom ;
to react with a compound of general formula **IVa** :
R³-Y **IVa**
in which
R³ is as defined for general formula **I** in any one of claims 1 to 3;
Y is a substituent which is displaced in a coupling reaction ;
thereby giving a compound of general formula I : 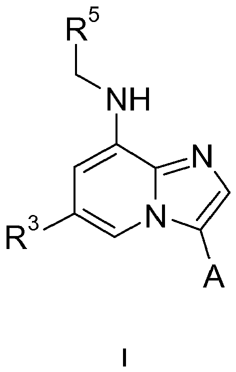 in which
R³, R⁵, and A are as defined for general formula **I** in any one of claims 1 to 3.

5. A method of preparing a compound according to any one of claims 1 to 3, said method comprising the step :
- allowing an intermediate compound of general formula **II** : 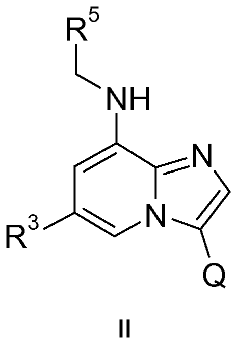 in which
R³ and R⁵ are as defined for general formula **I** in any one of claims 1 to 3; and Q is a halogen atom ;
to react with a compound of general formula **IIa** :
A-Y **IIa**
in which
A is as defined for general formula **I** in any one of claims 1 to 3;
Y is a substituent which is displaced in a coupling reaction ;
thereby giving a compound of general formula I : 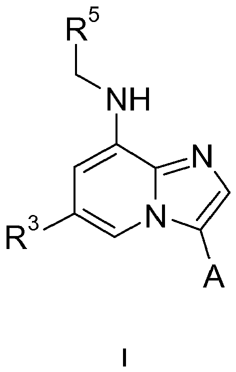
in which R³, R⁵ and A are as defined for general formula **I** in any one of claims 1 to 3.

6. A method of preparing a compound according to any one of claims 1 to 3, said method comprising the step :
- allowing an intermediate compound of general formula VII : 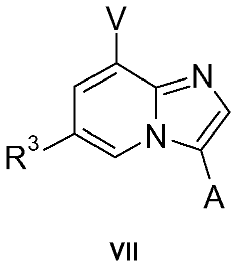 in which
R³ and A are as defined for general formula **I** in any one of claims 1 to 3; and V is a leaving group;
to react with a compound of general formula IIa :
R⁵-CH₂-NH₂ **VIIa**
in which
R⁵ is as defined for general formula **I** in any one of claims 1 to 3;
thereby giving a compound of general formula I : 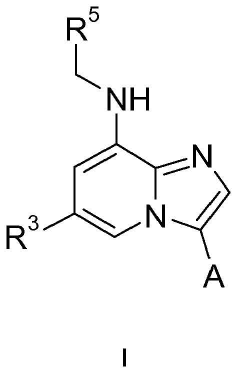
in which R³, R⁵ and A are as defined for general formula **I** in any one of claims 1 to 3.

7. A method of preparing a compound according to any one of claims 1 to 3, said method comprising the step :
- allowing an intermediate compound of general formula VII : 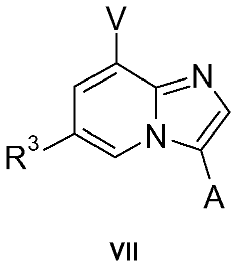 in which
R³ and A are as defined for general formula **I** in any one of claims 1 to 3; and V is a NH₂-group;
to react with a compound of general formula VIIb :
O=CHR⁵ **VIIb**
in which
R⁵ is as defined for general formula **I** in any one of claims 1 to 3;
thereby giving a compound of general formula I : 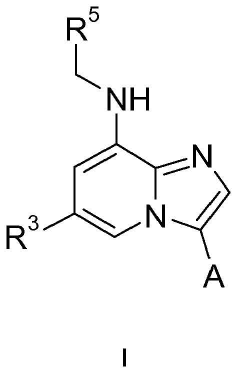
in which R³, R⁵ and A are as defined for general formula **I** in any one of claims 1 to 3.

8. A compound according to any one of the claims 1 to 3, or a stereoisomer, a tautomer, an N-oxide, a hydrate, a solvate, or a salt thereof, particularly a pharmaceutically acceptable salt thereof, or a mixture of same, for use in the treatment or prophylaxis of a disease.

9. A compound for use according to claim 8, wherein said disease is a disease of uncontrolled cell growth, proliferation and/or survival, an inappropriate cellular immune response, or an inappropriate cellular inflammatory response, in which the disease of uncontrolled cell growth, proliferation and/or survival, inappropriate cellular immune response, or inappropriate cellular inflammatory response is a haemotological tumour, a solid tumour and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

10. A pharmaceutical composition comprising a compound according to any one of the claims 1 to 3, or a stereoisomer, a tautomer, an N-oxide, a hydrate, a solvate, or a salt thereof, particularly a pharmaceutically acceptable salt thereof, or a mixture of same, and a pharmaceutically acceptable diluent or carrier.

11. A pharmaceutical composition comprising :
- one or more compounds according to any one of the claims 1 to 3, or a stereoisomer, a tautomer, an N-oxide, a hydrate, a solvate, or a salt thereof, particularly a pharmaceutically acceptable salt thereof, or a mixture of same; and
- one or more agents selected from : a taxane, such as Docetaxel, Paclitaxel, or Taxol; an epothilone, such as Ixabepilone, Patupilone, or Sagopilone; Mitoxantrone; Predinisolone; Dexamethasone; Estramustin; Vinblastin; Vincristin; Doxorubicin; Adriamycin; Idarubicin; Daunorubicin; Bleomycin; Etoposide; Cyclophosphamide; Ifosfamide; Procarbazine; Melphalan; 5-Fluorouracil; Capecitabine; Fludarabine; Cytarabine; Ara-C; 2-Chloro-2'-deoxyadenosine; Thioguanine; an anti-androgen, such as Flutamide, Cyproterone acetate, or Bicalutamide; Bortezomib; a platinum derivative, such as Cisplatin, or Carboplatin; Chlorambucil; Methotrexate; and Rituximab.

12. Use of a compound according to any one of the claims 1 to 3, or a stereoisomer, a tautomer, an N-oxide, a hydrate, a solvate, or a salt thereof, particularly a pharmaceutically acceptable salt thereof, or a mixture of same, for the preparation of a medicament for the prophylaxis or treatment of a disease.

13. Use according to claim 12, wherein said disease is a disease of uncontrolled cell growth, proliferation and/or survival, an inappropriate cellular immune response, or an inappropriate cellular inflammatory response, in which the disease of uncontrolled cell growth, proliferation and/or survival, inappropriate cellular immune response, or inappropriate cellular inflammatory response is a haemotological tumour, a solid tumour and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

14. A compound of general formula IV : 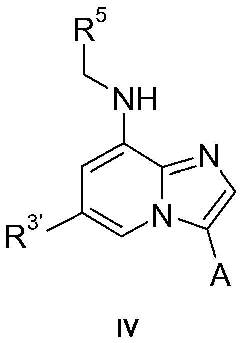 in which R⁵ and A are as defined for general formula **I** in any one of claims 1 to 3, and R^{3'} is a halogen atom.

15. A compound of general formula VII : 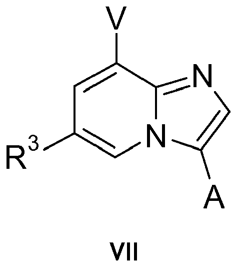 in which R³ and A are as defined for general formula **I** in any one of claims 1 to 3 and V is a NH₂-group or a halogen atom.

## Patentansprüche

1. Verbindung der allgemeinen Formel I: 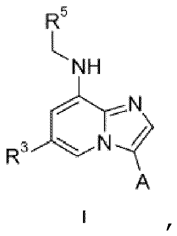 in welcher:
A für eine 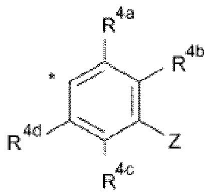 -Gruppe steht;
wobei * die Stelle der Anbindung der Gruppen an den Rest des Moleküls kennzeichnet;
Z für eine -C(=O)N(H)R¹- oder -C(=S)N(H)R¹-Gruppe steht;
R¹ für eine C₁-C₃-Alkyl- oder eine Cyclopropylgruppe steht;
wobei die Cyclopropylgruppe gegebenenfalls durch 1, 2 oder 3 gleiche oder verschiedene, aus Halogen, -OH, -CN und C₁-C₃-Alkyl- ausge-wählte Gruppen substituiert ist;
wobei die C₁-C₃-Alkylgruppe gegebenenfalls durch 1, 2 oder 3 gleiche oder verschiedene, aus Halogen, -OH, -CN, C₁-C₃-Alkoxy- ausgewählte Gruppen substituiert ist;
R³ für ein Wasserstoffatom oder eine -CN-, C₁-C₆-Alkyl-, - (CH₂)ₘ-C₂-C₆-Alkenyl-, - (CH₂)ₘ-C₂-C₆-Alkinyl-, Aryl-, Heteroaryl- oder Aryl-X-Gruppe steht;
wobei die C₁-C₆-Alkyl-, -(CH₂)ₘ-C₂-C₆-Alkenyl-, - (CH₂)ₘ-C₂-C₆-Alkinyl-, Aryl-, Aryl-X- bzw. Heteroarylgruppe gegebenenfalls durch 1, 2 oder 3 gleiche oder verschiedene R⁷-Gruppen substituiert ist;
R^{4a}, R^{4b}, R^{4d} für ein Wasserstoffatom stehen;
R^{4c} für ein Halogenatom oder eine -CN-, -OH-, C₁-C₃-Alkyl-, C₁-C₃-Alkoxy-, Halogen-C₁-C₃-alkyl-, Halogen-C₁-C₃-alkoxy-, R^{6a}(R^{6b}) N-C₁-C₃-Alkyl-, HO-C₁-C₃-Alkyl-, NC-C₁-C₃-Alkyl-, C₁-C₃-Alkoxy-C₁-C₃-alkyl- oder Halogen-C₁-C₃-alkoxy-C₁-C₃-alkylgruppe steht;
R⁵ für ein Wasserstoffatom oder eine C₁-C₆-Alkyl-, - (CH₂)ₙ-C₂-C₆-Alkenyl-, -(CH₂)ₙ-C₂-C₆-Alkinyl-, - (CH₂)ₘ-C₃-C₆-Cycloalkyl-, - (CH₂)ₘ-(3- bis 7-gliedriges Heterocycloalkyl)-, Aryl-C₁-C₆-alkyl-, Heteroaryl-C₁-C₆-alkyl-, Halogen-C₁-C₆-alkyl-, R^{6a}(R^{6b})N-C₁-C₆-Alkyl-, HO-C₁-C₆-Alkyl-, -C₁-C₆-Alkyl-CN-, C₁-C₆-Alkoxy-C₁-C₆-alkyl-, Halogen-C₁-C₆-alkoxy-C₁-C₆-alkyl-, C₃-C₆-Cyclo-alkyl-, 3- bis 7-gliedrige Heterocycloalkyl-, C₄-C₈-Cycloalkenyl-, Aryl- oder Heteroarylgruppe steht;
wobei die C₁-C₆-Alkyl-, -(CH₂)ₙ-C₂-C₆-Alkenyl-, -(CH₂)ₙ-C₂-C₆-Alkinyl-, -(CH₂)ₘ-C₃-C₆-Cycloalkyl-, -(CH₂)ₘ-(3- bis 7-gliedriges Heterocycloalkyl)-, Aryl-C₁-C₆-alkyl-, Heteroaryl-C₁-C₆-alkyl-, Halogen-C₁-C₆-alkyl-, R^{6a}(R^{6b})N-C₁-C₆-Alkyl-, HO-C₁-C₆-Alkyl-, -C₁-C₆-Alkyl-CN-, C₁-C₆-Alkoxy-C₁-C₆-alkyl-, Halogen-C₁-C₆-alkoxy-C₁-C₆-alkyl-, C₃-C₆-Cycloalkyl-, 3- bis 7-gliedrige Hetero-cycloalkyl-, C₄-C₈-Cycloalkenyl-, Aryl- bzw. Heteroarylgruppe gegebenenfalls durch 1, 2, 3, 4 oder 5 gleiche oder verschiedene R⁸-Gruppen substituiert ist;
R⁶, R^{6a}, R^{6b}, R^{6c} unabhängig voneinander für ein Wasserstoffatom oder eine C₁-C₆-Alkyl-, C₃-C₆-Cycloalkyl- oder Aryl-C₁-C₆-alkylgruppe stehen; wobei die C₃-C₆-Cycloalkylgruppe gegebenenfalls durch 1 oder 2 gleiche oder verschiedene, aus Halogen, -OH, -CN, C₁-C₆-Alkyl- und HO-C₁-C₆-Alkyl- ausgewählte Gruppen substituiert ist;
R⁷ für ein Halogenatom oder eine HO-, C₁-C₆-Alkoxy-, C₁-C₆-Alkyl-, Halogen-C₁-C₆-alkyl-, Halogen-C₁-C₆-alkoxy-, HO-C₁-C₆-Alkyl-, -C(=O)N(H)R^{6a}-, -N(R^{6a})R^{6b}-, -O(C=O)OR⁶- oder -OR⁶-Gruppe steht;
R⁸ für ein Wasserstoff- oder Halogenatom oder eine -CN-, C₁-C₆-Alkoxy-, C₁-C₆-Alkyl-, Halogen-C₁-C₆-alkyl-, R^{6a} (R^{6b})N-C₁-C₆-Alkyl-, HO-C₁-C₆-Alkyl-, C₁-C₆-Alkoxy-C₁-C₆-alkyl-, Halogen-C₁-C₆-alkoxy-C₁-C₆-alkyl-, C₂-C₆-Alkenyl-, C₂-C₆-Alkinyl-, 3-bis 7-gliedrige Heterocycloalkyl-, Aryl-, Heteroaryl-, -C(=O)R⁶-, -C(=O)N(H)R^{6a}-, -C(=O)N(R^{6a})R^{6b}-, -C(=O)O-R⁶-, -N(R^{6a})R^{6b}-, -NO₂-, -N(H) C(=O)R⁶-, -N(R^{6c})C (=O)R⁶-, -N(H) C (=O)N(R^{6a})R^{6b}-, -N(R^{6c})C (=O)N(R^{6a})R^{6b}-, -N (H) C (=O)OR⁶-, -N (R^{6c})C (=O)OR⁶-, -N (H) S (=O)R⁶-, -N(R^{6c})S (=O)R⁶-, -N(H) S (=O)₂R⁶-, -N(R^{6c})S (=O)₂R⁶-, -N=S(=O)(R^{6a})R^{6b}-, -OR⁶-, -O(C=O)R⁶-, -O(C=O)N(R^{6a})R^{6b}-, -O(C=O)OR⁶-, -SR⁶-, -S(=O)R⁶-, -S(=O)N(H)R⁶-, -S(=O)N(R^{6a})R^{6b}-, -S(=O₂)R⁶-, -S(=O)₂N(H)R⁶-, -S(=O)₂N(R^{6a})R^{6b}-, -S(=O)(=NR^{6c})R⁶-oder -S(=O)₂-(3- bis 7-gliedriges Heterocyclo-alkyl)gruppe steht; wobei die 3- bis 7-gliedrige Heterocycloalkyl-bzw. Heteroarylgruppe gegebenenfalls durch 1, 2, 3 oder 4 gleiche oder verschiedene C₁-C₆-Alkylgruppen substituiert ist;
m für eine ganze Zahl 0, 1, 2, 3, 4, 5 oder 6 steht;
n für eine ganze Zahl 0, 1, 2, 3, 4 oder 5 steht; und
X für S, S(=O), S(=O)₂, O, NR⁶, C(=O) oder CR^{6a}R^{6b} steht;
oder ein Stereoisomer, ein Tautomer, ein N-Oxid, ein Hydrat, ein Solvat oder ein Salz davon, oder eine Mischung dieser.

2. Verbindung nach Anspruch 1, wobei:
A für 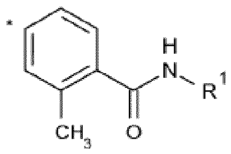 steht;
wobei * die Stelle der Anbindung der Gruppe an den Rest des Moleküls kennzeichnet;
R¹ für eine Methylgruppe oder eine Cyclopropylgruppe steht;
wobei die Cyclopropylgruppe gegebenenfalls durch ein Fluoratom substituiert ist;
R³ für eine Pyridyl-, Phenyl-, Phenyl-O- oder Phenyl-S-Gruppe steht;
wobei die Phenylgruppe entweder durch eine Methylgruppe und eine -C(=O)N(H)R^{6a}-Gruppe oder durch eine HO-CH₂-Gruppe substituiert ist;
wobei die Phenyl-O-Gruppe gegebenenfalls durch 1 oder 2 gleiche oder verschiedene, aus -F, H₃C-O-, HO- und H₃C- ausgewählte Gruppen substituiert ist;
wobei die Phenyl-S-Gruppe gegebenenfalls durch 1 oder 2 gleiche oder verschiedene, aus -F, H₃C-O-, HO- und H₃C- ausgewählte Gruppen substituiert ist;
R⁵ für eine Gruppe ausgewählt aus 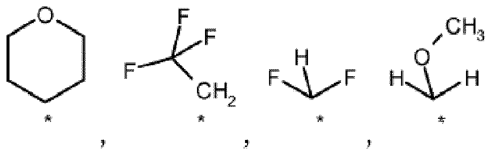 steht; wobei *
die Stelle der Anbindung der Gruppen an den Rest des Moleküls kennzeichnet;
R^{6a} für ein Wasserstoffatom oder eine Methylgruppe oder eine C₃-C₆-Cyclopropylgruppe steht, wobei die C₃-C₆-Cyclopropylgruppe gegebenenfalls durch eine -CN-Gruppe substituiert ist;
oder ein Stereoisomer, ein Tautomer, ein N-Oxid, ein Hydrat, ein Solvat oder ein Salz davon, oder eine Mischung dieser.

3. Verbindung nach Anspruch 1, ausgewählt aus der Gruppe bestehend aus:
N-Cyclopropyl-4-{8-[(2-methylpropyl)amino]imidazo-[1,2-a]pyridin-3-yl}benzamid,
N-Cyclopropyl-4-{6-[2-(hydroxymethyl)phenyl]-8-[(3,3,3-trifluorpropyl)amino]imidazo[1,2-a]-pyridin-3-yl}-2-methylbenzamid,
N-Cyclopropyl-2-methyl-4-{6-(pyridin-4-yl)-8-[3,3,3-trifluorpropyl)amino]imidazo[1,2-a]pyridin-3-yl}benzamid,
N-Cyclopropyl-4-{6-[(3-fluor-5-methylphenyl)-sulfanyl]-8-[(3,3,3-trifluorpropyl)amino]imidazo-[1,2-a]pyridin-3-yl}-2-methylbenzamid,
N-Cyclopropyl-4-{6-[(2,3-difluorphenyl)sulfanyl]-8-[(3,3,3-trifluorpropyl)amino]imidazo[1,2-a]-pyridin-3-yl}-2-methylbenzamid,
N-Cyclopropyl-2-methyl-4-{6-(phenylsulfanyl)-8-[(3,3,3-trifluorpropyl)amino]imidazo[1,2-a]pyridin-3-yl}benzamid,
4-{6-[(2-Aminophenyl)sulfanyl]-8-[(3,3,3-trifluorpropyl)amino]imidazo[1,2-a]pyridin-3-yl}-N-cyclo-propyl-2-methylbenzamid,
N-cyclopropyl-4-{6-[(3-fluorphenyl)sulfanyl]-8-[(3,3,3-trifluorpropyl)amino]imidazo[1,2-a]-pyridin-3-yl}-2-methylbenzamid,
N-Cyclopropyl-4-{6-[(2-hydroxyphenyl)sulfanyl]-8-[(3,3,3-trifluorpropyl)amino]imidazo[1,2-a]-pyridin-3-yl}-2-methylbenzamid,
N-Cyclopropyl-2-methyl-4-{6-(methylsulfonyl)-8-[(3,3,3-trifluorpropyl)amino]imidazo[1,2-a]-pyridin-3-yl}benzamid,
N-Cyclopropyl-4-{6-(2-fluor-4-methoxyphenoxy)-8-[(3,3,3-trifluorpropyl)amino]imidazo[1,2-a]-pyridin-3-yl}-2-methylbenzamid,
N-Cyclopropyl-4-{6-(3-fluorphenoxy)-8-[(3,3,3-trifluorpropyl)amino]imidazo[1,2-a]pyridin-3-yl}-2-methylbenzamid,
4-{6-(3-Fluorphenoxy)-8-[(3,3,3-trifluorpropyl)-amino]imidazo[1,2-a]pyridin-3-yl}-N,2-dimethyl-benzamid,
4,4'-{8-[(Tetrahydro-2H-pyran-4-ylmethyl)amino]-imidazo[1,2-a]pyridin-3,6-diyl}bis[N-cyclopropyl-2-methylbenzamid),
N-Ethyl-4-{6-(3-fluorphenoxy)-8-[(3,3,3-trifluor-propyl)amino]imidazo[1,2-a]pyridin-3-yl}-2-methyl-benzamid,
4-{6-(3-Fluorphenoxy)-8-[(3,3,3-trifluorpropyl)-amino]imidazo[1,2-a]pyridin-3-yl}-2-methyl-N-(1-methylcyclopropyl)benzamid,
N-[rel-(1S,2S)-2-Fluorcyclopropyl]-4-{6-(3-fluorphenoxy)-8-[(3,3,3-trifluorpropyl)amino]-imidazo[1,2-a]pyridin-3-yl}-2-methylbenzamid,
N-{[(1R,2R)- oder -(1S,2S)]-2-Fluorcyclopropyl}-4-{6-(3-fluorphenoxy)-8-[(3,3,3-trifluorpropyl)-amino]imidazo[1,2-a]pyridin-3-yl}-2-methyl-benzamid,
N-{[(1S,2S)- oder -(1R,2R)]-2-Fluorcyclopropyl}-4-{6-(3-fluorphenoxy)-8-[(3,3,3-trifluorpropyl)-amino]imidazo[1,2-a]pyridin-3-yl}-2-methyl-benzamid,
N-(1-Cyanocyclopropyl)-4-{6-(3-fluorphenoxy)-8-[(3,3,3-trifluorpropyl)amino]imidazo[1,2-a]-pyridin-3-yl}-2-methylbenzamid,
4-{6-Ethinyl-8-[(3,3,3-trifluorpropyl)amino]-imidazo[1,2-a]pyridin-3-yl}-N,2-dimethylbenzamid,
N-Ethyl-4-{6-ethinyl-8-[(3,3,3-trifluorpropyl)-amino]imidazo[1,2-a]pyridin-3-yl}-2-methyl-benzamid,
4-{6-Ethinyl-8-[(3,3,3-trifluorpropyl)amino]-imidazo[1,2-a]pyridin-3-yl}-N-[rel-(1R,2R)-2-fluorcyclopropyl]-2-methylbenzamid,
4-{6-Ethinyl-8-[(3,3,3-trifluorpropyl)amino]-imidazo[1,2-a]pyridin-3-yl}-2-methyl-N-(1-methyl-cyclopropyl)benzamid,
N-(1-Cyanocyclopropyl)-4-{6-ethinyl-8-[(3,3,3-trifluorpropyl)amino]imidazo[1,2-a]pyridin-3-yl}-2-methylbenzamid,
N-Cyclopropyl-4-{6-(5-fluor-2-methylphenoxy)-8-[(3,3,3-trifluorpropyl)amino]imidazo[1,2-a]-pyridin-3-yl}-2-methylbenzamid,
4-{6-(3-Hydroxyprop-1-in-1-yl)-8-[(3,3,3-trifluorpropyl)amino]imidazo[1,2-a]pyridin-3-yl}-N,2-dimethylbenzamid,
N-Ethyl-4-{6-(3-hydroxyprop-1-in-1-yl)-8-[(3,3,3-trifluorpropyl)amino]imidazo[1,2-a]pyridin-3-yl}-2-methylbenzamid,
4-{6-(3-Hydroxyprop-1-in-1-yl)-8-[(3,3,3-trifluorpropyl)amino]imidazo[1,2-a]pyridin-3-yl}-2-methyl-N-(1-methylcyclopropyl)benzamid,
N-(1-Cyanocyclopropyl)-4-{6-(3-hydroxyprop-1-in-1-yl)-8-[3,3,3-trifluorpropyl)amino]imidazo[1,2-a]-pyridin-3-yl}-2-methylbenzamid,
N-Cyclobutyl-4-{6-(3-fluorphenoxy)-8-[(3,3,3-trifluorpropyl)amino]imidazo[1,2-a]pyridin-3-yl}-2-methylbenzamid,
4-{6-(5-Fluor-2-methylphenoxy)-8-[(3,3,3-trifluorpropyl)amino]imidazo[1,2-a]pyridin-3-yl}-N,2-dimethylbenzamid,
N-Ethyl-4-[6-(5-fluor-2-methylphenoxy)-8-[(3,3,3-trifluorpropyl)amino]imidazo[1,2-a]pyridin-3-yl}-2-methylbenzamid,
4-{6-(5-Fluor-2-methylphenoxy)-8-[(3,3,3-trifluorpropyl)amino]imidazo[1,2-a]pyridin-3-yl}-2-methyl-N-(1-methylcyclopropyl)benzamid,
N-(1-Cyanocyclopropyl)-4-{6-(5-fluor-2-methyl-phenoxy)-8-[3,3,3-trifluorpropyl)amino]imidazo-[1,2-a]pyridin-3-yl}-2-methylbenzamid,
N-[rel-(1S,2S)-2-Fluorcyclopropyl]-4-{6-(5-fluor-2-methylphenoxy)-8-[(3,3,3-trifluorpropyl)amino]-imidazo[1,2-a]pyridin-3-yl}-2-methylbenzamid,
N-Cyclopropyl-4-{6-(2,3-difluorphenoxy)-8-[(3,3,3-trifluorpropyl)amino]imidazo[1,2-a]pyridin-3-yl}-2-methylbenzamid,
4-{6-(3-Chlorphenoxy)-8-[(3,3,3-trifluorpropyl)-amino]imidazo[1,2-a]pyridin-3-yl}-N-cyclopropyl-2-methylbenzamid,
4,4'-{8-[(3,3,3-Trifluorpropyl)amino]imidazo[1,2-a]pyridin-3,6-diyl}bis(N-cyclopropyl-2-methyl-benzamid),
N-Cyclopropyl-2-methyl-4-{6-phenoxy-8-[(3,3,3-trifluorpropyl)amino]imidazo[1,2-a]pyridin-3-yl}benzamid,
4-{6-(3-Chlorphenoxy)-8-[(3,3,3-trifluorpropyl)-amino]imidazo[1,2-a]pyridin-3-yl}-N,2-dimethyl-benzamid,
4-{6-(3-Chlorphenoxy)-8-[(3,3,3-trifluorpropyl)-amino]imidazo[1,2-a]pyridin-3-yl}-N-ethyl-2-methylbenzamid,
4-{6-(3-Chlorphenoxy)-8-[(3,3,3-trifluorpropyl)-amino]imidazo[1,2-a]pyridin-3-yl}-2-methyl-N-(1-methylcyclopropyl)benzamid,
4-{6-(3-Chlorphenoxy)-8-[(3,3,3-trifluorpropyl)-amino]imidazo[1,2-a]pyridin-3-yl}-N-(1-cyanocyclo-propyl)-2-methylbenzamid,
4-{6-(3-Fluorphenoxy)-8-[(3,3,3-trifluorpropyl)-amino]imidazo[1,2-a]pyridin-3-yl}-2-methyl-N-(1-methylcyclobutyl)benzamid,
4-{6-(3-Chlorphenoxy)-8-[(3,3,3-trifluorpropyl)-amino]imidazo[1,2-a]pyridin-3-yl}-N-[rel-(1R,2R)-2-fluorcyclopropyl]-2-methylbenzamid,
4-{6-(3-Chlorphenoxy)-8-[(3,3,3-trifluorpropyl)-amino]imidazo[1,2-a]pyridin-3-yl}-N-{[(1S,2S)-oder -(1R,2R)]-2-fluorcyclopropyl}-2-methylbenzamid,
4-{6-(3-Chlorphenoxy)-8-[(3,3,3-trifluorpropyl)-amino]imidazo[1,2-a]pyridin-3-yl}-N-{[(1R,2R)-oder -(1S,2S)]-2-fluorcyclopropyl}-2-methylbenzamid,
N-(2,6-Diethylphenyl)-4-{6-(3-fluorphenoxy)-8-[(3,3,3-trifluorpropyl)amino]imidazo[1,2-a]-pyridin-3-yl}-2-methylbenzamid,
4,4'-{8-[Methyl(3,3,3-trifluorpropyl)amino]-imidazo[1,2-a]pyridin-3,6-diyl}bis(N,2-dimethylbenzamid),
N-Cyclopropyl-4-{6-(3-fluor-4-methoxyphenoxy)-8-[(3,3,3-trifluorpropyl)amino]imidazo[1,2-a]-pyridin-3-yl}-2-methylbenzamid,
4,4'-{8-[Methyl(3,3,3-trifluorpropyl)amino]-imidazo[1,2-a]pyridin-3,6-diyl}bis[N-(1-cyanocyclopropyl)-2-methylbenzamid],
4,4'-{8-[Methyl(3,3,3-trifluorpropyl)amino]-imidazo[1,2-a]pyridin-3,6-diyl}bis[2-methyl-N-[1-methylcyclopropyl)benzamid],
4,4'-{8-[Methyl(3,3,3-trifluorpropyl)amino]-imidazo[1,2-a]pyridin-3,6-diyl}bis(N-ethyl-2-methylbenzamid),
N-(2,6-Diethylphenyl)-4-{6-(5-fluor-2-methyl-phenoxy)-8-[(3,3,3-trifluorpropyl)amino]imidazo-[1,2-a]pyridin-3-yl}-2-methylbenzamid,
N-Cyclobutyl-4-{6-(5-fluor-2-methylphenoxy)-8-[(3,3,3-trifluorpropyl)amino]imidazo[1,2-a]-pyridin-3-yl}-2-methylbenzamid,
rel-4-{6-(3-Fluorphenoxy)-8-[(3,3,3-trifluorpropyl)amino]imidazo[1,2-a]pyridin-3-yl}-2-methyl-N-[(1R,2R)-2-methylcyclopropyl]benzamid,
N-[1,1'-Bi(cyclopropyl)-1-yl]-4-{6-(3-fluorphenoxy)-8-[(3,3,3-trifluorpropyl)amino]imidazo-[1,2-a]pyridin-3-yl}-2-methylbenzamid,
4-{6-(3-Fluorphenoxy)-8-[(3,3,3-trifluorpropyl)-amino]imidazo[1,2-a]pyridin-3-yl}-N-[1-(hydroxymethyl)cyclopropyl]-2-methylbenzamid,
N-(1-Cyanocyclobutyl)-4-{6-(3-fluorphenoxy)-8-[(3,3,3-trifluorpropyl)amino]imidazo[1,2-a]pyridin-3-yl}-2-methylbenzamid,
N-Cyclopropyl-4-{6-(3-methoxyphenoxy)-8-[(3,3,3-trifluorpropyl)amino]imidazo[1,2-a]pyridin-3-yl}-2-methylbenzamid,
N-(1-Cyanocyclopropyl)-4-{6-(3-methoxyphenoxy)-8-[(3,3,3-trifluorpropyl)amino]imidazo[1,2-a]pyridin-3-yl}-2-methylbenzamid,
4-{6-(3-Methoxyphenoxy)-8-[(3,3,3-trifluorpropyl)-amino]imidazo[1,2-a]pyridin-3-yl}-2-methyl-N-(1-methylcyclopropyl)benzamid,
4-{6-(3-Methoxyphenoxy)-8-[(3,3,3-trifluorpropyl)-amino]imidazo[1,2-a]pyridin-3-yl}-N,2-dimethyl-benzamid,
N-Ethyl-4-{6-(3-methoxyphenoxy)-8-[(3,3,3-trifluorpropyl)amino]imidazo[1,2-a]pyridin-3-yl}-2-methylbenzamid,
N-(1-Cyanocyclopropyl)-2-methyl-4-{6-phenoxy-8-[(3,3,3-trifluorpropyl)amino]imidazo[1,2-a]-pyridin-3-yl}benzamid,
2-Methyl-N-(1-methylcyclopropyl)-4-{6-phenoxy-8-[(3,3,3-trifluorpropyl)amino]imidazo[1,2-a]-pyridin-3-yl}benzamid,
N,2-Dimethyl-4-{6-phenoxy-8-[(3,3,3-trifluorpropyl)amino]imidazo[1,2-a]pyridin-3-yl}benzamid,
N-Ethyl-2-methyl-4-{6-phenoxy-8-[(3,3,3-trifluorpropyl)amino]imidazo[1,2-a]pyridin-3-yl}benzamid,
rel-4-{6-(5-Fluor-2-methylphenoxy)-8-[(3,3,3-trifluorpropyl)amino]imidazo[1,2-a]pyridin-3-yl}-2-methyl-N-[(1R,2R)-2-methylcyclopropyl]benzamid,
N-[1,1'-Bi[cyclopropyl)-1-yl]-4-{6-(5-fluor-2-methylphenoxy)-8-[(3,3,3-trifluorpropyl)amino]-imidazo[1,2-a]pyridin-3-yl}-2-methylbenzamid,
N-(1-Cyanocyclobutyl)-4-{6-(5-fluor-2-methyl-phenoxy)-8-[(3,3,3-trifluorpropyl)amino]imidazo-[1,2-a]pyridin-3-yl}-2-methylbenzamid,
N-Cyclopropyl-2-methyl-4-{6-[4-(trifluormethoxy)-phenoxy]-8-[(3,3,3-trifluorpropyl)amino]imidazo-[1,2-a]pyridin-3-yl}benzamid,
N-(1-Cyanocyclopropyl)-2-methyl-4-{6-[4-trifluormethoxy)phenoxy]-8-[(3,3,3-trifluorpropyl)amino]-imidazo[1,2-a]pyridin-3-yl}benzamid,
2-Methyl-N-(1-methylcyclopropyl)-4-{6-[4-(trifluormethoxy)phenoxy]-8-[(3,3,3-trifluorpropyl)amino]imidazo[1,2-a]pyridin-3-yl}benzamid,
N,2-Dimethyl-4-{6-[4-(trifluormethoxy)phenoxy]-8-[(3,3,3-trifluorpropyl)amino]imidazo[1,2-a]-pyridin-3-yl}benzamid,
N-Ethyl-2-methyl-4-{6-[4-(trifluormethoxy)-phenoxy]-8-[(3,3,3-trifluorpropyl)amino]imidazo-[1,2-a]pyridin-3-yl}benzamid,
N-Cyclopropyl-4-{6-(2-fluor-4-methoxyphenoxy)-8-[(3,3,3-trifluorpropyl)amino]imidazo[1,2-a]-pyridin-3-yl}-2-methylbenzamid,
N-(1-Cyanocyclopropyl)-4-{6-(2,3-difluorphenoxy)-8-[(3,3,3-trifluorpropyl)amino]imidazo[1,2-a]-pyridin-3-yl}-2-methylbenzamid,
4-{6-(2,3-Difluorphenoxy)-8-[(3,3,3-trifluorpropyl)amino]imidazo[1,2-a]pyridin-3-yl}-2-methyl-N-(1-methylcyclopropyl)benzamid,
4-{6-(2,3-Difluorphenoxy)-8-[(3,3,3-trifluorpropyl)amino]imidazo[1,2-a]pyridin-3-yl}-N,2-dimethylbenzamid,
4-{6-(2,3-Difluorphenoxy)-8-[(3,3,3-trifluorpropyl)amino]imidazo[1,2-a]pyridin-3-yl}-N-ethyl-2-methylbenzamid,
N-Cyclopropyl-2-methyl-4-{6-[3-(trifluormethoxy)-phenoxy]-8-[(3,3,3-trifluorpropyl)amino]imidazo-[1,2-a]pyridin-3-yl}benzamid,
N-(1-Cyanocyclopropyl)-2-methyl-4-{6-[3-(trifluormethoxy)phenoxy]-8-[(3,3,3-trifluorpropyl)amino]-imidazo[1,2-a]pyridin-3-yl}benzamid,
2-Methyl-N-(1-methylcyclopropyl)-4-{6-[3-(trifluormethoxy)phenoxy]-8-[(3,3,3-trifluorpropyl)amino]imidazo[1,2-a]pyridin-3-yl}benzamid,
N-Ethyl-2-methyl-4-{6-[3-(trifluormethoxy)-phenoxy]-8-[(3,3,3-trifluorpropyl)amino]imidazo-[1,2-a]pyridin-3-yl}benzamid,
N,2-Dimethyl-4-{6-[3-(trifluormethoxy)phenoxy]-8-[(3,3,3-trifluorpropyl)amino]imidazo[1,2-a]-pyridin-3-yl}benzamid,
N-Cyclopropyl-4-{6-(2-methoxyphenyl)-8-[(3,3,3-trifluorpropyl)amino]imidazo[1,2-a]pyridin-3-yl}-2-methylbenzamid,
4-{6-(2-Methoxyphenyl)-8-[(3,3,3-trifluorpropyl)-amino]imidazo[1,2-a]pyridin-3-yl}-2-methyl-N-(1-methylcyclopropyl)benzamid,
4-{6-(2-Methoxyphenyl)-8-[(3,3,3-trifluorpropyl)-amino]imidazo[1,2-a]pyridin-3-yl}-N,2-dimethyl-benzamid,
N-Ethyl-4-{6-(2-methoxyphenyl)-8-[(3,3,3-trifluorpropyl)amino]imidazo[1,2-a]pyridin-3-yl}-2-methyl-benzamid,
N-Cyclopropyl-4-{6-[3-fluor-4-(trifluormethoxy)-phenoxy]-8-[(3,3,3-trifluorpropyl)amino]imidazo-[1,2-a]pyridin-3-yl}-2-methylbenzamid,
N-(1-Cyanocyclopropyl)-4-{6-[3-fluor-4-(trifluormethoxy)phenoxy]-8-[(3,3,3-trifluorpropyl)amino]-imidazo[1,2-a]pyridin-3-yl}-2-methylbenzamid,
4-{6-[3-Fluor-4-(trifluormethoxy)phenoxy]-8-[(3,3,3-trifluorpropyl)amino]imidazo[1,2-a]-pyridin-3-yl}-2-methyl-N-(1-methylcyclopropyl)-benzamid,
4-{6-[3-Fluor-4-(trifluormethoxy)phenoxy]-8-[(3,3,3-trifluorpropyl)amino]imidazo[1,2-a]-pyridin-3-yl}-N,2-dimethylbenzamid,
N-Ethyl-4-{6-[3-fluor-4-(trifluormethoxy)phenoxy]-8-[(3,3,3-trifluorpropyl)amino]imidazo[1,2-a]-pyridin-3-yl}-2-methylbenzamid,
N,2-Dimethyl-4-{6-(pyridin-4-yl)-8-[(3,3,3-trifluorpropyl)amino]imidazo[1,2-a]pyridin-3-yl}-benzamid,
N-Ethyl-2-methyl-4-{6-(pyridin-4-yl)-8-[(3,3,3-trifluorpropyl)amino]imidazo[1,2-a]pyridin-3-yl}-benzamid,
2-Methyl-N-(1-methylcyclopropyl)-4-{6-(pyridin-4-yl)-8-[(3,3,3-trifluorpropyl)amino]imidazo[1,2-a]-pyridin-3-yl}benzamid,
N-(1-Cyanocyclopropyl)-2-methyl-4-{6-(pyridin-4-yl)-8-[(3,3,3-trifluorpropyl)amino]imidazo[1,2-a]-pyridin-3-yl}benzamid,
N-Cyclopropyl-2-methyl-4-{8-[(3,3,3-trifluorpropyl)amino]-6-vinylimidazo[1,2-a]pyridin-3-yl}-benzamid,
N,2-Dimethyl-4-{8-[(3,3,3-trifluorpropyl)amino]-6-vinylimidazo[1,2-a]pyridin-3-yl}benzamid,
N-Ethyl-2-methyl-4-{8-[(3,3,3-trifluorpropyl)-amino]-6-vinylimidazo[1,2-a]pyridin-3-yl}benzamid, 2-Methyl-N-(1-methylcyclopropyl)-4-{8-[(3,3,3-trifluorpropyl)amino]-6-vinylimidazo[1,2-a]-pyridin-3-yl}benzamid,
N-(1-Cyanocyclopropyl)-2-methyl-4-{8-[(3,3,3-trifluorpropyl)amino]-6-vinylimidazo[1,2-a]-pyridin-3-yl}benzamid,
N-[rel-(1S,2S)-2-Fluorcyclopropyl]-2-methyl-4-{8-[(3,3,3-trifluorpropyl)amino]-6-vinylimidazo[1,2-a]pyridin-3-yl}benzamid,
2-Methyl-N-[rel-(1S,2S)-2-methylcyclopropyl]-4-{8-[(3,3,3-trifluorpropyl)amino]-6-vinylimidazo[1,2-a]pyridin-3-yl}benzamid,
N-Cyclopropyl-4-{6-ethyl-8-[(3,3,3-trifluorpropyl)amino]imidazo[1,2-a]pyridin-3-yl}-2-methyl-benzamid,
4-{6-Ethyl-8-[(3,3,3-trifluorpropyl)amino]imidazo-[1,2-a]pyridin-3-yl}-N,2-dimethylbenzamid,
N-Ethyl-4-{6-ethyl-8-[(3,3,3-trifluorpropyl)-amino]imidazo[1,2-a]pyridin-3-yl}-2-methylbenzamid,
4-{6-Ethyl-8-[3,3,3-trifluorpropyl)amino]imidazo[1,2-a]pyridin-3-yl}-2-methyl-N-(1-methylcyclo-propyl)benzamid,
N-(1-Cyanocyclopropyl)-4-{6-ethyl-8-[(3,3,3-trifluorpropyl)amino]imidazo[1,2-a]pyridin-3-yl}-2-methylbenzamid,
4-{6-Ethyl-8-[(3,3,3-trifluorpropyl)amino]imidazo-[1,2-a]pyridin-3-yl}-N-[rel-(1S,2S)-2-fluorcyclo-propyl]-2-methylbenzamid,
4-{6-Ethyl-8-[(3,3,3-trifluorpropyl)amino]imidazo-[1,2-a]pyridin-3-yl}-2-methyl-N-[rel-(1S,2S)-2-methylcyclopropyl]benzamid,
4-{6-(3-Fluor-4-methoxyphenoxy)-8-[(3,3,3-trifluorpropyl)amino]imidazo[1,2-a]pyridin-3-yl}-N,2-dimethylbenzamid,
N-Ethyl-4-{6-(3-fluor-4-methoxyphenoxy)-8-[(3,3,3-trifluorpropyl)amino]imidazo[1,2-a]pyridin-3-yl}-2-methylbenzamid,
N-Cyclopropyl-4-{6-[(1Z)-3-hydroxyprop-1-en-1-yl]-8-[(3,3,3-trifluorpropyl)amino]imidazo[1,2-a]-pyridin-3-yl}-2-methylbenzamid,
4-{6-[4-(Benzyloxy)phenoxy]-8-[(3,3,3-trifluorpropyl)amino]imidazo[1,2-a]pyridin-3-yl}-N-cyclopropyl-2-methylbenzamid,
N-Cyclopropyl-4-{6-(4-hydroxyphenoxy)-8-[(3,3,3-trifluorpropyl)amino]imidazo[1,2-a]pyridin-3-yl}-2-methylbenzamid,
N-Cyclopropyl-2-methyl-4-{6-[(1E)-prop-1-en-1-yl]-8-[(3,3,3-trifluorpropyl)amino]imidazo[1,2-a]-pyridin-3-yl}benzamid,
N,2-Dimethyl-4-{6-[(1E)-prop-1-en-1-yl]-8-[(3,3,3-trifluorpropyl)amino]imidazo[1,2-a]pyridin-3-yl}-benzamid,
N-Ethyl-2-methyl-4-{6-[(1E)-prop-1-en-1-yl]-8-[(3,3,3-trifluorpropyl)amino]imidazo[1,2-a]-pyridin-3-yl}benzamid,
2-Methyl-N-(1-methylcyclopropyl)-4-{6-[(1E)-prop-1-en-1-yl]-8-[(3,3,3-trifluorpropyl)amino]imidazo-[1,2-a]pyridin-3-yl}benzamid,
N-(1-Cyanocyclopropyl)-2-methyl-4-{6-[(1E)-prop-1-en-1-yl]-8-[(3,3,3-trifluorpropyl)amino]imidazo-[1,2-a]pyridin-3-yl}benzamid,
N-[rel-(1S,2S)-2-Fluorcyclopropyl)-2-methyl-4-{6-[(1E)-prop-1-en-1-yl]-8-[(3,3,3-trifluorpropyl)-amino]imidazo[1,2-a]pyridin-3-yl}benzamid,
2-Methyl-N-[rel-(1S,2S)-2-methylcyclopropyl]-4-{6-[(1E)-prop-1-en-1-yl]-8-[(3,3,3-trifluorpropyl)-amino]imidazo[1,2-a]pyridin-3-yl}benzamid,
N-[rel-(1R,2R)-2-Fluorcyclopropyl]-4-{6-[(1Z)-3-hydroxyprop-1-en-1-yl]-8-[(3,3,3-trifluorpropyl)-amino]imidazo[1,2-a]pyridin-3-yl}-2-methyl-benzamid,
4-{6-[(1Z)-3-Hydroxyprop-1-en-1-yl]-8-[(3,3,3-trifluorpropyl)amino]imidazo[1,2-a]pyridin-3-yl}-N,2-dimethylbenzamid,
N-Ethyl-4-{6-[(1Z)-3-hydroxyprop-1-en-1-yl]-8-[(3,3,3-trifluorpropyl)amino]imidazo[1,2-a]-pyridin-3-yl}-2-methylbenzamid,
4-{6-(3-Fluor-4-methoxyphenoxy)-8-[(tetrahydro-2H-pyran-4-ylmethyl)amino]imidazo[1,2-a]pyridin-3-yl]-N,2-dimethylbenzamid,
N-Ethyl-4-{6-(3-fluor-4-methoxyphenoxy)-8-[(tetra-hydro-2H-pyran-4-ylmethyl)amino]imidazo[1,2-a]-pyridin-3-yl}-2-methylbenzamid,
N-Cyclopropyl-4-{6-(3-fluor-4-methoxyphenoxy)-8-[(tetrahydro-2H-pyran-4-ylmethyl)amino]imidazo-[1,2-a]pyridin-3-yl}-2-methylbenzamid
N-[rel-(1S,2S)-2-Fluorcyclopropyl]-4-{6-(3-fluor4-methoxyphenoxy)-8-[(3,3,3-trifluorpropyl)amino]-imidazo[1,2-a]pyridin-3-yl}-2-methylbenzamid,
4-{6-(3-Fluor-4-methoxyphenoxy)-8-[(3,3,3-trifluorpropyl)amino]imidazo[1,2-a]pyridin-3-yl}-2-methyl-N-[rel-(1S,2S)-2-methylcyclopropyl]-benzamid,
4-{6-(3-Hydroxypropyl)-8-[(3,3,3-trifluorpropyl)-amino]imidazo[1,2-a]pyridin-3-yl}-N,2-dimethyl-benzamid,
N-Ethyl-4-{6-(3-hydroxypropyl)-8-[(3,3,3-trifluorpropyl)amino]imidazo[1,2-a]pyridin-3-yl}-2-methyl-benzamid,
N-Cyclopropyl-4-{6-(3-hydroxypropyl)-8-[(3,3,3-trifluorpropyl)amino]imidazo[1,2-a]pyridin-3-yl}-2-methylbenzamid,
N-Cyclopropyl-4-{6-(3-fluor-4-hydroxyphenoxy)-8-[(3,3,3-trifluorpropyl)amino]imidazo[1,2-a]-pyridin-3-yl}-2-methylbenzamid,
4-{6-(3-Fluor-4-hydroxyphenoxy)-8-[(3,3,3-trifluorpropyl)amino]imidazo[1,2-a]pyridin-3-yl}-N,2-dimethylbenzamid,
N-Ethyl-4-{6-(3-fluor-4-hydroxyphenoxy)-8-[(3,3,3-trifluorpropyl)amino]imidazo[1,2-a]pyridin-3-yl}-2-methylbenzamid,
N-Cyclopropyl-4-{6-(2-hydroxyphenyl)-8-[(3,3,3-trifluorpropyl)amino]imidazo[1,2-a]pyridin-3-yl}-2-methylbenzamid,
4-{6-(2-Hydroxyphenyl)-8-[(3,3,3-trifluorpropyl)-amino]imidazo[1,2-a]pyridin-3-yl}-N,2-dimethyl-benzamid,
N-Ethyl-4-{6-(2-hydroxyphenyl)-8-[(3,3,3-trifluorpropyl)amino]imidazo[1,2-a]pyridin-3-yl}-2-methyl-benzamid,
N-[rel-(1S,2S)-2-Fluorcyclopropyl]-4-{6-(2-hydroxyphenyl)-8-[(3,3,3-trifluorpropyl)amino]-imidazo[1,2-a]pyridin-3-yl}-2-methylbenzamid,
N-Cyclopropyl-4-{6-(3-hydroxyphenoxy)-8-[(3,3,3-trifluorpropyl)amino]imidazo[1,2-a]pyridin-3-yl}-2-methylbenzamid,
4-{6-(3-Hydroxyphenoxy)-8-[(3,3,3-trifluorpropyl)-amino]imidazo[1,2-a]pyridin-3-yl}-2-methyl-N-(1-methylcyclopropyl)benzamid,
N-Ethyl-4-{6-(3-hydroxyphenoxy)-8-[(3,3,3-trifluorpropyl)amino]imidazo[1,2-a]pyridin-3-yl}-2-methylbenzamid,
4-{6-(3-Hydroxyphenoxy)-8-[(3,3,3-trifluorpropyl)-amino]imidazo[1,2-a]pyridin-3-yl}-N,2-dimethyl-benzamid,
2-Methyl-4-{3-[3-methyl-4-(methylcarbamoyl)-phenyl]-8-[(3,3,3-trifluorpropyl)amino]imidazo-[1,2-a]pyridin-6-yl}benzoesäuremethylester,
N-Cyclopropyl-4-{6-(2-hydroxyethyl)-8-[(3,3,3-trifluorpropyl)amino]imidazo[1,2-a]pyridin-3-yl}-2-methylbenzamid,
4-{6-(2-Hydroxyethyl)-8-[(3,3,3-trifluorpropyl)-amino]imidazo[1,2-a]pyridin-3-yl}-N,2-dimethyl-benzamid,
N-Cyclopropyl-4-{6-[(1RS)-1-hydroxyethyl]-8-[(3,3,3-trifluorpropyl)amino]imidazo[1,2-a]-pyridin-3-yl}-2-methylbenzamid,
4-{6-[(1RS)-1-Hydroxyethyl]-8-[(3,3,3-trifluorpropyl)amino]imidazo[1,2-a]pyridin-3-yl}-N,2-dimethylbenzamid,
N-Ethyl-4-{6-[(1RS)-1-hydroxyethyl]-8-[(3,3,3-trifluorpropyl)amino]imidazo[1,2-a]pyridin-3-yl}-2-methylbenzamid,
N-Cyclopropyl-4-{6-(3-fluor-2-methoxyphenoxy)-8-[(3,3,3-trifluorpropyl)amino]imidazo[1,2-a]-pyridin-3-yl}-2-methylbenzamid,
4-{3-[4-(Cyclopropylcarbamoyl)-3-methylphenyl]-8-[(3,3,3-trifluorpropyl)amino]imidazo[1,2-a]-pyridin-6-yl}-N,2-dimethylbenzamid,
4-{3-[4-(Cyclopropylcarbamoyl)-3-methylphenyl]-8-[(3,3,3-trifluorpropyl)amino]imidazo[1,2-a]-pyridin-6-yl}-N-ethyl-2-methylbenzamid,
4-{3-[4-(Cyclopropylcarbamoyl)-3-methylphenyl]-8-[(3,3,3-trifluorpropyl)amino]imidazo[1,2-a]-pyridin-6-yl}-2-methyl-N-(1-methylcyclopropyl)-benzamid,
4-(6-{4-[(1-Cyanocyclopropyl)carbamoyl]-3-methyl-phenyl}-8-[(3,3,3-trifluorpropyl)amino]imidazo-[1,2-a]pyridin-3-yl)-N-cyclopropyl-2-methyl-benzamid,
4-{3-[4-(Cyclopropylcarbamoyl)-3-methylphenyl]-8-[(3,3,3-trifluorpropyl)amino]imidazo[1,2-a]-pyridin-6-yl}-N-[1-(hydroxymethyl)cyclopropyl]-2-methylbenzamid,
4-{3-[4-(Cyclopropylcarbamoyl)-3-methylphenyl]-8-[(3,3,3-trifluorpropyl)amino]imidazo[1,2-a]-pyridin-6-yl}-N-[rel-(1S,2S)-2-fluorcyclopropyl]-2-methylbenzamid,
4-{6-(4-Carbamoyl-3-methylphenyl)-8-[(3,3,3-trifluorpropyl)amino]imidazo[1,2-a]pyridin-3-yl}-N-cyclopropyl-2-methylbenzamid,
4-{6-(4-{[rel-(1S,2S)-2-Fluorcyclopropyl]-carbamoyl}-3-methylphenyl)-8-[(3,3,3-trifluorpropyl)amino]imidazo[1,2-a]pyridin-3-yl}-N,2-dimethylbenzamid,
4-{6-(4-{[1-(Hydroxymethyl)cyclopropyl]carbamoyl}-3-methylphenyl)-8-[(3,3,3-trifluorpropyl)amino]-imidazo[1,2-a]pyridin-3-yl}-N,2-dimethylbenzamid,
4-(6-{4-[(1-Cyanocyclopropyl)carbamoyl]-3-methyl-phenyl}-8-[(3,3,3-trifluorpropyl)amino]imidazo-[1,2-a]pyridin-3-yl}-N,2-dimethylbenzamid,
2-Methyl-N-(1-methylcyclopropyl)-4-{3-[3-methyl-4-(methylcarbamoyl)phenyl]-8-[(3,3,3-trifluorpropyl)amino]imidazo[1,2-a]pyridin-6-yl}benzamid,
4-{6-[4-(Cyclopropylcarbamoyl)-3-methylphenyl]-8-[(3,3,3-trifluorpropyl)amino]imidazo[1,2-a]-pyridin-3-yl}-N,2-dimethylbenzamid,
4-{6-[4-(Ethylcarbamoyl)-3-methylphenyl]-8-[(3,3,3-trifluorpropyl)amino]imidazo[1,2-a]-pyridin-3-yl}-N,2-dimethylbenzamid,
4-{6-(4-Carbamoyl-3-methylphenyl)-8-[(3,3,3-trifluorpropyl)amino]imidazo[1,2-a]pyridin-3-yl}-N,2-dimethylbenzamid,
N-Cyclopropyl-4-{8-[(2,2-difluorethyl)amino]-6-(3-fluorphenoxy)imidazo[1,2-a]pyridin-3-yl}-2-methyl-benzamid,
N-Cyclopropyl-4-{8-[(2,2-difluorethyl)amino]-6-(4-methoxyphenoxy)imidazo[1,2-a]pyridin-3-yl}-2-methylbenzamid,
N-Cyclopropyl-4-{8-[(2,2-difluorethyl)amino]-6-(2,3-difluorphenoxy)imidazo[1,2-a]pyridin-3-yl}-2-methylbenzamid,
N-Cyclopropyl-4-{8-[(2,2-difluorethyl)amino]-6-(5-fluor-2-methylphenoxy)imidazo[1,2-a]pyridin-3-yl}-2-methylbenzamid,
N-Cyclopropyl-4-{8-[(2,2-difluorethyl)amino]-6-(2,5-difluorphenoxy)imidazo[1,2-a]pyridin-3-yl}-2-methylbenzamid,
N-Cyclopropyl-4-{8-[(2,2-difluorethyl)amino]-6-(3-fluor-2-methylphenoxy)imidazo[1,2-a]pyridin-3-yl}-2-methylbenzamid,
N-Cyclopropyl-4-{8-[(2,2-difluorethyl)amino]-6-(3-fluor-4-methoxyphenoxy)imidazo[1,2-a]pyridin-3-yl}-2-methylbenzamid,
N-Cyclopropyl-4-{8-[(2,2-difluorethyl)amino]-6-(2-hydroxybenzyl)imidazo[1,2-a]pyridin-3-yl}-2-methylbenzamid,
N-Cyclopropyl-4-{6-(3-fluorphenoxy)-8-[(2-methoxy-ethyl)amino]imidazo[1,2-a]pyridin-3-yl}-2-methyl-benzamid,
N-Cyclopropyl-4-{8-[(2-methoxyethyl)amino]-6-(2-methylphenoxy)imidazo[1,2-a]pyridin-3-yl}-2-methylbenzamid,
N-Cyclopropyl-4-{6-(3,4-difluorphenoxy)-8-[(2-methoxyethyl)amino]imidazo[1,2-a]pyridin-3-yl}-2-methylbenzamid,
N-Cyclopropyl-4-{6-(5-fluor-2-methylphenoxy)-8-[(2-methoxyethyl)amino]imidazo[1,2-a]pyridin-3-yl}-2-methylbenzamid,
N-Cyclopropyl-4-{6-(2,3-difluorphenoxy)-8-[(2-methoxyethyl)amino]imidazo[1,2-a]pyridin-3-yl}-2-methylbenzamid,
N-Cyclopropyl-4-{6-(5-fluor-2-hydroxybenzoyl)-8-[(2-methoxyethyl)amino]imidazo[1,2-a]pyridin-3-yl}-2-methylbenzamid,
N-Cyclopropyl-4-{8-[(2,2-difluorethyl)amino]-6-(3-fluorphenoxy)imidazo[1,2-a]pyridin-3-yl}-2-methyl-benzamid,
N-Cyclopropyl-4-{6-(5-fluor-2-hydroxyphenoxy)-8-[(3,3,3-trifluorpropyl)amino]imidazo[1,2-a]-pyridin-3-yl}-2-methylbenzamid,
N-Cyclopropyl-4-{6-(3-fluor-2-hydroxyphenoxy)-8-[(3,3,3-trifluorpropyl)amino]imidazo[1,2-a]-pyridin-3-yl}-2-methylbenzamid,
N-Cyclopropyl-4-{6-[(5-fluorpyridin-3-yl)oxy]-8-[(3,3,3-trifluorpropyl)amino]imidazo[1,2-a]-pyridin-3-yl}-2-methylbenzamid,
N-Cyclopropyl-4-{6-[(5-fluor-6-methoxypyridin-3-yl)oxy]-8-[(3,3,3-trifluorpropyl)amino]imidazo-[1,2-a]pyridin-3-yl}-2-methylbenzamid,
oder ein Stereoisomer, ein Tautomer, ein N-Oxid, ein Hydrat, ein Solvat oder ein Salz davon, oder eine Mischung dieser.

4. Verfahren zur Herstellung einer Verbindung nach einem der Ansprüche 1 bis 3, wobei das Verfahren den Schritt umfasst, bei dem man:
- ein Zwischenprodukt der allgemeinen Formel IV: 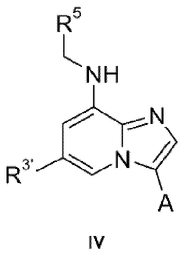 in welcher
R⁵ und A wie für die allgemeine Formel I in einem der Ansprüche 1 bis 3 definiert sind; und
R^{3'} für ein Halogenatom steht,
mit einer Verbindung der allgemeinen Formel IVa:
R³-Y IVa,
in welcher
R³ wie für die allgemeine Formel I in einem der Ansprüche 1 bis 3 definiert ist;
Y für einen Substituenten steht, der in einer Kupplungsreaktion verdrängt wird;
reagieren lässt, unter Erhalt einer Verbindung der allgemeinen Formel I: 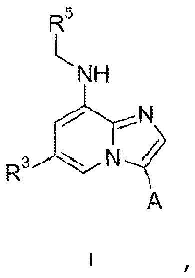 in welcher
R³, R⁵ und A wie für die allgemeine Formel I in einem der Ansprüche 1 bis 3 definiert sind.

5. Verfahren zur Herstellung einer Verbindung nach einem der Ansprüche 1 bis 3, wobei das Verfahren den Schritt umfasst, bei dem man:
- ein Zwischenprodukt der allgemeinen Formel II: 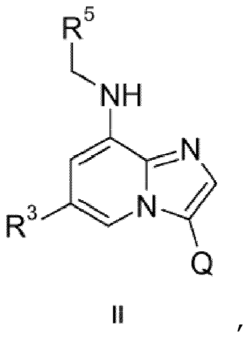 in welcher
R³ und R⁵ wie für die allgemeine Formel I in einem der Ansprüche 1 bis 3 definiert sind;
und Q für ein Halogenatom steht;
mit einer Verbindung der allgemeinen Formel IIa:
A-Y IIa,
in welcher
A wie für die allgemeine Formel I in einem der Ansprüche 1 bis 3 definiert ist,
Y für einen Substituenten steht, der in einer Kupplungsreaktion verdrängt wird;
reagieren lässt, unter Erhalt einer Verbindung der allgemeinen Formel I: 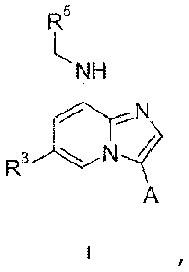 in welcher R³, R⁵ und A wie für die allgemeine Formel I in einem der Ansprüche 1 bis 3 definiert sind.

6. Verfahren zur Herstellung einer Verbindung nach einem der Ansprüche 1 bis 3, wobei das Verfahren den Schritt umfasst, bei dem man:
- ein Zwischenprodukt der allgemeinen Formel VII: 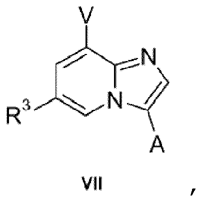 in welcher
R³ und A wie für die allgemeine Formel I in einem der Ansprüche 1 bis 3 definiert sind; und
V für eine Abgangsgruppe steht;
mit einer Verbindung der allgemeinen Formel IIa:
R⁵-CH₂-NH₂ **VIIa,**
in welcher
R⁵ wie für die allgemeine Formel I in einem der Ansprüche 1 bis 3 definiert ist;
reagieren lässt, unter Erhalt einer Verbindung der allgemeinen Formel I: 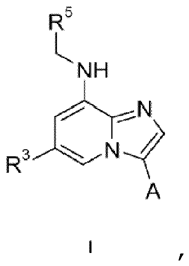 in welcher R³, R⁵ und A wie für die allgemeine Formel I in einem der Ansprüche 1 bis 3 definiert sind.

7. Verfahren zur Herstellung einer Verbindung nach einem der Ansprüche 1 bis 3, wobei das Verfahren den Schritt umfasst, bei dem man:
- ein Zwischenprodukt der allgemeinen Formel VII: 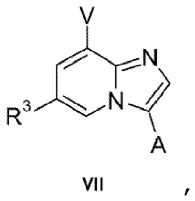 in welcher
R³ und A wie für die allgemeine Formel I in einem der Ansprüche 1 bis 3 definiert sind; und
V für eine NH₂-Gruppe steht;
mit einer Verbindung der allgemeinen Formel VIIb:
O=CHR⁵ VIIb,
in welcher
R⁵ wie für die allgemeine Formel I in einem der Ansprüche 1 bis 3 definiert ist;
reagieren lässt, unter Erhalt einer Verbindung der allgemeinen Formel I: 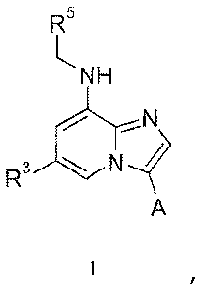
in welcher R³, R⁵ und A wie für die allgemeine Formel I in einem der Ansprüche 1 bis 3 definiert sind.

8. Verfahren nach einem der Ansprüche 1 bis 3 oder ein Stereoisomer, ein Tautomer, ein N-Oxid, ein Hydrat, ein Solvat oder ein Salz davon, insbesondere ein pharmazeutisch unbedenkliches Salz davon, oder eine Mischung dieser, zur Verwendung bei der Behandlung oder Prophylaxe einer Krankheit.

9. Verbindung zur Verwendung nach Anspruch 8, wobei es sich bei der Krankheit um eine Krankheit mit unkontrolliertem Zellwachstum, unkontrollierter Zellproliferation und/oder unkontrolliertem Zell-überleben, eine unangemessene zelluläre Immunreaktion oder eine unangemessene zelluläre Entzündungsreaktion handelt, wobei es sich bei der Krankheit mit unkontrolliertem Zellwachstum, unkontrollierter Zellproliferation und/oder unkontrolliertem Zellüberleben, der unangemessenen zellulären Immunreaktion bzw. der unangemessenen zellulären Entzündungsreaktion um einen hämatologischen Tumor, einen soliden Tumor und/oder Metastasen davon, z.B. Leukämien und myelodysplastisches Syndrom, maligne Lymphome, Kopf- und Halstumore einschließlich Hirntumoren und Hirnmetastasen, Tumore des Thorax einschließlich nichtkleinzelliger und kleinzelliger Lungentumore, Tumore des Magen-Darm-Trakts, endokrine Tumore, Brust- und andere gynäkologische Tumore, urologische Tumore einschließlich Nieren-, Blasen- und Prostatatumoren, Hauttumore und Sarkome und/oder Metastasen davon handelt.

10. Pharmazeutische Zusammensetzung, umfassend eine Verbindung nach einem der Ansprüche 1 bis 3 oder ein Stereoisomer, ein Tautomer, ein N-Oxid, ein Hydrat, ein Solvat oder ein Salz davon, insbesondere ein pharmazeutisch unbedenkliches Salz davon, oder eine Mischung dieser, und ein pharmazeutisch unbedenkliches Verdünnungsmittel oder einen pharmazeutisch unbedenklichen Träger.

11. Pharmazeutische Zusammensetzung, umfassend:
- eine oder mehrere Verbindungen nach einem der Ansprüche 1 bis 3 oder ein Stereoisomer, ein Tautomer, ein N-Oxid, ein Hydrat, ein Solvat oder ein Salz davon, insbesondere ein pharmazeutisch unbedenkliches Salz davon, oder eine Mischung dieser;
und
- ein oder mehrere Mittel ausgewählt aus: einem Taxan wie Docetaxel, Paclitaxel oder Taxol; einem Epothilon wie Ixabepilon, Patupilon oder Sagopilon; Mitoxantron; Predinisolon; Dexamethason; Estramustin; Vinblastin; Vincristin; Doxorubicin; Adriamycin; Idarubicin; Daunorubicin; Bleomycin; Etoposid; Cyclophosphamid; Ifosfamid; Procarbazin; Melphalan; 5-Fluoruracil; Capecitabin; Fludarabin; Cytarabin; Ara-C; 2-Chlor-2'-desoxyadenosin; Thioguanin; einem Antiandrogen wie Flutamid, Cyproteronacetat oder Bicalutamid; Bortezomib; einem Platinderivat wie Cisplatin oder Carboplatin; Chlorambucil; Methotrexat und Rituximab.

12. Verwendung einer Verbindung nach einem der Ansprüche 1 bis 3 oder eines Stereoisomers, eines Tautomers, eines N-Oxids, eines Hydrats, eines Solvats oder eines Salzes davon, insbesondere eines pharmazeutisch unbedenklichen Salzes davon, oder einer Mischung dieser, zur Herstellung eines Medikaments zur Prophylaxe oder Behandlung einer Krankheit.

13. Verwendung nach Anspruch 12, wobei es sich bei der Krankheit um eine Krankheit mit unkontrolliertem Zellwachstum, unkontrollierter Zellproliferation und/oder unkontrolliertem Zellüberleben, eine unangemessene zelluläre Immunreaktion oder eine unangemessene zelluläre Entzündungsreaktion handelt, wobei es sich bei der Krankheit mit unkontrolliertem Zellwachstum, unkontrollierter Zellproliferation und/oder unkontrolliertem Zellüberleben, der unangemessenen zellulären Immunreaktion bzw. der unangemessenen zellulären Entzündungsreaktion um einen hämatologischen Tumor, einen soliden Tumor und/oder Metastasen davon, z.B. Leukämien und myelodysplastisches Syndrom, maligne Lymphome, Kopf- und Halstumore einschließlich Hirntumoren und Hirnmetastasen, Tumore des Thorax einschließlich nichtkleinzelliger und kleinzelliger Lungentumore, Tumore des Magen-Darm-Trakts, endokrine Tumore, Brust-und andere gynäkologische Tumore, urologische Tumore einschließlich Nieren-, Blasen- und Prostatatumoren, Hauttumore und Sarkome und/oder Metastasen davon handelt.

14. Verbindung der allgemeinen Formel IV: 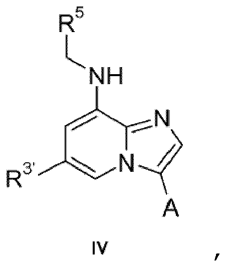 in welcher R⁵ und A wie für die allgemeine Formel I in einem der Ansprüche 1 bis 3 definiert sind und R³' für ein Halogenatom steht.

15. Verbindung der allgemeinen Formel VII: 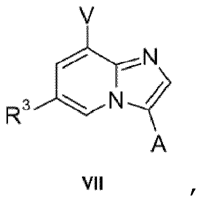 in welcher R³ und A wie für die allgemeine Formel I in einem der Ansprüche 1 bis 3 definiert sind und V für eine NH₂-Gruppe oder ein Halogenatom steht.

## Revendications

1. Composé de formule générale I : 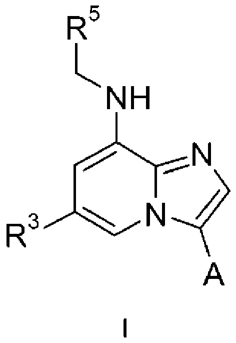 dans lequel :
A représente un groupement 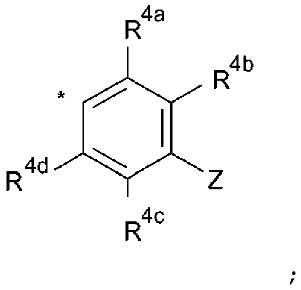
où * indique le point de liaison desdits groupements au reste de la molécule ;
Z représente un groupement -C(=O)N(H)R¹ ou - C(=S)N(H)R¹ ;
R¹ représente un groupement (alkyle en C₁-C₃)- ou cyclopropyl- ;
où ledit groupement cyclopropyl- est éventuellement substitué, de façon identique ou différente, par 1, 2 ou 3 groupements choisis parmi les suivants : halogène, -OH, -CN, (alkyle en C₁-C₃)- ;
où ledit groupement (alkyle en C₁-C₃)- est éventuellement substitué, de façon identique ou différente, par 1, 2 ou 3 groupements choisis parmi les suivants : halogène, -OH, -CN, (alkoxy en C₁-C₃)- ;
R³ représente un atome d'hydrogène ou un groupement - CN, (alkyle en C₁-C₆)-, -(CH₂)ₘ-(alcényle en C₂-C₆), - (CH₂)ₘ-(alcynyle en C₂-C₆), aryl-, hétéroaryl-, aryl-X- ;
où ledit groupement (alkyle en C₁-C₆)-, -(CH₂)ₘ-(alcényle en C₂-C₆), -(CH₂)ₘ-(alcynyle en C₂-C₆), aryl-aryl-X- ou hétéroaryl- est éventuellement substitué de façon identique ou différente par 1, 2 ou 3 groupements R⁷ ;
R^{4a}, R^{4b}, R^{4d}
représentent un atome d'hydrogène ;
R^{4c} représente un atome d'halogène ou un groupement - CN, -OH, (alkyle en C₁-C₃)-, (alkoxy en C₁-C₃)-, halogéno-(alkyle en C₁-C₃)-, halogéno-(alkoxy en C₁-C₃)-, R^{6a}(R^{6b})N-(alkyle en C₁-C₃)-, HO-(alkyle en C₁-C₃)-, NC-(alkyle en C₁-C₃)-, (alkoxy en C₁-C₃)-(alkyle en C₁-C₃)-, halogéno-(alkoxy en C₁-C₃)-(alkyle en C₁-C₃)- ;
R⁵ représente un atome d'hydrogène ou un groupement (alkyle en C₁-C₆)-, -(CH₂)ₙ-(alcényle en C₂-C₆), -(CH₂)ₙ-(alcynyle en C₂-C₆), -(CH₂)ₘ-(cycloalkyle en C₃-C₆), - (CH₂)ₘ-(hétérocycloalkyle comportant 3 à 7 chaînons), aryl-(alkyle en C₁-C₆)-, hétéroaryl- (alkyle en C₁-C₆)-, halogéno-(alkyle en C₁-C₆)-, R^{6a}(R^{6b})N-(alkyle en C₁-C₆)-, HO-(alkyle en C₁-C₆)-, -(alkyle en C₁-C₆)-CN, (alkoxy en C₁-C₆)-(alkyle en C₁-C₆)-, halogéno-(alkoxy en C₁-C₆)-(alkyle en C₁-C₆)-, (cycloalkyle en C₃-C₆)-, (hétérocycloalkyle comportant 3 à 7 chaînons)-, (cycloalcényle en C₄-C₈)-, aryl- ou hétéroaryl- ;
où ledit groupement (alkyle en C₁-C₆)-, -(CH₂)ₙ-(alcényle en C₂-C₆), - (CH₂)ₙ-(alcynyle en C₂-C₆), -(CH₂)ₘ-(cycloalkyle en C₃-C₆), -(CH₂)ₘ-(hétérocycloalkyle comportant 3 à 7 chaînons), aryl-(alkyle en C₁-C₆)-, hétéroaryl- (alkyle en C₁-C₆)-, halogéno-(alkyle en C₁-C₆)-, R^{6a}(R^{6b})N-(alkyle en C₁-C₆)-, HO-(alkyle en C₁-C₆)-, - (alkyle en C₁-C₆)-CN, (alkoxy en C₁-C₆)-(alkyle en C₁-C₆)-, halogéno-(alkoxy en C₁-C₆)- (alkyle en C₁-C₆)-, (cycloalkyle en C₃-C₆)-, (hétérocycloalkyle comportant 3 à 7 chaînons)-, (cycloalcényle en C₄-C₈)-, aryl- ou hétéroaryl- est éventuellement substitué de façon identique ou différente par 1, 2, 3, 4 ou 5 groupements R⁸ ;
R⁶, R^{6a}, R^{6b}, R^{6C},
représentent indépendamment les uns des autres un atome d'hydrogène ou un groupement (alkyle en C₁-C₆)-, (cycloalkyle en C₃-C₆)- ou aryl- (alkyle en C₁-C₆)- ; où ledit groupement (cycloalkyle en C₃-C₆)- est éventuellement substitué de façon identique différente par 1 ou 2 groupements choisis parmi les suivants : halogène, -OH, -CN, (alkyle en C₁-C₆)-, HO-(alkyle en C₁-C₆)- ;
R⁷ représente un atome d'halogène ou un groupement HO-, (alkoxy en C₁-C₆)-, (alkyle en C₁-C₆)-, halogéno-(alkyle en C₁-C₆)-, halogéno-(alkoxy en C₁-C₆)-, HO-(alkyle en C₁-C₆)-, -C(=O)N(H)R^{6a}, -N(R^{6a})R^{6b}, -O(C=O)OR⁶ ou -OR⁶ ;
R⁸ représente un atome d'hydrogène ou d'halogène ou un groupement -CN, (alkoxy en C₁-C₆)-, (alkyle en C₁-C₆)-, halogéno-(alkyle en C₁-C₆)-, R^{6a}(R^{6b})N-(alkyle en C₁-C₆)-, HO-(alkyle en C₁-C₆)-, (alkoxy en C₁-C₆)-(alkyle en C₁-C₆)-, halogéno-(alkoxy en C₁-C₆)-(alkyle en C₁-C₆)-, (alcényle en C₂-C₆)-, (alcynyle en C₂-C₆)-, (hétérocycloalkyle comportant 3 à 7 chaînons)-, aryl-, hétéroaryl-, -C(=O)R⁶, -C(=O)N(H)R^{6a}, -C(=O)N(R^{6a})R^{6b}, - C(=O)O-R⁶, -N(R^{6a})R^{6b}, -NO₂, -N(H)C(=O)R⁶, -N(R^{6c})C(=O)R⁶, -N(H)C(=O)N(R^{6a})R^{6b}, -N(R^{6c})C(=O)N(R^{6a})R^{6b}, -N(H)C(=O)OR⁶, -N (R^{6c}) C (=O) OR⁶, -N (H) S (=O) R⁶, -N (R^{6c}) S (=O) R⁶,-N(H)S(=O)₂R⁶, -N(R^{6c})S(=O)₂R⁶, -N=S(=O) (R^{6a})R^{6b}, -OR⁶, - O(C=O)R⁶, -O(C=O)N(R^{6a})R^{6b}, -O(C=O)OR⁶, -SR⁶, -S(=O)R⁶, - S(=O)N(H)R⁶, -S(=O)N(R^{6a})R^{6b}, -S(=O₂)R⁶, -S(=O)₂N(H)R⁶, - S (=O) ₂N (R^{6a}) R^{6b}, -S (=O) (=NR^{6c}) R⁶ ou-S (=O) ₂-(hétérocycloalkyle comportant 3 à 7 chaînons) ; où chacun desdits groupements (hétérocycloalkyle comportant 3 à 7 chaînons)- ou hétéroaryl- est éventuellement substitué de façon identique ou différente par 1, 2, 3 ou 4 groupements (alkyle en C₁-C₆)- ;
m représente un entier choisi parmi 0, 1, 2, 3, 4, 5 ou 6 ;
n représente un entier choisi parmi 0, 1, 2, 3, 4 ou 5 ; et
X représente un groupement S, S(=O), S(=O)₂, O, NR⁶, C(=O) ou CR^{6a}R^{6b} ;
ou l'un des stéréoisomères, tautomères, N-oxydes, hydrates, solvates ou sels de celui-ci, ou l'un de leurs mélanges.

2. Composé selon la revendication 1, où :
A représente 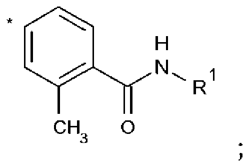
où * indique le point de liaison dudit groupement au reste de la molécule ;
R¹ représente un groupement méthyl- ou un groupement cyclopropyl- ;
où ledit groupement cyclopropyl- est éventuellement substitué par un atome de fluor ;
R³ représente un groupement pyridyl-, phényl-, phényl-O- ou phényl-S- ;
où le groupement phényl- est substitué soit par un groupement méthyl- et un groupement -C(=O)N(H)R^{6a}, soit par un groupement HO-CH₂- ;
où ledit groupement phényl-O- est éventuellement substitué, de façon identique ou différente, par 1 ou 2 groupements choisis parmi les suivants : -F, H₃C-O-, HO-, H₃C- ;
où le groupement phényl-S- est éventuellement substitué, de façon identique ou différente, par 1 ou 2 groupements choisis parmi les suivants : -F, H₃C-O-, HO-, H₃C- ;
R⁵ représente un groupement choisi parmi les suivants : 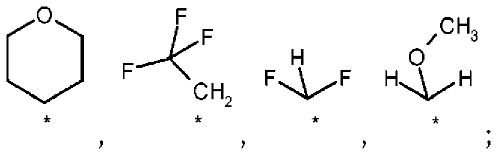
où * indique le point de liaison desdits groupements au reste de la molécule
R^{6a} représente un atome d'hydrogène ou un groupement méthyl- ou un groupement (cyclopropyle en C₃-C₆)- ; où ledit groupement (cyclopropyle en C₃-C₆)- est éventuellement substitué par un groupement -CN ;
ou l'un des stéréoisomères, tautomères, N-oxydes, hydrates, solvates ou sels de celui-ci, ou l'un de leurs mélanges.

3. Composé selon la revendication 1, choisi dans le groupe constitué par les suivants :
N-cyclopropyl-4-{8-[(2-méthylpropyl)amino]imidazo[1,2-a]pyridin-3-yl}benzamide,
N-cyclopropyl-4-{6-[2-(hydroxyméthyl)phényl]-8-[(3,3,3-trifluoropropyl)amino]imidazo[1,2-a]pyridin-3-yl}-2-méthylbenzamide,
N-cyclopropyl-2-méthyl-4-{6-(pyridin-4-yl)-8-[(3,3,3-trifluoropropyl)amino]imidazo[1,2-a]pyridin-3-yl}benzamide,
N-cyclopropyl-4-{6-[(3-fluoro-5-méthylphényl)sulfanyl]-8-[(3,3,3-trifluoropropyl)amino]imidazo[1,2-a]pyridin-3-yl}-2-méthylbenzamide,
N-cyclopropyl-4-{6-[(2,3-difluorophényl)sulfanyl]-8-[(3,3,3-trifluoropropyl)amino]imidazo[1,2-a]pyridin-3-yl}-2-méthylbenzamide,
N-cyclopropyl-2-méthyl-4-{6-(phénylsulfanyl)-8-[(3,3,3-trifluoropropyl)amino]imidazo[1,2-a]pyridin-3-yl}benzamide,
4-{6-[(2-aminophényl)sulfanyl]-8-[(3,3,3-trifluoropropyl)amino]imidazo[1,2-a]pyridin-3-yl}-N-cyclopropyl-2-méthylbenzamide,
N-cyclopropyl-4-{6-[(3-fluorophényl)sulfanyl]-8-[(3,3,3-trifluoropropyl)amino]imidazo[1,2-a]pyridin-3-yl}-2-méthylbenzamide,
N-cyclopropyl-4-{6-[(2-hydroxyphényl)sulfanyl]-8-[(3,3,3-trifluoropropyl)amino]imidazo[1,2-a]pyridin-3-yl}-2-méthylbenzamide,
N-cyclopropyl-2-méthyl-4-{6-(méthylsulfonyl)-8-[(3,3,3-trifluoropropyl)amino]imidazo[1,2-a]pyridin-3-yl}benzamide,
N-cyclopropyl-4-{6-(2-fluoro-4-méthoxyphénoxy)-8-[(3,3,3-trifluoropropyl)amino]imidazo[1,2-a]pyridin-3-yl}-2-méthylbenzamide,
N-cyclopropyl-4-{6-(3-fluorophénoxy)-8-[(3,3,3-trifluoropropyl)amino]imidazo[1,2-a]pyridin-3-yl}-2-méthylbenzamide,
4-{6-(3-fluorophénoxy)-8-[(3,3,3-trifluoropropyl)amino]imidazo[1,2-a]pyridin-3-yl}-N,2-diméthylbenzamide,
4,4'-{8-[(tétrahydro-2H-pyran-4-ylméthyl)amino]imidazo[1,2-a]pyridine-3,6-diyl}bis(N-cyclopropyl-2-méthylbenzamide),
N-éthyl-4-{6-(3-fluorophénoxy)-8-[(3,3,3-trifluoropropyl)amino]imidazo[1,2-a]pyridin-3-yl}-2-méthylbenzamide,
4-{6-(3-fluorophénoxy)-8-[(3,3,3-trifluoropropyl)amino]imidazo[1,2-a]pyridin-3-yl}-2-méthyl-N-(1-méthylcyclopropyl)benzamide,
N-[rel-(1S,2S)-2-fluorocyclopropyl]-4-{6-(3-fluorophénoxy)-8-[(3,3,3-trifluoropropyl)amino]imidazo[1,2-a]pyridin-3-yl}-2-méthylbenzamide,
N-{[(1R,2R) ou (1S,2S)]-2-fluorocyclopropyl}-4-{6-(3-fluorophénoxy)-8-[(3,3,3-trifluoropropyl)amino]imidazo[1,2-a]pyridin-3-yl}-2-méthylbenzamide,
N-{[(1S,2S) ou (1R,2R)]-2-fluorocyclopropyl}-4-{6-(3-fluorophénoxy)-8-[(3,3,3-trifluoropropyl)amino]imidazo[1,2-a]pyridin-3-yl}-2-méthylbenzamide,
N-(1-cyanocyclopropyl)-4-{6-(3-fluorophénoxy)-8-[(3,3,3-trifluoropropyl)amino]imidazo[1,2-a]pyridin-3-yl}-2-méthylbenzamide,
4-{6-éthynyl-8-[(3,3,3-trifluoropropyl)amino]imidazo[1,2-a]pyridin-3-yl}-N,2-diméthylbenzamide,
N-éthyl-4-{6-éthynyl-8-[(3,3,3-trifluoropropyl)amino]imidazo[1,2-a]pyridin-3-yl}-2-méthylbenzamide,
4-{6-éthynyl-8-[(3,3,3-trifluoropropyl)amino]imidazo[1,2-a]pyridin-3-yl}-N-[rel-(1R,2R)-2-fluorocyclopropyl]-2-méthylbenzamide,
4-{6-éthynyl-8-[(3,3,3-trifluoropropyl)amino]imidazo[1,2-a]pyridin-3-yl}-2-méthyl-N-(1-méthylcyclopropyl)benzamide,
N-(1-cyanocyclopropyl)-4-{6-éthynyl-8-[(3,3,3-trifluoropropyl)amino]imidazo[1,2-a]pyridin-3-yl}-2-méthylbenzamide,
N-cyclopropyl-4-{6-(5-fluoro-2-méthylphénoxy)-8-[(3,3,3-trifluoropropyl)amino]imidazo[1,2-a]pyridin-3-yl}-2-méthylbenzamide,
4-{6-(3-hydroxyprop-1-yn-1-yl)-8-[(3,3,3-trifluoropropyl)amino]imidazo[1,2-a]pyridin-3-yl}-N,2-diméthylbenzamide,
N-éthyl-4-{6-(3-hydroxyprop-1-yn-1-yl)-8-[(3,3,3-trifluoropropyl)amino]imidazo[1,2-a]pyridin-3-yl}-2-méthylbenzamide,
4-{6-(3-hydroxyprop-1-yn-1-yl)-8-[(3,3,3-trifluoropropyl)amino]imidazo[1,2-a]pyridin-3-yl}-2-méthyl-N-(1-méthylcyclopropyl)benzamide,
N-(1-cyanocyclopropyl)-4-{6-(3-hydroxyprop-1-yn-1-yl)-8-[(3,3,3-trifluoropropyl)amino]imidazo[1,2-a]pyridin-3-yl}-2-méthylbenzamide,
N-cyclobutyl-4-{6-(3-fluorophénoxy)-8-[(3,3,3-trifluoropropyl)amino]imidazo[1,2-a]pyridin-3-yl}-2-méthylbenzamide,
4-{6-(5-fluoro-2-méthylphénoxy)-8-[(3,3,3-trifluoropropyl)amino]imidazo[1,2-a]pyridin-3-yl}-N,2-diméthylbenzamide,
N-éthyl-4-{6-(5-fluoro-2-méthylphénoxy)-8-[(3,3,3-trifluoropropyl)amino]imidazo[1,2-a]pyridin-3-yl}-2-méthylbenzamide,
4-{6-(5-fluoro-2-méthylphénoxy)-8-[(3,3,3-trifluoropropyl)amino]imidazo[1,2-a]pyridin-3-yl}-2-méthyl-N-(1-méthylcyclopropyl)benzamide,
N-(1-cyanocyclopropyl)-4-{6-(5-fluoro-2-méthylphénoxy)-8-[(3,3,3-trifluoropropyl)amino]imidazo[1,2-a]pyridin-3-yl}-2-méthylbenzamide,
N-[rel-(1S,2S)-2-fluorocyclopropyl]-4-{6-(5-fluoro-2-méthylphénoxy)-8-[(3,3,3-trifluoropropyl)amino]imidazo[1,2-a]pyridin-3-yl}-2-méthylbenzamide,
N-cyclopropyl-4-{6-(2,3-difluorophénoxy)-8-[(3,3,3-trifluoropropyl)amino]imidazo[1,2-a]pyridin-3-yl}-2-méthylbenzamide,
4-{6-(3-chlorophénoxy)-8-[(3,3,3-trifluoropropyl)amino]imidazo[1,2-a]pyridin-3-yl}-N-cyclopropyl-2-méthylbenzamide,
4,4'-{8-[(3,3,3-trifluoropropyl)amino]imidazo[1,2-a]pyridine-3,6-diyl}bis(N-cyclopropyl-2-méthylbenzamide),
N-cyclopropyl-2-méthyl-4-{6-phénoxy-8-[(3,3,3-trifluoropropyl)amino]imidazo[1,2-a]pyridin-3-yl}benzamide,
4-{6-(3-chlorophénoxy)-8-[(3,3,3-trifluoropropyl)amino]imidazo[1,2-a]pyridin-3-yl}-N,2-diméthylbenzamide,
4-{6-(3-chlorophénoxy)-8-[(3,3,3-trifluoropropyl)amino]imidazo[1,2-a]pyridin-3-yl}-N-éthyl-2-méthylbenzamide,
4-{6-(3-chlorophénoxy)-8-[(3,3,3-trifluoropropyl)amino]imidazo[1,2-a]pyridin-3-yl}-2-méthyl-N-(1-méthylcyclopropyl)benzamide,
4-{6-(3-chlorophénoxy)-8-[(3,3,3-trifluoropropyl)amino]imidazo[1,2-a]pyridin-3-yl}-N-(1-cyanocyclopropyl)-2-méthylbenzamide,
4-{6-(3-fluorophénoxy)-8-[(3,3,3-trifluoropropyl)amino]imidazo[1,2-a]pyridin-3-yl}-2-méthyl-N-(1-méthylcyclobutyl)benzamide,
4-{6-(3-chlorophénoxy)-8-[(3,3,3-trifluoropropyl)amino]imidazo[1,2-a]pyridin-3-yl}-N-[rel-(1R,2R)-2-fluorocyclopropyl]-2-méthylbenzamide,
4-{6-(3-chlorophénoxy)-8-[(3,3,3-trifluoropropyl)amino]imidazo[1,2-a]pyridin-3-yl}-N-{[(1S,2S) ou (1R,2R)]-2-fluorocyclopropyl}-2-méthylbenzamide,
4-{6-(3-chlorophénoxy)-8-[(3,3,3-trifluoropropyl)amino]imidazo[1,2-a]pyridin-3-yl}-N-{[(1R,2R) ou (1S,2S)]-2-fluorocyclopropyl}-2-méthylbenzamide,
N-(2,6-diéthylphényl)-4-{6-(3-fluorophénoxy)-8-[(3,3,3-trifluoropropyl)amino]imidazo[1,2-a]pyridin-3-yl}-2-méthylbenzamide,
4,4'-{8-[méthyl(3,3,3-trifluoropropyl)amino]imidazo[1,2-a]pyridine-3,6-diyl}bis(N,2-diméthylbenzamide),
N-cyclopropyl-4-{6-(3-fluoro-4-méthoxyphénoxy)-8-[(3,3,3-trifluoropropyl)amino]imidazo[1,2-a]pyridin-3-yl}-2-méthylbenzamide,
4,4'-{8-[méthyl(3,3,3-trifluoropropyl)amino]imidazo[1,2-a]pyridine-3,6-diyl}bis[N-(1-cyanocyclopropyl)-2-méthylbenzamide],
4,4'-{8-[méthyl(3,3,3-trifluoropropyl)amino]imidazo[1,2-a]pyridine-3,6-diyl}bis[2-méthyl-N-(1-méthylcyclopropyl)benzamide],
4,4'-{8-[méthyl(3,3,3-trifluoropropyl)amino]imidazo[1,2-a]pyridine-3,6-diyl}bis(N-éthyl-2-méthylbenzamide),
N-(2,6-diéthylphényl)-4-{6-(5-fluoro-2-méthylphénoxy)-8-[(3,3,3-trifluoropropyl)amino]imidazo[1,2-a]pyridin-3-yl}-2-méthylbenzamide,
N-cyclobutyl-4-{6-(5-fluoro-2-méthylphénoxy)-8-[(3,3,3-trifluoropropyl)amino]imidazo[1,2-a]pyridin-3-yl}-2-méthylbenzamide,
rel-4-{6-(3-fluorophénoxy)-8-[(3,3,3-trifluoropropyl)amino]imidazo[1,2-a]pyridin-3-yl}-2-méthyl-N-[(1R,2R)-2-méthylcyclopropyl]benzamide,
N-[1,1'-bi(cyclopropyl)-1-yl]-4-{6-(3-fluorophénoxy)-8-[(3,3,3-trifluoropropyl)amino]imidazo[1,2-a]pyridin-3-yl}-2-méthylbenzamide,
4-{6-(3-fluorophénoxy)-8-[(3,3,3-trifluoropropyl)amino]imidazo[1,2-a]pyridin-3-yl}-N-[1-(hydroxyméthyl)cyclopropyl]-2-méthylbenzamide,
N-(1-cyanocyclobutyl)-4-{6-(3-fluorophénoxy)-8-[(3,3,3-trifluoropropyl)amino]imidazo[1,2-a]pyridin-3-yl}-2-méthylbenzamide,
N-cyclopropyl-4-{6-(3-méthoxyphénoxy)-8-[(3,3,3-trifluoropropyl)amino]imidazo[1,2-a]pyridin-3-yl}-2-méthylbenzamide,
N-(1-cyanocyclopropyl)-4-{6-(3-méthoxyphénoxy)-8-[(3,3,3-trifluoropropyl)amino]imidazo[1,2-a]pyridin-3-yl}-2-méthylbenzamide,
4-{6-(3-méthoxyphénoxy)-8-[(3,3,3-trifluoropropyl)amino]imidazo[1,2-a]pyridin-3-yl}-2-méthyl-N-(1-méthylcyclopropyl)benzamide,
4-{6-(3-méthoxyphénoxy)-8-[(3,3,3-trifluoropropyl)amino]imidazo[1,2-a]pyridin-3-yl}-N,2-diméthylbenzamide,
N-éthyl-4-{6-(3-méthoxyphénoxy)-8-[(3,3,3-trifluoropropyl)amino]imidazo[1,2-a]pyridin-3-yl}-2-méthylbenzamide,
N-(1-cyanocyclopropyl)-2-méthyl-4-{6-phénoxy-8-[(3,3,3-trifluoropropyl)amino]imidazo[1,2-a]pyridin-3-yl}benzamide,
2-méthyl-N-(1-méthylcyclopropyl)-4-{6-phénoxy-8-[(3,3,3-trifluoropropyl)amino]imidazo[1,2-a]pyridin-3-yl}benzamide,
N,2-diméthyl-4-{6-phénoxy-8-[(3,3,3-trifluoropropyl)amino]imidazo[1,2-a]pyridin-3-yl}benzamide,
N-éthyl-2-méthyl-4-{6-phénoxy-8-[(3,3,3-trifluoropropyl)amino]imidazo[1,2-a]pyridin-3-yl}benzamide,
rel-4-{6-(5-fluoro-2-méthylphénoxy)-8-[(3,3,3-trifluoropropyl)amino]imidazo[1,2-a]pyridin-3-yl}-2-méthyl-N-[(1R,2R)-2-méthylcyclopropyl]benzamide,
N-[1,1'-bi(cyclopropyl)-1-yl]-4-{6-(5-fluoro-2-méthylphénoxy)-8-[(3,3,3-trifluoropropyl)amino]imidazo[1,2-a]pyridin-3-yl}-2-méthylbenzamide,
N-(1-cyanocyclobutyl)-4-{6-(5-fluoro-2-méthylphénoxy)-8-[(3,3,3-trifluoropropyl)amino]imidazo[1,2-a]pyridin-3-yl}-2-méthylbenzamide,
N-cyclopropyl-2-méthyl-4-{6-[4-(trifluorométhoxy)phénoxy]-8-[(3,3,3-trifluoropropyl)amino]imidazo[1,2-a]pyridin-3-yl}benzamide,
N-(1-cyanocyclopropyl)-2-méthyl-4-{6-[4-(trifluorométhoxy)phénoxy]-8-[(3,3,3-trifluoropropyl)amino]imidazo[1,2-a]pyridin-3-yl}benzamide,
2-méthyl-N-(1-méthylcyclopropyl)-4-{6-[4-(trifluorométhoxy)phénoxy]-8-[(3,3,3-trifluoropropyl)amino]imidazo[1,2-a]pyridin-3-yl}benzamide,
N,2-diméthyl-4-{6-[4-(trifluorométhoxy)phénoxy]-8-[(3,3,3-trifluoropropyl)amino]imidazo[1,2-a]pyridin-3-yl}benzamide,
N-éthyl-2-méthyl-4-{6-[4-(trifluorométhoxy)phénoxy]-8-[(3,3,3-trifluoropropyl)amino]imidazo[1,2-a]pyridin-3-yl}benzamide,
N-cyclopropyl-4-{6-(2-fluoro-4-méthoxyphénoxy)-8-[(3,3,3-trifluoropropyl)amino]imidazo[1,2-a]pyridin-3-yl}-2-méthylbenzamide,
N-(1-cyanocyclopropyl)-4-{6-(2,3-difluorophénoxy)-8-[(3,3,3-trifluoropropyl)amino]imidazo[1,2-a]pyridin-3-yl}-2-méthylbenzamide,
4-{6-(2,3-difluorophénoxy)-8-[(3,3,3-trifluoropropyl)amino]imidazo[1,2-a]pyridin-3-yl}-2-méthyl-N-(1-méthylcyclopropyl)benzamide,
4-{6-(2,3-difluorophénoxy)-8-[(3,3,3-trifluoropropyl)amino]imidazo[1,2-a]pyridin-3-yl}-N,2-diméthylbenzamide,
4-{6-(2,3-difluorophénoxy)-8-[(3,3,3-trifluoropropyl)amino]imidazo[1,2-a]pyridin-3-yl}-N-éthyl-2-méthylbenzamide,
N-cyclopropyl-2-méthyl-4-{6-[3-(trifluorométhoxy)phénoxy]-8-[(3,3,3-trifluoropropyl)amino]imidazo[1,2-a]pyridin-3-yl}benzamide,
N-(1-cyanocyclopropyl)-2-méthyl-4-{6-[3-(trifluorométhoxy)phénoxy]-8-[(3,3,3-trifluoropropyl)amino]imidazo[1,2-a]pyridin-3-yl}benzamide,
2-méthyl-N-(1-méthylcyclopropyl)-4-{6-[3-(trifluorométhoxy)phénoxy]-8-[(3,3,3-trifluoropropyl)amino]imidazo[1,2-a]pyridin-3-yl}benzamide,
N-éthyl-2-méthyl-4-{6-[3-(trifluorométhoxy)phénoxy]-8-[(3,3,3-trifluoropropyl)amino]imidazo[1,2-a]pyridin-3-yl}benzamide,
N,2-diméthyl-4-{6-[3-(trifluorométhoxy)phénoxy]-8-[(3,3,3-trifluoropropyl)amino]imidazo[1,2-a]pyridin-3-yl}benzamide,
N-cyclopropyl-4-{6-(2-méthoxyphényl)-8-[(3,3,3-trifluoropropyl)amino]imidazo[1,2-a]pyridin-3-yl}-2-méthylbenzamide,
4-{6-(2-méthoxyphényl)-8-[(3,3,3-trifluoropropyl)amino]imidazo[1,2-a]pyridin-3-yl}-2-méthyl-N-(1-méthylcyclopropyl)benzamide,
4-{6-(2-méthoxyphényl)-8-[(3,3,3-trifluoropropyl)amino]imidazo[1,2-a]pyridin-3-yl}-N,2-diméthylbenzamide,
N-éthyl-4-{6-(2-méthoxyphényl)-8-[(3,3,3-trifluoropropyl)amino]imidazo[1,2-a]pyridin-3-yl}-2-méthylbenzamide,
N-cyclopropyl-4-{6-[3-fluoro-4-(trifluorométhoxy)phénoxy]-8-[(3,3,3-trifluoropropyl)amino]imidazo[1,2-a]pyridin-3-yl}-2-méthylbenzamide,
N-(1-cyanocyclopropyl)-4-{6-[3-fluoro-4-(trifluorométhoxy)phénoxy]-8-[(3,3,3-trifluoropropyl)amino]imidazo[1,2-a]pyridin-3-yl}-2-méthylbenzamide,
4-{6-[3-fluoro-4-(trifluorométhoxy)phénoxy]-8-[(3,3,3-trifluoropropyl)amino]imidazo[1,2-a]pyridin-3-yl}-2-méthyl-N-(1-méthylcyclopropyl)benzamide,
4-{6-[3-fluoro-4-(trifluorométhoxy)phénoxy]-8-[(3,3,3-trifluoropropyl)amino]imidazo[1,2-a]pyridin-3-yl}-N,2-diméthylbenzamide,
N-éthyl-4-{6-[3-fluoro-4-(trifluorométhoxy)phénoxy]-8-[(3,3,3-trifluoropropyl)amino]imidazo[1,2-a]pyridin-3-yl}-2-méthylbenzamide,
N,2-diméthyl-4-{6-(pyridin-4-yl)-8-[(3,3,3-trifluoropropyl)amino]imidazo[1,2-a]pyridin-3-yl}benzamide,
N-éthyl-2-méthyl-4-{6-(pyridin-4-yl)-8-[(3,3,3-trifluoropropyl)amino]imidazo[1,2-a]pyridin-3-yl}benzamide,
2-méthyl-N-(1-méthylcyclopropyl)-4-{6-(pyridin-4-yl)-8-[(3,3,3-trifluoropropyl)amino]imidazo[1,2-a]pyridin-3-yl}benzamide,
N-(1-cyanocyclopropyl)-2-méthyl-4-{6-(pyridin-4-yl)-8-[(3,3,3-trifluoropropyl)amino]imidazo[1,2-a]pyridin-3-yl}benzamide,
N-cyclopropyl-2-méthyl-4-{8-[(3,3,3-trifluoropropyl)amino]-6-vinylimidazo[1,2-a]pyridin-3-yl}benzamide,
N,2-diméthyl-4-{8-[(3,3,3-trifluoropropyl)amino]-6-vinylimidazo[1,2-a]pyridin-3-yl}benzamide,
N-éthyl-2-méthyl-4-{8-[(3,3,3-trifluoropropyl)amino]-6-vinylimidazo[1,2-a]pyridin-3-yl}benzamide,
2-méthyl-N-(1-méthylcyclopropyl)-4-{8-[(3,3,3-trifluoropropyl)amino]-6-vinylimidazo[1,2-a]pyridin-3-yl}benzamide,
N-(1-cyanocyclopropyl)-2-méthyl-4-{8-[(3,3,3-trifluoropropyl)amino]-6-vinylimidazo[1,2-a]pyridin-3-yl}benzamide,
N-[rel-(1S,2S)-2-fluorocyclopropyl]-2-méthyl-4-{8-[(3,3,3-trifluoropropyl)amino]-6-vinylimidazo[1,2-a]pyridin-3-yl}benzamide,
2-méthyl-N-[rel-(1S,2S)-2-méthylcyclopropyl]-4-{8-[(3,3,3-trifluoropropyl)amino]-6-vinylimidazo[1,2-a]pyridin-3-yl}benzamide,
N-cyclopropyl-4-{6-éthyl-8-[(3,3,3-trifluoropropyl)amino]imidazo[1,2-a]pyridin-3-yl}-2-méthylbenzamide,
4-{6-éthyl-8-[(3,3,3-trifluoropropyl)amino]imidazo[1,2-a]pyridin-3-yl}-N,2-diméthylbenzamide,
N-éthyl-4-{6-éthyl-8-[(3,3,3-trifluoropropyl)amino]imidazo[1,2-a]pyridin-3-yl}-2-méthylbenzamide,
4-{6-éthyl-8-[(3,3,3-trifluoropropyl)amino]imidazo[1,2-a]pyridin-3-yl}-2-méthyl-N-(1-méthylcyclopropyl)benzamide,
N-(1-cyanocyclopropyl)-4-{6-éthyl-8-[(3,3,3-trifluoropropyl)amino]imidazo[1,2-a]pyridin-3-yl}-2-méthylbenzamide,
4-{6-éthyl-8-[(3,3,3-trifluoropropyl)amino]imidazo[1,2-a]pyridin-3-yl}-N-[rel-(1S,2S)-2-fluorocyclopropyl]-2-méthylbenzamide,
4-{6-éthyl-8-[(3,3,3-trifluoropropyl)amino]imidazo[1,2-a]pyridin-3-yl}-2-méthyl-N-[rel-(1S,2S)-2-méthylcyclopropyl]benzamide,
4-{6-(3-fluoro-4-méthoxyphénoxy)-8-[(3,3,3-trifluoropropyl)amino]imidazo[1,2-a]pyridin-3-yl}-N,2-diméthylbenzamide,
N-éthyl-4-{6-(3-fluoro-4-méthoxyphénoxy)-8-[(3,3,3-trifluoropropyl)amino]imidazo[1,2-a]pyridin-3-yl}-2-méthylbenzamide,
N-cyclopropyl-4-{6-[(1Z)-3-hydroxyprop-1-én-1-yl]-8-[(3,3,3-trifluoropropyl)amino]imidazo[1,2-a]pyridin-3-yl}-2-méthylbenzamide,
4-{6-[4-(benzyloxy)phénoxy]-8-[(3,3,3-trifluoropropyl)amino]imidazo[1,2-a]pyridin-3-yl}-N-cyclopropyl-2-méthylbenzamide,
N-cyclopropyl-4-{6-(4-hydroxyphénoxy)-8-[(3,3,3-trifluoropropyl)amino]imidazo[1,2-a]pyridin-3-yl}-2-méthylbenzamide,
N-cyclopropyl-2-méthyl-4-{6-[(1E)-prop-1-én-1-yl]-8-[(3,3,3-trifluoropropyl)amino]imidazo[1,2-a]pyridin-3-yl}benzamide,
N,2-diméthyl-4-{6-[(1E)-prop-1-én-1-yl]-8-[(3,3,3-trifluoropropyl)amino]imidazo[1,2-a]pyridin-3-yl}benzamide,
N-éthyl-2-méthyl-4-{6-[(1E)-prop-1-én-1-yl]-8-[(3,3,3-trifluoropropyl)amino]imidazo[1,2-a]pyridin-3-yl}benzamide,
2-méthyl-N-(1-méthylcyclopropyl)-4-{6-[(1E)-prop-1-én-1-yl]-8-[(3,3,3-trifluoropropyl)amino]imidazo[1,2-a]pyridin-3-yl}benzamide,
N-(1-cyanocyclopropyl)-2-méthyl-4-{6-[(1E)-prop-1-én-1-yl]-8-[(3,3,3-trifluoropropyl)amino]imidazo[1,2-a]pyridin-3-yl}benzamide,
N-[rel-(1S,2S)-2-fluorocyclopropyl]-2-méthyl-4-{6-[(1E)-prop-1-én-1-yl]-8-[(3,3,3-trifluoropropyl)amino]imidazo[1,2-a]pyridin-3-yl}benzamide,
2-méthyl-N-[rel-(1S,2S)-2-méthylcyclopropyl]-4-{6-[(1E)-prop-1-én-1-yl]-8-[(3,3,3-trifluoropropyl)amino]imidazo[1,2-a]pyridin-3-yl}benzamide,
N-[rel-(1R,2R)-2-fluorocyclopropyl]-4-{6-[(1Z)-3-hydroxyprop-1-én-1-yl]-8-[(3,3,3-trifluoropropyl)amino]imidazo[1,2-a]pyridin-3-yl}-2-méthylbenzamide,
4-{6-[(1Z)-3-hydroxyprop-1-én-1-yl]-8-[(3,3,3-trifluoropropyl)amino]imidazo[1,2-a]pyridin-3-yl}-N,2-diméthylbenzamide,
N-éthyl-4-{6-[(1Z)-3-hydroxyprop-1-én-1-yl]-8-[(3,3,3-trifluoropropyl)amino]imidazo[1,2-a]pyridin-3-yl}-2-méthylbenzamide,
4-{6-(3-fluoro-4-méthoxyphénoxy)-8-[(tétrahydro-2H-pyran-4-ylméthyl)amino]imidazo[1,2-a]pyridin-3-yl}-N,2-diméthylbenzamide,
N-éthyl-4-{6-(3-fluoro-4-méthoxyphénoxy)-8-[(tétrahydro-2H-pyran-4-ylméthyl)amino]imidazo[1,2-a]pyridin-3-yl}-2-méthylbenzamide,
N-cyclopropyl-4-{6-(3-fluoro-4-méthoxyphénoxy)-8-[(tétrahydro-2H-pyran-4-ylméthyl)amino]imidazo[1,2-a]pyridin-3-yl}-2-méthylbenzamide,
N-[rel-(1S,2S)-2-fluorocyclopropyl]-4-{6-(3-fluoro-4-méthoxyphénoxy)-8-[(3,3,3-trifluoropropyl)amino]imidazo[1,2-a]pyridin-3-yl}-2-méthylbenzamide,
4-{6-(3-fluoro-4-méthoxyphénoxy)-8-[(3,3,3-trifluoropropyl)amino]imidazo[1,2-a]pyridin-3-yl}-2-méthyl-N-[rel-(1S,2S)-2-méthylcyclopropyl]benzamide,
4-{6-(3-hydroxypropyl)-8-[(3,3,3-trifluoropropyl)amino]imidazo[1,2-a]pyridin-3-yl}-N,2-diméthylbenzamide,
N-éthyl-4-{6-(3-hydroxypropyl)-8-[(3,3,3-trifluoropropyl)amino]imidazo[1,2-a]pyridin-3-yl}-2-méthylbenzamide,
N-cyclopropyl-4-{6-(3-hydroxypropyl)-8-[(3,3,3-trifluoropropyl)amino]imidazo[1,2-a]pyridin-3-yl}-2-méthylbenzamide,
N-cyclopropyl-4-{6-(3-fluoro-4-hydroxyphénoxy)-8-[(3,3,3-trifluoropropyl)amino]imidazo[1,2-a]pyridin-3-yl}-2-méthylbenzamide,
4-{6-(3-fluoro-4-hydroxyphénoxy)-8-[(3,3,3-trifluoropropyl)amino]imidazo[1,2-a]pyridin-3-yl}-N,2-diméthylbenzamide,
N-éthyl-4-{6-(3-fluoro-4-hydroxyphénoxy)-8-[(3,3,3-trifluoropropyl)amino]imidazo[1,2-a]pyridin-3-yl}-2-méthylbenzamide,
N-cyclopropyl-4-{6-(2-hydroxyphényl)-8-[(3,3,3-trifluoropropyl)amino]imidazo[1,2-a]pyridin-3-yl}-2-méthylbenzamide,
4-{6-(2-hydroxyphényl)-8-[(3,3,3-trifluoropropyl)amino]imidazo[1,2-a]pyridin-3-yl}-N,2-diméthylbenzamide,
N-éthyl-4-{6-(2-hydroxyphényl)-8-[(3,3,3-trifluoropropyl)amino]imidazo[1,2-a]pyridin-3-yl}-2-méthylbenzamide,
N-[rel-(1S,2S)-2-fluorocyclopropyl]-4-{6-(2-hydroxyphényl)-8-[(3,3,3-trifluoropropyl)amino]imidazo[1,2-a]pyridin-3-yl}-2-méthylbenzamide,
N-cyclopropyl-4-{6-(3-hydroxyphénoxy)-8-[(3,3,3-trifluoropropyl)amino]imidazo[1,2-a]pyridin-3-yl}-2-méthylbenzamide,
4-{6-(3-hydroxyphénoxy)-8-[(3,3,3-trifluoropropyl)amino]imidazo[1,2-a]pyridin-3-yl}-2-méthyl-N-(1-méthylcyclopropyl)benzamide,
N-éthyl-4-{6-(3-hydroxyphénoxy)-8-[(3,3,3-trifluoropropyl)amino]imidazo[1,2-a]pyridin-3-yl}-2-méthylbenzamide,
4-{6-(3-hydroxyphénoxy)-8-[(3,3,3-trifluoropropyl)amino]imidazo[1,2-a]pyridin-3-yl}-N,2-diméthylbenzamide,
2-méthyl-4-{3-[3-méthyl-4-(méthylcarbamoyl)phényl]-8-[(3,3,3-trifluoropropyl)amino]imidazo[1,2-a]pyridin-6-yl}benzoate de méthyle,
N-cyclopropyl-4-{6-(2-hydroxyéthyl)-8-[(3,3,3-trifluoropropyl)amino]imidazo[1,2-a]pyridin-3-yl}-2-méthylbenzamide,
4-{6-(2-hydroxyéthyl)-8-[(3,3,3-trifluoropropyl)amino]imidazo[1,2-a]pyridin-3-yl}-N,2-diméthylbenzamide,
N-cyclopropyl-4-{6-[(1RS)-1-hydroxyéthyl]-8-[(3,3,3-trifluoropropyl)amino]imidazo[1,2-a]pyridin-3-yl}-2-méthylbenzamide,
4-{6-[(1RS)-1-hydroxyéthyl]-8-[(3,3,3-trifluoropropyl)amino]imidazo[1,2-a]pyridin-3-yl}-N,2-diméthylbenzamide,
N-éthyl-4-{6-[(1RS)-1-hydroxyéthyl]-8-[(3,3,3-trifluoropropyl)amino]imidazo[1,2-a]pyridin-3-yl}-2-méthylbenzamide,
N-cyclopropyl-4-{6-(3-fluoro-2-méthoxyphénoxy)-8-[(3,3,3-trifluoropropyl)amino]imidazo[1,2-a]pyridin-3-yl}-2-méthylbenzamide,
4-{3-[4-(cyclopropylcarbamoyl)-3-méthylphényl]-8-[(3,3,3-trifluoropropyl)amino]imidazo[1,2-a]pyridin-6-yl}-N,2-diméthylbenzamide,
4-{3-[4-(cyclopropylcarbamoyl)-3-méthylphényl]-8-[(3,3,3-trifluoropropyl)amino]imidazo[1,2-a]pyridin-6-yl}-N-éthyl-2-méthylbenzamide,
4-{3-[4-(cyclopropylcarbamoyl)-3-méthylphényl]-8-[(3,3,3-trifluoropropyl)amino]imidazo[1,2-a]pyridin-6-yl}-2-méthyl-N-(1-méthylcyclopropyl)benzamide,
4-(6-{4-[(1-cyanocyclopropyl)carbamoyl]-3-méthylphényl}-8-[(3,3,3-trifluoropropyl)amino]imidazo[1,2-a]pyridin-3-yl)-N-cyclopropyl-2-méthylbenzamide,
4-{3-[4-(cyclopropylcarbamoyl)-3-méthylphényl]-8-[(3,3,3-trifluoropropyl)amino]imidazo[1,2-a]pyridin-6-yl}-N-[1-(hydroxyméthyl)cyclopropyl]-2-méthylbenzamide,
4-{3-[4-(cyclopropylcarbamoyl)-3-méthylphényl]-8-[(3,3,3-trifluoropropyl)amino]imidazo[1,2-a]pyridin-6-yl}-N-[rel-(1S,2S)-2-fluorocyclopropyl]-2-méthylbenzamide,
4-{6-(4-carbamoyl-3-méthylphényl)-8-[(3,3,3-trifluoropropyl)amino]imidazo[1,2-a]pyridin-3-yl}-N-cyclopropyl-2-méthylbenzamide,
4-{6-(4-{[rel-(1S,2S)-2-fluorocyclopropyl]carbamoyl}-3-méthylphényl)-8-[(3,3,3-trifluoropropyl)amino]imidazo[1,2-a]pyridin-3-yl}-N,2-diméthylbenzamide,
4-{6-(4-{[1-(hydroxyméthyl)cyclopropyl]carbamoyl}-3-méthylphényl)-8-[(3,3,3-trifluoropropyl)amino]imidazo[1,2-a]pyridin-3-yl}-N,2-diméthylbenzamide,
4-(6-{4-[(1-cyanocyclopropyl)carbamoyl]-3-méthylphényl}-8-[(3,3,3-trifluoropropyl)amino]imidazo[1,2-a]pyridin-3-yl)-N,2-diméthylbenzamide,
2-méthyl-N-(1-méthylcyclopropyl)-4-{3-[3-méthyl-4-(méthylcarbamoyl)phényl]-8-[(3,3,3-trifluoropropyl)amino]imidazo[1,2-a]pyridin-6-yl}benzamide,
4-{6-[4-(cyclopropylcarbamoyl)-3-méthylphényl]-8-[(3,3,3-trifluoropropyl)amino]imidazo[1,2-a]pyridin-3-yl}-N,2-diméthylbenzamide,
4-{6-[4-(éthylcarbamoyl)-3-méthylphényl]-8-[(3,3,3-trifluoropropyl)amino]imidazo[1,2-a]pyridin-3-yl}-N,2-diméthylbenzamide,
4-{6-(4-carbamoyl-3-méthylphényl)-8-[(3,3,3-trifluoropropyl)amino]imidazo[1,2-a]pyridin-3-yl}-N,2-diméthylbenzamide,
N-cyclopropyl-4-{8-[(2,2-difluoroéthyl)amino]-6-(3-fluorophénoxy)imidazo[1,2-a]pyridin-3-yl}-2-méthylbenzamide,
N-cyclopropyl-4-{8-[(2,2-difluoroéthyl)amino]-6-(4-méthoxyphénoxy)imidazo[1,2-a]pyridin-3-yl}-2-méthylbenzamide,
N-cyclopropyl-4-{8-[(2,2-difluoroéthyl)amino]-6-(2,3-difluorophénoxy)imidazo[1,2-a]pyridin-3-yl}-2-méthylbenzamide,
N-cyclopropyl-4-{8-[(2,2-difluoroéthyl)amino]-6-(5-fluoro-2-méthylphénoxy)imidazo[1,2-a]pyridin-3-yl}-2-méthylbenzamide,
N-cyclopropyl-4-{8-[(2,2-difluoroéthyl)amino]-6-(2,5-difluorophénoxy)imidazo[1,2-a]pyridin-3-yl}-2-méthylbenzamide,
N-cyclopropyl-4-{8-[(2,2-difluoroéthyl)amino]-6-(3-fluoro-2-méthylphénoxy)imidazo[1,2-a]pyridin-3-yl}-2-méthylbenzamide,
N-cyclopropyl-4-{8-[(2,2-difluoroéthyl)amino]-6-(3-fluoro-4-méthoxyphénoxy)imidazo[1,2-a]pyridin-3-yl}-2-méthylbenzamide,
N-cyclopropyl-4-{8-[(2,2-difluoroéthyl)amino]-6-(2-hydroxybenzoyl)imidazo[1,2-a]pyridin-3-yl}-2-méthylbenzamide,
N-cyclopropyl-4-{6-(3-fluorophénoxy)-8-[(2-méthoxyéthyl)amino]imidazo[1,2-a]pyridin-3-yl}-2-méthylbenzamide,
N-cyclopropyl-4-{8-[(2-méthoxyéthyl)amino]-6-(2-méthylphénoxy)imidazo[1,2-a]pyridin-3-yl}-2-méthylbenzamide,
N-cyclopropyl-4-{6-(3,4-difluorophénoxy)-8-[(2-méthoxyéthyl)amino]imidazo[1,2-a]pyridin-3-yl}-2-méthylbenzamide,
N-cyclopropyl-4-{6-(5-fluoro-2-méthylphénoxy)-8-[(2-méthoxyéthyl)amino]imidazo[1,2-a]pyridin-3-yl}-2-méthylbenzamide,
N-cyclopropyl-4-{6-(2,3-difluorophénoxy)-8-[(2-méthoxyéthyl)amino]imidazo[1,2-a]pyridin-3-yl}-2-méthylbenzamide,
N-cyclopropyl-4-{6-(5-fluoro-2-hydroxybenzoyl)-8-[(2-méthoxyéthyl)amino]imidazo[1,2-a]pyridin-3-yl}-2-méthylbenzamide,
N-cyclopropyl-4-{8-[(2,2-difluoroéthyl)amino]-6-(3-fluorophénoxy)imidazo[1,2-a]pyridin-3-yl}-2-méthylbenzamide,
N-cyclopropyl-4-{6-(5-fluoro-2-hydroxyphénoxy)-8-[(3,3,3-trifluoropropyl)amino]imidazo[1,2-a]pyridin-3-yl}-2-méthylbenzamide,
N-cyclopropyl-4-{6-(3-fluoro-2-hydroxyphénoxy)-8-[(3,3,3-trifluoropropyl)amino]imidazo[1,2-a]pyridin-3-yl}-2-méthylbenzamide,
N-cyclopropyl-4-{6-[(5-fluoropyridin-3-yl)oxy]-8-[(3,3,3-trifluoropropyl)amino]imidazo[1,2-a]pyridin-3-yl}-2-méthylbenzamide,
N-cyclopropyl-4-{6-[(5-fluoro-6-méthoxypyridin-3-yl)oxy]-8-[(3,3,3-trifluoropropyl)amino]imidazo[1,2-a]pyridin-3-yl}-2-méthylbenzamide,
ou l'un des stéréoisomères, tautomères, N-oxydes, hydrates, solvates ou sels de celui-ci, ou l'un de leurs mélanges.

4. Procédé de synthèse d'un composé selon l'une quelconque des revendications 1 à 3, ledit procédé comprenant l'étape suivante :
- réaction d'un composé intermédiaire de formule générale **IV** : 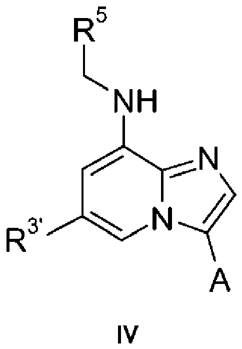 dans lequel
R⁵ et A sont tels que définis pour la formule générale I dans l'une quelconque des revendications 1 à 3 ; et
R^{3'} représente un atome d'halogène ;
avec un composé de formule générale **IVa** :
R³-Y **IVa**
dans lequel
R³ est tel que défini pour la formule générale I dans l'une quelconque des revendications 1 à 3 ;
Y représente un substituant qui est déplacé dans une réaction de couplage ;
ce qui permet d'obtenir un composé de formule générale **I** : 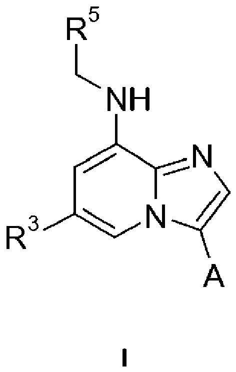 dans lequel
R³, R⁵ et A sont tels que définis pour la formule générale I dans l'une quelconque des revendications 1 à 3.

5. Procédé de synthèse d'un composé selon l'une quelconque des revendications 1 à 3, ledit procédé comprenant l'étape suivante :
- réaction d'un composé intermédiaire de formule générale **II :** 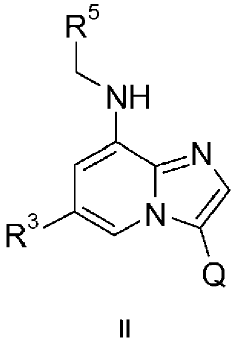 dans lequel
R³ et R⁵ sont tels que définis pour la formule générale I dans l'une quelconque des revendications 1 à 3 ;
et Q représente un atome d'halogène ;
avec un composé de formule générale **IIa** :
A-Y **IIa**
dans lequel
A est tel que défini pour la formule générale I dans l'une quelconque des revendications 1 à 3 ;
Y représente un substituant qui est déplacé dans une réaction de couplage ;
ce qui permet d'obtenir un composé de formule générale **I** : 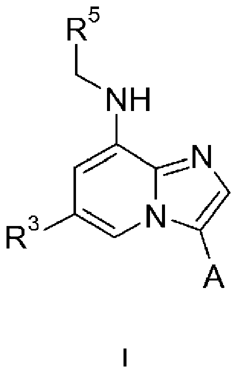
dans lequel R³, R⁵ et A sont tels que définis pour la formule générale **I** dans l'une quelconque des revendications 1 à 3.

6. Procédé de synthèse d'un composé selon l'une quelconque des revendications 1 à 3, ledit procédé comprenant l'étape suivante :
- réaction d'un composé intermédiaire de formule générale VII : 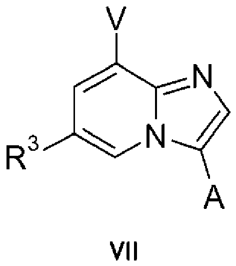 dans lequel
R³ et A sont tels que définis pour la formule générale I dans l'une quelconque des revendications 1 à 3 ; et
V représente un groupement partant ;
avec un composé de formule générale **VIIa :**
R⁵-CH₂-NH₂ **VIIa**
dans lequel
R⁵ est tel que défini pour la formule générale I dans l'une quelconque des revendications 1 à 3 ;
ce qui permet d'obtenir un composé de formule générale **I** : 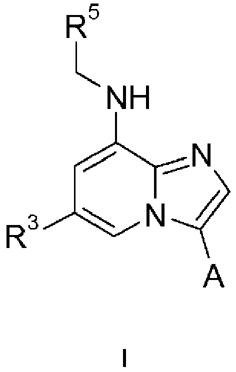
dans lequel R³, R⁵ et A sont tels que définis pour la formule générale **I** dans l'une quelconque des revendications 1 à 3.

7. Procédé de synthèse d'un composé selon l'une quelconque des revendications 1 à 3, ledit procédé comprenant l'étape suivante :
- réaction d'un composé intermédiaire de formule générale VII : 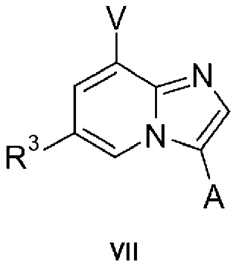 dans lequel
R³ et A sont tels que définis pour la formule générale I dans l'une quelconque des revendications 1 à 3 ; et
V représente un groupement NH₂- ;
avec un composé de formule générale VIIb :
O=CHR⁵ **VIIb**
dans lequel
R⁵ est tel que défini pour la formule générale **I** dans l'une quelconque des revendications 1 à 3 ;
ce qui permet d'obtenir un composé de formule générale **I** : 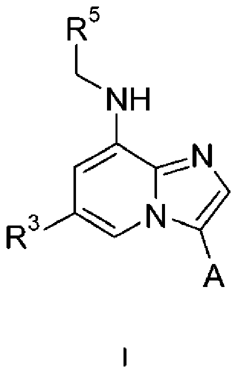
dans lequel R³, R⁵ et A sont tels que définis pour la formule générale **I** dans l'une quelconque des revendications 1 à 3.

8. Composé selon l'une quelconque des revendications 1 à 3, ou l'un des stéréoisomères, tautomères, N-oxydes, hydrates, solvates ou sels de celui-ci, en particulier l'un des sels pharmaceutiquement acceptables de celui-ci, ou l'un de leurs mélanges, pour utilisation dans le traitement prophylactique ou thérapeutique d'une maladie.

9. Composé pour utilisation selon la revendication 8, où ladite maladie est une maladie de croissance, prolifération et/ou survie cellulaire incontrôlée, une réponse immunitaire cellulaire inappropriée, ou une réponse inflammatoire cellulaire inappropriée, dans laquelle la maladie de croissance, prolifération et/ou survie cellulaire incontrôlée, la réponse immunitaire cellulaire inappropriée, ou la réponse inflammatoire cellulaire inappropriée est une tumeur hématologique, une tumeur solide et/ou leurs métastases, par exemple les leucémies et le syndrome myélodysplasique, les lymphomes malins, les tumeurs de la tête et du cou y compris les tumeurs cérébrales et les métastases cérébrales, les tumeurs du thorax y compris les tumeurs pulmonaires à petites cellules et non à petites cellules, les tumeurs gastro-intestinales, les tumeurs endocrines, les tumeurs mammaires et autres tumeurs gynécologiques, les tumeurs urologiques y compris les tumeurs rénales, vésicales et prostatiques, les tumeurs cutanées et les sarcomes, et/ou leurs métastases.

10. Composition pharmaceutique comprenant un composé selon l'une quelconque des revendications 1 à 3, ou l'un des stéréoisomères, tautomères, N-oxydes, hydrates, solvates ou sels de celui-ci, en particulier l'un des sels pharmaceutiquement acceptables de celui-ci, ou l'un de leurs mélanges, ainsi qu'un diluant ou vecteur pharmaceutiquement acceptable.

11. Composition pharmaceutique comprenant :
-- un ou plusieurs composés selon l'une quelconque des revendications 1 à 3, ou l'un des stéréoisomères, tautomères, N-oxydes, hydrates, solvates ou sels de celui-ci, en particulier l'un des sels pharmaceutiquement acceptables de celui-ci, ou l'un de leurs mélanges ;
et
- - un ou plusieurs agents choisis parmi les suivants : un taxane, par exemple Docétaxel, Paclitaxel ou Taxol ; une épothilone, par exemple Ixabépilone, Patupilone ou Sagopilone ; la Mitoxantrone ; la Prednisolone ; la Dexaméthasone ; l'Estramustine ; la Vinblastine ; la Vincristine ; la Doxorubicine ; l'Adriamycine ; l'Idarubicine ; la Daunorubicine ; la Bléomycine ; l'Étoposide ; le Cyclophosphamide ; l'Ifosfamide ; la Procarbazine ; le Melphalan ; le 5-Fluorouracile ; la Capécitabine ; la Fludarabine ; la Cytarabine ; Ara-C ; la 2-Chloro-2'-désoxyadénosine ; la Thioguanine ; un antiandrogène, par exemple Flutamide, Cyprotérone acétate, ou Bicalutamide ; le Bortézomib ; un dérivé de platine, par exemple Cisplatine ou Carboplatine ; le Chlorambucile ; le Méthotrexate ; et le Rituximab.

12. Utilisation d'un composé selon l'une quelconque des revendications 1 à 3, ou de l'un des stéréoisomères, tautomères, N-oxydes, hydrates, solvates ou sels de celui-ci, en particulier de l'un des sels pharmaceutiquement acceptables de celui-ci, ou l'un de leurs mélanges, pour l'élaboration d'un médicament destiné au traitement prophylactique ou thérapeutique d'une maladie.

13. Utilisation selon la revendication 12, où ladite maladie est une maladie de croissance, prolifération et/ou survie cellulaire incontrôlée, une réponse immunitaire cellulaire inappropriée, ou une réponse inflammatoire cellulaire inappropriée, dans laquelle la maladie de croissance, prolifération et/ou survie cellulaire incontrôlée, la réponse immunitaire cellulaire inappropriée, ou la réponse inflammatoire cellulaire inappropriée est une tumeur hématologique, une tumeur solide et/ou leurs métastases, par exemple les leucémies et le syndrome myélodysplasique, les lymphomes malins, les tumeurs de la tête et du cou y compris les tumeurs cérébrales et les métastases cérébrales, les tumeurs du thorax y compris les tumeurs pulmonaires à petites cellules et non à petites cellules, les tumeurs gastro-intestinales, les tumeurs endocrines, les tumeurs mammaires et autres tumeurs gynécologiques, les tumeurs urologiques y compris les tumeurs rénales, vésicales et prostatiques, les tumeurs cutanées et les sarcomes, et/ou leurs métastases.

14. Composé de formule générale **IV** : 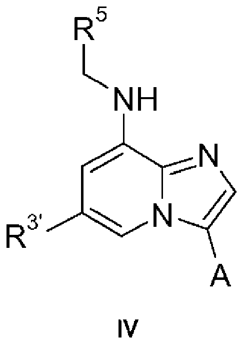 dans lequel R⁵ et A sont tels que définis pour la formule générale I dans l'une quelconque des revendications 1 à 3, et R^{3'} représente un atome d'halogène.

15. Composé de formule générale **VII** : 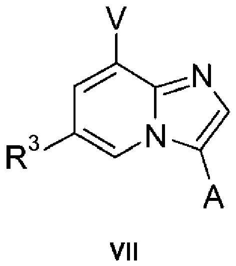 dans lequel R³ et A sont tels que définis pour la formule générale I dans l'une quelconque des revendications 1 à 3, et V représente un groupement NH₂-ou un atome d'halogène.
